# EUROPEAN PATENT APPLICATION

(11) **EP 1 857 455 A1**
(43) Date of publication of application: **21.11.2007**
(21) Application number: 06728805.0
(22) Date of filing: 02.03.2006
(51) Int. Cl.: C07D 401/06, C07D 405/06, C07D 409/06, C07D 207/34, C07D 307/68, C07D 333/40, A61K 31/341, A61K 31/381, A61K 31/40, A61K 31/443, A61K 31/4436, A61K 31/4439, A61P 9/04, A61P 13/12, A61P 17/16, A61P 43/00

(54) **CINNAMOYL COMPOUND AND USE THEREOF**

(30) Priority: 02.03.2005 JP 2005057363
(71) Applicant: Sumitomo Chemical Company, Limited, Tokyo 104-8260 (JP)
(72) Inventor: SHIRAKI, Hiroaki, Rockville, MD 20852 (US); HIGASHI, Kiyoshi, 5420012 (JP); TOMIGAHARA, Yoshitaka, Osaka 560-0013 (JP); TAKAHASHI, Junya, Hyogo 6660262 (JP)
(74) Representative: Vossius & Partner
(86) International application number: PCT/JP2006/304537
(87) International publication number: WO 2006/100922

(57) **Abstract**

Disclosed is an extracellular matrix genetranscription inhibitor composition or the like **characterized by** containing a cinnamoyl compound represented by the formula(I) below: 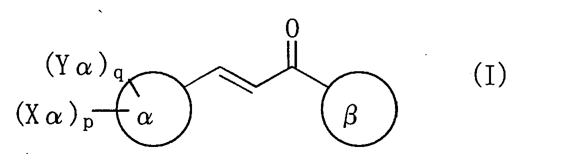 and an inert carrier.

## Description

### Technical Field

The present invention relates to a cinnamoyl compound and use of the same.

### Background Art

In diseases and disorders such as hepatic cirrhosis, chronic pancreatitis, scirrhous gastric cancer, interstitial pulmonary disease, asthma, chronic obstructive pulmonary diseases, glomerular nephritis, lupus nephritis, tubulointerstitial nephritis, IgA nephritis, renal sclerosis, diabetic nephropathy, hereditary renal disease, myocardial fibrosis, heart failure, restenosis after PTCA, atherosclerosis, marrow fibrosis, rheumatoid arthritis, hyperplasia scar after inflammation, postoperative scars or burn scars, atopic dermatitis, hypertrophic scar, hysteromyoma, prostate hypertrophy, scleroderma, Alzheimer disease, sclerotic peritonitis, diabetic retinopathy, and type I diabetes, excessive accumulation of an extracellular matrix, a representative of which is collagen or fibronectin, causes fibrosis and sclerosis of tissues, resulting in decreased functions, cicatrization and the like in the organs or tissues. Such excessive accumulation of an extracellular matrix is induced by increased production of the extracellular matrix due to a breakdown of balance between biosynthesis and degradation of the extracellular matrix. In fact, it has been observed that expression of an extracellular matrix gene such as a collagen gene (in particular, a Type I collagen gene, a Type III collagen gene, or a Type IV collagen gene), a fibronectin gene, a laminin gene, a proteoglycan gene or the like has been increased in a fibrotic tissue [J. Invest. Dermatol., 94, 365, (1990); Proc. Natl. Acad. Sci. USA, 88, 6642, (1991); J. Am. Soc. Nephrol., 15, 2637, (2004); Cardiovasc. Pathol., 13, 119, (2004); Clin. Nephrol., 44, 211, (1995); J. Hepatol., 29, 263, (1998))].

It has been also observed that the amount of TGF-β, which is a cytokine, has been increased in a fibrotic tissue [e.g. J. Invest. Dermatol., 94, 365, (1990) and Proc. Natl. Acad. Sci. USA, 88, 6642, (1991)]. It has been suggested that TGF-β has increased expression of an extracellular matrix gene and been involved in increased production of an extracellular matrix protein and, consequently, fibrosis of a tissue [e.g. J. Invest. Dermatol., 94, 365, (1990); and Lab. Invest., 63, 171, (1990)]. It has been also shown that by administering an anti-TGF-β antibody or a soluble anti-TGF-β receptor to a model animal of tissue fibrosis, improvement of tissue fibrosis has been achieved and thereby the tissue function has been also improved [e.g. Diabetes, 45, 522-530, (1996), Proc. Natl. Acad. Sci. USA, 96, 12719-12724, (1999); and Proc. Natl. Acad. Sci. USA, 97, 8015-8020, (2000)]. It has been also known that by administering a compound which suppressively acts on intracellular signal transduction via TGF-β, improvement in fibrosis of a tissue has been achieved and thereby the tissue function has been also improved [e.g. Autoimmunity, 35, 277-282, (2002); J. Hepatol., 37, 331-339, (2002); and Life Sci., 71, 1559-1606, (2002)].

On the other hand, it is believed that a cause of heart failure such as left ventricular diastolic failure or renal failure such as diabetic nephropathy or renal sclerosis is cardiac fibrosis under a hypertensive condition.

Thus, there is a need for development and provision of a drug which improves fibrosis of a tissue by decreasing expression of an extracellular matrix gene in the tissue to reduce accumulation of the extracellular matrix (i.e. an extracellular matrix accumulation-suppressing agent, a fibrosing disease-treating agent, or a heart failure-treating agent).

### Disclosure of Invention

The present invention relates to compounds represented by the following formulas (I) to (VI), (I'), (II') and (V') having the ability to suppress transcription of an extracellular matrix gene.

That is, the present invention provides:
1. A composition for suppressing transcription of an extracellular matrix gene which comprises an inert carrier and a cinnamoyl compound represented by the formula (I): 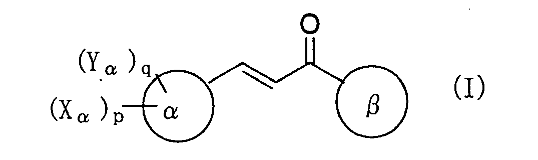 wherein,
   I. α represents an aromatic 5-membered ring, or an aromatic 6-membered ring having two or more nitrogen atoms; in (Y_{α})_{q}, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the α ring; in (X_{α})ₚ, X_{α} represents a substituent which does not belong to the following X₀ group, Y₀ group and Z₀ group, p represents 0, 1, 2 or 3 and, when p is not less than 2, X_{α}s are the same or different, and the sum of p and q is not more than 3;
      (1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ'')-NRₑ'''-R_{d}- group (wherein Rₑ, Rₑ' , Rₑ" and Rₑ''' are the same or different, Rₑ, Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
      (2) the Y₀ group: a M_{b0}-R_{d}- group, wherein M_{b0} represents a M_{c0}- group
         [wherein M_{co} represents a M_{do}-R_{d}'- group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 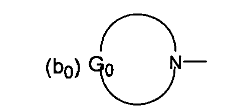 (wherein Go forms an optionally substituted, saturated or unsaturated, nonaromatic 5 to 14-membered hydrocarbon ring or heterocyclic ring), a (c₀)- group represented by 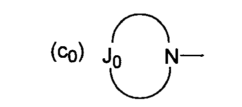 (wherein, J₀ forms a 5 to 7-membered aromatic ring optionally containing a nitrogen atom), a (do)- group represented by 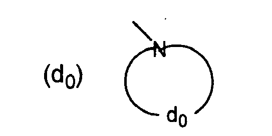 [wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group], or a (e₀)- group represented by 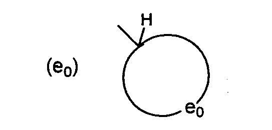 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}; and R_{d}' is the same as or different from R_{d} and has the same meaning as R_{d} has],
         a M_{c0}-Bₐ- group (wherein M_{c0} and Bₐ are as defined above), a M_{c0}-CO- group (wherein M_{c0} is as defined above), a M_{c0}-CO-O-group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ-group (wherein M_{c0} and Rₑ are as defined above), a M_{c0}RₑN-CO-group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
      (3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and
   II. β represents
      a group represented by formula (I-1): 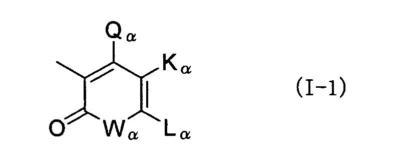 wherein,
      (1)Q_{α} represents an optionally substituted hydroxyl group, or an optionally substituted amino group,
      (2)W_{α} represents an oxygen atom or a -NT_{α}- group (wherein T_{α} represents a hydrogen atom, or a substituent on the nitrogen atom),
      (3)K_{α} and L_{α} are the same or different, and represent a hydrogen atom, or a substituent on the carbon atom, or K_{α} and L_{α} may form an optionally substituted C3-C10 alkylene group or an optionally substituted C4-C10 alkenylene group; a group represented by formula (1-2): 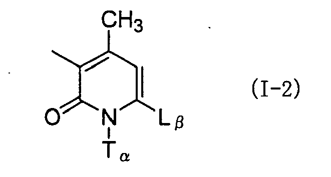 wherein T_{α} is as defined above, and L_{β} represents a hydroxyl group or a methyl group;
         a group represented by formula (I-3): 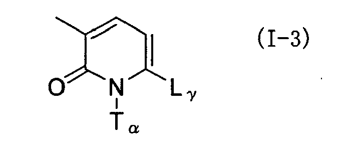 wherein T_{α} is as defined above, and L_{γ} represents a C1-C10 alkyl group;
         a group represented by formula (I-4): 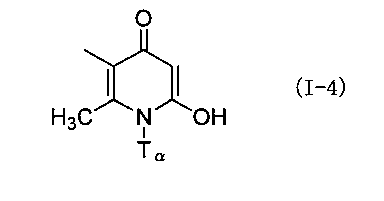 wherein T_{α} is as defined above;
         a group represented by formula (I-5): 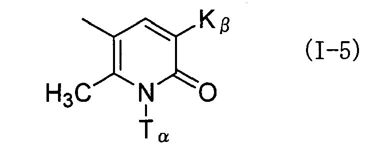 wherein T_{α} is as defined above, and K_{β} represents a cyano group or a UOCO- group (wherein U represents a hydrogen atom or a C1-C10 alkyl group);
         a group represented by formula (1-6): 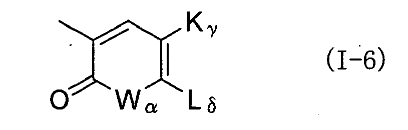 wherein W_{α} is as defined above, and K_{γ} and L_{δ} form an optionally substituted C3-C10 alkylene group or an optionally substituted C4-C10 alkenylene group;
         a group represented by formula (I-7): 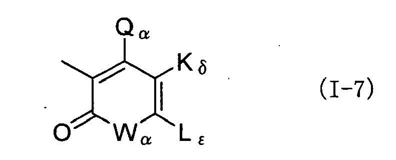 wherein Q_{α} and W_{α} are as defined above, and K_{δ} and L_{ε} form a -V_{α}=V_{α}'-V_{α}''=V_{α}'''- group (wherein V_{α}, V_{α}', V_{α}'' and V_{α}''' are the same or different, and represent an optionally substituted methine group or a -N= group, and at least one of V_{α}, V_{α}', V_{α}'' and V_{α}''' represents a -N= group);
         a group represented by formula (I-8): 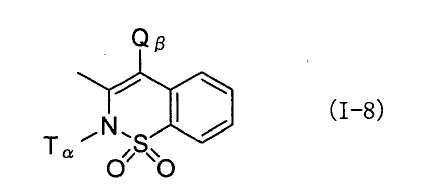 wherein T_{α} is as defined above, and Q_{β} represents an optionally substituted hydroxyl group; or
         a group represented by formula (I-9): 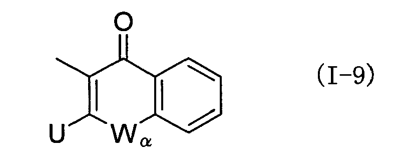 wherein U and W_{α} are as defined above; and
         the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
2. A cinnamoyl compound represented by the formula (II): 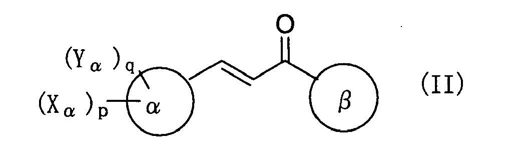 wherein,
   I. α represents an aromatic 5-membered ring, or an aromatic 6-membered ring having two or more nitrogen atoms; in (Y_{α})_{q}, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the α ring; in (X_{α})p, X_{α} represents a substituent which does not belong to the following X₀ group, Y₀ group and Z₀ group, p represents 0, 1, 2 or 3 and, when p is not less than 2, X_{α}s are the same or different, and the sum of p and q is not more than 3;
      (1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ'' are the same or different, Rₑ and Rₑ' are as defined above, Rₑ" has the same meaning as Rₑ has, and R_{d} is as defined above), a RₑRₑ'N-C(=NRₑ")-NRₑ'''-R_{d}- group (wherein Rₑ, Rₑ', Rₑ'' and Rₑ''' are the same or different, Rₑ, Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
      (2) the Y₀ group: a M_{b0}-R_{d}- group, wherein M_{b0} represents a M_{c0}- group [wherein M_{co} represents a M_{do}-R_{d}'-group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 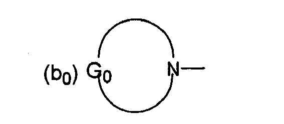 (wherein Go forms an optionally substituted, saturated or unsaturated, nonaromatic 5 to 14-membered hydrocarbon ring or heterocyclic ring), a (c₀)- group represented by 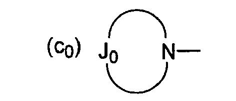 (wherein, J₀ forms a 5 to 7-membered aromatic ring optionally containing a nitrogen atom), a (do)- group represented by 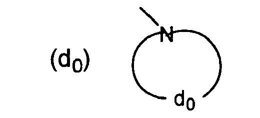 {wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group}, or a (e₀)- group represented by 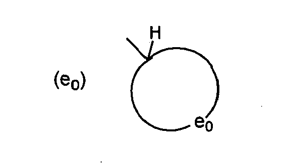 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}, and R_{d}' is the same as or different from R_{d} and has the same meaning as R_{d} has], a M_{c0}-Ba- group (wherein M_{c0} and Bₐ are as defined above), a M_{c0}-CO- group (wherein M_{c0} is as defined above), a M_{c0}-CO-O- group (wherein M_{c0} is as defined above), a M_{c0}O-CO- group (wherein M_{c0} is as defined above), a M_{c0}ReN- group (wherein M_{c0} and Rₑ are as defined above), a M_{c0}-CO-NRₑ- group (wherein M_{c0} and Rₑ are as defined above), a M_{c0}O-CO-NRₑ- group (wherein M_{c0} and Rₑ are as defined above), a M_{c0}RₑN-CO- group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
      (3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and
   II. β represents
      a group represented by formula (II-1): 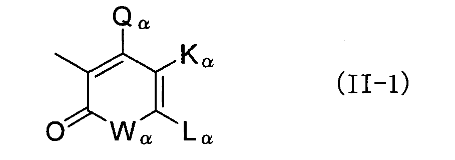
   wherein,
   (1)Q_{α} represents an optionally substituted hydroxyl group, or an optionally substituted amino group,
   (2)W_{α} represents an oxygen atom or a -NT_{α}- group (wherein T_{α} represents a hydrogen atom, or a substituent on the nitrogen atom),
   (3)K_{α} and L_{α} are the same or different, and represent a hydrogen atom, or a substituent on the carbon atom, or K_{α} and L_{α} may form an optionally substituted C3-C10 alkylene group or an optionally substituted C4-C10 alkenylene group; a group represented by formula (II-2): 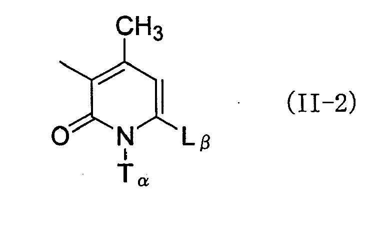 wherein T_{α} is as defined above, and L_{β} represents a hydroxyl group or a methyl group;
      a group represented by formula (II-3): 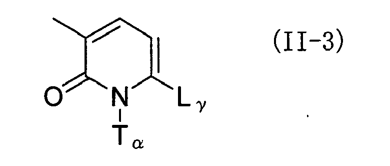 wherein T_{α} is as defined above, and L_{γ} represents a C1-C10 alkyl group;
      a group represented by formula (II-4): 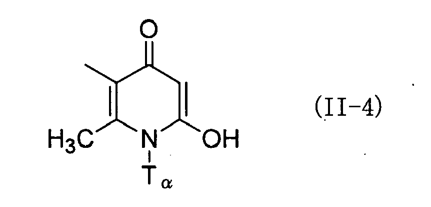 wherein T_{α} is as defined above;
      a group represented by formula (II-5): 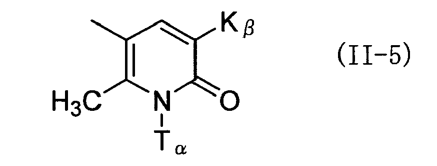 wherein T_{α} is as defined above, and K_{β} represents a cyano group or a UOCO- group (wherein U represents a hydrogen atom or a C1-C10 alkyl group);
      a group represented by formula (II-6): 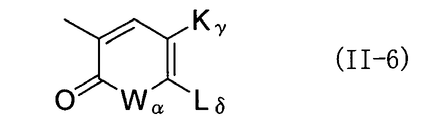 wherein W_{α} is as defined above, and K_{γ} and L_{δ} form an optionally substituted C3-C10 alkylene group or an optionally substituted C4-C10 alkenylene group;
      a group represented by formula (II-7): 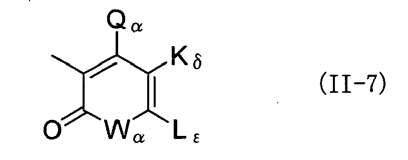 wherein Q_{α} and W_{α} are as defined above, and K_{δ} and L_{ε} form a -V_{α}=V_{α}'-V_{α}'' =V_{α}''' - group (wherein V_{α}, V_{α}', V_{α}'' and V_{α}''' are the same or different, and represent an optionally substituted methine group or a -N= group, and at least one of V_{α}, V_{α}', V_{α}'' and V_{α}''' represents a -N= group);
      a group represented by formula (II-8): 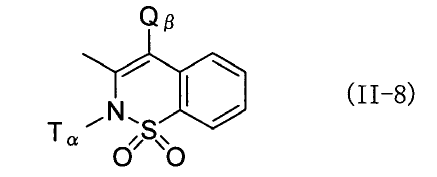 wherein T_{α} is as defined above, and Q_{β} represents an optionally substituted hydroxyl group; or
      a group represented by formula (II-9): 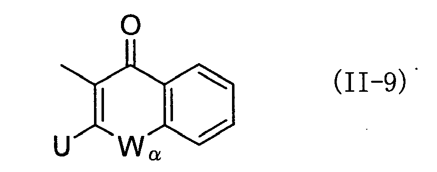 wherein U and W_{α} are as defined above; provided that p and q are not 0 at the same time when α is a furan ring or a thiophene ring; and
      the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
3. A cinnamoyl compound represented by the formula (III): 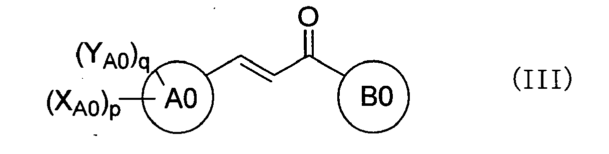 wherein,
   I. A0 represents an aromatic 5-membered ring, or an aromatic 6-membered ring having two or more nitrogen atoms,
   II. in (X_{A0})ₚ, X_{A0} represents a group included in any group of the following A₀ group to N₀ group, p represents 0, 1, 2 or 3 and, when p is not less than 2, X_{A0}s are the same or different,
      (1) the A₀ group:
         a D₁-R₄- group, wherein D₁ represnts a (R₁-(O)ₖ-)A₁N-(O)_{k'}- group [wherein R₁ represents a hydrogen atom, or a C1-C10 alkyl group, or a C2-C10 alkyl group substituted with a halogen atom or a R₂-B₁-group (wherein R₂ represents a C1-C10 alkyl group, a C3-C10 alkenyl group or a C3-C10 alkynyl group, and B₁ represents an oxy group, a thio group, a sulfinyl group or a sulfonyl group), or a C3-C10 alkenyl group, or a C3-C10 alkynyl group, k represents 0 or 1, A₁ represents a R₃-(CHR₀)ₘ-(B₂-B₃)_{m'}- group {wherein R₃ represents a hydrogen atom, or a C1-C10 alkyl group optionally substituted with a halogen atom or a R₂-B₁-group (wherein R₂ and B₁ are as defined above), or a C2-C10 alkenyl group, or a C2-C10 alkynyl group, R₀ represents a hydrogen atom, a C1-C10 alkyl group or a C2-C10 haloalkyl group, m represents 0 or 1, B₂ represents a single bond, an oxy group, a thio group or a -N((O)ₙR₁')- group (wherein R₁' is the same as or different from R₁, and has the same meaning as R₁ has, and n represents 0 ro 1), B₃ represents a carbonyl group, a thiocarbonyl group or a sulfonyl group, m' represents 0 or 1, and B₃ is not a sulfonyl group when m is 0 and R₃ is a hydrogen atom}, and k' represents 0 or 1], and R₄ represents a C1-C10 alkylene group, provided that a R₀'R₀"N-R₄- group (wherein R₀' and R₀" are the same as or different from R₀ and have the same meaning as R₀ has, and R₄ is as defined above) is excluded,
         a D₂-R₄- group, wherein D₂ represents a cyano group, a R₁R₁'NC(=N-(O)ₙ-A₁)- group (wherein R₁, R₁', n and A₁ are as defined above), an A₁N=C(-OR₂)- group (wherein A₁ and R₂ are as defined above) or a NH₂-CS- group, and R₄ is as defined above,
         a D₃-R₄- group, wherein D₃ represents a nitro group or a R₁OSO₂- group (wherein R₁ is as defined above), and R₄ is as defined above, and
         a R₁OSO₂- group, wherein R₁ is as defined above;
      (2) the B₀ group: an (a₀)- group represented by 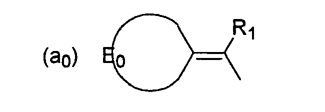 wherein E₀ forms an optionally substituted, saturated or unsaturated, aromatic or nonaromatic 5 to 14-membered hydrocarbon ring or heterocyclic ring, and R₁ is as defined above;
      (3) the C₀ group: a C2-C10 alkenyl group substituted with a halogen atom, a R₂-B₁- group (wherein R₂ and B₁ are as defined above), a D₄-R₄- group [wherein D₄ represents a hydroxyl group or an A₁-O- group (wherein A₁ is as defined above), and R₄ is as defined above], a D₅- group [wherein D₅ represents a O=C(R₃)- group (wherein R₃ is as defined above), an A₁-(O)ₙ-N=C(R₃)- group (wherein A₁, n and R₃ are as defined above), a R₁-B₀-CO-R₄-(O)ₙ-N=C(R₃)- group {wherein R₁, R₄, n and R₃ are as defined above, and B₀ represents an oxy group, a thio group or a -N((O)ₘR₁')-group (wherein R₁' and m are as defined above)}, a D₂-R₄-(O)ₙ-N=C(R₃)- group (wherein D₂, R₄, n and R₃ are as defined above) or a R₁A₁N-N=C(R₃)- group (wherein R₁, A₁ and R₃ are as defined above)], a R₁A₁N-O-R₄- group (wherein R₁, A₁ and R₄ are as defined above), a R₁(A₁-(O)ₙ-)N- group (wherein R₁, A₁ and n are as defined above), a D₂- group (wherein D₂ is as defined above) or a D₃- group (wherein D₃ is as defined above);
      (4) the D₀ group: a C2-C10 alkynyl group substituted with a (b₀)-R₄- group (in (b₀) 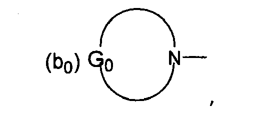 Go forms an optionally substituted, saturated or unsaturated, nonaromatic 5 to 14-membered hydrocarbon ring or heterocyclic ring), a (c₀)-R₄- group (in (c₀) 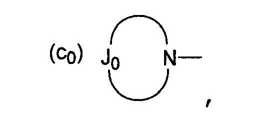 J₀ forms an aromatic 5 to 7-membered ring optionally containing a nitrogen atom and R₄ is as defined above), a halogen atom, a R₂-B₁-R₄- group (wherein R₂, B₁ and R₄ are as defined above), a D₄-R₄- group (wherein D₄ and R₄ are as defined above), a D₅- group (wherein D₅ is as defined above), a D₁-R₄- group (wherein D₁ and R₄ are as defined above), a D₂- group (wherein D₂ is as defined above) or a D₃-R₄- group (wherein D₃ and R₄ are as defined above);
      (5) the E₀ group: an A₂-CO-R₅- group, provided that R₅ is not a vinylene group when A₂ is a hydroxyl group [wherein A₂ represents
         (i) an A₃-B₄- group
            wherein A₃ represents a hydrogen atom, or a C1-C10 alkyl group, or a C2-C10 haloalkyl group, or a C2-C10 alkenyl group optionally substituted with a halogen atom; or a C3-C10 alkynyl group optionally substituted with a halogen atom, or a Rₐ₀-(R₄)ₘ- group (wherein Rₐ₀ represents an optionally substituted 5 to 7-membered aryl group or heteroaryl group, and R₄ and m are as defined above), or a C1-C10 alkyl group substituted with a (b₀)-R₄- group (wherein (b₀) and R₄ are as defined above), a (c₀)-R₄- group (wherein (c₀) and R₄ are as defined above), a R₂-B₁-R₄-group (wherein R₂, B₁ and R₄ are as defined above), a D₄-R₄-group (wherein D₄ and R₄ are as defined above), a D₅- group (wherein D₅ is as defined above), a D₁-R₄- group (wherein D₁ and R₄ are as defined above), a D₂- group (wherein D₂ is as defined above), a D₃-R₄- group (wherein D₃ and R₄ are as defined above) or an A₄-SO₂-R₄- group {wherein A₄ represents a (b₀)- group (wherein (b₀) is as defined above), a (c₀)-group (wherein (c₀) is as defined above) or a R₁R₁'N- group (wherein R₁ and R₁' are as defined above), and R₄ is as defined above}, and
            B₄ represents an oxy group, a thio group or a -N((O)ₘR₁)-group (wherein R₁ and m are as defined above), provided that A₃ is not a hydrogen atom when B₄ is a thio group;
         (ii) a R₁-B₄-CO-R₄-B₄'- group (wherein R₁, B₄ and R₄ are as defined above, B₄' is the same as or different from B₄ and has the same meaning as B₄ has, provided that R₂ is not a hydrogen atom when B₄ is a thio group) or a D₂-R₄-B₄-group (wherein D₂, R₄ and B₄ are as defined above);
         (iii) a R₂-SO₂-NR₁- group (wherein R₂ is as defined above, provided that a hydrogen atom is excluded, and R₁ is as defined above);
         (iv) a (b₀)- group, wherein (b₀) is as defined above;
         (v) a (c₀)- group, wherein (c₀) is as defined above; or
         (vi) a R₁A₁N-NR₁'- group, wherein R₁, A₁ and R₁' are as defined above; and
            R₅ represents a C2-C10 alkenylene group optionally substituted with a halogen atom or a C2-C10 alkynylene group];
      (6) the F₀ group: an A₅-B₅-R₆- group, wherein
         A₅ represents a C2-C10 alkyl group substituted with a D₄- group (wherein D₄ is as defined above), a D₁- group (wherein D₁ is as defined above), a D₃- group (wherein D₃ is as defined above) or an A₄-SO₂- group (wherein A₄ is as defined above), or a C1-C10 alkyl group substituted with a R₂-B₁- group (wherein R₂ and B₁ are as defined above), a D₂-group (wherein D₂ is as defined above), a D₅- group (wherein D₅ is as defined above) or an A₂-CO- group (wherein A₂ is as defined above), and
         B₅ represents a B₁- group (wherein B₁ is as defined above) or a -NA₁- group (wherein A₁ is as defined above), and
         R₆ represent a single bond or a C1-C10 alkylene group;
      (7) the G₀ group: an A₆-B₅-R₆- group wherein
         A₆ represents an (a₀)-R₄- group (wherein (a₀) and R₄ are as defined above), or a C2-C10 alkenyl group, or a C2-C10 alkynyl group, or a C2-C10 alkenyl group substituted with a halogen atom, a R₂-B₁- group (wherein R₂ and B₁ are as defined above), a D₅- group (wherein D₅ is as defined above), a D₂- group (wherein D₂ is as defined above) or an A₂-CO- group (wherein A₂ is as defined above), or a C2-C10 alkynyl group substituted with a halogen atom, a R₂-B₁-group (wherein R₂ and B₁ are as defined above), a D₅- group (wherein D₅ is as defined above), D₂- group (wherein D₂ is as defined above) or an A₂-CO- group (wherein A₂ is as defined above), or a C3-C10 alkenyl group substituted with a (b₀)- group (wherein (b₀) is as defined above), a (c₀)-group (wherein (c₀) is as defined above), a D₄- group (wherein D₄ is as defined above), a D₁- group (wherein D₁ is as defined above) or a D₃- group (wherein D₃ is as defined above), or a C3-C10 alkynyl group substituted with a D₄-group (wherein D₄ is as defined above), a D₁- group (wherein D₁ is as defined above) or a D₃- group (wherein D₃ is as defined above), and
         B₅ and R₆ are as defined above;
      (8) the H₀ group:
         a D₂-N(-(O)ₙ-A₁)-R₆- group (wherein D₂, n, A₁ and R₆ are as defined above),
         a D₂- group (wherein D₂ is as defined above, provided that a cyano group is excluded),
         a R₁(R₁' (O)ₙ)N-CR₁"=N-R₆- group (wherein R₁, R₁', n and R₆ are as defined above, R₁" is the same as or different from R₁ and has the same meaning as R₁ has),
         a R₁-(O)ₙ-N=CR₁'-NR₂-R₆- group (wherein R₁, n, R₁', R₂ and R₆ are as defined above),
         a R₂-B₃-NR₁-CO-NR₁'-R₆- group (wherein R₂, B₃, R₁, R₁' and R₆ are as defined above),
         a D₂-CO-NR₁-R₆- group (wherein D₂, R₁ and R₆ are as defined above), and
         an A₂-COCO-NR₁-R₆- group (wherein A₂, R₁ and R₆ are as defined above);
      (9) the I₀ group:
         an A₇-B₆-N((O)ₙR₁)-R₆- group [wherein A₇ represents a C2-C10 alkenyl group optionally substituted with a halogen atom, or a C2-C10 alkynyl group, or a C3-C10 haloalkynyl group, or a R₂-B₁-R₄- group (wherein R₂, B₁ and R₄ are as defined above), or a D₄-R₄- group (wherein D₄ and R₄ are as defined above), or a D₅-R₄- group (wherein D₅ and R₄ are as defined above), or a D₁-R₄- group (wherein D₁ and R₄ are as defined above), or a (b₀)-R₄- group (wherein (b₀) and R₄ are as defined above), or a (c₀)-R₄- group (wherein (c₀) and R₄ are as defined above), or a D₂-R₄- group (wherein D₂ and R₄ are as defined above), or a D₃-R₄- group (wherein D₃ and R₄ are as defined above), or an A₄-SO₂-R₄- group (wherein A₄ and R₄ are as defined above), or an A₂-CO-R₄- group (wherein A₂ and R₄ are as defined above), B₆ represents a carbonyl group or a thiocarbonyl group, and n, R₁ and R₆ are as defined above],
         an A₈-CS-N((O)ₙR₁)-R₆- group [wherein A₈ represents a hydrogen atom or a C1-C10 alkyl group optionally substituted with a halogen atom, and n, R₁ and R₄ are as defined above],
         an A₇' -B₂' -B₃-N ((O)ₙR₁) -R₆- group [wherein A₇' represents a C3-C10 alkenyl group optionally substituted with a halogen atom, or a C3-C10 alkynyl group optionally substituted with a halogen atom, or a R₂-B₁-R₄'- group (wherein R₂ and B₁ are as defined above, and R₄' represents a C2-C10 alkylene group), or a D₄-R₄'- group (wherein D₄ and R₄' are as defined above), or a D₁-R₄'- group (wherein D₁ and R₄' are as defined above), or a (b₀)-R₄'- group (wherein (b₀) and R₄' are as defined above), or a (c₀)-R₄'- group (wherein (c₀) and R₄' are as defined above), or a D₂-R₄-group (wherein D₂ and R₄ are as defined above), or a D₃-R₄'-group (wherein D₃ and R₄' are as defined above), or an A₂-CO-R₄- group (wherein A₂ and R₄ are as defined above), B₂' represents an oxy group, a thio group or a -N((O)_{n'}R₁')-group (wherein n' is the same as or different from n and has the same meaning as n has, and R₁' is as defined above), and B₃, n, R₁ and R₆ are as defined above],
         an A₈' -B₂' -CS-N ((O)ₙR₁)-R₆- group [wherein A₈' represents a C1-C10 alkyl group or a C2-C10 haloalkyl group, B₂' is as defined above, and n, R₁ and R₆ are as defined above],
         an A₈'-S-B₃'-N((O)ₙR₁)-R₆- group [wherein A₈', n, R₁ and R₆ are as defined above, and B₃' represents a carbonyl group or a sulfonyl group], and
         an A₇"-SO₂-N((O)ₙR₁)-R₆- group [wherein A₇" represents a C2-C10 alkenyl group, or a C3-C10 alkenyl group substituted with a halogen atom, or a C3-C10 alkynyl group optionally substituted with a halogen atom, or a R₂-B₁-R₄'- group (wherein R₂, B₁ and R₄' are as defined above), or a D₄-R₄'-group (wherein D₄ and R₄' are as defined above), or a D₅-R₄-group (wherein D₅ and R₄ are as defined above), or a D₁-R₄'-group (wherein D₁ and R₄' are as defined above), or a (b₀)-R₄'- group (wherein (b₀) and R₄' are as defined above), or a (c₀)-R₄'- group (wherein (c₀) and R₄' are as defined above), or a D₂-R₄- group (wherein D₂ and R₄ are as defined above), or a NO₂-R₄- group (wherein R₄ is as defined above), or an A₂-CO-R₄- group (wherein A₂ and R₄ are as defined above), and n, R₁ and R₆ are as defined above];
      (10) the J₀ group:
         an A₇-CO- group (wherein A₇ is as defined above),
         an A₉-CS- group (wherein A₉ represents A₇ or A₈),
         an A₉' (O)ₘN=C(A₉)- group (wherein A₉' represents A₇' or A₈', and m and A₉ are as defined above),
         a D₂-CO- group (wherein D₂ is as defined above),
         an A₂-COCO- group (wherein A₂ is as defined above),
         an A₉-CO-B₁'-R₆- group (wherein A₉ and R₆ are as defined above, and B₁' represents an oxy group or a thio group, provided that A₉ is not A₈ when B₁' is an oxy group),
         an A₉-CS-B₁'-R₆- group (wherein A₉, B₁' and R₆ are as defined above),
         an A₇"-SO₂-B₁'-R₆- group (wherein A₇", B₁' and R₆ are as defined above),
         an A₈-SO₂-B₁'-R₆- group (wherein A₈, B₁' and R₆ are as defined above, provided that A₈ is not a hydrogen atom),
         an A₉'-B₂'-B₃-B₁'-R₆- group (wherein A₉' , B₂', B₃, B₁' and R₆ are as defined above), and
         a C2-C10 alkenyl group substituted with a (b₀)- group (wherein (b₀) is as defined above) or a (c₀)- group (wherein (c₀) is as defined above);
      (11) the K₀ group: an A₁₀-N((O)ₙR₁)-CO-R₆- group, wherein A₁₀ represents a hydrogen atom (provided that n is not 0), an A₇"-SO₂- group (wherein A₇" is as defined above), an A₈-SO₂- group (wherein A₈ is as defined above, provided that A₈ is not a hydrogen atom), an Ag'O- group (wherein A₉' is as defined above, (provided that n is not 1), an A₉'-group (wherein A₉' is as defined above, provided that A₈' is excluded when n is 0), a R₂OCH₂- group (wherein R₂ is as defined above), an A₂-CO-R₄- group (wherein A₂ and R₄ are as defined above) or an A₂-CO-CH(CH₂CO-A₂)- group (wherein A₂ is as defined above), and n, R₁ and R₆ are as defined above;
      (12) the L₀ group:
         an A₁₀'-N((O)ₙR₁)-SO₂-R₆- group [wherein A₁₀' represents a hydrogen atom (provided that n is not 1), an A₉'O- group (wherein Ag' is as defined above, provided that n is not 0), an A₉'- group (wherein A₉' is as defined above, provided that A₈' is excluded when n is 0), a R₂-CO- group (wherein R₂ is as defined above), an A₂-CO-R₄- group (wherein A₂ and R₄ are as defined above) or an A₂-CO-CH (CH₂CO-A₂)- group (wherein A₂ is as defined above), and n, R₁ and R₆ are as defined above],
         an A₉"R₁N-SO₂-N((O)ₙR₁')-R₆- group [wherein A₉" represents a hydrogen atom or an A₉'- group (wherein A₉' is as defined above), and R₁, n, R₁' and R₆ are as defined above] and
         a (b₀)-SO₂-N((O)ₙR₁')-R₆- group [wherein (b₀), n, R₁' and R₆ are as defined above];
      (13) the M₀ group:
         a R₁(R₂S)C=N-R₆- group (wherein R₁, R₂ and R₆ are as defined above),
         a R₂B (R₂'B')C=N-R₆- group (wherein R₂ and R₆ are as defined above, R₂' is the same as or different from R₂ and has the same meaning as R₂ has, and B and B' are the same or different and represent an oxy group or a thio group),
         a R₁R₁' N- (R₂S) C=N-R₆- group (wherein R₁, R₁', R₂ and R₆ are as defined above);
         a R₁N=C (SR₂) -NR₂' -R₆- group (wherein R₁, R₂, R₂' and R₆ are as defined above), and
         a R₁(R₁'O)N-R₆- group (wherein R₁, R₁' and R₆ are as defined above);
      (14) the N₀ group: a A₁₁-P(=O) (OR₁')-R₄- group, wherein A₁₁ represents a R₁- group (wherein R₁ is as defined above), a R₁O-R₆- group (wherein R₁ and R₆ are as defined above) or a R₁OCO-CHR₀- group (wherein R₁ and R₀ are as defined above), and R₁' and R₄ are as defined above;
   III. in (Y_{A0})_{q}, Y_{A0} represents a group included in the following X₀ group and Y₀ group, q represents 0, 1, 2 or 3, the sum of p (wherein p is as defined above) and q is not more than 3, and when q is not less than 2, Y_{A0}s are the same or different, and when q is not less than 2, adjacent two same or different Y_{A0}s may together form a group included in the Z₀ group to be fused to the A0 ring,
      (1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ")-NRₑ'''-R_{d}- group (wherein Rₑ, Rₑ' , Rₑ" and Rₑ''' are the same or different, Rₑ, Rₑ' and Rₑ" are as defined above, Rₑ' ' ' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
      (2) the Y₀ group: a M_{b0}-R_{d}- group, wherein M_{b0} represents a M_{c0}- group [wherein M_{co} represents a M_{do}-R_{d}'-group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 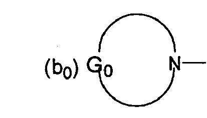 (wherein (b₀) is as defined above), a (c₀) - group represented by 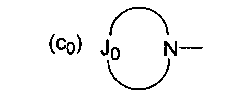 (wherein, (c₀) is as defined above), a (d₀)- group represented by 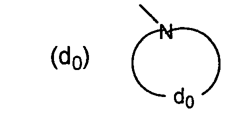 {wherein d₀ forms a 5 to 12-membered hydrocarbon ring which is substituted with a carbonyl group or a thiocarbonyl group and further which may be optionally substituted with an oxy group, a thio group, a -NR₁- group (wherein R₁ is as defined above), a sulfinyl group or a sulfonyl group}, or a (e₀)- group represented by 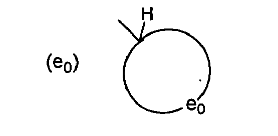 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}, and R_{d}' is the same as or different from R_{d} and has the same meaning as R_{d} has], a M_{c0}-Bₐ- group (wherein M_{c0} and Bₐ are as defined above), a M_{c0}-CO- group (wherein M_{c0} is as defined above), a M_{c0}-CO-O- group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ- group (wherein M_{c0} and Rₑ are as defined above), a M_{c0}RₑN-CO- group (wherein M_{c0} and Rₑ are as defined above), a M_{c0}RₑN-CO-NRₑ'- group (wherein M_{c0}, Rₑ and Rₑ' are as defined above) , a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above), or a M_{c0}RₑN-SO₂- group (wherein M_{c0} and Rₑ are as defined above), and R_{d} is as defined above;
      (3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the A0 ring; and
   IV. B0 is a group represented by formula (III-1): 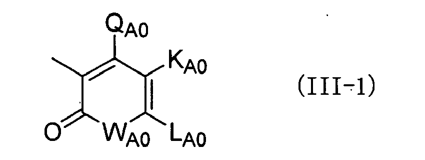 [wherein,
      (1) Q_{A0} represents a hydroxyl group, a (b₀)- group (wherein (b₀) is as defined above), an A₉-B₆-B_{c}- group [wherein A₉ and B₆ are as defined above, and B_{c} represents an oxy group or a N-((O)ₘR₁)- group (wherein m and R₁ are as defined above), provided that B_{c} is not a sulfonyl group when A₉ is a hydrogen atom], an A₇"-SO₂-B_{c}- group (wherein A₇" and B_{c} are as defined above), an A₈-SO₂-B_{c}- group (wherein A₈ and B_{c} are as defined above, provided that A₈ is not a hydrogen atom), a R₁R₁'N-SO₂-B_{c}- group (wherein R₁, R₁' and B_{c} are as defined above), a (b₀)-SO₂-B_{c}- group (wherein (b₀) and B_{c} are as defined above), an A₉'-B_{c}- group (wherein A₉' and B_{c} are as defined above), a D₅-R₄-B_{c}- group (wherein D₅, R₄ and B_{c} are as defined above), a M_{c0}-B₃-B_{c}- group (wherein M_{c0}, B₃ and B_{c} are as defined above) or a M_{c0}-B_{c}- group (wherein M_{c0} and B_{c} are as defined above),
      (2) W_{A0} represents an oxygen atom or a -NT_{A0}- group wherein T_{A0} represents a hydrogen atom, an A₉'- group (wherein A₉' is as defined above), a D₅-R₄- group (wherein D₅ and R₄ are as defined above) or a M_{cO}- group (wherein M_{cO} is as defined above) ,
      (3) K_{A0} represents a hydrogen atom, a halogen atom or a C1-C10 alkyl group, L_{A0} represents a hydrogen atom, a C1-C10 alkyl group or a M_{bO}- group (wherein M_{bO} is as defined above), or K_{A0} and L_{A0} may form a C3-C10 alkylene group, or a C4-C10 alkenelyne group optionally substituted with 1 or more same or different Mₐ- groups (wherein Mₐ is as defined above)],
         a group represented by formula (III-2): 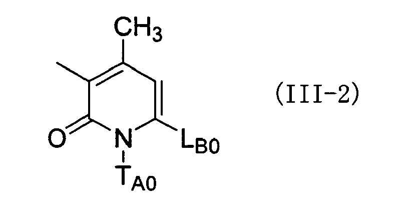 [wherein T_{A0} is as defined above, and L_{B0} represents a hydroxyl group or a methyl group],
         a group represented by formula (III-3): 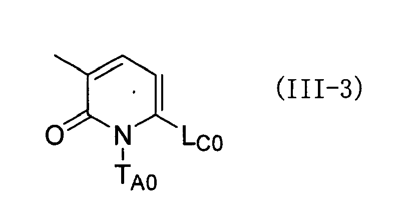 [wherein T_{A0} is as defined above, and L_{C0} represents a C1-C10 alkyl group],
         a group represented by formula (III-4): 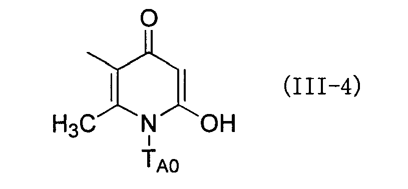 [wherein T_{A0} is as defined above],
         a group represented by formula (III-5): 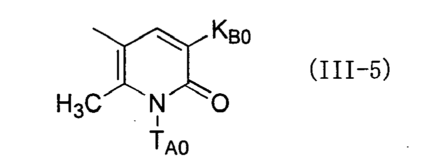 [wherein T_{A0} is as defined above, and K_{B0} represents a cyano group or a UOCO- group (wherein U represents a hydrogen atom or a C1-C10 alkyl group)],
         a group represented by formula (III-6): 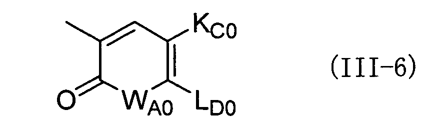 [wherein W_{A0} is as defined above, and K_{C0} and L_{D0} form a C3-C10 alkylene group, or a C4-C10 alkenylyne optionally substituted with 1 or more same or different Mₐ- groups (wherein Mₐ is as defined above)],
         a group represented by formula (III-7): 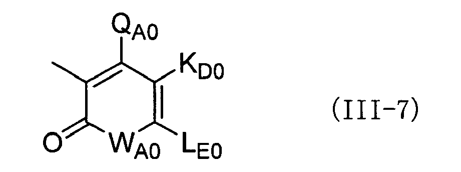 [wherein Q_{A0} and W_{A0} are as defined above, K_{D0} and L_{E0} form a -V_{A0}=V_{A0}'-V_{A0}=V_{A0}'''- group {wherein V_{A0}, V_{A0}', V_{A0}" and V_{A0}''' are same or different, and represent a methine group optionally substituted with a Mₐ- group (wherein Mₐ is as defined above), or a -N= group, and at least one of V_{A0}, V_{A0}', V_{A0}" and V_{A0}''' represents a -N= group}],
         a group represented by formula (III-8): 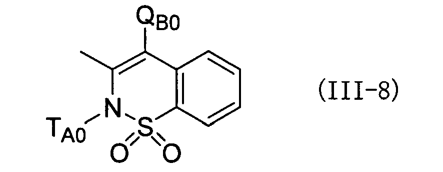 [wherein T_{A0} is as defined above, and Q_{B0} represents a hydroxyl group, a A₉-B₆-O- group (wherein A₉ and B₆ are as defined above), an A₇"-SO₂-O- group (wherein A₇" is as defined above), an A₈-SO₂-O- group, (wherein A₈ is as defined above, provided that A₈ is not a hydrogen), a R₁R₁'N-SO₂-O- group (wherein R₁ and R₁' are as defined above), a (b₀)-SO₂-O- group (wherein (b₀) is as defined above), an A₉'-O- group (wherein A₉' is as defined above), a D₅-R₄-O- group (wherein D₅ and R₄ are as defined above), a M_{cO}-B₃-O- group (wherein M_{cO} and B₃ are as defined above) or a M_{c0}-O- group (wherein M_{co} is as defined above)], or
         a group represented by formula (III-9): 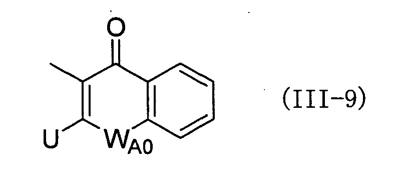 [wherein U and W_{A0} are as defined above], provided that when A0 is a furan ring or a thiophene ring, p and q are not 0 at the same time; and
         the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
4. A cinnamoyl compound represented by the formula (IV): 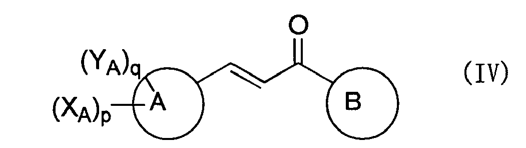 wherein,
   I. A represents an aromatic 5-membered ring, or an aromatic 6-membered ring having two or more nitrogen atoms;
   II. in (X_{A})ₚ, X_{A} represents a group included in any group of the following A group to the N group, p represents 0, 1, 2 or 3, and when p is not less than 2, X_{A}s are the same or different;
      (1) the A group:
         a D₁-R₄- group, wherein D₁ represents a (R₁-(O)ₖ-(A₁N-(O)ₖ'- group [wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), or a C3-C10 alkenyl group, or a C3-C10 alkynyl group, k represents 0 or 1, A₁ represents a R₃-(CHR₀)ₘ-(B₂-B₃)_{m'}- group {wherein R₃ represents a hydrogen atom, or a C1-C10 alkyl group optionally substituted with a halogen atom or a R₂-B₁-group (wherein R₂ and B₁ are as defined above), or a C2-C10 alkenyl group, or a C2-C10 alkynyl group, R₀ represents a hydrogen atom, a C1-C10 alkyl group or a C2-C10 haloalkyl group, m represents 0 or 1, B₂ represents a single bond, an oxy group, a thio group or a -N((O)ₙR₁')- group (wherein R₁' is the same as or different from R₁ and has the same meaning as R₁ has, and n represents 0 or 1), B₃ represents a carbonyl group, a thiocarbonyl group or a sulfonyl group, m' represents 0 or 1, and B₃ is not a sulfonyl group when m is 0 and R₃ is a hydrogen atom}, and k' represents 0 or 1], and R₄ represents a C1-C10 alkylene group, provided that a R₀'R₀"N-R₄- group (wherein R₀' and R₀'' are the same as or different from R₀ and have the same meaning as R₀ has, and R₄ is as defined above) is excluded,
         a D₂-R₄- group, wherein D₂ represents a cyano group, a R₁R₁'NC(=N-(O)ₙ-A₁)- group (wherein R₁, R₁', n and A₁ are as defined above), an A₁N=C(-OR₂)- group (wherein A₁ and R₂ are as defined above) or a NH₂-CS- group, and R₄ is as defined above,
         a D₃-R₄- group, wherein D₃ represents a nitro group or a R₁OSO₂- group (wherein R₁ is as defined above), and R₄ is as defined above, and
         a R₁OSO₂- group, wherein R₁ is as defined above;
      (2) the B group: an (a)-group represented by 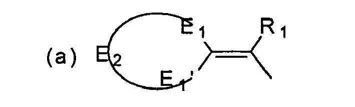 wherein E₁ and E₁' represent a methylene group optionally substituted with a C1-C10 alkyl group or a C1-C10 alkoxy group, or a carbonyl group, provided that E₁ and E₁' are not a carbonyl group at the same time, E₂ represents a C2-C10 alkylene group optionally substituted with an oxy group, a thio group, a sulfinyl group, a sulfonyl group or a - NR₁'- group (wherein R₁' is as defined above), or a C3-C10 alkenylene group optionally substituted with an oxy group, a thio group, a sulfinyl group, a sulfonyl group or a - NR₁'- group (wherein R₁' is as defined above), and R₁ is as defined above;
      (3) the C group: a C2-C10 alkenyl group substituted with a halogen atom, a R₂-B₁- group (wherein R₂ and B₁ are as defined above), a D₄-R₄- group [wherein D₄ represents a hydroxyl group or an A₁-O- group (wherein A₁ is as defined above), and R₄ is as defined above], a D₅- group [wherein D₅ represents an O=C(R₃)- group (wherein R₃ is as defined above), an A₁-(O)ₙ-N=C(R₃)- group (wherein A₁, n and R₃ are as defined above), a R₁-B₀-CO-R₄-(O)ₙ-N=C(R₃)- group {wherein R₁, R₄, n and R₃ are as defined above, and B₀ represents an oxy group, a thio group or a -N((O)ₘR₁')-group (wherein R₁' and m are as defined above)}, a D₂-R₄-(O)ₙ-N=C(R₃)- group (wherein D₂, R₄, n and R₃ are as defined above) or a R₁A₁N-N=C(R₃)- group (wherein R₁, A₁ and R₃ are as defined above)], a R₁A₁N-O-R₄- group (wherein R₁, A₁ and R₄ are as defined above), a R₁ (A₁-(O)ₙ-)N- group (wherein R₁, A₁ and n are as defined above), a D₂- group (wherein D₂ is as defined above) or a D₃- group (wherein D₃ is as defined above);
      (4) the D group: a C2-C10 alkynyl group substituted with a (b)-R₄- group [wherein in (b) 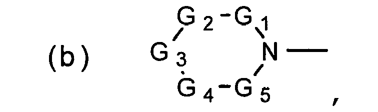 G₁, G₂, G₄ and G₅ represent a methylene group which is connected with the adjacent atom via a single bond and which may be optionally substituted with a methyl group, or a methine group which is connected with the adjacent atom via a double bond and which may be optionally substituted with a methyl group, and G₃ represents a single bond, or a double bond, or a C1-C10 alkylene group optionally substituted with a methyl group, an oxy group, a thio group, a sulfinyl group, a sulfonyl group or a -NR₁- group (wherein R₁ is as defined above), or a C2-C10 alkenylene group optionally substituted with a methyl group, an oxy group, a thio group, a sulfinyl group, a sulfonyl group or a -NR₁- group (wherein R₁ is as defined above); and R₄ is as defined above], a (c)-R₄- group [wherein, in (c) 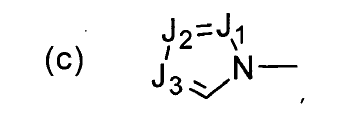 J₁, J₂ and J₃ are the same or different, and represent a methine group optionally substituted with a methyl group, or a nitrogen atom; and R₄ is as defined above), a halogen atom, a R₂-B₁-R₄- group (wherein R₂, B₁ and R₄ are as defined above), a D₄-R₄- group (wherein D₄ and R₄ are as defined above), a D₅- group (wherein D₅ is as defined above), a D₁-R₄- group (wherein D₁ and R₄ are as defined above), a D₂- group (wherein D₂ is as defined above) or a D₃-R₄- group (wherein D₃ and R₄ are as defined above);
      (5) the E group: an A₂-CO-R₅- group, provided that R₅ is not a vinylene group when A₂ is a hydroxyl group, [wherein A₂ represents
         (i) an A₃-B₄- group
            wherein A₃ represents a hydrogen atom, or a C1-C10 alkyl group, or a C2-C10 haloalkyl group, or a C2-C10 alkenyl group optionally substituted with a halogen atom, or a C3-C10 alkynyl group optionally substituted with a halogen atom, or Rₐ-(R₄)ₘ- group (wherein Rₐ represents a phenyl group, a pyridyl group, a furyl group or a thienyl group, which may be optionally substituted with a halogen atom, a C1-C10 alkyl group, a C1-C10 alkoxy group or a nitro group, and R₄ and m are as defined above), or a C1-C10 alkyl group substituted with a (b)-R₄- group (wherein (b) and R₄ are as defined above), a (c)-R₄- group (wherein (c) and R₄ are as defined above), a R₂-B₁-R₄- group (wherein R₂, B₁ and R₄ are as defined above), a D₄-R₄- group (wherein D₄ and R₄ are as defined above), a D₅- group (wherein D₅ is as defined above), a D₁-R₄- group (wherein D₁ and R₄ are as defined above), a D₂- group (wherein D₂ is as defined above), a D₃-R4- group (wherein D₃ and R₄ are as defined above) or an A₄-SO₂-R₄- group {wherein A₄ represents a (b)- group (wherein (b) is as defined above), a (c)- group (wherein (c) is as defined above) or a R₁R₁'N- group (wherein R₁ and R₁' are as defined above), and R₄ is as defined above}, and B₄ represents an oxy group, a thio group or a -N((O)ₘR₁)-group (wherein R₁ and m are as defined above), provided that A₃ is not a hydrogen atom when B₄ is a thio group;
         (ii) a R₁-B₄-CO-R₄-B₄'- group, wherein R₁, B₄ and R₄ are as defined above, B₄' is the same as or different from B₄ and has the same meaning as B₄ has, provided that R₂ is not a hydrogen atom when B₄ is a thio group, or
            a D₂-R₄-B₄- group, wherein D₂, R₄ and B₄ are as defined above;
         (iii) a R₂-SO₂-NR₁- group, wherein R₂ is as defined above, provided that a hydrogen atom is excluded, and R₁ is as defined above;
         (iv) a (b)- group, wherein (b) is as defined above,
         (v) a (c)- group, wherein (c) is as defined above, or
         (vi) a R₁A₁N-NR₁'- group, wherein R₁, A₁ and R₁' are as defined above; and
            R₅ represents a C2-C10 alkenylene group optionally substituted with a halogen atom or a C2-C10 alkynylene group];
      (6) the F group: an A₅-B₅-R₆- group
         [wherein A₅ represents a C2-C10 alkyl group substituted with a D₄- group (wherein D₄ is as defined above), a D₁-group (wherein D₁ is as defined above), a D₃- group (wherein D₃ is as defined above) or an A₄-SO₂- group (wherein A₄ is as defined above), or a C1-C10 alkyl group substituted with a R₂-B₁- group (wherein R₂ and B₁ are as defined above), a D₂- group (wherein D₂ is as defined above), a D₅- group (wherein D₅ is as defined above) or an A₂-CO- group (wherein A₂ is as defined above),
         B₅ represents a B₁- group (wherein B₁ is as defined above) or a -NA₁- group (wherein A₁ is as defined above), and
         R₆ represents a single bond or a C1-C10 alkylene group];
      (7) the G group: an A₆ -B₅ -R₆- group
         [wherein A₆ represents an (a)-R₄- group (wherein (a) and R₄ are as defined above), or a C2-C10 alkenyl group, or a C2-C10 alkynyl group, or a C2-C10 alkenyl group substituted with a halogen atom, a R₂-B₁- group (wherein R₂ and B₁ are as defined above), a D₅- group (wherein D₅ is as defined above), a D₂- group (wherein D₂ is as defined above) or an A₂-CO- group (wherein A₂ is as define above), or a C2-C10 alkynyl group substituted with a halogen atom, a R₂-B₁-group (wherein R₂ and B₁ are as defined above), a D₅- group (wherein D₅ is as defined above), a D₂- group (wherein D₂ is as defined above) or an A₂-CO- group (wherein A₂ is as defined above), or a C3-C10 alkenyl group substituted with a (b)- group (wherein (b) is as defined above), a (c)-group (wherein (c) is as defined above), a D₄- group (wherein D₄ is as defined above), a D₁- group (wherein D₁ is as defined above) or a D₃- group (wherein D₃ is as defined above), or a C3-C10 alkynyl group substituted with a D₄-group (wherein D₄ is as defined above), a D₁- group (wherein D₁ is as defined above) or a D₃- group (wherein D₃ is as defined above), and B₅ and R₆ are as defined above];
      (8) the H group:
         a D₂-N(-(O)ₙ-A₁)-R₆- group, wherein D₂, n, A₁ and R₆ are as defined above,
         a D₂- group, wherein D₂ is as defined above, provided that a cyano group is excluded,
         a R₁(R₁' (O)ₙ)N-CR₁''=N-R₆- group, wherein R₁, R₁' , n and R₆ are as defined above, R₁" is the same as or different from R₁ and has the same meaning as R₁ has,
         a R₁-(O)ₙ-N=CR₁'-NR₂-R₆- group, wherein R₁, n, R₁' , R₂ and R₆ are as defined above,
         a R₂-B₃-NR₁-CO-NR₁'-R₆- group, wherein R₂, B₃, R₁, R₁' and R₆ are as defined above,
         a D₂-CO-NR₁-R₆- group, wherein D₂, R₁ and R₆ are as defined above, or
         an A₂-COCO-NR₁-R₆- group, wherein A₂, R₁ and R₆ are as defined above;
      (9) the I group:
         an A₇-B₆-N((O)ₙR₁)-R₆- group [wherein A₇ represents a C2-C10 alkenyl group optionally substituted with a halogen atom, or a C2-C10 alkynyl group, or a C3-C10 haloalkynyl group, or a R₂-B₁-R₄- group (wherein R₂, B₁ and R₄ are as defined above), or a D₄-R₄- group (wherein D₄ and R₄ are as defined above), or a D₅-R₄- group (wherein D₅ and R₄ are as defined above), or a D₁-R₄- group (wherein D₁ and R₄ are as defined above), or a (b)-R₄- group (wherein (b) and R₄ are as defined above), or a (c)-R₄- group (wherein (c) and R₄ are as defined above), or a D₂-R₄- group (wherein D₂ and R₄ are as defined above), or a D₃-R₄- group (wherein D₃ and R₄ are as defined above), or an A₄-SO₂-R₄- group (wherein A₄ and R₄ are as defined above), or an A₂-CO-R₄- group (wherein A₂ and R₄ are as defined above), B₆ represents a carbonyl group or a thiocarbonyl group, and n, R₁ and R₆ are as defined above],
         an A₈-CS-N((O)ₙR₁)-R₆- group [wherein A₈ represents a hydrogen atom or a C1-C10 alkyl group optionally substituted with a halogen atom, and n, R₁ and R₆ are as defined above],
         an A₇' -B₂' -B₃-N ((O)ₙR₁)-R₆- group [wherein A₇' represents a C3-C10 alkenyl group optionally substituted with a halogen atom, or a C3-C10 alkynyl group optionally substituted with a halogen atom, or a R₂-B₁-R₄'- group (wherein R₂ and B₁ are as defined above, and R₄' represents a C2-C10 alkylene group), or a D₄-R₄'- group (wherein D₄ and R₄' are as defined above), or a D₁-R₄'- group (wherein D₁ and R₄' are as defined above), or a (b)-R₄'- group (wherein (b) and R₄' are as defined above), or a (c)-R₄'- group (wherein (c) and R₄' are as defined above), or a D₂-R₄-group (wherein D₂ and R₄ are as defined above), or a D₃-R₄'-group (wherein D₃ and R₄' are as defined above), or an A₂-CO-R₄- group (wherein A₂ and R₄ are as defined above), B₂' represents an oxy group, a thio group or a -N((O)_{n'}R₁')-group (wherein n' is the same as or different from n and has the same meaning as n has, and R₁' is as defined above), and B₃, n, R₁ and R₆ are as defined above],
         an A₈'-B₂'-CS-N((O)ₙR₁)-R₄- group [wherein A₈' represents a C1-C10 alkyl group or a C2-C10 haloalkyl group, B₂' is as defined above, and n, R₁ and R₆ are as defined above],
         an A₈'-S-B₃'-N((O)ₙR₁)-R₆- group [wherein A₈', n, R₁ and R₆ are as defined above, and B₃' represents a carbonyl group or a sulfonyl group] or
         an A₇"-SO₂-N((O)ₙR₁)-R₆- group [wherein A₇" represents a C2-C10 alkenyl group, or a C3-C10 alkenyl group substituted with a halogen atom, or a C3-C10 alkynyl group optionally substituted with a halogen atom, or a R₂-B₁-R₄'- group (wherein R₂, B₁ and R₄' are as defined above), or a D₄-R₄'-group (wherein D₄ and R₄' are as defined above), or a D₅-R₄-group (wherein D₅ and R₄ are as defined above), or a D₁-R₄'-group (wherein D₁ and R₄' are as defined above), or a (b)-R₄'- group (wherein (b) and R₄' are as defined above), or a (c)-R₄'- group (wherein (c) and R₄' are as defined above), or a D₂-R₄- group (wherein D₂ and R₄ are as defined above), or a NO₂-R₄- group (wherein R₄ is as defined above), or an A₂-CO-R₄- group (wherein A₂ and R₄ are as defined above), and n, R₁ and R₄ are as defined above];
      (10) the J group:
         an A₇-CO- group (wherein A₇ is as defined above),
         an A₉-CS- group (wherein A₉ represents A₇ or A₈),
         an A₉' (O)ₘN=C(A₉)- group (wherein A₉' represents A₇' or A₈', and m and A₉ are as defined above),
         a D₂-CO- group (wherein D₂ is as defined above),
         an A₂-COCO- group (wherein A₂ is as defined above),
         an A₉-CO-B₁'-R₆- group (wherein A₉ and R₆ are as defined above, and B₁' represents an oxy group or a thio group, provided that A₉ is not A₈ when B₁' is an oxy group),
         an A₉-CS-B₁'-R₆- group (wherein A₉, B₁' and R₆ are as defined above),
         an A₇"-SO₂-B₁'-R₆- group (wherein A₇", B₁' and R₆ are as defined above),
         an A₈-SO₂-B₁'-R₆- group (wherein A₈, B₁' and R₆ are as defined above, provided that A₈ is not a hydrogen atom),
         an A₉'-B₂'-B₃-B₁'-R₆- group (wherein A₉' , B₂' , B₃, B₁' and R₆ are as defined above), and
         a C2-C10 alkenyl group substituted with a (b)- group (wherein (b) is as defined above) or a (c)- group (wherein (c) is as defined above);
      (11) the K group: an A₁₀-N((O)ₙR₁)-CO-R₆- group [wherein A₁₀ represents a hydrogen atom (provided that n is not 0), an A₇"-SO₂- group (wherein A₇" is as defined above), an A₈-SO₂- group (wherein A₈ is as defined above, provided that A₈ is not a hydrogen atom), an Ag'O- group (wherein A₉' is as defined above, provided that n is not 1), an Ag'-group (wherein A₉' is as defined above, provided that A₈' is excluded when n is 0), a R₂OCH₂- group (wherein R₂ is as defined above), an A₂-CO-R₄- group (wherein A₂ and R₄ are as defined above) or an A₂-CO-CH(CH₂CO-A₂)- group (wherein A₂ is as defined above), and n, R₁ and R₆ are as defined above];
      (12) the L group:
         an A₁₀'-N((O)ₙR₁)-SO₂-R₆- group [wherein A₁₀' represents a hydrogen atom (provided that n is not 0), an A₉'O- group (wherein A₉' is as defined above, provided that n is not 1), an A₉'- group (wherein A₉' is as defined above, provided that A₈' is excluded when n is 0), a R₂-CO- group (wherein R₂ is as defined above), an A₂-CO-R₄- group (wherein A₂ and R₄ are as defined above) or an A₂-CO-CH(CH₂CO-A₂)- group (wherein A₂ is as defined above), and n, R₁ and R₆ are as defined above],
         an A₉''R₁N-SO₂-N((O)ₙR₁')-R₆- group [wherein A₉" represents a hydrogen atom or an A₉'- group (wherein A₉' is as defined above), and R₁, n, R₁' and R₆ are as defined above], and
         a (b)-SO₂-N((O)ₙR₁')-R₆- group [wherein (b), n, R₁' and R₆ are as defined above];
      (13) the M group:
         a R₁(R₂S)C=N-R₆- group (wherein R₁, R₂ and R₆ are as defined above),
         a R₂B(R₂'B')C=N-R₆- group (wherein R₂ and R₆ are as defined above, R₂' is the same as or different from R₂ and has the same meaning as R₂ has, and B and B' are the same or different and represent an oxy group or a thio group),
         a R₁R₁'N-(R₂S)C=N-R₆- group (wherein R₁, R₁', R₂ and R₆ are as defined above),
         a R₁N=C(SR₂)-NR₂'-R₆- group (wherein R₁, R₂, R₂' and R₆ are as defined above), and
         a R₁(R₁'O)N-R₆- group (wherein R₁, R₁' and R₆ are as defined above);
      (14) the N group: an A₁₁-P(=O) (OR₁') -R₄- group [wherein A₁₁ represents a R₁- group (wherein R₁ is as defined above), a R₁O-R₆- group (wherein R₁ and R₆ are as defined above) or a R₁OCO-CHR₀- group (wherein R₁ and R₀ are as defined above), and R₁' and R₄ are as defined above];
   III. in (Y_{A})_{q}, Y_{A} represents a group included in the following X group or Y group, q is 0, 1, 2 or 3, the sum of p (wherein p is as defined above) and q is not more than 3, and when q is not less than 2, Y_{A}s are the same or different, and when q is not less than 2, adjacent two same or different Y_{A}s may together form a group included in the Z group to be fused to the A ring;
      (1) the X group: a Mₐ-group, wherein Mₐ represents a R_{b}- group (wherein R_{b} represents a C1-C10 alkyl group optionally substituted with a halogen atom), a halogen atom, a nitro group, a cyano group, a R_{c}-Bₐ-R_{d}- group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH- group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}-group (wherein Rₑ, Rₑ' and R_{d} are as defined above), a RₑRₑ'N-CO-NRₑ''-R_{d}- group (wherein Rₑ, Rₑ' and Rₑ'' are the same or different, Rₑ and Rₑ' are as defined above, Rₑ'' has the same meaning as Rₑ has, and R_{d} is as defined above), a RₑRₑ'N-C(=NRₑ'')-NRₑ'''-R_{d}- group (wherein Rₑ, Rₑ', Rₑ'' and Rₑ''' are the same or different, Rₑ, Rₑ' and Rₑ'' are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
      (2) the Y group: a M_{b}-R_{d}- group, wherein M_{b} represents a M_{c}-group [wherein M_{c} represents a M_{d}-R_{d}'- group {wherein M_{d} represents a phenyl group optionally substituted with a Mₐ- group (wherein Mₐ is as defined above), a pyridyl group optionally substituted with a Mₐ- group (wherein Mₐ is as defined above), a naphthyl group optionally substituted with a Mₐ- group (wherein Mₐ is as defined above), a (b)-group (wherein (b) is as defined above), a (c)- group (wherein (c) is as defined above), a (d)- group 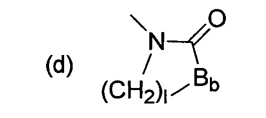 (wherein 1 is 2, 3 or 4, B_{b} represents an oxy group or a thio group) or an (e)- group 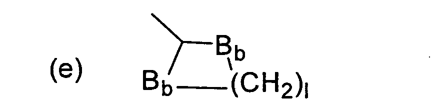 (wherein 1 and B_{b} are as defined above)}, and R_{d}' is the same as or different from R_{d} and has the same meaning as R_{d} has], a M_{c}-Bₐ- group (wherein M_{c} and Bₐ are as defined above), a M_{c}-CO- group (wherein M_{c} is as defined above), a M_{c}-CO-O- group (wherein M_{c} is as defined above), a M_{c}O-CO-group (wherein M_{c} is as defined above), a M_{c}RₑN- group (wherein M_{c} and Rₑ are as defined above), a M_{c}-CO-NRₑ- group (wherein M_{c} and Rₑ are as defined above), a M_{c}O-CO-NRₑ-group (wherein M_{c} and Rₑ are as defined above), a M_{c}RₑN-CO-group (wherein M_{c} and Rₑ are as defined above), a M_{c}RₑN-CO-NRₑ'- group (wherein M_{c}, Rₑ and Rₑ' are as defined above), a M_{c}RₑN-C(=NRₑ')-NRₑ"- group (wherein M_{c}, Rₑ, Rₑ' and Rₑ" are as defined above), a M_{c}-SO₂-NRₑ- group (wherein M_{c} and Rₑ are as defined above) or a M_{c}RₑN-SO₂- group (wherein M_{c} and Rₑ are as defined above), and
         R_{d} is as defined above;
      (3) Z group: a -Yₐ"=C(Yₐ)-Yₐ'- group (wherein Yₐ represents a hydrogen atom, or a C1-C10 alkyl group optionally substituted with a halogen atom, or a C1-C10 alkoxy group, Yₐ' represents an oxy group, or a thio group, or an imino group optionally substituted with a C1-C10 alkyl group, and Yₐ" represents a -N= group or a methine group),
         a C3-C10 alkylene group, and
         a -Y_{b}=Y_{b}'-Y_{b}"=Y_{b}'''- group, wherein Y_{b}, Y_{b}', Y_{b}" and Y_{b}''' are the same or different, and represent a methine group optionally substituted with a Mₐ- group (wherein Mₐ is as defined above), or a -N= group;
   IV. B is
      a group represented by formula (IV-1): 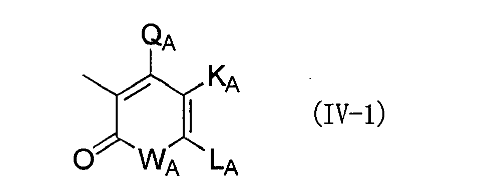 wherein,
      (1) Q_{A} represents a hydroxyl group, a (b)- group (wherein (b) is as defined above), an A₉-B₆-B_{c}- group [wherein A₉ and B₆ are as defined above, and B_{c} represents an oxy group or a -N((O)ₘR₁)- group (wherein m and R₁ are as defined above), provided that B_{c} is not a sulfonyl group when A₉ is a hydrogen atom], an A₇"-SO₂-B_{c}- group (wherein A₇" and B_{c} are as defined above), an A₈-SO₂-B_{c}- group (wherein A₈ and B_{c} are as defined above, provided that A_{B} is not a hydrogen atom), a R₁R₁'N-SO₂-B_{c}- group (wherein R₁ , R₁' and B_{c} are as defined above), a (b₀)-SO₂-O- group (wherein (b) and B_{c} are as defined above), an A₉'-B_{c}- group (wherein A₉' and B_{c} are as defined above), a D₅-R₄-B_{c}- group (wherein D₅ ,R₄ and B_{c} are as defined above), a M_{c}-B₃-B_{c}-group (wherein M_{c}, B₃ and B_{c} are as defined above) or a M_{c}-B_{c}- group (wherein M_{c} and B_{c} are as defined above),
      (2) W_{A} represents an oxygen atom or a -NT_{A}- group [wherein T_{A} represents a hydrogen atom, an A₉'- group (wherein A₉' is as defined above), a D₅-R₄- group (wherein D₅ and R₄ are as defined above) or a M_{c}- group (wherein M_{c} is as defined above)], and
      (3) K_{A} represents a hydrogen atom, a halogen atom or a C1-C10 alkyl group, L_{A} represents a hydrogen atom, a C1-C10 alkyl group or a M_{b}- group (wherein M_{b} is as defined above), or K_{A} and L_{A} may form a C3-C10 alkylene group or a - C(Mₐ')=C(Mₐ'')-C(Mₐ''')=C(Mₐ'''')- group (wherein Mₐ' , Mₐ'', Mₐ''' and Mₐ'''' are the same or different, and are the same as or different from Mₐ, and represent a hydrogen atom or Mₐ);
         a group represented by formula (IV-2): 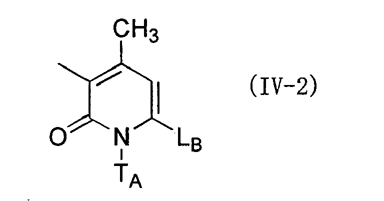
      wherein T_{A} is as defined above, and L_{B} represents a hydroxyl group or a methyl group;
      a group represented by formula (IV-3): 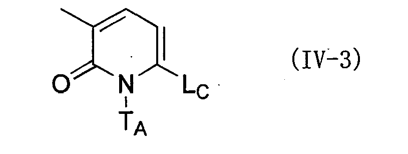 wherein T_{A} is as defined above, and L_{C} represents a C1-C10 alkyl group;
      a group represented by formula (IV-4): 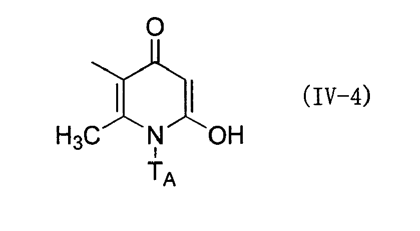 wherein T_{A} is as defined above;
      a group represented by formula (IV-5): 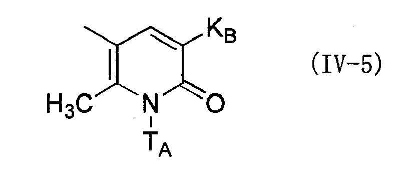 wherein T_{A} is as defined above, and K_{B} represents a cyano group or a UOCO- group (wherein U represents a hydrogen atom or C1-C10 alkyl group);
      a group represented by formula (IV-6): 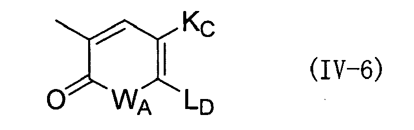 wherein W_{A} is as defined above, and K_{C} and L_{D} form a C3-C10 alkylene group, or a C4-C10 alkenylene group optionally substituted with 1 or more same or different Mₐ- groups (wherein Mₐ is as defined above);
      a group represented by formula (IV-7): 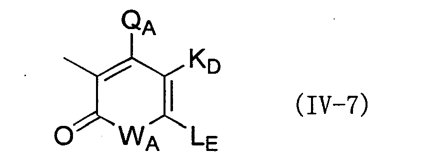 wherein Q_{A} and W_{A} are as defined above, and K_{D} and L_{E} form a -V_{A}=V_{A}'-V_{A}''=V_{A}'''- group {wherein V_{A}, V_{A}', VA'' and V_{A}''' are the same or different, and represent a methine group optionally substituted with a Mₐ- group (wherein Mₐ is as defined above), or a -N= group, and at least one of V_{A} V_{A}' V_{A}" and V_{A}''' represents a -N= group};
      a group represented by formula (IV-8): 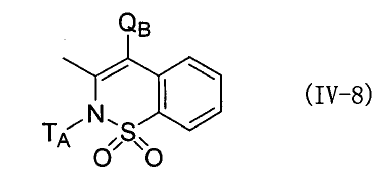
   wherein T_{A} is as defined above, Q_{B} represents a hydroxyl group, an A₉-B₆-O- group [wherein A₉ and B₆ are as defined above], an A₇''-SO₂-O- group (wherein A₇' ' is as defined above), an A₈-SO₂-O- group (wherein A₈ is as defined above, provided that A₈ is not a hydrogen atom), a R₁R₁'N-SO₂-O-group (wherein R₁ and R₁' are as defined above), a (b)-SO₂-O- group (wherein (b) is as defined above), an A₉'-O- group (wherein A₉' is as defined above), a D₅-R₄-O- group (wherein D₅ and R₄ are as defined above), a M_{c}-B₃-O- group (wherein M_{c} and B₃ are as defined above), or a M_{c}-O- group (wherein M_{c} is as defined above); or
   a group represented by formula (IV-9): 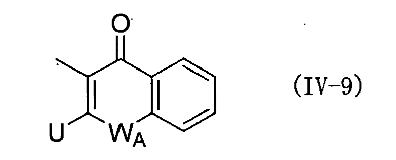 wherein U and W_{A} are as defined above, provided that when A is a furan ring or a thiophene ring, p and q are not 0 at the same time; and
   the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range];
5. A cinnamoyl compound represented by the formula (V): 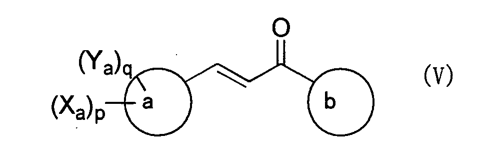 wherein,
   I. a represents a thiophene ring, a furan ring, a pyrrole ring, a pyrazole ring, a 1,2,3-triazole ring, a tetrazole ring, an isoxazole ring, a thiazole ring, a pyridazine ring or a pyrimidine ring; Xₐ represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an a₀-r₁-b-r₁'- group {wherein a₀ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a r₂O-CO- group (wherein r₂ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), a₁-NH-CO- group (wherein a₁ represents a C2-C10 alkyl group substituted with a C1-C10 alkoxy group), an a₁'-CO- group (wherein a₁' represents a morpholino group), a rr'N-CH₂- group (wherein r and r' are as defined above), a r₀-(O)₁-CONH-CH₂- group (wherein r₀ represents a C1-C10 alkyl group, and 1 represents 0 or 1), a r-OCH₂- group (wherein r is as defined above), a r₀-CO- group (wherein r₀ is as defined above), a cyano group or a sulfomethyl group, r₁ represents a C1-C10 alkylene group, r₁' represents a single bond or a C1-C10 alkylene group, and b represents an oxy group, a thio group, a sulfinyl group, a sulfonyl group, or an imino group optionally substituted with a methyl group}, or an a₂-y-CO-NH- group (wherein a₂ represents a C2-C10 alkyl group substituted with a C1-C10 alkoxy group, and y represents an oxy group or an imino group), or a rO-COCO-NH- group (wherein r is as defined above), or an a₃-z-NH- group (wherein a₃ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an a₄-Nr'CO- group {wherein a₄ represents a C1-C10 alkoxy group, or a C3-C10 alkenyloxy group, or a r₀-SO₂- group (wherein r₀ is as defined above), or a C2-C10 alkyl group substituted with a hydroxyl group or a C1-C10 alkoxy group, or a C2-C10 alkyl group substituted with a r₀r₀'N- group (wherein r₀ is as defined above, and r₀' is the same as or different from r₀, and represents the same meaning as r₀ has), or a C1-C10 alkyl group substituted with a rO-CO- group (wherein r is as defined above), a cyano group or an aminocarbonyl group, or a rO-CO-(rO-COCH₂)CH- group (wherein r is as defined above), and r' is as defined above}, or an a₅-NHSO₂- group (wherein a₅ represents a C2-C10 alkyl group substituted with a C1-C10 alkoxyl group), or a r₀ON=CH- group (wherein r₀ is as defined above), or a r₀NHCSNH- group (wherein r₀ is as defined above), or a r₀NHC(-Sr₀')=N- group (wherein r₀ and r₀' are as defined above), or a (rO)₂P(=O)CH₂- group (wherein r is as defined above), p represents 0, 1, 2 or 3 and, and when p is not less than 2, Xₐs are same or different,
      Yₐ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an a₀'-b'- group (wherein a₀' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a r₀r₀'N- group (wherein r₀ and r₀' are as defined above), or a r₀CO-NH- group (wherein r₀ is as defined above), or a r₀r₀'NCONH- group (wherein r₀ and r₀' are as defined above), or a rr'NCO- group (wherein r and r' are as defined above), or a hydroxyl group, q represents 0, 1, 2 or 3, and when q is not less than 2, Yₐs are the same or different, and when q is not less than 2, adjacent Yₐs may be fused to the a ring to form a 4,5,6,7-tetrahydrobenzo[b]thiophene ring;
   II. b is
      a group represented by formula (V-1): 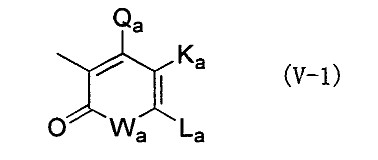
   wherein Qₐ represents a rₐ-O- group {rₐ represents a hydrogen atom, or a C1-C10 alkyl group, or a C3-C10 alkenyl group, or a C3-C10 alkynyl, or a C1-C10 alkyl group substituted with a r₀r₀'N-CH₂- group (wherein r₀ and r₀' are the same or different), a rOCH₂ group (wherein r is as defined above), a r₀-CO- group (wherein r₀ is as defined above), a C1-C10 alkoxycarbonyl group, a carboxy group, an aminocarbonyl group or a cyano group, or a r₃-r₁- group (wherein r₃ represents a phenyl group or a pyridyl group, and r₁ is as defined above)}, or a piperidino group, or a morpholino group, or a r₄r₄' N-group (wherein r₄ and r₄' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); Wₐ represents an oxygen atom or a - NTₐ- group [wherein Tₐ represents a r_{b}- group (wherein r_{b} is the same as or different from rₐ, and represents the same meaning as rₐ has) or a r₃'- group (wherein r₃' is the same as or different from r₃, and represents the same meaning as r₃ has)], and Kₐ represents a hydrogen atom, halogen atom or C1-C10 alkyl group, and Lₐ represents hydrogen atom or C1-C10 alkyl group, or Kₐ and Lₐ may form a 1,3-butadienylene group;
   a group represented by formula (V-2): 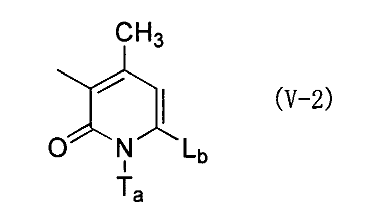 wherein Tₐ is as defined above, and L_{b} represents a hydroxyl group or a methyl group;
   a group represented by formula (V-3): 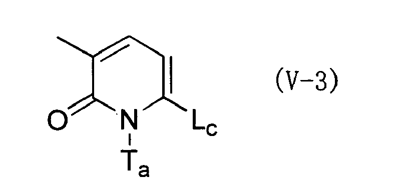 wherein Tₐ is as defined above, and L_{c} represents a C1-C10 alkyl group;
   a group represented by formula (V-4): 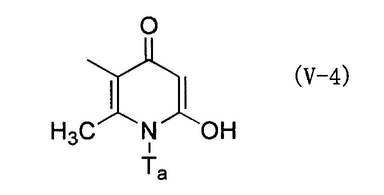 wherein Tₐ is as defined above;
   a group represented by formula (V-5): 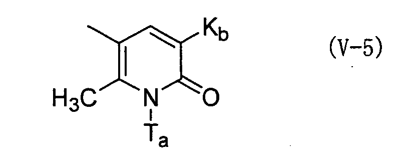 wherein Tₐ is as defined above, and K_{b} represents a cyano group or a UOCO- group (wherein U represents a hydrogen atom or C1-C10 alkyl group);
   a group represented by formula (V-6): 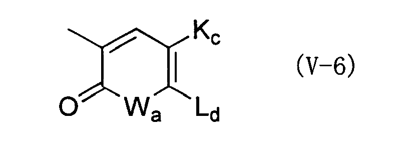 wherein Wₐ is as defined above, and Kₑ and L_{d} form a C3-C10 alkylene group or a C4-C10 alkenylene group;
   a group represented by formula (V-7): 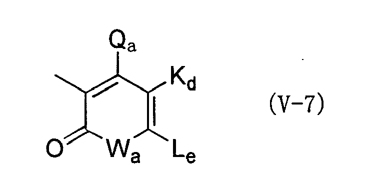 wherein Qₐ and Wₐ are as defined above, and K_{d} and Lₑ form a -Vₐ=Vₐ'-Vₐ''=Vₐ'''- group (wherein Vₐ, Vₐ', Vₐ'' and Vₐ''' are the same or different, and represent a methine group, or a -N= group, and at least one of Vₐ, Vₐ', Vₐ'' and Vₐ''' represents a -N= group);
   a group represented by formula (V-8): 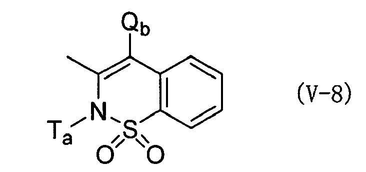 wherein Tₐ is as defined above, and Q_{b} represents a rₐ-O-group (wherein rₐ is as defined above); or
   a group represented by formula (V-9): 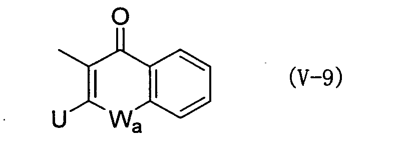 wherein U and Wₐ are as defined above, provided that when A is a furan ring or a thiophene ring, p and q are not 0 at the same time; and
   the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
6. A cinnamoyl compound represented by the formula (VI): 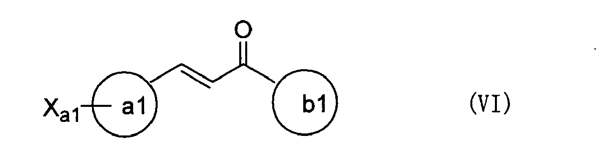 wherein
   a1 represents a thiophene ring, a furan ring, a pyrrole ring or a thiazole ring,
   Xₐ₁ represents an a₀'-r₁-b₀-r₁'- group {wherein a₀' represents a rO-CO- group (wherein r represents a hydrogen atom or a C1-C10 alkyl group), a r₀r₀'N-CH₂- group (wherein r₀ and r₀' are the same or different and represent a C1-C10 alkyl group), or a hydroxymethyl group, r₁ represents a C1-C10 alkylene group, r₁' represents a single bond or a C1-C10 alkylene group, b₀ represents an oxy group, a thio group, a sulfinyl group, a sulfonyl group, or an imino group substituted with one methyl group}, or an a₂-O-CO-NH-group (wherein a₂ represents a C2-C10 alkyl group substituted with a C1-C10 alkoxy group), or an a₃'-CO-NH-group (wherein a₃' represents a C1-C10 alkyl group substituted with a C1-C10 alkoxy group), or an a₄'-NrCO-group (wherein a₄' represents a C2-C10 alkyl group substituted with a hydroxyl group or a C1-C10 alkoxy group, or a C1-C10 alkyl group substituted with an aminocarbonyl group, and r is as defined above), and
   b1 represents a group represented by formula (VI-1): 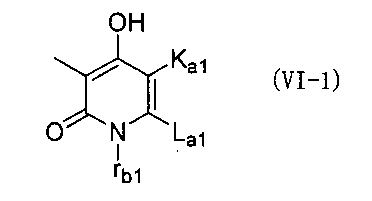 (wherein r_{b1} represents a hydrogen atom or C1-C10 alkyl group, Kₐ₁ represents a hydrogen atom, and Lₐ₁ represents C1-C10 alkyl group, or Kₐ₁ and Lₐ₁ may form a 1,3-butadienylene group),
   a group represented by formula (VI-7): 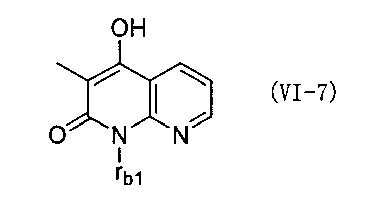 (wherein r_{b1} is as defined above), or
   a group represented by formula (VI-8): 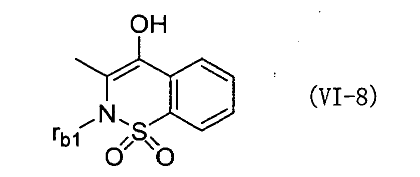 (wherein r_{b1} is as defined above); and
   the term "as defined above" used for the same symbols among plural substituents means that the plural substituents independently represent the same meaning as
   that described above and, among the plural substituents, although the selection range of substituents to be selected is the same, selected substituents may be the same or different as long as they are selected within the range;
7. An aldehyde derivative represented by the formula (VII) : 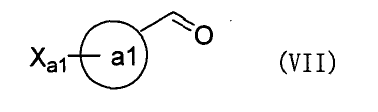 wherein
   a1 represents a thiophene group, a furan ring, a pyrrole group or a thiazole ring, and
   Xₐ₁ represents an a₀'-r₁-b₀-r₁'- group {wherein a₀' represents a r₀O-CO- group (wherein r₀ represents a C1-C10 alkyl group), a r₀r₀'N-CH₂- group (wherein r₀ and r₀' are the same or different, and r₀' represents the same meaning as that of r₀), or a hydroxymethyl group, r₁ represents a C1-C10 alkylene group, r₁' represents a single bond or a C1-C10 alkylene group, b₀ represents an oxy group, a thio group, a sulfinyl group, a sulfonyl group, or an imino group substituted with one methyl group}, or an a₂-O-CO-NH-group (wherein a₂ represents a C2-C10 alkyl group substituted with a C1-C10 alkoxy group), or an a₃'-CO-NH-group (wherein a₃' represents a C1-C10 alkyl group substituted with a C1-C10 alkoxy group), or an a₄'-NrCO-group (wherein a₄' represents a C2-C10 alkyl group substituted with a hydroxyl group or a C1-C10 alkoxy group, or a C1-C10 alkyl group substituted with an aminocarbonyl group, r represents a hydrogen atom or a C1-C10 alkyl group),
   provided that a1 is not a thiophene ring when a₀' is a dimethylaminomethyl group or a hydroxymethyl group, r₁ is a methylene group, r₁' is a single bond and b₀ is an oxy group, a thio group or an imino group substituted with one methyl group, at the same time, and a1 is not a thiophene ring when a₀' is a methoxycarbonyl group, r₁ is a methylene group, r₁' is a single bond and b₀ is a thio group, at the same time;
8. A process for producing a cinnamoyl compound represented by the formula (VIII' ' ) : 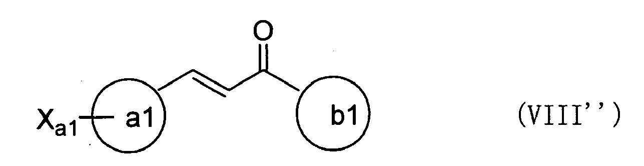 [wherein a1, Xₐ₁ and b1 are as defined below], which comprises reacting an aldehyde derivative represented by the formula (VIII): 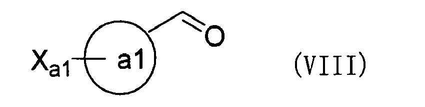 [wherein
   a1 represents a thiophene group, a furan ring, a pyrrole group or a thiazole ring, and
   Xₐ₁ represents an a₀'-r₁-b₀-r₁'- group {wherein a₀' represents a r₀O-CO- group (wherein r₀ represents a C1-C10 alkyl group), a r₀r₀'N-CH₂- group (wherein r₀ and r₀' are the same or different, and r₀' represents the same meaning as that of r₀), or a hydroxymethyl group, r₁ represents a C1-C10 alkylene group, r₁' represents a single bond or a C1-C10 alkylene group, b₀ represents an oxy group, a thio group, a sulfinyl group, a sulfonyl group, or an imino group substituted with one methyl group}, or an a₂-O-CO-NH-group (wherein a₂ represents a C2-C10 alkyl group substituted with a C1-C10 alkoxy group), or an a₃'-CO-NH-group (wherein a₃' represents a C1-C10 alkyl group substituted with a C1-C10 alkoxy group), or an a₄'-NrCO-group (wherein a₄' represents a C2-C10 alkyl group substituted with a hydroxyl group or a C1-C10 alkoxy group, or a C1-C10 alkyl group substituted with an aminocarbonyl group, r represents a hydrogen atom or a C1-C10 alkyl group),
   provided that a1 is not a thiophene ring when a₀' is a dimethylaminomethyl group or a hydroxymethyl group, r₁ is a methylene group, r₁' is a single bond and b₀ is an oxy group, a thio group or an imino group substituted with one methyl group, at the same time, and a1 is not a thiophene ring when a₀' is a methoxycarbonyl group, r₁ is a methylene group, r₁' is a single bond and b₀ is a thio group, at the same time]
   with a compound represented by formula (VIII') 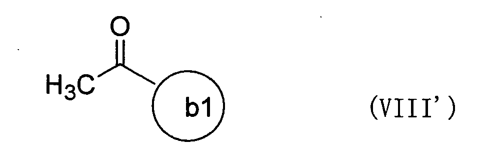 [wherein
   b1 is a group represented by formula (VIII-1): 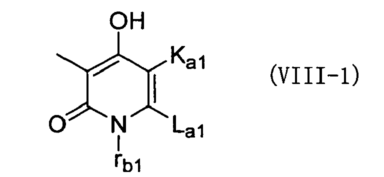 (wherein r_{b1} represents a hydrogen atom or C1-C10 alkyl group, Kₐ₁ represents a hydrogen atom, and Lₐ₁ represents C1-C10 alkyl group, or Kₐ₁ and Lₐ₁ may form a 1,3-butadienylene group),
   a group represented by formula (VIII-7): 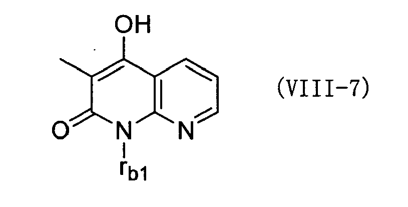 (wherein r_{b1} is as defined above), or
   a group represented by formula (VIII-8): 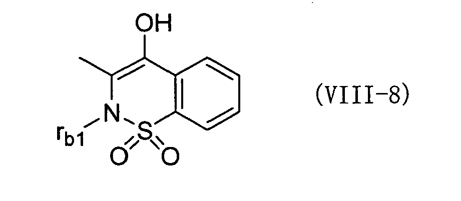 (wherein r_{b1} is as defined above)];
9. A composition for suppressing transcription of an extracellular matrix gene which comprises an inert carrier and a cinnamoyl compound represented by the formula (I'): 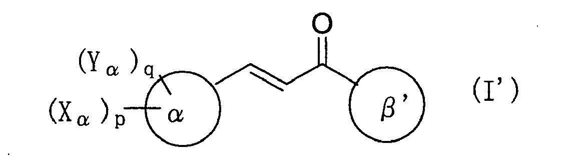 wherein,
   I. α represents an aromatic 5-membered ring, or an aromatic 6-membered ring having two or more nitrogen atoms; in (Y_{α})_{q}, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the α ring; in (X_{α})ₚ, X_{α} represents a substituent which does not belong to the following X₀ group, Y₀ group and Z₀ group, p represents 0, 1, 2 or 3 and, when p is not less than 2, X_{α}s are the same or different, and the sum of p and q is not more than 3;
      (1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ")-NRₑ'''-R_{d}- group (wherein Rₑ, Rₑ', Rₑ" and Rₑ''' are the same or different, Rₑ, Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
      (2) the Y₀ group: a M_{b0}-R_{d}- group, wherein M_{b0} represents a M_{c0}- group
         [wherein M_{co} represents a M_{do}-R_{d}'- group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 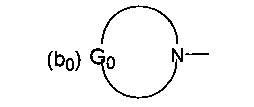 (wherein Go forms an optionally substituted, saturated or unsaturated, nonaromatic 5 to 14-membered hydrocarbon ring or heterocyclic ring), a (c₀)- group represented by 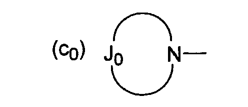 (wherein, J₀ forms a 5 to 7-membered aromatic ring optionally containing a nitrogen atom), a (do)- group represented by 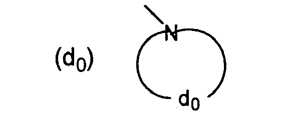 [wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group], or a (e₀)- group represented by 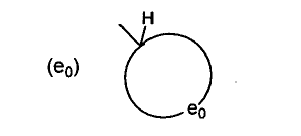 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}; and R_{d}' is the same as or different from R_{d} and has the same meaning as R_{d} has],
         a M_{c0}-Bₐ- group (wherein M_{c0} and Bₐ are as defined above), a M_{c0}-CO- group (wherein M_{c0} is as defined above), a M_{c0}-CO-O-group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ-group (wherein M_{c0} and Rₑ are as defined above), a M_{c0}RₑN-CO-group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
      (3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and
   II. β' is a group represented by formula (I'-1): 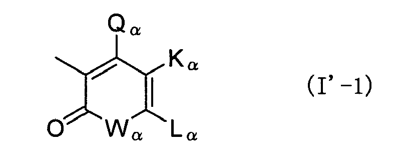 [wherein,
      (1)Q_{α} represents an optionally substituted hydroxyl group, or an optionally substituted amino group,
      (2)W_{α} represents an oxygen atom or a -NT_{α}- group (wherein T_{α} represents a hydrogen atom, or a substituent on the nitrogen atom),
      (3)K_{α} and L_{α} are the same or different, and represent a hydrogen atom, or a substituent on a carbon atom, or K_{α} and L_{α} may form an optionally substituted C3-C10 alkylene group or an optionally substituted C4-C10 alkenylene group],
         a group represented by formula (I'-2): 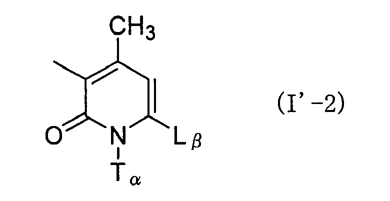 [wherein T_{α} is as defined above, and L_{β} represents a hydroxyl group or a methyl group],
         a group represented by formula (I'-3): 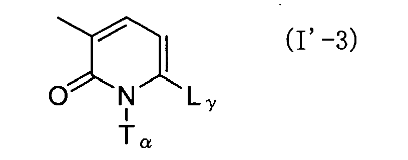 [wherein T_{α} is as defined above, and L_{γ} represents a C1-C10 alkyl group],
         a group represented by formula (I'-4): 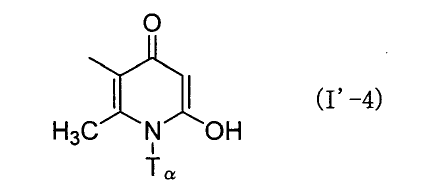 [wherein T_{α} is as defined above],
         a group represented by formula (I'-5): 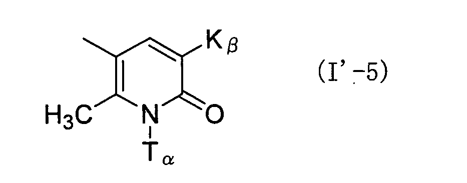 [wherein T_{α} is as defined above, and K_{β} represents a cyano group or a UOCO-group (wherein U represents a hydrogen atom or a C1-C10 alkyl group)],
         a group represented by formula (I'-6): 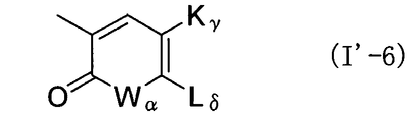 [wherein W_{α} is as defined above, and K_{γ} and L_{δ} form an optionally substituted C3-C10 alkylene group or an optionally substituted C4-C10 alkenylene group],
         a group represented by formula (I'-7): 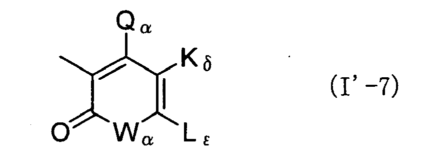 [wherein Q_{α} and W_{α} are as defined above, and K_{δ} and L_{ε} form a -V_{α}=V_{α}'-V_{α}'' =V_{α}''' - group (wherein V_{α}, V_{α}', V_{α}'' and V_{α}''' are the same or different, and represent an optionally substituted methine group or a -N= group, and at least one of V_{α}, V_{α}', V_{α}'' and V_{α}''' represents a -N= group)],
         a group represented by formula (1'-8): 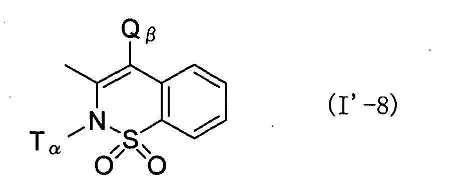 [wherein T_{α} is as defined above, and Q_{β} represents an optionally substituted hydroxyl group], or
         a group represented by formula (I'-9): 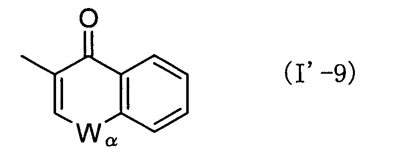 [wherein W_{α} is as defined above]; and
         the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
10. A cinnamoyl compound represented by formula (II'): 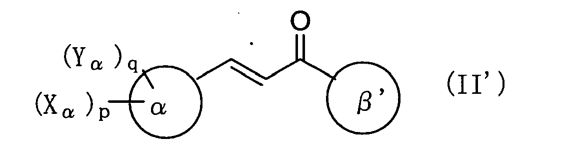 wherein,
   I. α represents an aromatic 5-membered ring, or an aromatic 6-membered ring having two or more nitrogen atoms; in (Y_{α})_{q}, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the α ring; in (X_{α})ₚ, X_{α} represents a substituent which does not belong to the following X₀ group, Y₀ group and Z₀ group, p represents 0, 1, 2 or 3 and, when p is not less than 2, X_{α}s are the same or different, and the sum of p and q is not more than 3;
      (1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ")-NRₑ'''-R_{d}- group (wherein Rₑ, Rₑ', Rₑ" and Rₑ''' are the same or different, Rₑ, Rₑ' and Rₑ" are as defined above, Rₑ' ' ' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
      (2) the Y₀ group: a M_{b0}-R_{d}- group, wherein M_{b0} represents a M_{c0}- group
         [wherein M_{co} represents a M_{do}-R_{d}'- group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 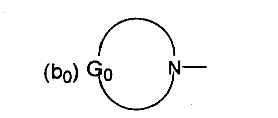 (wherein Go forms an optionally substituted, saturated or unsaturated, nonaromatic 5 to 14-membered hydrocarbon ring or heterocyclic ring), a (c₀)- group represented by 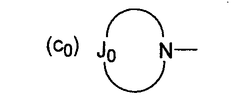 (wherein, J₀ forms a 5 to 7-membered aromatic ring optionally containing a nitrogen atom), a (do)- group represented by 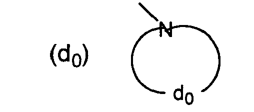 [wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group], or a (e₀)- group represented by 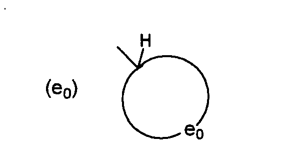 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}; and R_{d}' is the same as or different from R_{d} and has the same meaning as R_{d} has],
         a M_{c0}-Bₐ- group (wherein M_{c0} and Bₐ are as defined above), a M_{c0}-CO- group (wherein M_{c0} is as defined above), a M_{c0}-CO-O-group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ-group (wherein M_{c0} and Rₑ are as defined above), a M_{c0}RₑN-CO-group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
      (3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl, group or a sulfonyl group, and which is aromatic or nonaromatic and monocyclic or fused ring and is fused to the α ring; and
   II. β' is a group represented by formula (II'-1): 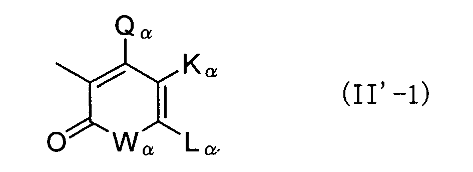 [wherein,
      (1)Q_{α} represents an optionally substituted hydroxyl group, or an optionally substituted amino group,
      (2)W_{α} represents an oxygen atom or a -NT_{α}- group (wherein T_{α} represents a hydrogen atom, or a substituent on the nitrogen atom),
      (3)K_{α} and L_{α} are the same or different, and represent a hydrogen atom, or a substituent on a carbon atom, or K_{α} and L_{α} may form an optionally substituted C3-C10 alkylene group or an optionally substituted C4-C10 alkenylene group],
         a group represented by formula (II'-2): 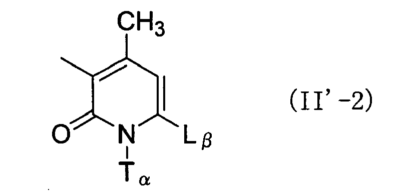 [wherein T_{α} is as defined above, and L_{β} represents a hydroxyl group or a methyl group],
         a group represented by formula (II'-3): 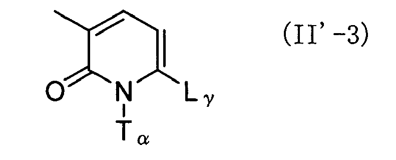 [wherein T_{α} is as defined above, and L_{γ} represents a C1-C10 alkyl group],
         a group represented by formula (II'-4): 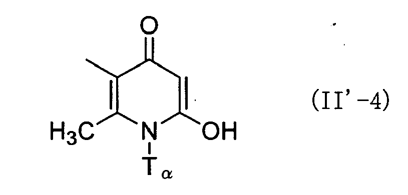 [wherein T_{α} is as defined above],
         a group represented by formula (II'-5): 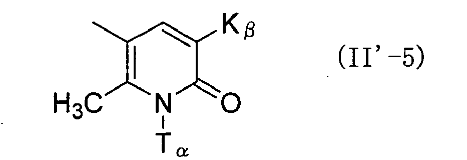 [wherein T_{α} is as defined above, and K_{β} represents a cyano group or a UOCO-group (wherein U represents a hydrogen atom or a C1-C10 alkyl group)],
         a group represented by formula (II'-6): 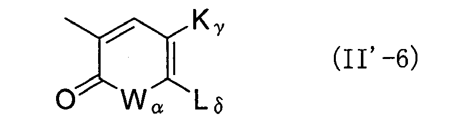 [wherein W_{α} is as defined above, and K_{γ} and L_{δ} form an optionally substituted C3-C10 alkylene group or an optionally substituted C4-C10 alkenylene group],
         a group represented by formula (II'-7): 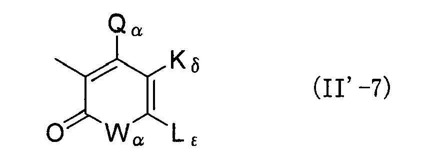 [wherein Q_{α} and W_{α} are as defined above, and K_{δ} and L_{ε} form a -V_{α}=V_{α} 'V_{α}'' =V_{α}'''- group (wherein V_{α}, V_{α}', V_{α}'' and V_{α}''' are the same or different, and represent an optionally substituted methine group or a -N= group, and at least one of V_{α}, V_{α}', V_{α}'' and V_{α}''' represents a -N= group)],
         a group represented by formula (II'-8): 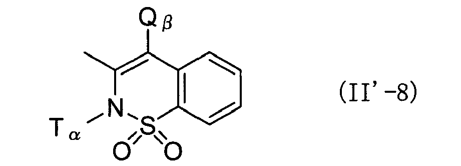 [wherein T_{α} is as defined above, and Q_{β} represents an optionally substituted hydroxyl group], or
         a group represented by formula (II'-9): 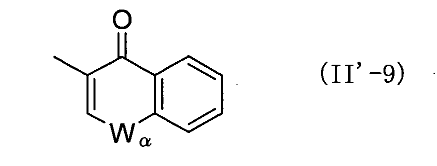 [wherein W_{α} is as defined above], provided that when α is a furan ring or a thiophene ring, p and q are not 0 at the same time; and
         the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
11. A cinnamoyl compound represented by formula (V'): 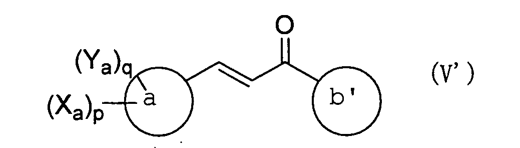 wherein,
   I. a represents a thiophene ring, a furan ring, a pyrrole ring or a tetrazole ring; Xₐ represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an a₀-r₁-b-r₁'- group {wherein a₀ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a r₂O-CO- group (wherein r₂ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), a₁-NH-CO- group (wherein a₁ represents a C2-C10 alkyl group substituted with a C1-C10 alkoxy group), an a₁'-CO- group (wherein a₁' represents a morpholino group), a rr'N-CH₂- group (wherein r and r' are as defined above), a r₀-(O)₁-CONH-CH₂- group (wherein r₀ represents a C1-C10 alkyl group, and 1 represents 0 or 1), a r-OCH₂- group (wherein r is as defined above), a r₀-CO- group (wherein r₀ is as defined above), a cyano group or a sulfomethyl group, r₁ represents a C1-C10 alkylene group, r₁' represents a single bond or a C1-C10 alkylene group, and b represents an oxy group, a thio group, a sulfinyl group, a sulfonyl group, or an imino group}, or an a₂-y-CO-NH- group (wherein a₂ represents a C2-C10 alkyl group substituted with a C1-C10 alkoxy group, and y represents an oxy group or an imino group), or a rO-COCO-NH- group (wherein r is as defined above), or an a₃-z-NH- group (wherein a₃ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an a₄-Nr'CO- group {wherein a₄ represents a C1-C10 alkoxy group, or a C3-C10 alkenyloxy group, or a r₀-SO₂- group (wherein r₀ is as defined above), or a C2-C10 alkyl group substituted with a hydroxyl group or a C1-C10 alkoxy group, or a C2-C10 alkyl group substituted with a r₀r₀'N- group (wherein r₀ is as defined above, and r₀' is the same as or different from r₀, and represents the same meaning as r₀ has), or a C1-C10 alkyl group substituted with a rO-CO- group (wherein r is as defined above), a cyano group or an aminocarbonyl group, or a rO-CO-(rO-COCH₂)CH- group (wherein r is as defined above), and r' is as defined above}, or an a₅-NHSO₂- group (wherein a₅ represents a C2-C10 alkyl group substituted with a C1-C10 alkoxyl group), or a r₀ON=CH- group (wherein r₀ is as defined above), or a r₀NHCSNH- group (wherein r₀ is as defined above), or a r₀NHC(-Sr₀')=N- group (wherein r₀ and r₀' are as defined above), or a (rO)₂P(=O)CH₂- group (wherein r is as defined above),
      p represents 0, 1, 2 or 3 and, and when p is not less than 2, Xₐs are same or different,
      Yₐ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an a₀'-b'- group (wherein a₀' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a r₀r₀'N- group (wherein r₀ and r₀' are as defined above), or a r₀CO-NH- group (wherein r₀ is as defined above), or a r₀r₀'NCONH- group (wherein r₀ and r₀' are as defined above), or a rr'NCO- group (wherein r and r' are as defined above), or a hydroxyl group,
      q represents 0, 1, 2 or 3, and when q is not less than 2, Yₐs are the same or different, and when q is not less than 2, adjacent Yₐs may be fused to the a ring to form a 2,3-dihydro-benzo[1,4]dioxine ring;
   II. b' is
      a group represented by formula (V'-1): 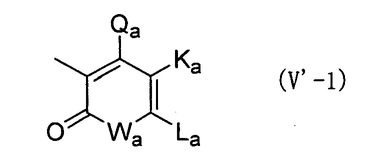
   wherein Qₐ represents a rₐ-O- group {rₐ represents a hydrogen atom, or a C1-C10 alkyl group, or a C3-C10 alkenyl group, or a C3-C10 alkynyl, or a C1-C10 alkyl group substituted with a r₀r₀'N-CH₂- group (wherein r₀ and r₀' are the same or different), a rOCH₂ group (wherein r is as defined above), a r₀-CO- group (wherein r₀ is as defined above), a C1-C10 alkoxycarbonyl group, a carboxy group, an aminocarbonyl group or a cyano group, or a r₃-r₁- group (wherein r₃ represents a phenyl group or a pyridyl group, and r₁ is as defined above)}, or a piperidino group, or a morpholino group, or a r₄r₄'N-group (wherein r₄ and r₄' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); Wₐ represents an oxygen atom or a - NTₐ- group [wherein Tₐ represents a r_{b}- group (wherein r_{b} is the same as or different from rₐ, and represents the same meaning as rₐ has) or a r₃' - group (wherein r₃' is the same as or different from r₃, and represents the same meaning as r₃ has)], and Kₐ represents a hydrogen atom, halogen atom or C1-C10 alkyl group, and Lₐ represents hydrogen atom or C1-C10 alkyl group, or Kₐ and Lₐ may form a 1,3-butadienylene group;
   a group represented by formula (V'-2): 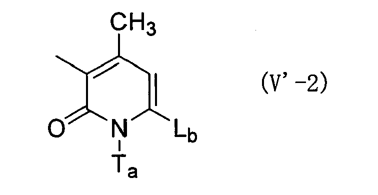 wherein Tₐ is as defined above, and L_{b} represents a hydroxyl group or a methyl group;
   a group represented by formula (V'-3): 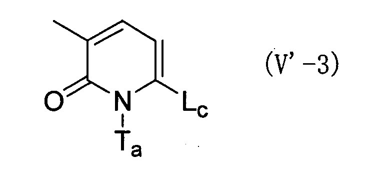 wherein Tₐ is as defined above, and L_{c} represents a C1-C10 alkyl group;
   a group represented by formula (V'-4): 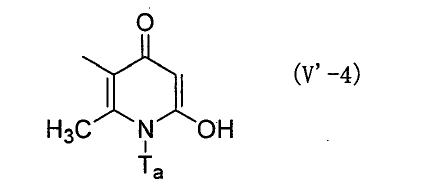 wherein Tₐ is as defined above;
   a group represented by formula (V'-5): 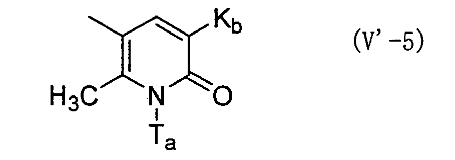 wherein Tₐ is as defined above, and K_{b} represents a cyano group or a UOCO- group (wherein U represents a hydrogen atom or C1-C10 alkyl group);
   a group represented by formula (V'-6): 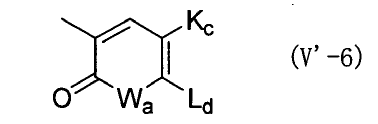 wherein Wₐ is as defined above, and K_{c} and L_{d} form a C3-C10 alkylene group or a C4-C10 alkenylene group;
   a group represented by formula (V'-7): 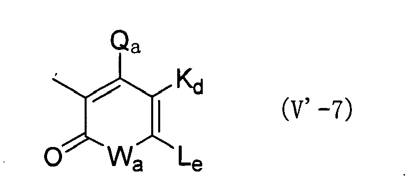 wherein Qₐ and Wₐ are as defined above, and K_{d} and Lₑ form a -Vₐ=Vₐ'-Vₐ''=Vₐ'''- group (wherein Vₐ, Vₐ' , Vₐ" and Vₐ''' are the same or different, and represent a methine group, or a -N= group, and at least one of Vₐ, Vₐ', Vₐ'' and Vₐ''' represents a -N= group);
   a group represented by formula (V'-8): 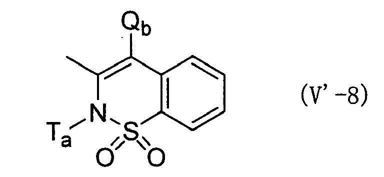 wherein Tₐ is as defined above, and Q_{b} represents a rₐ-O-group (wherein rₐ is as defined above); or
   a group represented by formula (V'-9): 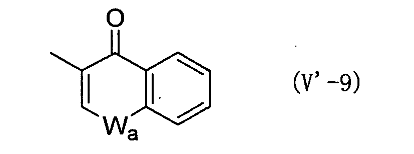 wherein Wₐ is as defined above, provided that when a is a furan ring or a thiophene ring, p and q are not 0 at the same time; and
   the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range;
12. A composition for suppressing transcription of an extracellular matrix gene, which comprises the compound according to any one of the above items 2 to 6, 10 and 11, and an inert carrier;
13. A composition for suppressing transcription of an extracellular matrix gene, which comprises the compound according to the above item 5 or 11, and an inert carrier;
14. Use of a cinnamoyl compound contained in the composition according to the above item 1 or 9 as an active ingredient, as an active ingredient for suppressing transcription of an extracellular matrix gene;
15. Use of the compound according to any one of the above items 2 to 6, 10 and 11, as an active ingredient for suppressing transcription of an extracellular matrix gene;
16. Use of the compound according to the above item 5 or 11, as an active ingredient for suppressing transcription of an extracellular matrix gene;
17. Use of a cinnamoyl compound contained in the composition according to the above item 1 or 9 as an active ingredient, as an active ingredient for decreasing expression of an extracellular matrix gene to induce a reduction in accumulation of an extracellular matrix and thereby improving tissue fibrosis;
18. Use of the compound according to any one of the above items 2 to 6, 10 and 11, as an active ingredient for decreasing expression of an extracellular matrix gene to induce a reduction in accumulation of an extracellular matrix and thereby improving tissue fibrosis;
19. A method for improving tissue fibrosis, which comprises administering an effective amount of a cinnamoyl compound contained in the composition according to the above item 1 or 9 as an active ingredient to a mammalian patient in need thereof;
20. A method for improving tissue fibrosis, which comprises administering an effective amount of the compound according to any one of the above items 2 to 6, 10 and 11 to a mammalian patient in need thereof;
21. An agent for treating chronic renal failure, which comprises the compound according to any one of the above items 2 to 6, 10 and 11 and an inert carrier;
22. Use of a cinnamoyl compound contained in the composition according to the above item 1 or 9 as an active ingredient, or the compound according to any one of the above items 2 to 6, 10 and 11, as an active ingredient for treating chronic renal failure;
23. A method for treating chronic renal failure, which comprises administering an effective amount of a cinnamoyl compound contained in the composition according to the above item 1 or 9 as an active ingredient, or the compound according to any one of the above items 2 to 6, 10 and 11 to a mammalian patient in need thereof;
24. An agent for treating heart failure, which comprises a cinnamoyl compound contained in the composition according to the above item 1 or 9 as an active ingredient, or the compound according to any one of the above items 2 to 6, 10 and 11, and an inert carrier;
25. Use of a cinnamoyl compound contained in the composition according to the above item 1 or 9 as an active ingredient, or the compound according to the above items 2 to 6, 10 and 11, as an active ingredient for treating heart failure;
26. A method for treating heart failure, which comprises administering an effective amount of a cinnamoyl compound contained in the composition according to the above item 1 or 9 as an active ingredient, or the composition according to any one of the above items 2 to 6, 10 and 11 to a mammalian patient in need thereof;
27. A composition for suppressing the activity of TGF-β, which comprises a cinnamoyl compound contained in the composition according to the above item 1 or 9 as an active ingredient, and an inert carrier;
28. A composition for suppressing the activity of TGF-β, which comprises the compound according to any one of the above items 2 to 6, 10 and 11, and an inert carrier;
29. Use of a cinnamoyl compound contained in the composition according to the above item 1 or 9 as an active ingredient, as an active ingredient for suppressing the activity of TGF-β;
30. Use of the compound according to any one of the above items 2 to 6, 10 and 11, as an active ingredient for suppressing the activity of TGF-β;
31. A composition for hair growth which comprises a cinnamoyl compound contained in the composition according to the above item 1 or 9 as an active ingredient, or the compound according to any one of the above items 2 to 6, 10 and 11, and an inert carrier;
32. Use of a cinnamoyl compound contained in the composition according to the above item 1 or 9 as an active ingredient, or the compound according to any one of the above items 2 to 6, 10 and 11, as an active ingredient for inhibiting a promoting effect of TGF-β on transition to a hair regression phase to induce extension of a hair growth phase and thereby providing a hair-growing effect; and
33. A method for growing hair, which comprises administering an effective amount of a cinnamoyl compound contained in the composition according to the above item 1 or 9 as an active ingredient, or the compound according to any one of the above items 2 to 6, 10 and 11, to a mammalian patient in need thereof.

### Best Mode for Carrying Out the Invention

The present invention will be explained in detail below.

In the present invention, a saturated hydrocarbon group in an alkyl group, a haloalkyl group, an alkoxy group, an alkoxycarbonyl group, an alkylthio group, an alkylsulfinyl group, an alkylsulfonyl group and an alkylene group may be branched, or a part or all of carbon atoms thereof may form a ring. An unsaturated hydrocarbon group in an alkenyl group, an alkenyloxy group, an alkynyl group, an alkynyloxy group, an alkenylene group and an alkynylene group may be branched, or a part or all of carbon atoms thereof may form a ring, and the number of unsaturated bonds thereof may be singular or plural.

In the present invention, examples of an alkyl group include a methyl group, an ethyl group, an isopropyl group, a cyclohexyl group, a cyclopropylmethyl group and the like. Examples of a haloalkyl group include a 2,2,2-trifluoroethyl group and the like. Examples of an alkoxy group include a methoxy group, an ethoxy group, a cyclopentyloxy group, a 2-cyclohexylethoxy group and the like. Examples of an alkylthio group include a methylthio group and the like. Examples of an alkylsulfinyl group include a methylsulfinyl group and the like. Examples of an alkylsulfonyl group include a methylsulfonyl group and the like. Examples of an alkylene group include a methylene group, an ethylethylene group, a 1,4-cyclohexylene group and the like. Examples of an alkenyl group include a vinyl group, a 2-propenyl group, a 3-methyl-2-butenyl group, a 1,3-butadienyl group, a 3-cyclohexenyl group and the like. Examples of an alkynyl group include an ethynyl group, a 2-propynyl group, a 2-penten-4-ynyl group and the like. Examples of an alkenylene group include a vinylene group, a propenylene group, a 1,3-butadienylene group and the like. Examples of an alkynylene group include an ethynylene group, a propynylene group and the like.

In the present invention, examples of a halogen atom include a fluorine atom, a chlorine atom, a bromine atom and an iodine atom.

In the present invention, a pyridyl group includes a 2-pyridyl group, a 3-pyridyl group and a 4-pyridyl group. A furyl group includes a 2-furyl group and a 3-furyl group. A thienyl group includes a 2-thienyl group and a 3-thienyl group. A naphthyl group includes a 1-naphthyl group and a 2-naphthyl group.

In a cinnamoyl compound represented by the formula (I), (II), (III), (IV), (V), (I') or (II') (hereinafter, referred to as the compound (I), (II), (III), (IV), (V), (I') or (II'), respectively), when the α ring, the A0 ring, the A ring or the a ring is an aromatic 6-memberd ring having two or more nitrogen atoms, an N-oxide thereof is also included.

In the α ring, the A0 ring, the A ring and the a ring of the compounds (I) to (V), (I') and (II'), examples of the aromatic 5-memberd ring include a thiophene ring, a furan ring, a pyrrole ring, an imidazole ring, a pyrazole ring, a 1,2,3-triazole ring, a 1,2,4-triazole ring, a tetrazole ring, an oxazole ring, an isoxazole ring, a thiazole ring, an isothiazole ring, a furazane ring, a 1,2,5-thiadiazole ring and the like, and examples of the aromatic 6-membered ring having two or more nitrogen atoms include a pyridazine ring, a pyrimidine ring, a pyrazine ring, a 1,3,5-triazine ring, a 1,2,4-triazine ring and the like.

The cinnamoyl compound represented by the formulas (I) to (VI), (I'), (II') or (V') (hereinafter, referred to as the compound (I) to (VI), (I'), (II') or (V'), respectively) includes a pharmaceutically acceptable salt thereof. The pharmaceutically acceptable salt includes a salt of the compound (I) to (VI), (I'), (II') or (V') (hereinafter, referred to as the present compound in some cases) with an inorganic acid, an organic acid, an inorganic base, or an organic base. Examples of a salt with an inorganic acid include hydrochloride, hydrobromide and the like. Examples of a salt with an organic acid include acetate, benzoate and the like. Examples of a salt with an inorganic base include a potassium salt, a sodium salt and the like. Examples of a salt with an organic base include a pyridine salt, a morpholine salt and the like.

X_{A0}, Y_{A0}, Q_{A0}, K_{A0}, L_{A0} and T_{A0} in the compound (III) are independently represented by a group represented by D₁, D₂, D₃, D₄, D₅, R₀, R₀', R₀'', R₁, R₁', R₁''. R₂, R₂', R₃, R₄, R₄', R₅, R₆, A₁, A₂, A₃, A₄, A₅, A₆, A₇, A₇', A₇'', A₈, A₈', A₉, A₉' A₉'', A₁₀, A₁₀', A₁₁, B, B', B₀, B₁, B₁', B₂, B₂', B₃, B₃', B₄, B₄', B₅, B₆, (a₀), (b₀), (C₀), (d₀), (e₀), Mₐ, Mₐ', Mₐ'', Mₐ''', Mₐ''', M_{b0}, Mc₀, M_{d0}, Rₐ₀, R_{b}, R_{c}, R_{d}, R_{d}', Rₑ, Rₑ', Pₑ'', Rₑ''', Bₐ, B_{b}, B_{c}, Yₐ, Yₐ', Y_{b}, Y_{b}', Y_{b}'', Y_{c} and Y_{c}', and an integer represented by k, k', 1, m, m', n and n'.

X_{A}, Y_{A}, Q_{A}, K_{A}, L_{A} and T_{A} in the compound (IV) are independently represented by a group represented by D₁, D₂, D₃, D₄, D₅, R₀, R₀', R₀'', R₁, R₁', R₁'', R₂, R₂', R₃, R₄, R₄', R₅, R₆, A₁, A₂, A₃, A₄, A₅, A₆, A₇, A₇', A₇'', A₈, A₈', A₉, A₉', A₉'', A₁₀, A₁₀', A₁₁, B, B', B₀, B₁, B₁', B₂, B₂', B₃, B₃', B₄, B₄' B₅, B₆, (a), (b), (c), (d), (e), Mₐ, Mₐ', Mₐ", Mₐ''', Mₐ'''', M_{b}, M_{c}, M_{d}, Rₐ, R_{b}, R_{c}, R_{d}, R_{d}', Rₑ, Rₑ', Rₑ'', Rₑ''', Bₐ, B_{b}, B_{c}, Yₐ, Yₐ', Y_{b}, Y_{b}', Y_{b}'', Y_{c} and Y_{c}', and an integer represented by k, k', 1, m, m', n and n'.

Xₐ, Yₐ, qₐ and tₐ in the compounds (V) and (V') are independently represented by a group represented by a₀, a₀', a₁, a₁', a₂, a₃, a₄, a₅, b, b', r, r',r₀, r₀', r₁, r₁', r₂, r₃, r₃', r₄, r₄', rₐ, r_{b}, y and z, and an integer represented by 1.

The "6- to 10-membered aryl group", in the Y₀ group of substituents which may be selected as Y_{α} of the compounds (I), (II), (I') and (II'), represents a group forming a monocyclic or fused aromatic hydrocarbon ring, and examples thereof include a phenyl group, a 1-naphthyl group, a 2-naphtyl group, a 6-indanyl group and the like. The "5- to 10-memberd heteroaryl group" represents a group forming a monocyclic or fused aromatic heterocycle, and examples thereof include a 2-furyl group, a 3-furyl group, a 2-thienyl group, a 3-thienyl group, a 2-pyridyl group, a 3-pyridyl group, a 4-pyridyl group, a 2-quinolyl group, and the like. The "3- to 10-memberd cyclic hydrocarbon or heterocyclic group optionally containing an unsaturated bond" includes a monocyclic or fused ring, and examples thereof include a 2-cyclohexenyl group, a 2-morpholinyl group, a 4-piperidyl group and the like. The "3- to 10-memberd cyclic hydrocarbon or heterocyclic group optionally containing an unsaturated bond" may be substituted with 1 or more same or different Mₐ-groups as describe above.

The "group which is fused to the A ring", in the Z₀ group of substituents which may be selected as Y_{α} of the compounds (I) and (II), may have 1 or more same or different atoms or groups selected from a halogen atom, a C1-C10 alkoxy group, a C3-C10 alkenyloxy group, a C3-C10 alkynyloxy group, a carbonyl group, a thiocarbonyl group, an oxy group, a thio group, a sulfinyl group and a sulfonyl group.

In Rₐ₀ of the Eₒ group of substituents which may be selected as X_{A0} of the compound (III), the "optionally substituted 5- to 7-membered aryl group or heteroaryl group" represents a group forming a monocyclic or fused aromatic hydrocarbon ring or a group forming a monocyclic or fused aromatic heterocycle, and examples thereof include a phenyl group, a 1-naphthyl group, a 2-naphthyl group, a 6-indanyl group, a 2-furyl group, a 3-furyl group, a 2-thienyl group, a 3-thienyl group, a 2-pyridyl group, a 3-pyridyl group, a 4-pyridyl group, a 2-quinolyl group and the like. The "optionally substituted 5- to 7-membered aryl group or heteroaryl group" may be substituted with 1 or more same or different Mₐ-groups as described above.

In (do) of the Y₀ group of substituents which may be selected as Y_{α} or Y_{A0} of the compounds (I), (II), (III), (I') and (II'), the phrase "forming a 5 to 12-membered hydrocarbon ring which is substituted with a carbonyl group or a thiocarbonyl group and further which may be optionally substituted with an oxy group, a thio group, a -NR₁- group (wherein R₁ is as defined above), a sulfinyl group or a sulfonyl group" means forming a 5- to 12-membered hydrocarbon ring in which one or more of carbon atoms are substituted with a carbonyl group or a thiocarbonyl group, and further, one or more of carbon atoms may be substituted with one or more same or different groups selected from an oxy group, a thio group, a -NR₁- group (wherein R₁ is as defined above), a sulfinyl group and a sulfonyl group.

In (e₀) of the Y₀ group of substituents which may be selected as Y_{α} or Y_{A0} of the compounds (I), (II), (III), (I') and (II'), the phrase "forming a 5 to 12-membered hydrocarbon ring optionally substituted with a carbonyl group, a thiocarbonyl group, an oxy group, a thio group, a -NR₁- group (wherein R₁ is as defined above), a sulfinyl group or a sulfonyl group" means forming a 5- to 12-membered hydrocarbon ring in which one or more of carbon atoms are optionally substituted with one or more same or different groups selected from a carbonyl group, a thiocarbonyl group, an oxy group, a thio group, a -NR₁-group (wherein R₁ is as defined above), a sulfinyl group and a sulfonyl group.

In (a) of the B group of substituents which may be selected as X_{A} of the compound (IV), the "C2-C10 alkylene group optionally substituted with an oxy group, a thio group, a sulfinyl group, a sulfonyl group or a -NR₁'- group (wherein R₁' is as defined above)" means a C2-C10 alkylene group in which one or more of carbon atoms may be substituted with one or more same or different groups selected from an oxy group, a thio group, a sulfinyl group, a sulfonyl group and a -NR₁'- group (wherein R₁' is as defined above), and the "C3-C10 alkenylene group optionally substituted with an oxy group, a thio group, a sulfinyl group, a sulfonyl group or a -NR₁'- group (wherein R₁' is as defined above)" means a C3-C10 alkenylene group in which one or more of carbon atoms may be substituted with oen or more same or different groups selected from an oxy group, a thio group, a sulfinyl group, a sulfonyl group and a -NR₁'-group (wherein R₁' is as defined above).

In (b) of the D group of substituents which may be selected as X_{A} of the compound (IV), the "C1-C10 alkylene group optionally substituted with a methyl group, an oxy group, a thio group, a sulfinyl group, a sulfonyl group or a -NR₁- group (wherein R₁ is as defined above)" means a C2-C10 alkylene group in which one or more of carbon atoms may be substituted with a methyl group, or one or more of carbon atoms may be substituted with one or more same or different groups selected from an oxy group, a thio group, a sulfinyl group, a sulfonyl group and a -NR₁- group (wherein R₁ is as defined above), and the "C2-C10 alkenylene group optionally substituted with a methyl group, an oxy group, a thio group, a sulfinyl group, a sulfonyl group or a -NR₁- group (wherein R₁ is as defined above)" means a C2-C10 alkenylene group in which one or more of carbon atoms may be substituted with a methyl group, or one or more carbon atoms may be substituted with one or more same or different groups selected from an oxy group, a thio group, a sulfinyl group, a sulfonyl group and a -NR₁- group (wherein R₁ is as defined above).

Examples of groups belonging to the X₀ group, the Y₀ group and the Z₀ group which may be selected as Y_{α} of the compounds (I), (II), (I') and (II') are shown in the following Table 23, Table 24 and Tables 25 to 26, respectively.

Examples of groups belonging to the A₀ group, the B₀ group, the C₀ group, the D₀ group, the E₀ group, the F₀ group, the G₀ group, the H₀ group, the I₀ group, the J₀ group, the K₀ group, the L₀ group, the M₀ group and the N₀ group which may be selected as X_{A0} of the compound (III) are shown in the following Table 1, Table 2, Table 3, Table 4, Tables 5 to 7, Tables 8 to 11, Tables 12 to 15, Table 16, Table 17, Table 18, Table 19, Table 20, Table 21 and Table 22, respectively. Examples of groups belonging to the X₀ group, the Y₀ group and the Z₀ group which may be selected as Y_{A0} of the compound (III) are shown in the following Table 23, Table 24 and Tables 25 to 26. Examples of groups which may be selected as Q_{A0} and T_{A0} of the compound (III) are shown in the following Table 27 to Table 28 and Table 29, respectively.

Examples of groups belonging to the A group, the B group, the C group, the D group, the E group, the F group, the G group, the H group, the I group, the J group, the K group, the L group, the M group and the N group which may be selected as X_{A} of the compound (IV) are shown in the following Table 1, Table 2, Table 3, Table 4, Tables 5 to 7, Tables 8 to 11, Tables 12 to 15, Table 16, Table 17, Table 18, Table 19, Table 20, Table 21 and Table 22, respectively. Examples of groups belonging to the X group, the Y group and the Z group which may be selected as Y_{A} of the compound (IV) are shown in the following Table 23, Table 24 and Tables 25 to 26, respectively. Examples of groups which may be selected as Q_{A} and T_{A} of the compound (IV) are shown in the following Table 27 to Table 28 and Table 29, respectively.

Examples of the aforementioned groups belonging to the A₀ group to N₀ group and the A group to N group are shown in the following Table 1 to Table 22. When said groups have geometrical isomers, all of the geometrical isomers are included, and when said groups have tautomers, all of the tautomers are included.

Examples of groups belonging to the A₀ group and the A group are shown in Table 1.

**Table 1**

| No. | Group |
|---|---|
| A-1 | -CH₂ONH₂ |
| A-2 | -CH₂ON(CH₃)₂ |
| A-3 | -CH₂ONHCOCH₃ |
| A-4 | -CH₂NHOCH₂CH=CH₂ |
| A-5 | -CH₂CN |
| A-6 | -CH₂CH₂CN |
| A-7 | -CH₂CH₂C (=NH) NH₂ |
| A-8 | -CH₂CH₂C (=NCH₂C≡CH)N(CH₃)₂ |
| A-9 | -CH₂C(=NH)NHCOCH₃ |
| A-10 | -CH₂C(=NOCOCH₃)-NH₂ |
| A-11 | -CH₂C(=NCOCH₃)-OCH₃ |
| A-12 | -CH₂CSNH₂ |
| A-13 | -CH₂NO₂ |
| A-14 | -CH₂SO₃H |
| A-15 | -SO₃H |

Examples of groups belonging to the B₀ group and the B group are shown in Table 2.

**Table 2**

| No. | Group | No. | Group |
|---|---|---|---|
| B-1 | 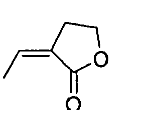 | B-4 | 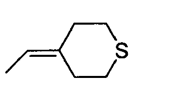 |
| B-2 | 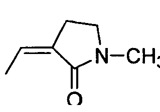 | B-5 | 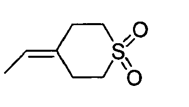 |
| B-3 | 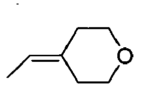 | B-6 | 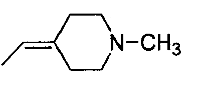 |

Examples of groups belonging to the C₀ group and the C group are shown in Table 3.

**Table 3**

| No. | Group |
|---|---|
| C-1 | -CH=CF₂ |
| C-2 | -CH=CHOCH₃ |
| C-3 | -CH=CHSCH₃ |
| C-4 | -CH=CHSOCH₃ |
| C-5 | -CH=CHSO₂CH₃ |
| C-6 | -CH=CHCH₂OH |
| C-7 | -CH=CHCH₂OCOCH₃ |
| C-8 | -CH=CHCHO |
| C-9 | -CH=CHCH=NCH₂CH=CH₂ |
| C-10 | -CH=CHCH=NOH |
| C-11 | -CH=CHCH=NOCH₂COOCH₃ |
| C-12 | -CH=CHCH=NOCH₂CN |
| C-13 | -CH=CHCH=NN(CH₃)₂ |
| C-14 | -CH=CHCH=NNHCOCH₃ |
| C-15 | -CH=CHCOCH₃ |
| C-16 | -CH=C(CH₃)COCH₃ |
| C-17 | -CH=CHCOCF₃ |
| C-18 | -CH=CHCH₂ON(CH₃)₂ |
| C-19 | -CH=CHCH₂ON(SO₂CH₃)CH₃ |
| C-20 | -CH=CHCH₂N(CH₂CH=CH₂)₂ |
| C-21 | -CH=CHCH₂N(OH)CH₃ |
| C-22 | -CH=CHNHCOCH₃ |
| C-23 | -CH=CHCN |
| C-24 | -CH=CHC(=NH)N(CH₃)₂ |
| C-25 | -CH=CHC(=NH)NHOCH3 |
| C-26 | -CH=CHCSNH₂ |
| C-27 | -CH=CHNO₂ |
| C-28 | -CH=CHSO₃H |

Examples of groups belonging to the D₀ group and the D group are shown in Table 4.

**Table 4**

| No. | Group |
|---|---|
| D-1 | 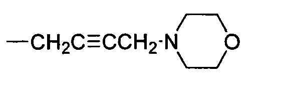 |
| D-2 | 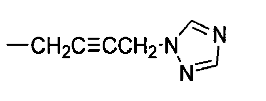 |
| D-3 | -C≡CI |
| D-4 | -C≡CCH₂SCH₃ |
| D-5 | -C≡CC(CH₃)₂OH |
| D-6 | -C≡CCH₂OCOOCH₃ |
| D-7 | -C≡CCH=NCH₃ |
| D-8 | -C≡CCH=NOCH₃ |
| D-9 | -C≡CCH=NN(CH₃)₂ |
| D-10 | -C≡CCH₂ON(CH₃)₂ |
| D-11 | -C≡CCH₂N(CH₃)₂ |
| D-12 | -C≡CCH₂CH₂NO₂ |

Examples of groups belonging to the E₀ group and the E group are shown in Table 5 to Table 7.

**Table 5**

| No. | Group |
|---|---|
| E-1 | -CH=CHCOOCH₃ |
| E-2 | -CH=CHCOOC₂H₅ |
| E-3 | -CH=CHCOOCH₂CH₂Cl |
| E-4 | -CH=CHCOOCH₂CF₃ |
| E-5 | -CH=CHCOOCH₂CH=CH₂ |
| E-6 | -CH=CHCOOCH₂C=CH |
| E-7 | 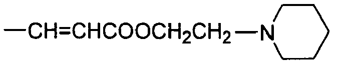 |
| E-8 | 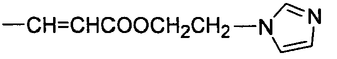 |
| E-9 | -CH=CHCOOCH₂CH₂OCH₃ |
| E-10 | -CH=CHCOOCH₂CH₂SCH₃ |
| E-11 | -CH=CHCOOCH₂CH₂SOCH₃ |
| E-12 | -CH=CHCOOCH₂CH₂SO₂CH₃ |
| E-13 | -CH=CHCOOCH₂CH₂OH |
| E-14 | -CH=CHCOOCH₂CH₂OSO₂N (CH₃)₂ |
| E-15 | -CH=CHCOOCH₂CH₂COCH₃ |
| E-17 | -CH=CHCOOCH₂CH₂ON(CH₃)₂ |
| E-18 | -CH=CHCOOCH₂CH₂N(CH₃) ₂ |
| E-19 | -CH=CHCOOCH₂CH₂N (OC₂H₅)C₂H₅ |
| E-20 | -CH=CHCOOCH₂CH₂NHCOCH₃ |
| E-21 | -CH=CHCOOCH₂CH₂N (CH₃) COCH₃ |
| E-22 | -CH=CHCOOCH₂CH₂NHCOOCH₂CH₂OCH₃ |
| E-23 | -CH=CHCOOCH₂CH₂NHCOSCH₂CH=CH₂ |
| E-24 | -CH=CHCOOCH₂CH₂NHCONHC₂H₅ |
| E-25 | -CH=CHCOOCH₂CH₂NHCON (CH₃) ₂ |
| E-26 | -CH=CHCOOCH₂CH₂NHCON(OCH₃)CH₃ |
| E-27 | -CH=CHCOOCH₂CH₂NHCSNHCH₂CH₂Cl |
| E-28 | -CH=CHCOOCH₂CH₂NHSO₂N(CH₃)₂ |
| E-29 | -CH=CHCOOCH₂CH₂CN |
| E-30 | -CH=CHCOOCH₂CH₂NO₂ |

**Table 6**

| No. | Group |
|---|---|
| E-31 | -CH=CHCOOCH₂CH₂SO₃H |
| E-32 | 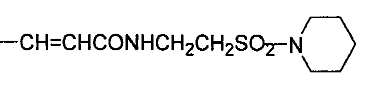 |
| E-33 | -CH=CHCONHCH₂CH₂SO₂N (CH₃) ₂ |
| E-34 | -CH=CHCOSCH₃ |
| E-35 | -CH=CHCON(CH₃)CH₂C≡CH |
| E-36 | -CH=CHCON(OCH₃)CH₃ |
| E-37 | -CH=CHCONHOCH₃ |
| E-38 | -CH=CHCONHOCH₂CH=CH₂ |
| E-39 | -CH=CHCOOCH₂COOCH₃ |
| E-40 | -CH=CHCOSCH₂COOCH₃ |
| E-41 | -CH=CHCONHCH₂COOCH₃ |
| E-42 | -CH=CHCONHCH₂CON (CH₃) ₂ |
| E-43 | -CH=CHCONHCH₂CN |
| E-44 | -CH=CHCONHCH₂C (=NH)N(CH₃) CH₂CH=CH₂ |
| E-45 | -CH=CHCONHCH₂C(=NH)NHOH |
| E-46 | -CH=CHCONHS0₂CH₃ |
| E-47 | 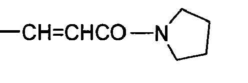 |
| E-48 | 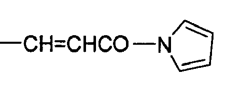 |
| E-49 | 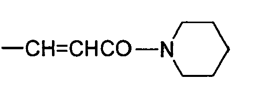 |
| E-50 | 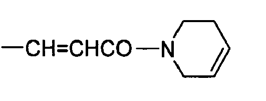 |

**Table 7**

| No. | Group |
|---|---|
| E-51 | 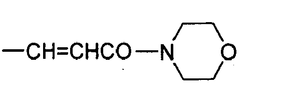 |
| E-52 | 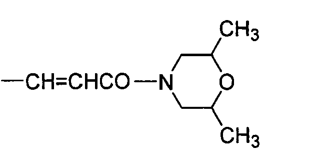 |
| E-53 | 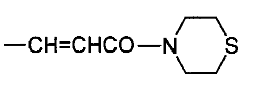 |
| E-54 | 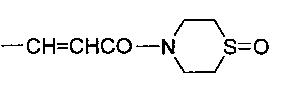 |
| E-55 | 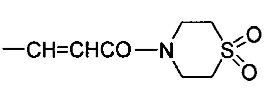 |
| E-56 | 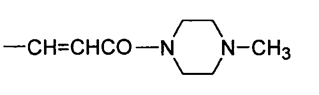 |
| E-57 | 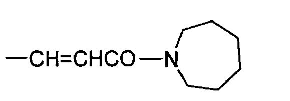 |
| E-58 | 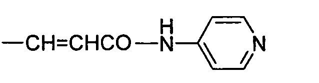 |
| E-59 | -CH=CHCONHN(CH₃)₂ |
| E-60 | -CH=CHCONHNHCOOC₂H₅ |
| E-61 | -CH=CHCONHNHCSNH(c)C₆H₁₁ |
| E-62 | -CH=CFCOOCH₃ |

Examples of groups belonging to the F₀ group and the F group are shown in Table 8 to Table11.

**Table 8**

| No. | Group |
|---|---|
| F-1 | -OCH₂CH₂OH |
| F-2 | -OCH₂CH₂CH₂OH |
| F-3 | -CH₂OCH₂CH₂OH |
| F-4 | -OCH₂CH₂OCON (CH₃) ₂ |
| F-5 | -CH₂OCH₂CH₂ONH₂ |
| F-6 | -OCH₂CH₂N(CH₃)₂ |
| F-7 | -OCH₂CH₂CH₂N(CH₃)₂ |
| F-8 | -OCH₂CH₂N (OCH₃) CH₃ |
| F-9 | -CH₂OCH₂CH₂NH₂ |
| F-10 | -CH₂OCH₂CH2NHCOCH₃ |
| F-11 | -CH₂OCH₂CH₂N(CH₃)COCH₃ |
| F-12 | -CH₂OCH₂CH₂NHCOO(t)C₄H₉ |
| F-13 | -CH₂OCH₂CH₂NHCOSCH₂CH=CH₂ |
| F-14 | -CH₂OCH₂CH₂NHCONHC₂H₅ |
| F-15 | -CH₂OCH₂CH₂NHCON (CH₃) ₂ |
| F-16 | -CH₂OCH₂CH₂NHCON (OCH₃) CH₃ |
| F-17 | -CH₂OCH₂CH₂NHCSNHCH₂CH₂Cl |
| F-18 | -CH₂OCH₂CH₂NO₂ |
| F-19 | -CH₂OCH₂CH₂SO₃H |
| F-20 | -CH₂OCH₂CH₂CH₂SO₃H |
| F-21 | -CH₂OCH₂CH₂CH₂CH₂SO₃H |
| F-22 | -CH₂OCH₂CH₂NHSO₂N (CH₃) 2 |
| F-23 | 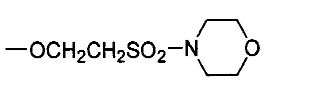 |
| F-24 | -OCH₂CH₂OCH₃ |
| F-25 | -OCH₂CH₂SCH₃ |

**Table 9**

| No. | Group |
|---|---|
| F-26 | -OCH₂CH₂SOCH₃ |
| F-27 | -OCH₂CH₂SO₂CH₃ |
| F-28 | -CH₂OCH₂CN |
| F-29 | -CH₂OCH₂C (=NH) NH₂ |
| F-30 | -CH₂OCH₂CSNH₂ |
| F-31 | -CH₂OCH₂COCH₃ |
| F-32 | -CH₂OCH₂COCF₃ |
| F-33 | -CH₂OCH₂CHO |
| F-34 | -CH₂OCH₂CH=NOCH₂C≡CH |
| F-35 | -CH₂OCH₂CH=NN(CH₃)₂ |
| F-36 | -OCH₂COOH |
| F-37 | -OCH₂COOCH₃ |
| F-38 | -CH₂OCH₂COOCH₂CH₂Cl |
| F-39 | -CH₂OCH₂COOCH₂CH=CH₂ |
| F-40 | -CH₂OCH₂COOCH₂C=CH |
| F-41 | 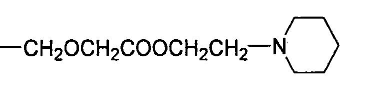 |
| F-42 | 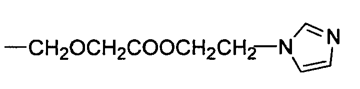 |
| F-43 | -CH₂OCH₂COOCH₂CH₂OCH₃ |
| F-44 | -CH₂OCH₂COOCH₂CH₂SCH₃ |
| F-45 | -CH₂OCH₂COOCH₂CH₂SOCH₃ |
| F-46 | -CH₂OCH₂COOCH₂CH₂SO₂CH₃ |
| F-47 | -CH₂OCH₂COOCH₂CH₂OH |
| F-48 | -CH₂OCH₂COO(CH₂)₉OH |
| F-49 | -CH₂OCH₂COOCH₂CH₂OSO₂N (CH₃) ₂ |
| F-50 | -CH₂OCH₂COOCH₂CH₂COCH₃ |

**Table 10**

| No. | Group |
|---|---|
| F-51 | -CH₂OCH₂COOCH₂CH₂ON (CH₃)₂ |
| F-52 | -CH₂OCH₂COOCH₂CH₂N (CH₃)₂ |
| F-53 | -CH₂OCH₂COOCH₂CH₂N (OC₂H₅) C₂H₅ |
| F-54 | -CH₂OCH₂COOCH₂CH₂NHCOCH₃ |
| F-55 | -CH₂OCH₂COOCH₂CH₂N (CH₃) COCH₃ |
| F-56 | -CH₂OCH₂COOCH₂CH₂NHCOOCH₂CH₂OCH₃ |
| F-57 | -CH₂OCH₂COOCH₂CH₂NHCOSCH₂CH=CH₂ |
| F-58 | -CH₂OCH₂COOCH₂CH₂NHCONHC₂H₅ |
| F-59 | -CH₂OCH₂COOCH₂CH₂NHCON (CH₃) ₂ |
| F-60 | -CH₂OCH₂COOCH₂CH₂NHCON (OCH₃) CH₃ |
| F-61 | -CH₂OCH₂COOCH₂CH₂NHCSNHCH₂CH₂Cl |
| F-62 | -CH₂OCH₂COOCH₂CH₂NHSO₂N (CH₃)₂ |
| F-63 | -CH₂OCH₂COOCH₂CH₂CN |
| F-64 | -CH₂OCH₂COOCH₂CH₂NO₂ |
| F-65 | -CH₂OCH₂COOCH₂CH₂SO₃H |
| F-66 | 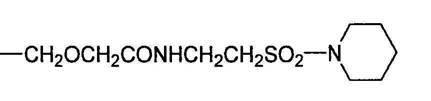 |
| F-67 | -CH₂OCH₂CONHCH₂CH₂SO₂N(CH₃)₂ |
| F-68 | -CH₂OCH₂COSCH₃ |
| F-69 | -CH₂OCH₂CONH₂ |
| F-70 | -CH₂OCH₂CONHCH₃ |
| F-71 | -CH₂OCH₂CON(CH₃)₂ |
| F-72 | -OCH₂CON(CH₃)CH₂C≡CH |
| F-73 | -OCH₂CON(OCH₃)CH₃ |
| F-74 | -OCH₂CONHOCH₃ |
| F-75 | -OCH₂CONHOCH₂CH=CH₂ |

**Table 11**

| No. | Group |
|---|---|
| F-76 | -CH₂OCH₂COOCH₂COOCH₃ |
| F-77 | -CH₂OCH₂COSCH₂COOCH₃ |
| F-78 | -CH₂OCH₂CONHCH₂COOCH₃ |
| F-79 | -CH₂OCH₂CONHCH₂CON(CH₃)₂ |
| F-80 | -CH₂OCH₂CONHCH₂CN |
| F-81 | -CH₂OCH₂CONHCH₂C (=NH) NH₂ |
| F-82 | -CH₂OCH₂CONHSO₂CH₃ |
| F-83 | 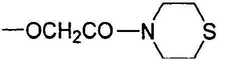 |
| F-84 | -OCH₂CONHN(CH₃)₂ |
| F-85 | -OCH₂CONHNHCOOC₂H₅ |
| F-86 | -OCH₂CONHNHCSNH(c) C₆H₁₁ |
| F-87 | -CH₂SCH₂CN |
| F-88 | -CH₂SCH₂COOCH₃ |
| F-89 | -CH₂SOCH₂COOCH₃ |
| F-90 | -CH₂SO₂CH₂COOCH₃ |
| F-91 | -NHCH₂COOCH₃ |
| F-92 | -NHCH₂CH₂N(CH₃)₂ |
| F-93 | -N (COCH₃) CH₂CH₂OH |
| F-94 | -CH₂OCH₂COOCH₃ |
| F-94 | -CH₂OCH₂COOH |
| F-95 | -CH₂SCH₂COOH |
| F-96 | -CH₂SOCH₂COOH |
| F-97 | -CH₂SO₂CH₂COOH |
| F-98 | -SCH₂COOCH₃ |
| F-99 | -SOCH₂COOCH₃ |
| F-100 | -SO_{z}CH₂COOCH₃ |
| F-101 | -SCH₂COOH |
| F-102 | -SOCH₂COOH |
| F-103 | -SO₂CH₂COOH |

Examples of groups belonging to the Go group and the G group are shown in Table 12 to Table 15.

**Table 12**

| No. | Group | No. | Group |
|---|---|---|---|
| G-1 | 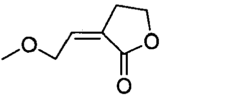 | G-4 | 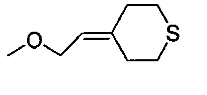 |
| G-2 | 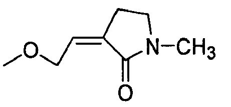 | G-5 | 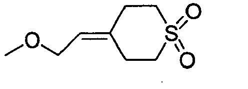 |
| G-3 | 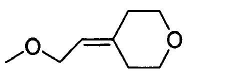 | G-6 | 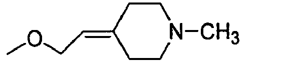 |

| No. | Group | | |
|---|---|---|---|
| G-7 | -OCH₂CH=CH₂ | | |
| G-8 | -OCH₂C≡CH | | |
| G-9 | -CH₂OCH₂CH=CHCl | | |
| G-10 | -CH₂SCH=CHOCH₃ | | |
| G-11 | -CH₂SO₂CH=CHOCH₃ | | |
| G-12 | -CH₂OCH=CHCOCH₃ | | |
| G-13 | -CH₂OCH=CHCHO | | |
| G-14 | -CH₂OCH=CHCH=NCH₂CH=CH₂ | | |
| G-15 | -CH₂OCH=CHCH=NOCH₃ | | |
| G-16 | -CH₂OCH=CHCH=NN (CH₃)₂ | | |
| G-17 | -CH₂OCH=CHCN | | |
| G-18 | -CH₂OCH=CHC(=NH)NH₂ | | |
| G-19 | -CH₂OCH=CHCOOH | | |
| G-20 | -CH₂OCH₂C=CCOOH | | |
| G-21 | -CH₂OCH=CHCOOCH₃ | | |
| G-22 | -CH₂OCH=CHCOOCH₂CH₂Cl | | |
| G-23 | -CH₂OCH=CHCOOCH₂CH=CH₂ | | |
| G-24 | -CH₂OCH=CHCOOCH₂C=CH | | |
| G-25 | 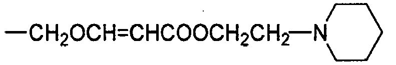 | | |

**Table 13**

| No. | Group |
|---|---|
| G-26 | 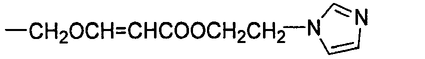 |
| G-27 | -CH₂OCH=CHCOOCH₂CH₂OCH₃ |
| G-28 | -CH₂OCH=CHCOOCH₂CH₂SCH₃ |
| G-29 | -CH₂OCH=CHCOOCH₂CH₂SOCH₃ |
| G-30 | -CH₂OCH=CHCOOCH₂CH₂SO₂CH₃ |
| G-31 | -CH₂OCH=CHCOOCH₂CH₂OH |
| G-32 | -CH₂OCH=CHCOOCH₂CH₂OSO₂N (CH₃)₂ |
| G-33 | -CH₂OCH=CHCOOCH₂CH₂COCH₃ |
| G-34 | -CH₂OCH=CHCOOCH₂CH₂ON (CH₃)₂ |
| G-35 | -CH₂OCH=CHCOOCH₂CH₂N (CH₃)₂ |
| G-36 | -CH₂OCH=CHCOOCH₂CH₂N (OC₂H₅) C₂H₅ |
| G-37 | -CH₂OCH=CHCOOCH₂CH₂NHCOCH₃ |
| G-38 | -CH₂OCH=CHCOOCH₂CH₂N (CH₃) COCH₃ |
| G-39 | -CH₂OCH=CHCOOCH₂CH₂NHCOOCH₂CH₂OCH₃ |
| G-40 | -CH₂OCH=CHCOOCH₂CH₂NHCOSCH₂CH=CH₂ |
| G-41 | -CH₂OCH=CHCOOCH₂CH₂NHCONHC₂H₅ |
| G-42 | -CH₂OCH=CHCOOCH₂CH₂NHCON(CH₃)₂ |
| G-43 | -CH₂OCH=CHCOOCH₂CH₂NHCON (OCH₃) CH₃ |
| G-44 | -CH₂OCH=CHCOOCH₂CH₂NHCSNHCH₂CH₂Cl |
| G-45 | -CH₂OCH=CHCOOCH₂CH₂NHSO₂N(CH₃)₂ |
| G-46 | -CH₂OCH=CHCOOCH₂CH₂C(=NH)NH₂ |
| G-47 | -CH₂OCH=CHCOOCH₂CH₂NO₂ |
| G-48 | -CH₂OCH=CHCOOCH₂CH₂SO₃H |
| G-49 | 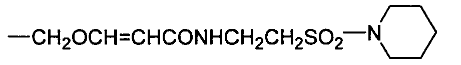 |
| G-50 | -CH₂OCH=CHCONHCH₂CH₂SO₂N (CH₃)₂ |

**Table 14**

| No. | Group |
|---|---|
| G-51 | -CH₂OCH=CHCOSCH₃ |
| G-52 | -CH₂OCH=CHCON (CH₃)CH₂C≡CH |
| G-53 | -CH₂OCH=CHCON(OCH₃)CH₃ |
| G-54 | -CH₂OCH=CHCONHOCH₃ |
| G-55 | -CH₂OCH=CHCONHOCH₂CH=CH₂ |
| G-56 | -CH₂OCH=CHCONHCH₂COOCH₃ |
| G-57 | -CH₂OCH=CHCONHCH₂CON(CH₃)₂ |
| G-58 | -CH₂OCH=CHCONHSO₂CH₃ |
| G-59 | 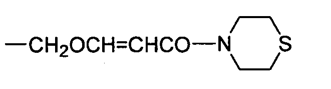 |
| G-60 | -CH₂OCH=CHCONHN (CH₃)₂ |
| G-61 | -CH₂OCH=CHCONHNHCOOC₂H₅ |
| G-62 | -CH₂OCH=CHCONHNHCSNH(c)C₆H₁₁ |
| G-63 | 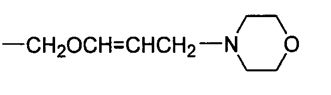 |
| G-64 | 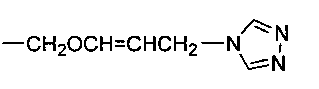 |
| G-65 | -CH₂OCH=CHCH₂OCH₃ |
| G-66 | -CH₂OCH=CHCH₂SCH₃ |
| G-67 | -CH₂OCH=CHCH₂SOCH₃ |
| G-68 | -CH₂OCH=CHCH₂SO₂CH₃ |
| G-69 | -CH₂OCH=CHCH₂OH |
| G-70 | -CH₂OCH=CHCH₂OCOCH₃ |

**Table 15**

| No. | Group |
|---|---|
| G-71 | -OCH₂C=CCH₂OH |
| G-72 | -CH₂OCH=CHCH₂ON(CH₃)₂ |
| G-73 | -CH₂OCH=CHCH₂N(CH₃)₂ |
| G-7 4 | -CH₂OCH=CHCH₂N(CH₂CH=CH₂)₂ |
| G-75 | -CH₂OCH=CHCH₂N(OH)CH₃ |
| G-76 | -CH₂OCH=CHCH₂NO₂ |
| G-77 | -CH₂OCH=CHCH₂SO₃H |
| G-78 | -SCH₂CH=CH₂ |
| G-79 | -SOCH₂CH=CH₂ |
| G-80 | -SO₂CH₂CH=CH₂ |
| G-81 | -CH₂SCH=CHCOOH |
| G-82 | -CH₂NHCH=CHCOOH |
| G-83 | -CH₂OCH₂CH=CH₂ |
| G-84 | -CH₂OCH₂C≡CH |

Examples of groups belonging to the H₀ group and the H group are shown in Table 16.

**Table 16**

| No. | Group |
|---|---|
| H-1 | -CH₂NHCN |
| H-2 | -N(COCH₃)CN |
| H-3 | -NHC(=NH)NHOH |
| H-4 | -NHC(=NH)N(CH₂CH=CH₂)CH₃ |
| H-5 | -C(=NH)NHCH₂CH=CH₂ |
| H-6 | -N=CHN(CH₃)₂ |
| H-7 | -N(CH₃)C(CH₃)=NOCH₂C≡CH |
| H-8 | -NHCONHCOCH₃ |
| H-9 | -NHCONHSO₂CH₃ |
| H-10 | -NHCOCN |
| H-11 | -NHCOCOOCH₃ |
| H-12 | -NHCOCOOH |

Examples of groups belonging to the I₀ group and the I group are shown in Table 17.

**Table 17**

| No. | Group |
|---|---|
| I-1 | -NHCOCH=CH₂ |
| 1-2 | -NHCSCH=CH₂ |
| 1-3 | -NHCOCF=CH₂ |
| 1-4 | -NHCOC=CH |
| 1-5 | -NHCOCH₂OCH₃ |
| I-6 | -NHCOCH₂SCH₃ |
| 1-7 | -NHCOCH₂COCH₃ |
| I-8 | -NHCOCH₂OH |
| 1-9 | -NHCOCH₂ONH₂ |
| I-10 | -NHCOCH₂N (CH₃) CH₂C=CH |
| I-11 | -NHCOCH₂NHCOCH₃ |
| 1-12 | -NHCOCH₂COOCH₃ |
| I-13 | -NHCOCH₂CN |
| I-14 | -NHCOCH₂NO₂ |
| I-15 | -NHCOCH₂SO₃H |
| I-16 | -NHCOCH₂SO₂N(CH₃)₂ |
| I-17 | -NHCSCH₃ |
| I-18 | -NHCSCH₂N(CH₃)₂ |
| I-19 | -NHCOOCH₂CH₂OCH₃ |
| I-20 | -NHCOOCH₂CN |
| I-21 | -NHCOOCH₂CH₂NO₂ |
| I-22 | -NHCOOCH₂CH₂NHCOCH₃ |
| I-23 | -NH(CS)OCH₃ |
| I-24 | -NH(CO)SCH₃ |
| I-24 | -NHCONHCH₂CH₂OCH₃ |
| I-25 | -NHCSNHCH₃ |
| I-26 | -NHSO₂CH=CH₂ |
| I-27 | -NHSO₂CH₂CH=CH₂ |
| I-28 | -NHSO₂CH₂C≡CH |
| I-29 | -NHSO₂CH₂COCH₃ |
| I-30 | -NHSO₂CH₂CN |
| I-31 | -NHSO₂CH₂NO₂ |
| I-32 | -NHSO₂CH₂COOH |
| I-33 | -NHSO₂CH₂COOCH₃ |

Examples of groups belonging to the J₀ group and the J group are shown in Table 18.

**Table 18**

| No. | Group |
|---|---|
| J-1 | -COCH=CH₂ |
| J-2 | -COC=CH |
| J-3 | -COC=CCF₃ |
| J-4 | -COCH₂SCH₃ |
| J-5 | -COCH₂OH |
| J-6 | -COCH₂N(CH₃)₂ |
| J-7 | -CSCH₃ |
| J-8 | -CSCF₃ |
| J-9 | -CH=NCH₃ |
| J-10 | -CH=NOCH₃ |
| J-11 | -COCN |
| J-12 | -COC(=NH)NH₂ |
| J-13 | -COCOOCH₃ |
| J-14 | -CH₂OCON(CH₃)₂ |

Examples of groups belonging to the K₀ group and the K group are shown in Table 19.

**Table 19**

| No. | Group |
|---|---|
| K-1 | -CONHSO₂CH₃ |
| K-2 | -CONHOH |
| K-3 | -CONHOCH₃ |
| K-4 | -CONHOCH₂CH=CH₂ |
| K-5 | -CONHCH₂CH₂OH |
| K-6 | -CONHCH₂CH₂OCH₃ |
| K-7 | -CONHCH₂OCH₃ |
| K-8 | -CONHCH₂CH=CH₂ |
| K-9 | -CONHCH₂C=CH |
| K-10 | -CONHCH₂CN |
| K-11 | -CONHCH₂COOH |
| K-12 | -CONHCH₂COOCH₃ |
| K-13 | -CONHCH₂CONH₂ |
| K-14 | -CONHCH₂CONHCH₃ |
| K-15 | -CONHCH₂CONH(CH₃)₂ |
| K-16 | -CONHCH(CH₂COOH)COOH |
| K-17 | -CONHCH (CH₂COOCH₃) COOCH₃ |
| K-18 | -CONHCH₂CH₂N(CH₃)₂ |
| K-19 | -CON(CH₃)CH₂CH₂OH |
| K-20 | -CON(CH₃)CH₂CH₂OCH₃ |

Examples of groups belonging to the L₀ group and the L group are shown in Table 20.

**Table 20**

| No. | Group |
|---|---|
| L-1 | -SO₂NHOH |
| L-2 | -SO₂NHOCH₃ |
| L-3 | -SO₂NHOCH₂CH=CH₂ |
| L-4 | -SO₂NHCH₂CH₂OCH₃ |
| L-5 | -SO₂NHCH₂CH=CH₂ |
| L-6 | -SO₂NHCH₂C=CH |
| L-7 | -SO₂NHCH₂CN |
| L-8 | -SO₂NHCOCH₃ |
| L-9 | -SO₂NHCH₂COOH |
| L-10 | -SO₂NHCH₂COOCH₃ |
| L-11 | -SO₂NHCH₂CONH₂ |
| L-12 | -SO₂NHCH₂CONHCH₃ |
| L-13 | -SO₂NHCH₂CON (CH₃)₂ |
| L-14 | -SO₂NHCH(CH₂COOH)COOH |
| L-15 | -NHSO₂N(CH₃)₂ |

Examples of groups belonging to the M₀ group and the M group are shown in Table 21.

**Table 21**

| No. | Group |
|---|---|
| M-1 | -N=C(-SCH₃)CH₃ |
| M-2 | -N=C(-OCH₃)OCH₃ |
| M-3 | -N=C (-SCH₃) OCH₃ |
| M-4 | -N=C(-SCH₃)SCH₃ |
| M-5 | -N=C (-SCH₃) NHCH₃ |
| M-6 | -N(CH₃)C(-SCH₃)=NCH₃ |
| M-7 | -N(CH₃)OCH₂CH=CH₂ |
| M-8 | -N (CH₂CH=CH₂) OCH₂CH=CH₂ |

Examples of groups belonging to the N₀ group and the N group are shown in Table 22.

**Table 22**

| No. | Group |
|---|---|
| N-1 | -CH₂P(=O)(OH)₂ |
| N-2 | -CH₂P(=O)(OCH₃)₂ |
| N-3 | -CH₂P(=O)(OCH₃)-CH₃ |
| N-4 | -CH₂P(=O)(OCH₃)-CH(OH)CH₃ |
| N-5 | -CH₂P(=O)(OCH₃)-CH₂CH₂OH |
| N-6 | -CH₂P(=O)(OCH₃)-CH₂COOCH₃ |

Examples of the aforementioned groups belonging to the X₀ group to the Z₀ group and the X group to the Z group are shown in the following Table 23 to Table 25. When said groups have geometrical isomers, all of the geometrical isomers are included, and when said groups have tautomers, all of the tautomers are included.

Examples of groups belonging to the X₀ group and the X group are shown in Table 23.

**Table 23**

| No. | Group | No. | Group |
|---|---|---|---|
| X-1 | -CH₃ | X-18 | -OCF₂CHF₂ |
| X-2 | -C₂H₅ | X-19 | -SCF₃ |
| X-3 | -CF₃ | X-20 | -CH₂OCH₃ |
| X-4 | -CH=CHCH₃ | X-21 | -COCH₃ |
| X-5 | -CH₂CH=CH₂ | X-22 | -OCOCH₃ |
| X-6 | -C≡CH | X-23 | -COOH |
| X-7 | -F | X-24 | -COOCH₃ |
| X-8 | -Cl | X-25 | -CH=CHCOOH |
| X-9 | -Br | X-26 | -N (CH₃) ₂ |
| X-10 | -NO₂ | X-27 | -NHCOCH₃ |
| X-11 | -CN | X-28 | -NHCOOCH₃ |
| X-12 | -OCH₃ | X-29 | -CONH₂ |
| X-13 | -SCH₃ | X-30 | -CON(CH₃)₂ |
| X-14 | -SOC₄H₉ | X-31 | -NHCON(CH₃)₂ |
| X-15 | -SO₂C₄H₉ | X-32 | -NHC(=NH)NH₂ |
| X-16 | -OCHF₂ | X-33 | -NHSO₂CF₃ |
| X-17 | -OCF₃ | X-34 | -SO₂N(CH₃)₂ |

Examples of groups belonging to the Y₀ group and the Y group are shown in Table 24.

**Table 24**

| No. | Group | No. | Group |
|---|---|---|---|
| Y-1 | 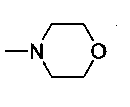 | Y-6 | 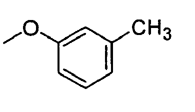 |
| Y-2 | 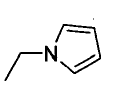 | Y-7 | 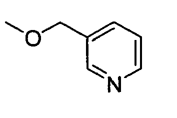 |
| Y-3 | 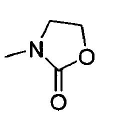 | Y-8 | 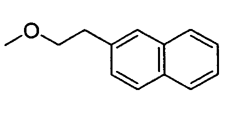 |
| Y-4 | 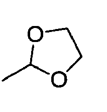 | Y-9 | 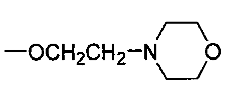 |
| Y-5 | 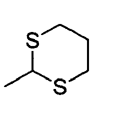 | Y-10 | 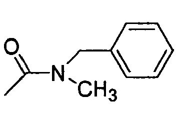 |

Examples of the α ring, the A0 ring or the A ring fused to the Z₀ group or the Z group are shown in Table 25 to Table 26.

**Table 25**

| No. | Z₀ group or Z group | α ring, A0 ring or A ring fused to Z₀ group or Z group |
|---|---|---|
| Z-1 | -CH=CH-CH=CH- | 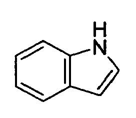 |
| Z-2 | -CH=CH-CH=CH- | 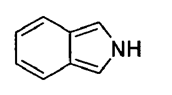 |
| Z-3 | -CH=CH-CH=CH- | 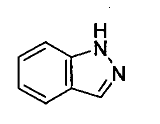 |
| Z-4 | -CH=CH-CH=CH- | 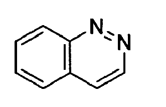 |
| Z-5 | -CH=CH-CH=CH- | 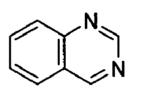 |
| Z-6 | -CH=CH-CH=CH- | 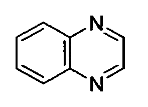 |
| Z-7 | -CH=CH-CH=CH- | 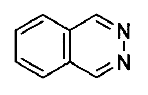 |
| Z-8 | -CH=N-CH=N- | 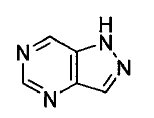 |

**Table 26**

| No. | Z₀ group or Z group | α ring, A0 ring or A ring fused to Z₀ group or Z group |
|---|---|---|
| Z-9 | -N=CH-CH=N- | 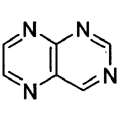 |
| Z-10 | -CH=CH-S- | 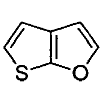 |
| Z-11 | -N=CH-NH- | 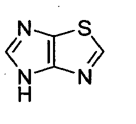 |
| Z-12 | -CH=N-N=CH- | 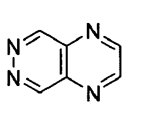 |
| Z-13 | -N=CH-CH=CH- | 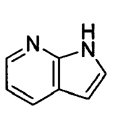 |
| Z-14 | -CH=CH-CH=N- | 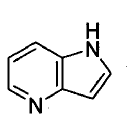 |
| Z-15 | -CH=CH-S- | 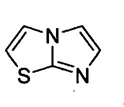 |
| Z-16 | -CH₂-CH₂-CH₂-CH₂- | 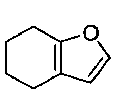 |
| Z-17 | -CH₂-CH₂-CH₂-CH₂- | 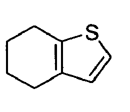 |

Examples of Q_{A0} and Q_{A} are shown in Table 27 to Table 28.

**Table 27**

| No. | Group |
|---|---|
| Q-1 | -OH |
| Q-2 | 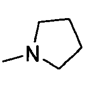 |
| Q-3 | 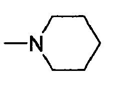 |
| Q-4 | 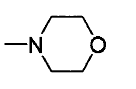 |
| Q-5 | -OCOCH₃ |
| Q-6 | -OSO₂N(CH₃)₂ |
| Q-7 | -NHCH₂CH=CH₂ |
| Q-8 | -NHCH₂C=CH |
| Q-9 | -NHCH₂CH₂OCH₃ |
| Q-10 | -OCH₃ |
| Q-11 | -OCH₂CH₂(c)C₆H₁₁ |
| Q-12 | -OCH₂CH=CH₂ |
| Q-13 | -OCH₂C≡CH |
| Q-14 | -OCH₂COOH |
| Q-15 | -OCH₂COOCH₃ |

**Table 28**

| No. | Group |
|---|---|
| Q-16 | -OCH₂CONH₂ |
| Q-17 | -OCH₂CN |
| Q-18 | -OCH₂CH₂OH |
| Q-19 | -OCH₂CH₂OCH₃ |
| Q-20 | -OCH₂CH₂N(CH₃)₂ |
| Q-21 | -OCH₂COCH₃ |
| Q-22 | -OCOC₆H₅ |
| Q-23 | -OCH₂C₆H₅ |
| Q-24 | 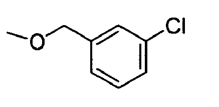 |
| Q-25 | 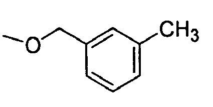 |
| Q-26 | 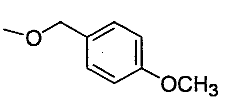 |

Examples of T_{A0} and T_{A} are shown in Table 29.

**Table 29**

| No. | Group |
|---|---|
| T-1 | -H |
| T-2 | -CH₃ |
| T-3 | -CH₂CH₂(c)C₆H₁₁ |
| T-4 | -CH₂CH=CH₂ |
| T-5 | -CH₂C≡CH |
| T-6 | -CH₂C₆H₅ |
| T-7 | -CH₂COOH |
| T-8 | -CH₂COOCH₃ |
| T-9 | -CH₂CONH₂ |
| T-10 | -CH₂CN |
| T-11 | -CH₂CH₂OH |
| T-12 | -CH₂CH₂OCH₃ |
| T-13 | -CH₂CH₂N(CH₃)₂ |
| T-14 | -CH₂COCH₃ |
| T-15 | -CH₂CF₃ |
| T-16 | -Ph |
| T-17 | 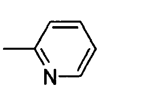 |

In some examples of the compound (II), β is a group represented by the formula (II-1), the formula (II-7) or the formula (II-8). A specific example of such compound (II) includes a cinnamoyl compound represented by the formula (II"): 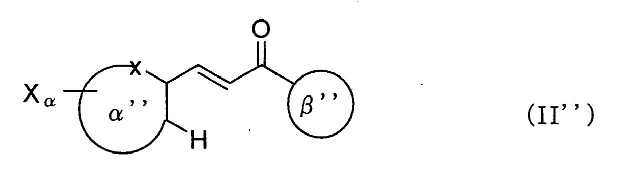 wherein α" represents an aromatic 5-membered ring, X_{α} is as defined above, x represents a methine group, an oxygen atom, a sulfur atom, a -N= group, or an imino group optionally substituted with one methyl group, and β" is a group represented by formula (II" -1): 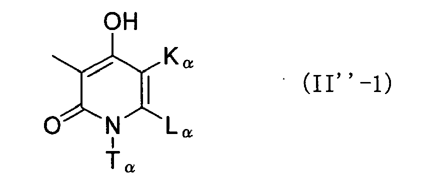 (wherein T_{α}, K_{α} and L_{α} are as defined above), a group represented by formula (II'' -7): 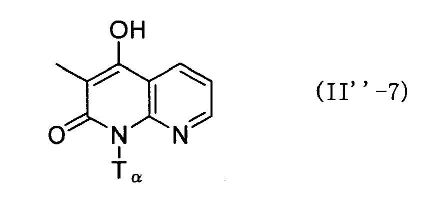 (wherein T_{α} is as defined above), or a group represented by formula (II"-8): 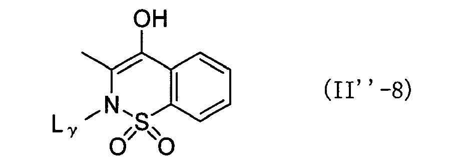 (wherein L_{γ} is as defined above). When x is a methine group in the cinnamoyl compound (II"), x dose not have a substituent.

In a further specific example of the cinnamoyl compound (II"), α" is a thiophene ring.

In a more specific example of the cinnamoyl compound (II"), α" is a thiophene ring, β" is a group (II"-1), T_{α} is a methyl group, K_{α} is a hydrogen atom and L_{α} is a methyl group.

In another specific example of the cinnamoyl compound (II"), α" is a thiophene ring, β" is a group (II"-7) and T_{α} is a methyl group.

In a still more specific example of the cinnamoyl compound (II''), α" is a thiophene ring, β" is a group (II"-8) and L_{γ} is a methyl group.

In some examples of the compound (III), B0 is a group represented by the formula (III-1), the formula (III-7) or the formula (III-8). A specific example of such compound (III) includes a cinnamoyl compound represented by the formula (III'): 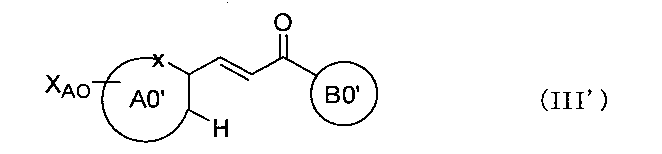 wherein A0' represents an aromatic 5-membered ring, X_{A0} and x are as defined above, B0' is a group represented by the formula (III'-1): 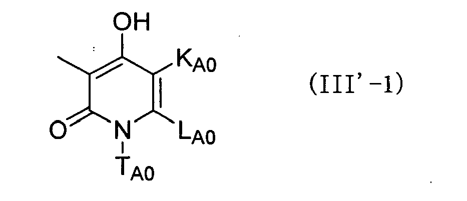 (wherein T_{A0}, K_{A0} and L_{A0} are as define above), a group represented by formula (III'-7): 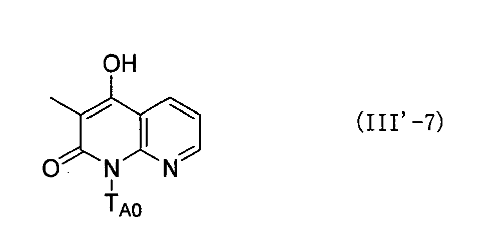 (wherein T_{A0} is as defined above), or a group represented by formula (III'-8): 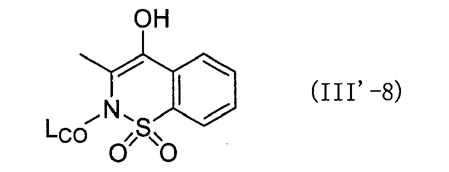 (wherein L_{C0} is as defined above). When x is a methine group in the cinnamoyl compound (III'), x dose not have a substituent.

In a further specific example of the cinnamoyl compound (III'), A0' is a thiophene ring.

In a more specific example of the cinnamoyl compound (III'), A0' is a thiophene ring, B0' is a group (III'-1), T_{A0} is a methyl group, K_{A0} is a hydrogen atom and L_{A0} is a methyl group.

In another specific example of the cinnamoyl compound (III'), A0' is a thiophene ring, B0' is a group (III'-7) and T_{A0} is a methyl group.

In a still more specific example of the cinnamoyl compound (III'), α" is a thiophene ring, β" is a group (III'-8) and L_{C0} is a methyl group.

In some examples of the compound (IV), B is a group represented by the formula (IV-1), the formula (IV-7) or the formula (IV-8). A specific example of such compound (IV) includes a cinnamoyl compound represented by the formula (IV'): 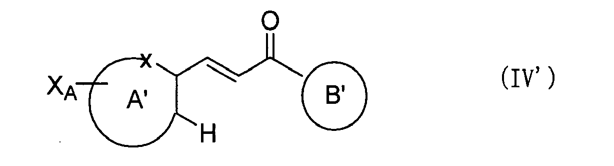 wherein A' is an aromatic 5-membered ring, X_{A} and x are as defined above, and B' is a group represented by the formula (IV'-1) : 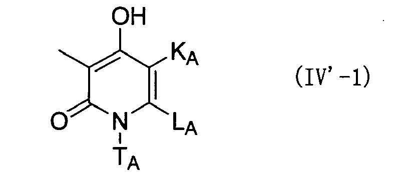 (wherein T_{A}, K_{A} and L_{A} are as defined above), a group represented by formula (IV'-7): 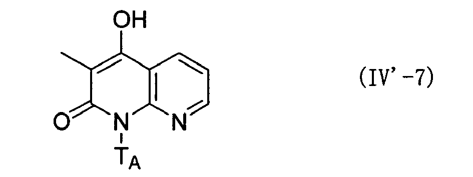 (wherein T_{A} is as defined above), or a group represented by formula (IV'-8): 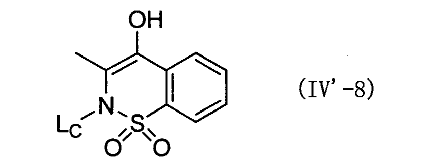 (wherein L_{c} is as defined above). When x is a methine group in the cinnamoyl compound (IV'), x does not have a substituent.

In a further specific example of the cinnamoyl compound (IV'), A' is a thiophene ring.

In a more specific example of the cinnamoyl compound (IV'), A' is a thiophene ring, B' is a group (IV'-1), T_{A} is a methyl group, K_{A} is a hydrogen atom and L_{A} is a methyl group.

In another specific example of the cinnamoyl compound (IV'), A' is a thiophene ring, B' is a group (IV-7) and T_{A} is a methyl group.

In a still more specific example of the cinnamoyl compound (IV'), A' is a thiophene ring, B' is a group (III'-8) and L_{c} is a methyl group.

In some examples of the compound (V), b is a group represented by the formula (V-1), (V-7) or the formula (V-8). A specific example of such compound (V) includes a cinnamoly compound represented by the formula (V"): 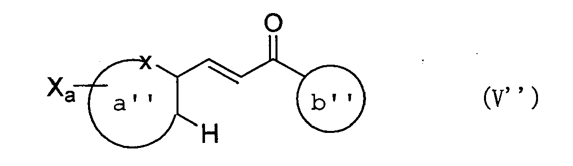 wherein a" represents a thiophene ring, a furan ring, a pyrrole ring or a thiazole ring, Xₐ and x are as defined above, and b" represents a group represented by the formula (V"-1): 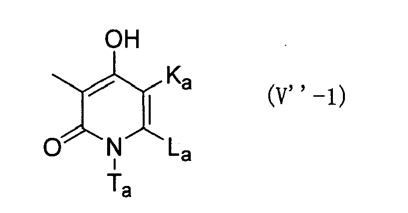 (wherein Tₐ, Kₐ and Lₐ are as defined above), a group represented by formula (V''-7): 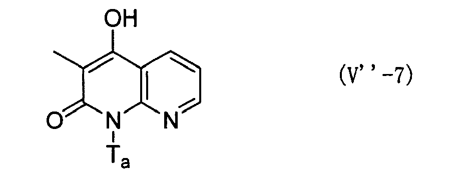 (wherein Tₐ is as defined above), or a group represented by formula (V''-8): 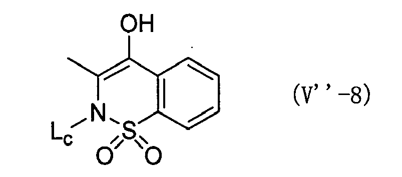 (wherein L_{c} is as defined above).

In a further specific example of the cinnamoyl compound (V"), a" is a thiophene ring.

In a more specific example of the cinnamoyl compound (V"), a" is a thiophene ring, b" is a group (V"-1), Tₐ is a methyl group, Kₐ is a hydrogen atom and Lₐ is a methyl group.

In another specific example of the cinnamoyl compound (V"), a" is a thiophene ring, b" is a group (V"-7) and Tₐ is a methyl group.

In a still more specific example of the cinnamoyl compound (V"), a" is a thiophene ring, b" is a group (V"-8) and L_{c} is a methyl group.

Examples of the compound (VI) includes a compound wherein a1 is a thiophene ring, b1 is a group (VI-1), r_{b1} is a methyl group, Kₐ₁ is a hydrogen atom and Lₐ₁ is a methyl group, a compound wherein a1 is a thiophene ring, b1 is a group (VI-7) and r_{b1} is a methyl group, and a compound wherein a1 is a thiophene ring, b1 is a group (VI-8) and r_{b1} is a methyl group.

Although WO 97/35565, JP09227547, WO 00/20371, JP2002371078, WO 01/79187 and WO 92/18483 disclose compounds having a certain conceptional skeleton, they do not describe the effect of suppressing transcription of an extracellular matrix gene in a tissue, consequently, the effect of suppressing the accumulation of an extracellular matrix.

The compound (I) or (II) can be produced, for example, by reacting a compound represented by the formula (α) (wherein α, X_{α}, Y_{α}, p and q are as defined above) and a compound represented by the formula (β) (wherein β is as defined above) (see Russian J. General Chem. (2001), 71, 1257, Indian J. Chem. (1974), 12, 956 and JP50046666).

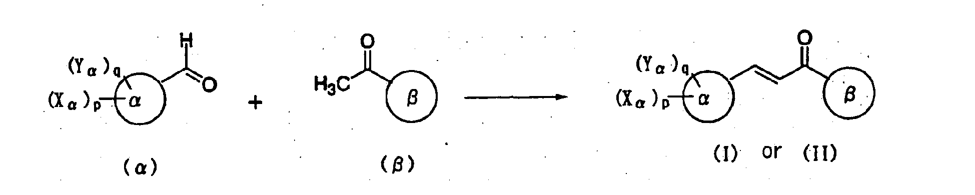

The compound (III) can be produced, for example, by reacting a compound represented by the formula (A0) (wherein A, X_{A0}, Y_{A0}, p and q are as defined above) and a compound represented by the formula (B0) (wherein B0 is as defined above).

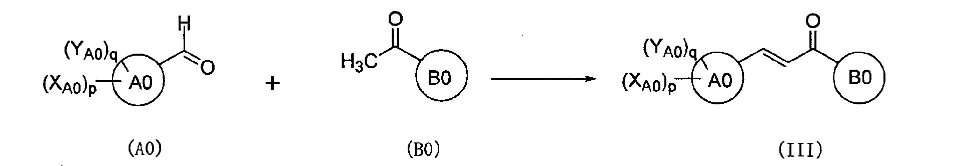

The compound (IV) can be produced, for example, by reacting a compound represented by the formula (A) (wherein A, X_{A}, Y_{A}, p and q are as defined above) and a compound represented by the formula (B) (wherein B is as defined above).

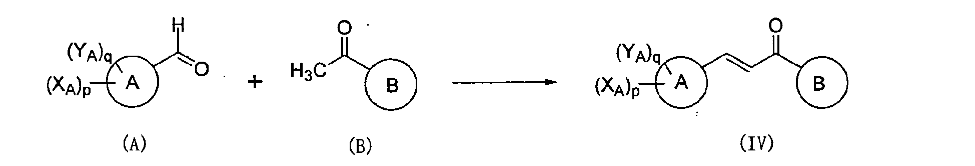

The compound (V) can be produced, for example, by reacting a compound represented by the formula (a) (wherein a, Xₐ, Yₐ, p and q are as defined above) and a compound represented by the formula (b) (wherein b is as defined above.

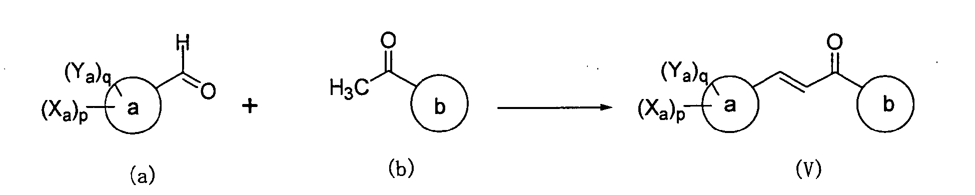

The compound (VI) can be produced, for example, by reacting a compound represented by the formula (a1) (wherein a1 and Xₐ₁ are as defined above) and a compound represented by the formula (b1) (wherein b1 is as defined above).

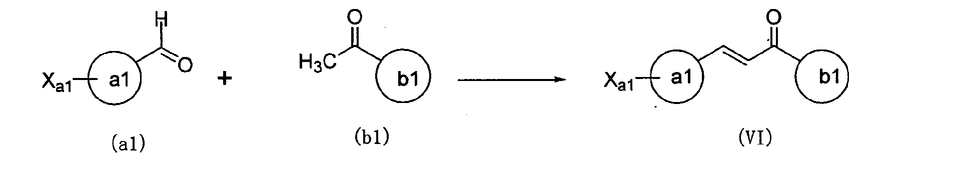

A part of compounds represented by the formula (a1) are known. However, an aldehyde derivative represented by the formula (VII) (hereinafter, referred to as the present aldehyde derivative in some cases) has never been reported, and is a novel substance.

Among the present aldehyde derivatives, a compound represented by the formula (VII-1): 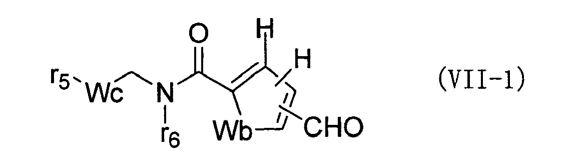 [wherein W_{b} represents an oxygen atom, a sulfur atom or a - NMe- group, r₅ is a hydroxyl group, a C1-C10 alkoxy group or a rr'N- group (wherein r and r' are as defined above), W_{c} represents a -CO- group or a -CH₂- group, and r₆ represents a hydrogen atom or a C1-C10 alkyl group, provided that when W_{c} is a -CH₂- group, r and r' are not a hydrogen atom] can be produced, for example, by reacting a compound represented by the formula (VII-2): 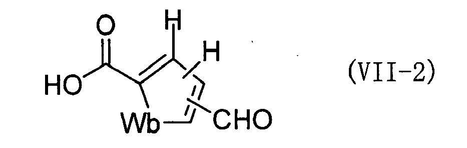 (wherein W_{b} is as defined above) and a compound represented by formula (VII-3): 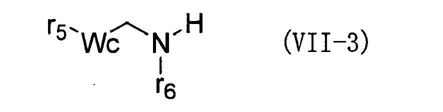 (wherein r₅, W_{c}, and r₆ are as defined above). In the reaction, the reaction temperature is usually from room temperature to the reflux temperature of a solvent, and the reaction time is usually from instant to about 24 hours. The reaction is usually performed in the presence of a dehydrating agent. Examples of a dehydrating agent used include carbonyldiimidazole and the like. In the reaction, usually, 1 to 2 mol of the compound (VII-3) and 1 to 7 mol of a dehydrating agent are used per 1 mol of the compound (VII-2). In the reaction, a solvent is not necessarily required, but the reaction is usually performed in the presence of a solvent. Examples of a solvent which may be used in the reaction include aliphatic hydrocarbons such as hexane, petroleum ether and the like; aromatic hydrocarbons such as benzene, toluene and the like; halogenated hydrocarbons such as chloroform, dichloroethane and the like; ethers such as diethyl ether, dioxane, tetrahydrofuran and the like; ketones such as acetone, methyl ethyl ketone and the like; esters such as ethyl acetate, diethyl carbonate and the like; nitriles such as acetonitrile, isobutyronitrile and the like; amides such as formamide, N,N-dimethylformamide and the like; sulfur compounds such as dimethyl sulfoxide and the like; and their mixtures. After completion of the reaction, a reaction solution is extracted with an organic solvent, washed with water, and subjected to a conventional posttreatment such as concentration of an organic layer under reduced pressure or the like, and then, if necessary, purified by chromatography, recrystallization or the like to obtain the present aldehyde derivative. The compound (VII-2) is known and described in, for example, Tetrahedron (1985) 41, 3803. Examples of the compound (VII-3) include 2-methoxyethylamine, 2-aminoethanol, N-(2-methoxyethyl)methylamine, 2-(methylamino)ethanol, glycine, glycine methyl ester, glycinamide and N,N-dimethylethylenediamine.

Specifically, the compound (VII-1) can be produced by reacting the compound (VII-2) with 2-methoxyethylamine, 2-aminoethanol, N-(2-methoxyethyl)methylamine or glycinamide as the compound (VII-3).

Among the present aldehyde derivatives, a compound represented by the formula (VII'-1): 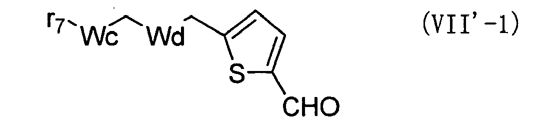 [wherein W_{d} represents an oxygen atom or a sulfur atom, W_{c} is as defined above, and r₇ represents a C1-C10 alkoxy group] can be produced, for example, by reacting a compound represented by the formula (VII'-2): 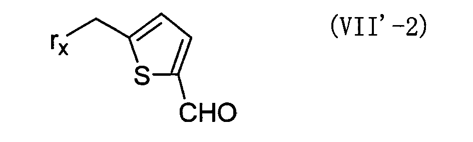 [wherein rₓ represents a halogen atom] and a compound represented by formula (VII'-3): 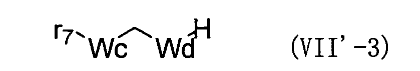 [wherein r₇, W_{c} and W_{d} are as defined above]. In the reaction, the reaction temperature is usually from room temperature to the reflux temperature of a solvent, and the reaction time is usually from instant to about 24 hours. The reaction is usually performed in the presence of a base. Examples of a base used include sodium hydride, potassium hydroxide, potassium carbonate, normalbutyllithium and the like. In the reaction, usually, 1 to 2 mol of the compound (VII'-3) and 1 to 7 mol of a base are used per 1 mol of the compound (VII'-2). In the reaction, a solvent is not necessarily required, but the reaction is usually performed in the presence of a solvent. Examples of a solvent usable in the reaction include ethers such as diethyl ether, dioxane, tetrahydrofuran and the like; ketones such as acetone, methyl ethyl ketone and the like; esters such as ethyl acetate, diethyl carbonate and the like; nitriles such as acetonitrile, isobutyronitrile and the like; amides such as formamide, N,N-dimethylformamide and the like; sulfur compounds such as dimethyl sulfoxide and the like; and their mixtures. After completion of the reaction, the present benzaldehyde derivative can be obtained in the same manner as described above. The compound (VII'-2) is known and described, for example, in WO 00/32598. Examples of the compound (VII'-3) include ethylene glycol, 2-methoxyethanol, methyl glycolate, methyl thioglycolate and the like.

Specifically, the compound (VII'-1) can be produced by reacting the compound (VII'-2) and ethylene glycol or methyl thioglycolate as the compound (VII'-3).

Among the present aldehyde derivatives, a compound represented by the formula (VII"-1): 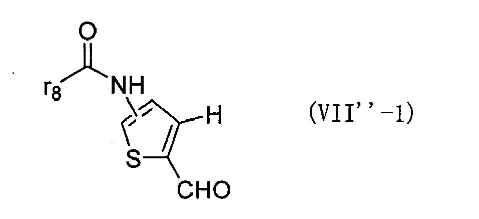 [wherein r₈ represents a C1-C10 alkyl group substituted with a C1-C10 alkoxy group or a C2-C10 alkoxy group substituted with a C1-C10 alkoxy group] can be produced by reacting a compound represented by the formula (VII''-2): 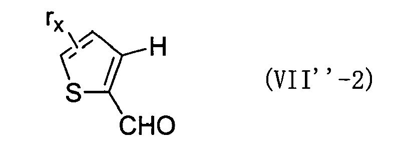 [wherein rₐ is as defined above] and a compound represented by (VII'-3): 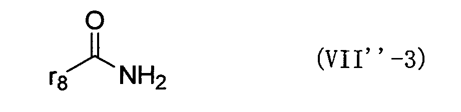 [wherein r₈ is as defined above]. In the reaction, the reaction temperature is usually from room temperature to the reflux temperature of a solvent, and the reaction time is usually from instant to about 24 hours. The reaction is usually performed in the presence of a base and a copper catalyst. Examples of a base used include sodium hydride, potassium hydroxide, potassium carbonate and the like. Examples of a copper catalyst include copper (powder), copper(I) bromide, and copper(I) iodide. The compound (VII'' -1) can be produced in good yield by adding aliphatic amine such as N,N'-dimethylethylenediamine to the reaction. In the reaction, usually, 1 to 2 mol of the compound (VII"-3), 1 to 7 mol of a base and 0.1 to 2 mol of a copper catalyst are used per 1 mol of the compound (VII"-2). In the reaction, a solvent is not necessarily required, but the reaction is usually performed in the presence of a solvent. Examples of a solvent usable in the reaction include ethers such as diethyl ether, dioxane, tetrahydrofuran and the like; ketones such as acetone, methyl ethyl ketone and the like; esters such as ethyl acetate, diethyl carbonate and the like; nitriles such as acetonitrile, isobutyronitrile and the like; amides such as formamide, N,N-dimethylformamide and the like; sulfur compounds such as dimethyl sulfoxide and the like; and their mixtures. After completion of the reaction, the present benzaldehyde derivative can be obtained in the same manner as described above. The compound (VII"-3) is known and described, for example, in US3449406. Examples of the compound (VII'-3) include methoxyacetamide, 2-methoxyethyl carbamate and the like.

Among the present aldehyde derivatives, a compound represented by the formula (VII'''-1): 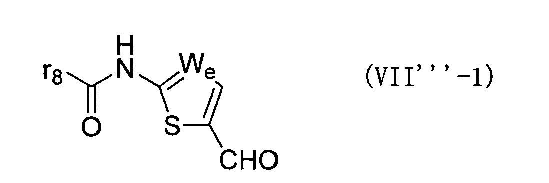 [wherein Wₑ represents a methine group or a -N= group, and r₈ is as defined above] can be produced by reacting a compound represented by the formula (VII"'-2): 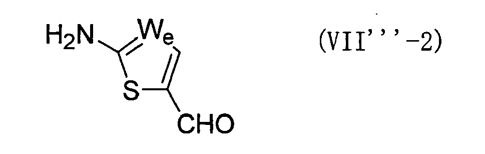 [wherein Wₑ is as defined above] and a compound represented by (VII'''-3) : 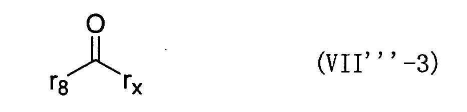 [wherein rₓ is as defined above]. In the reaction, the reaction temperature is usually from room temperature to the reflux temperature of a solvent, and the reaction time is usually from instant to about 24 hours. The reaction is usually performed in the presence of a base. Examples of a base used include inorganic bases such as potassium hydroxide, potassium carbonate and the like, and organic bases such as pyridine, triethylamine, N,N-diethylaniline and the like. In the reaction, usually, 1 to 2 mol of the compound (VII'''-3) and 1 to 7 mol of a base are used per 1 mol of the compound (VII"'-2). In the reaction, a solvent is not necessarily required, but the reaction is usually performed in the presence of a solvent. Examples of a solvent usable in the reaction include ethers such as diethyl ether, dioxane, tetrahydrofuran and the like; ketones such as acetone, methyl ethyl ketone and the like; esters such as ethyl acetate, diethyl carbonate and the like; nitriles such as acetonitrile, isobutyronitrile and the like; amides such as formamide, N,N-dimethylformamide and the like; sulfur compounds such as dimethyl sulfoxide and the like; and their mixtures. After completion of the reaction, the present benzaldehyde derivative can be obtained in the same manner as described above. The compound (VII'''-2) is known and described, for example, in Bioorg. Med. Chem. Lett. 14(1), 235(2004). Examples of the compound (VII'''-3) include methoxyacetyl chloride, 2-methoxyethyl chloroformate and the like.

The compound (VIII") can be produced by reacting the present aldehyde derivative and the compound (VIII').

Table 30 and Table 31 show examples of the novel present aldehyde derivative (VII-1) represented by the compound numbers (a) to (p).

**Table 30**

| Comp. No. | the present aldehyde derivative (VII-1) | Comp. No. | the present aldehyde derivative (VII-1) |
|---|---|---|---|
| (a) | 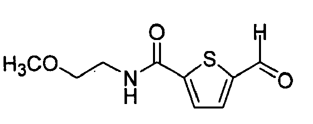 | (h) | 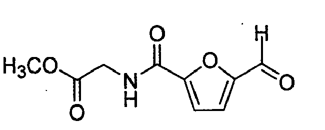 |
| (b) | 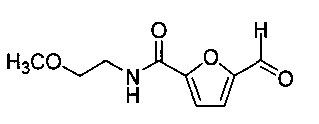 | (i) | 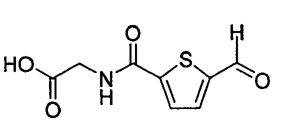 |
| (c) | 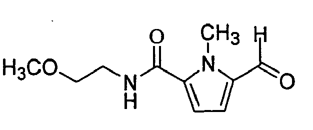 | (j) | 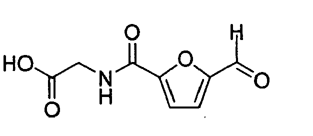 |
| (d) | 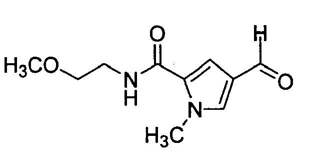 | (k) | 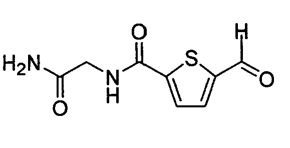 |
| (e) | 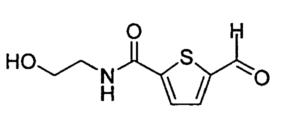 | (1) | 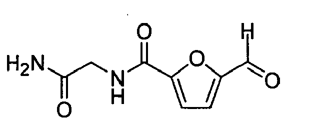 |
| (f) | 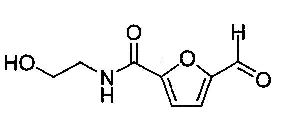 | (m) | 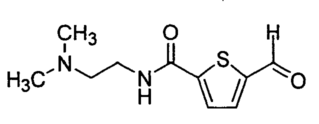 |
| (g) | 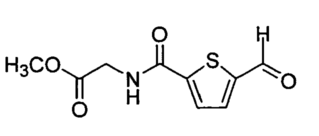 | (n) | 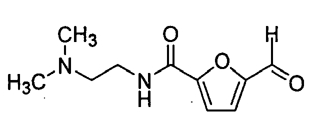 |

**Table 31**

| | |
|---|---|
| the present aldehyde derivative (VII-1) Compound No. (o) | 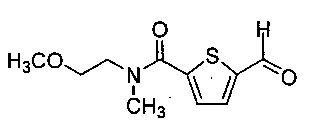 |
| the present aldehyde derivative (VII-1) Compound No. (p) | 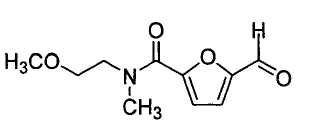 |

Table 32 shows examples of the novel present aldehyde derivative (VII'-1) represented by the compound numbers (q) to (r).

**Table 32**

| | |
|---|---|
| the present aldehyde derivative (VII'-1) Compound No. (q) | 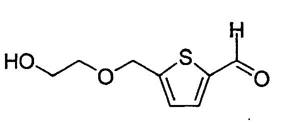 |
| the present aldehyde derivative (VII'-1) Compound No. (r) | 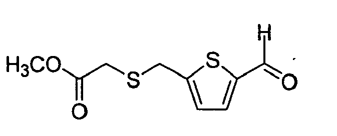 |

Table 33 to Table 34 show examples of the novel present aldehyde derivative (VII"-1) represented by the compound numbers (s) to (v).

**Table 33**

| | |
|---|---|
| the present aldehyde derivative (VII'-1) Compound No. (s) | 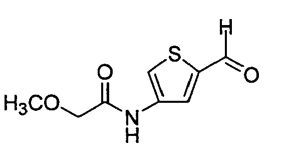 |
| the present aldehyde derivative (VII'-1) Compound No. (t) | 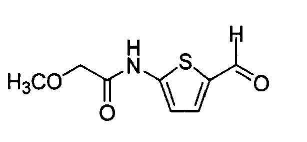 |

**Table 34**

| | |
|---|---|
| the present aldehyde derivative (VII''-1) Compound No. (u) | 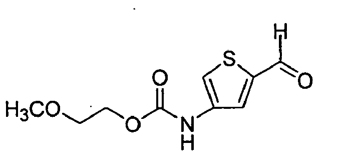 |
| the present aldehyde derivative (VII''-1) Compound No. (v) | 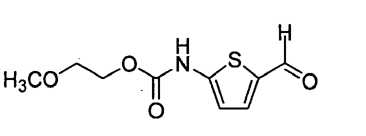 |

Table 35 shows an example of the novel present aldehyde derivative (VII'''-1) represented by the compound number (w).

**Table 35**

| | |
|---|---|
| the present aldehyde derivative (VII'''-1) Compound No. (w) | 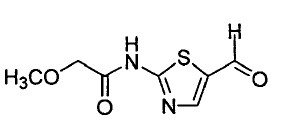 |

Among the compounds (V), examples of the compound (Va-1)-represented by the compound numbers (1a-1) to (72a-1), examples of the compound (Va-2) represented by the compound numbers (1a-2) to (16a-2), and examples of the compound (Va-3) represented by the compound numbers (1a-3) to (11a-3) are shown in Table 36 to Table 43.

### (Compounds shown in Table 36 to Table 43)

The present compounds (Va-1), (Va-2) and (Va-3)

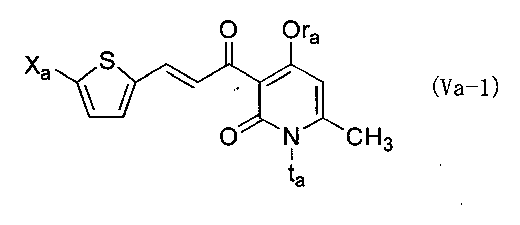

**Table 36**

| Compound No. | Xₐ | rₐ | tₐ |
|---|---|---|---|
| (1a-1) | -CH₂CH₂CN | -H | -CH₃ |
| (2a-1) | 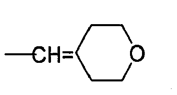 | -H | -CH₃ |
| (3a-1) | -CH=CF₂ | -H | -CH₃ |
| (4a-1) | -CH=CHCN | -H | -H |
| (5a-1) | -CH=CHCOOCH₃ | -H | -CH₃ |
| (6a-1) | -C≡CC(CH₃)₂OH | -H | -CH₃ |
| (7a-1) | -OCH₂CH₂SCH₃ | -H | -CH₃ |
| (8a-1) | -OCH₂CH₂SOCH₃ | -H | -CH₃ |
| (9a-1) | -OCH₂CH₂SO₂CH₃ | -H | -CH₃ |
| (10a-1) | -CH₂OCH₂CH=CH₂ | -H | -H |
| (11a-1) | -CH₂OCH₂C=CH | -H | -H |
| (12a-1) | -CH₂OCH₂COOCH₃ | -H | -CH₃ |
| (13a-1) | -CH₂OCH₂COOCH₃ | -CH₃ | -CH₃ |
| (14a-1) | -CH₂OCH₂COO(CH₂)₉-OH | -H | -CH₃ |
| (15a-1) | -CH₂OCH₂COOH•pyridine | -H | -H |
| (16a-1) | -CH₂OCH₂COO(Ca)_{0.5} | -(Ca)_{0.5} | -CH₃ |
| (17a-1) | -CH₂OCH₂COONa | -Na | -CH₃ |
| (18a-1) | -CH₂OCH₂COOH | -H | -CH₃ |
| (19a-1) | -CH₂OCH₂CONH₂ | -H | -CH₃ |
| (20a-1) | -CH₂OCH₂CON(CH₃)₂ | -H | -CH₃ |
| (21a-1) | -OCH₂CH₂N(CH₃)₂ | -H | -CH₃ |
| (22a-1) | -OCH₂CH₂CH₂N(CH₃)₂ | -H | -CH₃ |
| (23a-1) | -CH₂OCH₂CH₂NH₂ | -H | -CH₃ |
| (24a-1) | -CH₂OCH₂CH₂NH₂•HCl | -H | -CH₃ |
| (25a-1) | -CH₂OCH₂CH₂NHCOCH₃ | -H | -CH₃ |

**Table 37**

| Compound No. | Xₐ | rₐ | tₐ |
|---|---|---|---|
| (26a-1) | -CH₂OCH₂CH₂NHCOOC (CH₃) | -H | -CH₃ |
| (27a-1) | -CH₂OCH₂CH₂OH | -H | -CH₃ |
| (28a-1) | -OCH₂CH₂CH₂OH | -H | -CH₃ |
| (29a-1) | -OCH₂CH₂OCH₃ | -H | -CH₃ |
| (30a-1) | -CH₂OCH₂COCH₃ | -H | -CH₃ |
| (31a-1) | -CH₂OCH₂CN | -H | -H |
| (32a-1) | -CH₂OCH₂CH₂SO₃H | -H | -CH₃ |
| (33a-1) | -CH₂OCH₂CH₂CH₂SO₃Na | -Na | -H |
| (34a-1) | -CH₂SCH₂COOCH₃ | -H | -CH₃ |
| (35a-1) | -CH₂SOCH₂COOCH₃ | -H | -CH₃ |
| (36a-1) | -CH₂SO₂CH₂COOCH₃ | -H | -CH₃ |
| (37a-1) | -CH₂SOCH₂COOH | -H | -CH₃ |
| (38a-1) | -CH₂SO₂CH₂COOH | -H | -CH₃ |
| (39a-1) | -NHCH₂CH₂N(CH₃)2 | -H | -H |
| (40a-1) | -NHCOOCH₂CH₂OCH₃ | -H | -CH₃ |
| (41a-1) | -NHCONHCH₂CH₂OCH₃ | -H | -CH₃ |
| (42a-1) | -NHCOCOOCH₃ | -H | -CH₃ |
| (43a-1) | -NHCOCOOH | -H | -CH₃ |
| (44a-1) | -NHCOCH=CH₂ | -H | -CH₃ |
| (45a-1) | -NHSO₂CH₂CH=CH₂ | -H | -CH₃ |
| (46a-1) | -NHCOCH₂OCH₃ | -H | -CH₃ |
| (47a-1) | -NHCOCH₂OCH₃ | -CH₃ | -CH₃ |
| (48a-1) | -NHSO₂CH₂COOCH₃ | -H | -H |
| (49a-1) | -NHSO₂CH₂COOH | -H | -H |
| (50a-1) | -NHCOCH₂CN | -H | -H |

**Table 38**

| Compound No. | Xₐ | rₐ | tₐ |
|---|---|---|---|
| (51a-1) | -CONHOCH₃ | -H | -CH₃ |
| (52a-1) | -CONHOCH₂CH=CH₂ | -H | -CH₃ |
| (53a-1) | -CONHSO₂CH₃ | -H | -CH₃ |
| (54a-1) | -CONHCH₂CH₂OH | -H | -CH₃ |
| (55a-1) | -CON (CH₃) CH₂CH₂OH | -H | -CH₃ |
| (56a-1) | -CONHCH₂CH₂OCH₃ | -H | -CH₃ |
| (57a-1) | -CONHCH₂CH₂OCH₃ | -CH₃ | -CH₃ |
| (58a-1) | -CON (CH₃) CH₂CH₂OCH₃ | -H | -CH₃ |
| (59a-1) | -CONHCH₂CH₂N (CH₃)₂ | -H | -CH₃ |
| (60a-1) | -CONHCH₂COOCH₃ | -H | -CH₃ |
| (61a-1) | -CONHCH₂COOH | -H | -CH₃ |
| (62a-1) | -CONHCH₂CN | -H | -H |
| (63a-1) | -CONHCH₂CONH₂ | -H | -CH₃ |
| (64a-1) | -CONHCH (CO₂CH₃) -CH₂CO₂CH₃ | -H | -H |
| (65a-1) | -CONHCH(CO₂H)-CH₂CO₂H | -H | -H |
| (66a-1) | -CH=NOCH₃ | -H | -CH₃ |
| (67a-1) | -NHCSNHCH₃ | -H | -CH₃ |
| (68a-1) | -N=C (-SCH₃)NHCH₃ | -H | -CH₃ |
| (69a-1) | -CH₂P(=O)(OCH₃) ₂ | -H | -CH₃ |
| (70a-1) | -CH₂P(=O)(OH)₂ | -H | -CH₃ |
| (71a-1) | -CH₂SCH₂COOH | -H | -CH₃ |
| (72a-1) | -N (CH₃) CH₂CH₂N (CH₃)₂ | -H | -CH₃ |

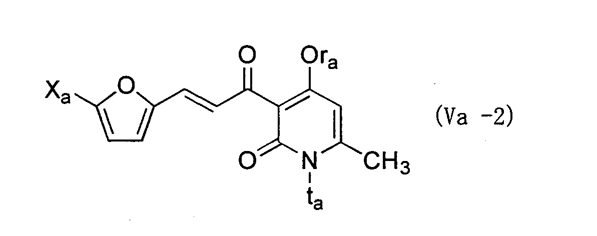

**Table 39**

| Compound No. | Xₐ | rₐ | tₐ |
|---|---|---|---|
| (1a-2) | -CH₂OCH₂COOCH₃ | -H | -CH₃ |
| (2a-2) | -CH₂OCH₂COOH | -H | -CH₃ |
| (3a-2) | -NHCOOCH₂CH₂OCH₃ | -H | -CH₃ |
| (4a-2) | -NHCONHCH₂CH₂OCH₃ | -H | -CH₃ |
| (5a-2) | -NHCOCH₂OCH₃ | -H | -CH₃ |
| (6a-2) | -NHCOCH₂OCH₃ | -CH₃ | -CH₃ |
| (7a-2) | -CONHCH₂CH₂OH | -H | -H |
| (8a-2) | -CONHCH₂CH₂OH | -H | -CH₃ |
| (9a-2) | -CON (CH₃) CH₂CH₂OH | -H | -CH₃ |
| (10a-2) | -CONHCH₂CH₂OCH₃ | -H | -CH₃ |
| (11a-2) | -CONHCH₂CH₂OCH₃ | -CH₃ | -CH₃ |
| (12a-2) | -CON (CH₃) CH₂CH₂OCH₃ | -H | -CH₃ |
| (13a-2) | -CONHCH₂CH₂N (CH₃) ₂ | -H | -CH₃ |
| (14a-2) | -CONHCH₂COOCH₃ | -H | -CH₃ |
| (15a-2) | -CONHCH₂COOH | -H | -CH₃ |
| (15a-2) | -CONHCH₂CONH₂ | -H | -CH₃ |
| (16a-2) | -SO₂NHCH₂CH₂OCH₃ | -H | -CH₃ |

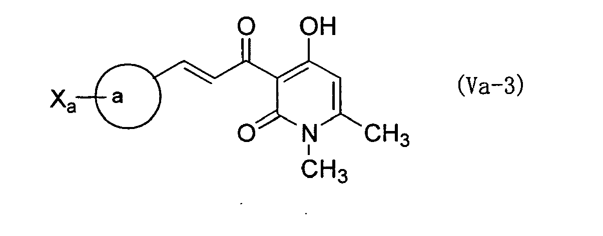

**Table 40**

| Compound No. | 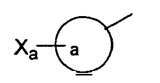 |
|---|---|
| (la-3) | 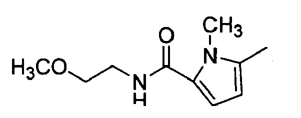 |
| (2a-3) | 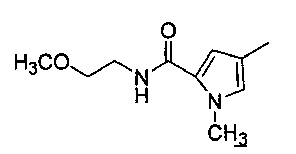 |
| (3a-3) | 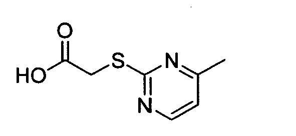 |
| (4a-3) | 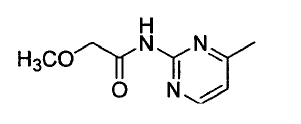 |
| (5a-3) | 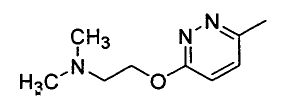 |
| (6a-3) | 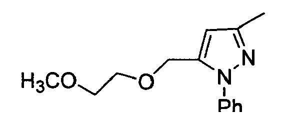 |
| (7a-3) | 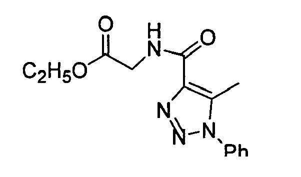 |
| (8a-3) | 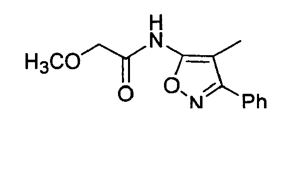 |

**Table 41**

| | |
|---|---|
| compound (Va-3) Compound No. (9a-3) | 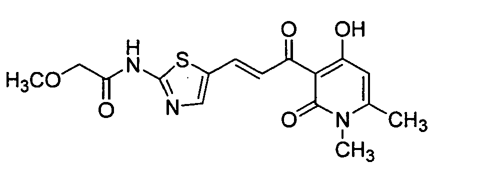 |

**Table 42**

| | |
|---|---|
| compound (Va-3) Compound No. (10a-3) | 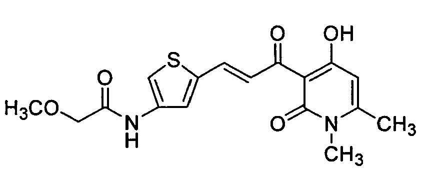 |

**Table 43**

| | |
|---|---|
| compound (Va-3) Compound No. (11a-3) | 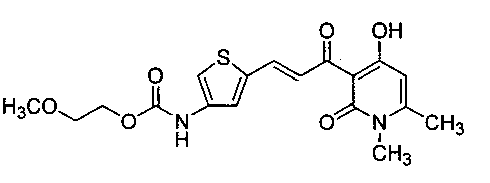 |

Among the compounds (V), examples of the compound (Va') represented by the compound numbers (1a') to (30a') are shown in Tables 44 to 45.

### (Compounds shown in Tables 44 to 45)

The compound (Va')

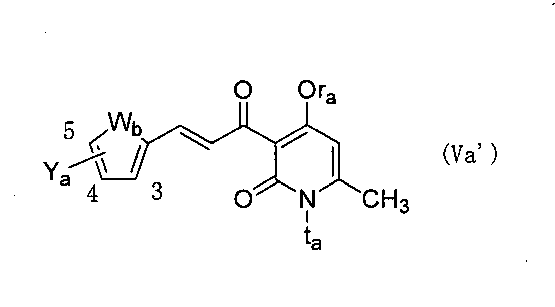

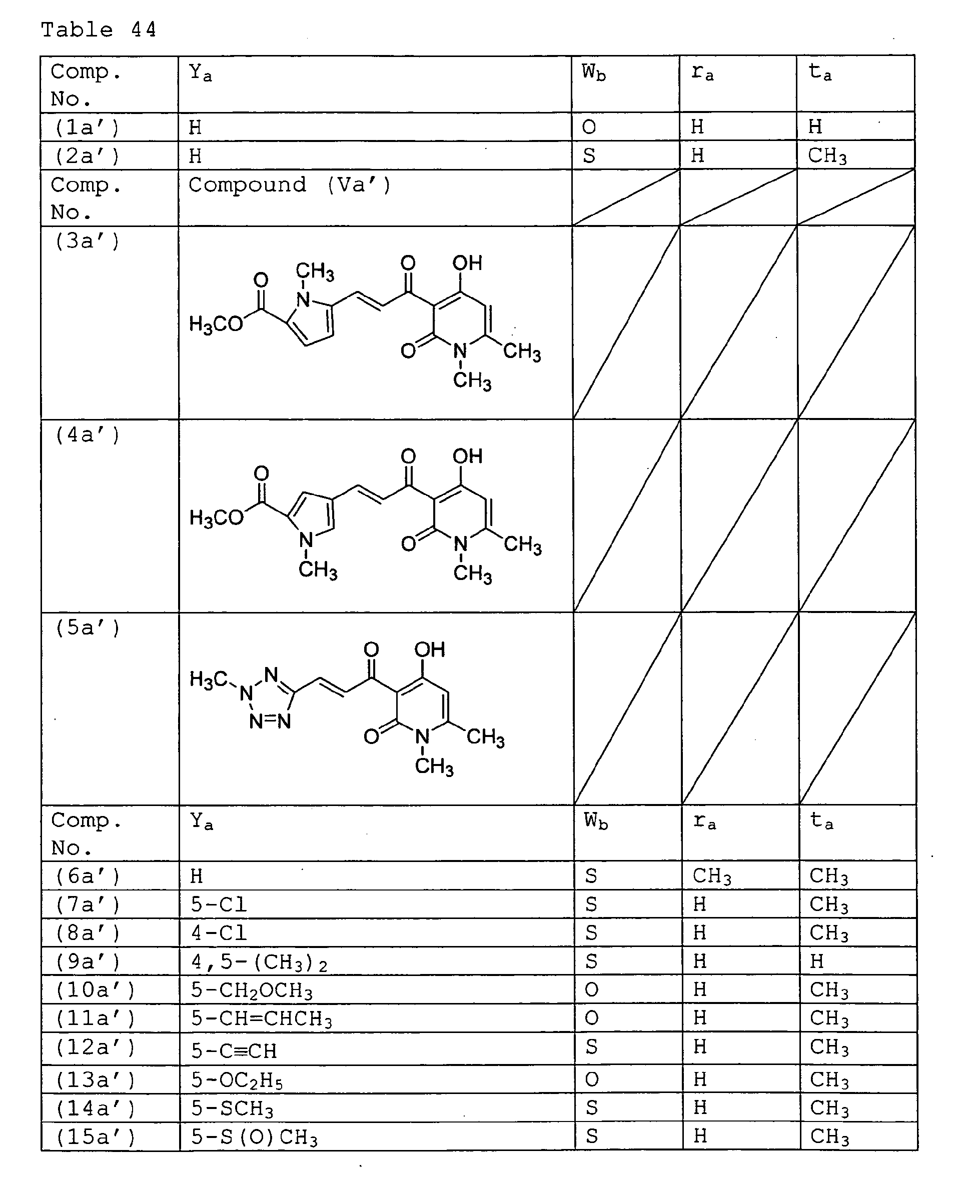

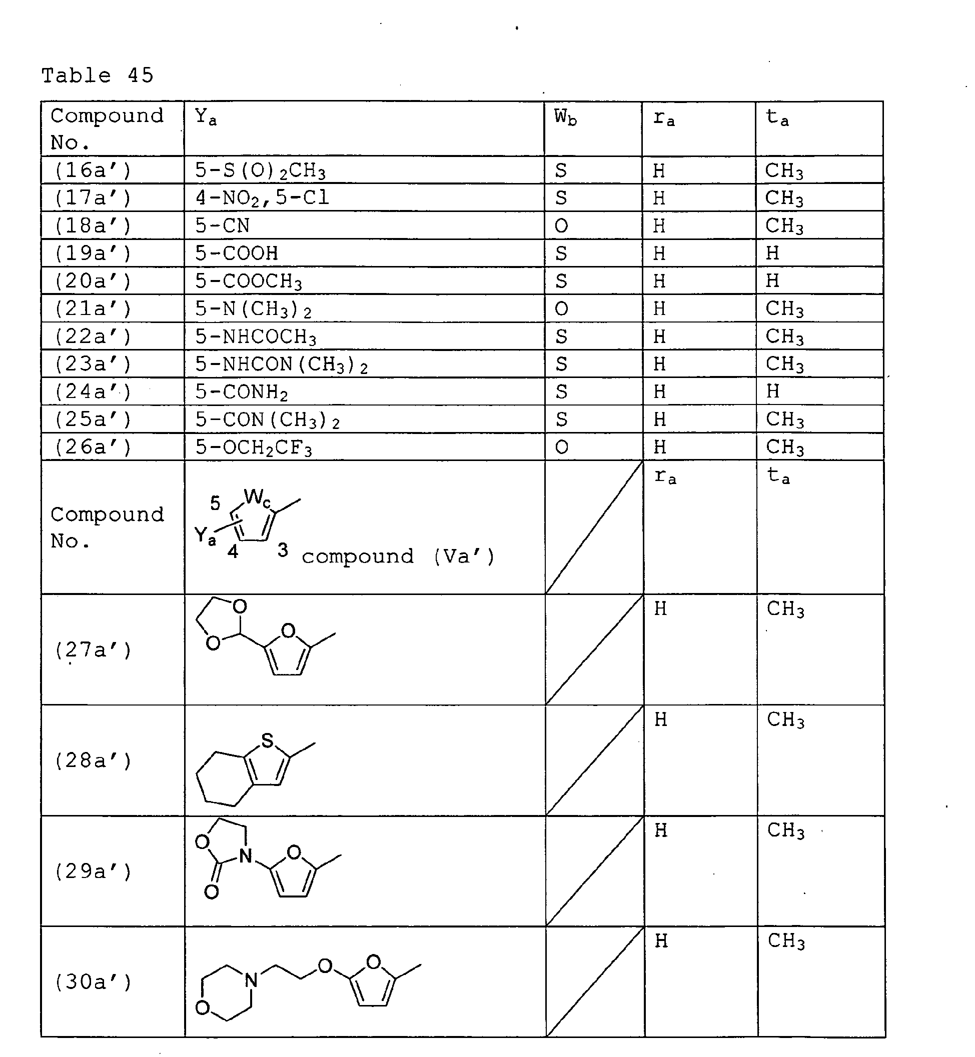

Among the compounds (V), examples of the compound (Vb) represented by the compound numbers (1b) to (13b) are shown in Table 46.

### (Compounds shown in Table 46)

The compound (Vb):

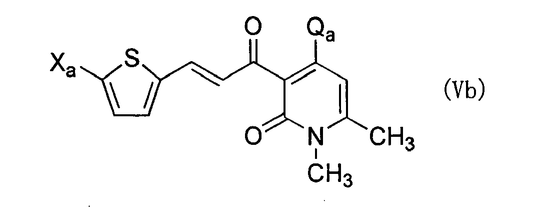

**Table 46**

| Comp. No. | Xₐ | Qₐ |
|---|---|---|
| (1b) | -CONHCH₂CH₂OCH₃ | -OCH₂CH=CH₂ |
| (2b) | -CONHCH₂CH₂OCH₃ | -OCH₂C≡CH |
| (3b) | -CONHCH₂COOCH₃ | -OCH₂COOCH₃ |
| (4b) | -CONHCH₂COOH | -OCH₂COOH |
| (5b) | -CONHCH₂CONH₂ | -OCH₂CONH₂ |
| (6b) | -CONHCH₂CH₂OCH₃ | -OCH₂CN |
| (7b) | -CONHCH₂CH₂OCH₃ | -OCH₂CH₂OH |
| (8b) | -CONHCH₂CH₂OCH₃ | -OCH₂Ph |
| (9b) | -CONHCH₂CH₂OCH₃ | -OCH₂CH₂N(CH₃)₂ |
| (10b) | -OCH₂CH₂OCH₃ | 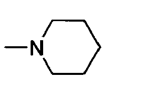 |
| (11b) | 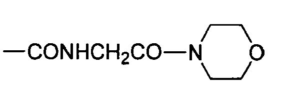 | 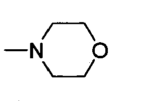 |
| (12b) | -CONHCH₂CH₂OCH₃ | -NHCH₂C≡CH |
| (13b) | -CONHCH₂CONHCH₂CH₂OCH₃ | -NHCH₂CH₂OCH₃ |

Among the compounds (V), examples of the compound (Vc) represented by the compound numbers (1c) to (11c) are shown in Table 47.

### (Compounds shown in Table 47)

The compound (Vc):

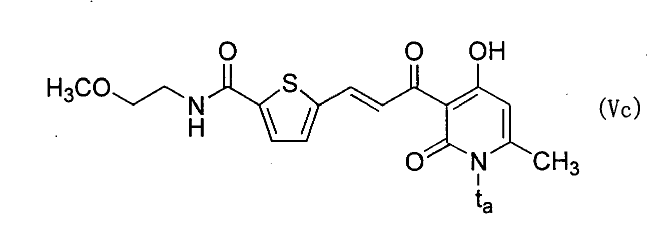

**Table 47**

| Compound No. | tₐ |
|---|---|
| (1c) | -CH₂CH=CH₂ |
| (2c) | -CH₂C≡CH |
| (3c) | -CH₂COOCH₃ |
| (4c) | -CH₂COOH |
| (5c) | -CH₂CONH₂ |
| (6c) | -CH₂CN |
| (7c) | -CH₂COCH₃ |
| (8c) | -CH₂CH₂OCH₃ |
| (9c) | -CH₂Ph |
| (10c) | -Ph |
| (11c) | 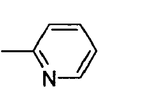 |

Among the compounds (V), examples of the compound (Vd) represented by the compound numbers (1d) to (3d) are shown in Table 48.

### (Compounds shown in Table 48)

The compound (Vd):

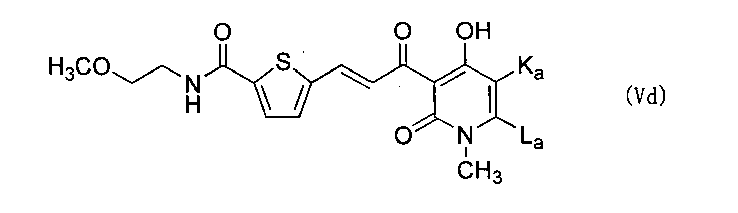

**Table 48**

| Compound No. | compound (Vd) |
|---|---|
| (1d) | 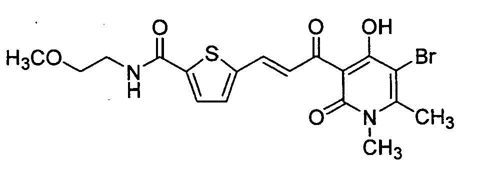 |
| (2d) | 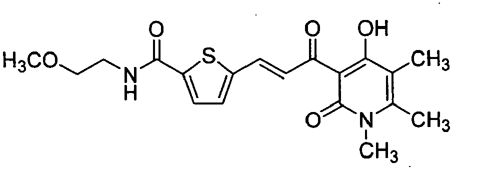 |
| (3d) | 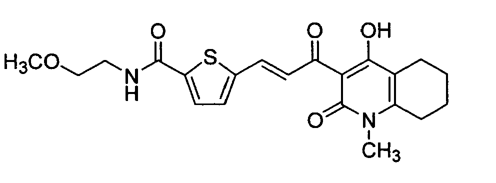 |

Among the compounds (V), examples of the compound (Ve) represented by the compound numbers (1e) to (70e) are shown in Table 49 to Table 51.

### (Compounds shown in Table 49 to Table 51)

The present compound (Ve):

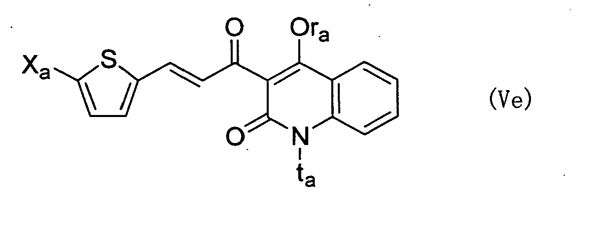

**Table 49**

| Compound No. | Xₐ | rₐ | tₐ |
|---|---|---|---|
| (1e) | -CH₂CH₂CN | -H | -CH₃ |
| (2e) | 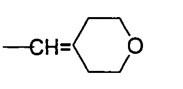 | -H | -CH₃ |
| (3e) | -CH=CF₂ | -H | -CH₃ |
| (4e) | -CH=CHCN | -H | -H |
| (5e) | -CH=CHCOOCH₃ | -H | -CH₃ |
| (6e) | -C=CC(CH₃)₂OH | -H | -CH₃ |
| (7e) | -OCH₂CH₂SCH3 | -H | -CH₃ |
| (8e) | -OCH₂CH₂SOCH₃ | -H | -CH₃ |
| (9e) | -OCH₂CH₂SO₂CH₃ | -H | -CH₃ |
| (10e) | -CH₂OCH₂CH=CH₂ | -H | -H |
| (11e) | -CH₂OCH₂C=CH | -H | -H |
| (12e) | -CH₂OCH₂COOCH₃ | -H | -CH₃ |
| (13e) | -CH₂OCH₂COOCH₃ | -CH₃ | -CH₃ |
| (14e) | -CH₂OCH₂COO(CH₂)₉-OH | -H | -CH₃ |
| (15e) | -CH₂OCH₂COOH•pyridine | -H | -H |
| (16e) | -CH₂OCH₂COO (Ca)_{0.5} | -(Ca)_{0.5} | -CH₃ |
| (17e) | -CH₂OCH₂COONa | -Na | -CH₃ |
| (18e) | -CH₂OCH₂COOH | -H | -CH₃ |
| (19e) | -CH₂OCH₂CONH₂ | -H | -CH₃ |
| (20e) | -CH₂OCH₂CONCH₃)₂ | -H | -CH₃ |
| (21e) | -OCH₂CH₂N(CH₃)₂ | -H | -CH₃ |
| (22e) | -OCH₂CH₂CH₂N (CH₃)₂ | -H | -CH₃ |
| (23e) | -CH₂OCH₂CH₂NH₂ | -H | -CH₃ |
| (24e) | -CH₂OCH₂CH₂NH₂•HCl | -H | -CH₃ |
| (25e) | -CH₂OCH₂CH₂NHCOCH₃ | -H | -CH₃ |

**Table 50**

| Compound No. | Xₐ | rₐ | tₐ |
|---|---|---|---|
| (26e) | -CH₂OCH₂CH₂NHCOOC(CH₃)₃ | -H | -CH₃ |
| (27e) | -CH₂OCH₂CH₂OH | -H | -CH₃ |
| (28e) | -OCH₂CH₂CH₂OH | -H | -CH₃ |
| (29e) | -OCH₂CH₂OCH₃ | -H | -CH₃ |
| (30e) | -CH₂OCH₂COCH₃ | -H | -CH₃ |
| (31e) | -CH₂OCH₂CN | -H | -H |
| (32e) | -CH₂OCH₂CH₂SO₃H | -H | -CH₃ |
| (33e) | -CH₂OCH₂CH₂CH₂SO₃Na | -Na | -H |
| (34e) | -CH₂SCH₂COOCH₃ | -H | -CH₃ |
| (35e) | -CH₂SOCH₂COOCH₃ | -H | -CH₃ |
| (36e) | -CH₂SO₂CH₂COOCH₃ | -H | -CH₃ |
| (37e) | -CH₂SOCH₂COOH | -H | -CH₃ |
| (38e) | -CH₂SO₂CH₂COOH | -H | -CH₃ |
| (39e) | -NHCH₂CH₂N(CH₃)₂ | -H | -H |
| (40e) | -NHCOOCH₂CH₂OCH₃ | -H | -CH₃ |
| (41e) | -NHCONHCH₂CH₂OCH₃ | -H | -CH₃ |
| (42e) | -NHCOCOOCH₃ | -H | -CH₃ |
| (43e) | -NHCOCOOH | -H | -CH₃ |
| (44e) | -NHCOCH=CH₂ | -H | -CH₃ |
| (45e) | -NHSO₂CH₂CH=CH₂ | -H | -CH₃ |
| (46e) | -NHCOCH₂OCH₃ | -H | -CH₃ |
| (47e) | -NHCOCH₂OCH₃ | -CH₃ | -CH₃ |
| (48e) | -NHSO₂CH₂COOCH₃ | -H | -H |
| (49e) | -NHSO₂CH₂COOH | -H | -H |
| (50e) | -NHCOCH₂CN | -H | -H |

**Table 51**

| Compound No. | Xₐ | rₐ | tₐ |
|---|---|---|---|
| (51e) | -CONHOCH₃ | -H | -CH₃ |
| (52e) | -CONHOCH₂CH=CH₂ | -H | -CH₃ |
| (53e) | -CONHSO₂CH₃ | -H | -CH₃ |
| (54e) | -CONHCH₂CH₂OH | -H | -CH₃ |
| (55e) | -CON (CH₃) CH₂CH₂OH | -H | -CH₃ |
| (56e) | -CONHCH₂CH₂OCH₃ | -H | -CH₃ |
| (57e) | -CONHCH₂CH₂OCH₃ | -CH₃ | -CH₃ |
| (58e) | -CON (CH₃) CH₂CH₂OCH₃ | -H | -CH₃ |
| (59e) | -CONHCH₂CH₂N (CH₃) ₂ | -H | -CH₃ |
| (60e) | -CONHCH₂COOCH₃ | -H | -CH₃ |
| (61e) | -CONHCH₂COOH | -H | -CH₃ |
| (62e) | -CONHCH₂CN | -H | -H |
| (63e) | -CONHCH₂CONH₂ | -H | -CH₃ |
| (64e) | -CONHCH (CO₂CH₃) -CH₂CO₂CH₃ | -H | -H |
| (65e) | -CONHCH(CO₂H)-CH₂CO_{2H} | -H | -H |
| (66e) | -CH=NOCH₃ | -H | -CH₃ |
| (67e) | -NHCSNHCH₃ | -H | -CH₃ |
| (68e) | -N=C(-SCH₃)NHCH₃ | -H | -CH₃ |
| (69e) | -CH₂P(=O) (OCH₃)₂ | -H | -CH₃ |
| (70e) | -CH₂P (=O) (OH) ₂ | -H | -CH₃ |

Among the compounds (V), examples of the compound (Vf) represented by the compound numbers (1f) to (70f) are shown in Tables 52 to 54.

### (Compounds shown in Tables 52 to 54)

The present compound (Vf):

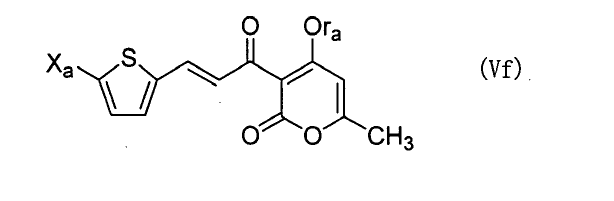

**Table 52**

| Compound No. | Xₐ | rₐ |
|---|---|---|
| (1f) | -CH₂CH₂CN | -H |
| (2f) | 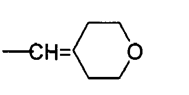 | -H |
| (3f) | -CH=CF₂ | -H |
| (4f) | -CH=CHCN | -H |
| (5f) | -CH=CHCOOCH₃ | -H |
| (6f) | -C≡CC(CH₃)₂OH | -H |
| (7f) | -OCH₂CH₂SCH₃ | -H |
| (8f) | -OCH₂CH₂SOCH₃ | -H |
| (9f) | -OCH₂CH₂SO₂CH₃ | -H |
| (10f) | -CH₂OCH₂CH=CH₂ | -H |
| (11f) | -CH₂OCH₂C≡CH | -H |
| (12f) | -CH₂OCH₂COOCH₃ | -H |
| (13f) | -CH₂OCH₂COOCH₃ | -CH₃ |
| (14f) | -CH₂OCH₂COO(CH₂)₉-OH | -H |
| (15f) | -CH₂OCH₂COOH•pyridine | -H |
| (16f) | -CH₂OCH₂COO (Ca) 0.5 | -(Ca)_{0.5} |
| (17f) | -CH₂OCH₂COONa | -Na |
| (18f) | -CH₂OCH₂COOH | -H |
| (19f) | -CH₂OCH₂CONH₂ | -H |
| (20f) | -CH₂OCH₂CON (CH₃) ₂ | -H |
| (21f) | -OCH₂CH₂N(CH₃)₂ | -H |
| (22f) | -OCH₂CH₂CH₂N (CH₃) 2 | -H |
| (23f) | -CH₂OCH₂CH₂NH₂ | -H |
| (24f) | -CH₂OCH₂CH₂NH₂•HCl | -H |
| (25f) | -CH₂OCH₂CH₂NHCOCH₃ | -H |

**Table 53**

| Compound No. | Xₐ | rₐ |
|---|---|---|
| (26f) | -CH₂OCH₂CH₂NHCOOC (CH₃) | -H |
| (27f) | -CH₂OCH₂CH₂OH | -H |
| (28f) | -OCH₂CH₂CH₂OH | -H |
| (29f) | -OCH₂CH₂OCH₃ | -H |
| (30f) | -CH₂OCH₂COCH₃ | -H |
| (31f) | -CH₂OCH₂CN | -H |
| (32f) | -CH₂OCH₂CH₂SO₃H | -H |
| (33f) | -CH₂OCH₂CH₂CH₂SO₃Na | -Na |
| (34f) | -CH₂SCH₂COOCH₃ | -H |
| (35f) | -CH₂SOCH₂COOCH₃ | -H |
| (36f) | -CH₂SO₂CH₂COOCH₃ | -H |
| (37f) | -CH₂SOCH₂COOH | -H |
| (38f) | -CH₂SO₂CH₂COOH | -H |
| (39f) | -NHCH₂CH₂N(CH₃)₂ | -H |
| (40f) | -NHCOOCH₂CH₂OCH₃ | -H |
| (41f) | -NHCONHCH₂CH₂OCH₃ | -H |
| (42f) | -NHCOCOOCH₃ | -H |
| (43f) | -NHCOCOOH | -H |
| (44f) | -NHCOCH=CH₂ | -H |
| (45f) | -NHSO₂CH₂CH=CH₂ | -H |
| (46f) | -NHCOCH₂OCH₃ | -H |
| (47f) | -NHCOCH₂OCH₃ | -CH₃ |
| (48f) | -NHSO₂CH₂COOCH₃ | -H |
| (49f) | -NHSO₂CH₂COOH | -H |
| (50f) | -NHCOCH₂CN | -H |

**Table 54**

| Compound No. | Xₐ | rₐ |
|---|---|---|
| (51f) | -CONHOCH₃ | -H |
| (52f) | -CONHOCH₂CH=CH₂ | -H |
| (53f) | -CONHSO₂CH₃ | -H |
| (54f) | -CONHCH₂CH₂OH | -H |
| (55f) | -CON (CH₃) CH₂CH₂OH | -H |
| (56f) | -CONHCH₂CH₂OCH₃ | -H |
| (57f) | -CONHCH₂CH₂OCH₃ | -CH₃ |
| (58f) | -CON (CH₃) CH₂CH₂OCH₃ | -H |
| (59f) | -CONHCH₂CH₂N (CH₃) ₂ | -H |
| (60f) | -CONHCH₂COOCH₃ | -H |
| (61f) | -CONHCH₂COOH | -H |
| (62f) | -CONHCH₂CN | -H |
| (63f) | -CONHCH₂CONH₂ | -H |
| (64f) | -CONHCH (CO₂CH₃) -CH₂CO₂CH₃ | -H |
| (65f) | -CONHCH (CO₂H)-CH₂CO₂H | -H |
| (66f) | -CH=NOCH₃ | -H |
| (67f) | -NHCSNHCH₃ | -H |
| (68f) | -N=C (-SCH3) NHCH3 | -H |
| (69f) | -CH₂P(=O)(OCH₃) ₂ | -H |
| (70f) | -CH₂P(=O)(OH)₂ | -H |

Among the compounds (V), examples of the compound (Vg) represented by the compound numbers (1g) to (70g) are shown in Tables 55 to 57.

### (Compounds shown in Tables 55 to 57)

The present compound (Vg):

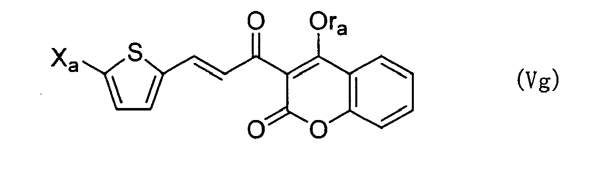

**Table 55**

| Compound No. | Xₐ | rₐ |
|---|---|---|
| (1g) | -CH₂CH₂CN | -H |
| (2g) | 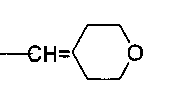 | -H |
| (3g) | -CH=CF₂ | -H |
| (4g) | -CH=CHCN | -H |
| (5g) | -CH=CHCOOCH₃ | -H |
| (6g) | -C≡CC(CH₃)₂OH | -H |
| (7g) | -OCH₂CH₂SCH₃ | -H |
| (8g) | -OCH₂CH₂SOCH₃ | -H |
| (9g) | -OCH₂CH₂SO₂CH₃ | -H |
| (10g) | -CH₂OCH₂CH=CH₂ | -H |
| (11g) | -CH₂OCH₂C=CH | -H |
| (12g) | -CH₂OCH₂COOCH₃ | -H |
| (13g) | -CH₂OCH₂COOCH₃ | -CH₃ |
| (14g) | -CH₂OCH₂COO (CH₂)₉-OH | -H |
| (15g) | -CH₂OCH₂COOH•pyridine | -H |
| (16g) | -CH₂OCH₂COO (Ca)_{0.5} | -(Ca)_{0.5} |
| (17g) | -CH₂OCH₂COONa | -Na |
| (18g) | -CH₂OCH₂COOH | -H |
| (19g) | -CH₂OCH₂CONH₂ | -H |
| (20g) | -CH₂OCH₂CON (CH₃)₂ | -H |
| (21g) | -OCH₂CH₂N(CH₃)₂ | -H |
| (22g) | -OCH₂CH₂CH₂N (CH₃)₂ | -H |
| (23g) | -CH₂OCH₂CH₂NH₂ | -H |
| (24g) | -CH₂OCH₂CH₂NH₂•HCl | -H |
| (25g) | -CH₂OCH₂CH₂NHCOCH₃ | -H |

**Table 56**

| Compound No. | Xₐ | rₐ |
|---|---|---|
| (26g) | -CH₂OCH₂CH₂NHCOOC(CH₃)₃ | -H |
| (27g) | -CH₂OCH₂CH₂OH | -H |
| (28g) | -OCH₂CH₂CH₂OH | -H |
| (29g) | -OCH₂CH₂OCH₃ | -H |
| (30g) | -CH₂OCH₂COCH₃ | -H |
| (31g) | -CH₂OCH₂CN | -H |
| (32g) | -CH₂OCH₂CH₂SO₃H | -H |
| (33g) | -CH₂OCH₂CH₂CH₂SO₃Na | -Na |
| (34g) | -CH₂SCH₂COOCH₃ | -H |
| (35g) | -CH₂SOCH₂COOCH₃ | -H |
| (36g) | -CH₂SO₂CH₂COOCH₃ | -H |
| (37g) | -CH₂SOCH₂COOH | -H |
| (38g) | -CH₂SO₂CH₂COOH | -H |
| (39g) | -NHCH₂CH₂N(CH₃)₂ | -H |
| (40g) | -NHCOOCH₂CH₂OCH₃ | -H |
| (41g) | -NHCONHCH₂CH₂OCH₃ | -H |
| (42g) | -NHCOCOOCH₃ | -H |
| (43g) | -NHCOCOOH | -H |
| (44g) | -NHCOCH=CH₂ | -H |
| (45g) | -NHSO₂CH₂CH=CH₂ | -H |
| (46g) | -NHCOCH₂OCH₃ | -H |
| (47g) | -NHCOCH₂OCH₃ | -CH₃ |
| (48g) | -NHSO₂CH₂COOCH₃ | -H |
| (49g) | -NHSO₂CH₂COOH | -H |
| (50g) | -NHCOCH₂CN | -H |

**Table 57**

| Compound No. | Xₐ | rₐ |
|---|---|---|
| (51g) | -CONHOCH₃ | -H |
| (52g) | -CONHOCH₂CH=CH₂ | -H |
| (53g) | -CONHSO₂CH₃ | -H |
| (54g) | -CONHCH₂CH₂OH | -H |
| (55g) | -CON (CH₃) CH₂CH₂OH | -H |
| (56g) | -CONHCH₂CH₂OCH₃ | -H |
| (57g) | -CONHCH₂CH₂OCH₃ | -CH₃ |
| (58g) | -CON (CH₃) CH₂CH₂OCH₃ | -H |
| (59g) | -CONHCH₂CH₂N (CH₃)₂ | -H |
| (60g) | -CONHCH₂COOCH₃ | -H |
| (61g) | -CONHCH₂COOH | -H |
| (62g) | -CONHCH₂CN | -H |
| (63g) | -CONHCH₂CONH₂ | -H |
| (64g) | -CONHCH (CO₂CH₃) -CH₂CO₂CH₃ | -H |
| (65g) | -CONHCH (CO₂H) -CH₂CO₂H | -H |
| (66g) | -CH=NOCH₃ | -H |
| (67g) | -NHCSNHCH₃ | -H |
| (68g) | -N=C(-SCH₃)NHCH₃ | -H |
| (69g) | -CH₂P(=O)(OCH₃)₂ | -H |
| (70g) | -CH₂P(=O)(OH)₂ | -H |

Among the compounds (V), examples of the compound (Vh) represented by the compound numbers (1b) to (16h) are shown in Table 58 to Table 59.

### (Compounds shown in Table 58 to Table 59)

The compound (Vh):

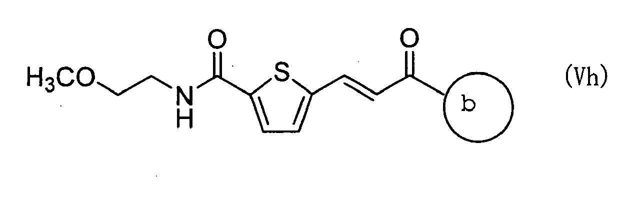

**Table 58**

| Compound No. | 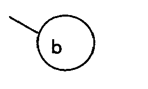 | Compound No. | 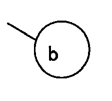 |
|---|---|---|---|
| (1h) | 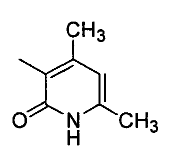 | (9h) | 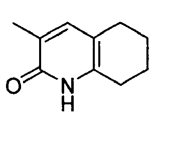 |
| (2h) | 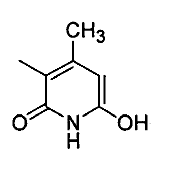 | (10h) | 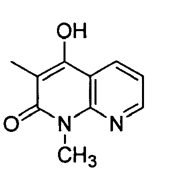 |
| (3h) | 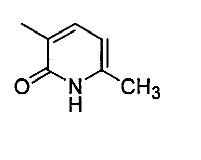 | (11h) | 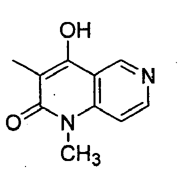 |
| (4h) | 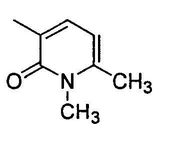 | (12h) | 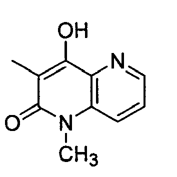 |

**Table 59**

| Compound No. | 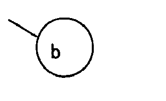 | Compound No. | 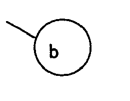 |
|---|---|---|---|
| (5h) | 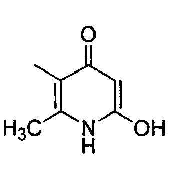 | (13h) | 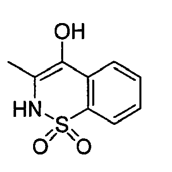 |
| (6h) | 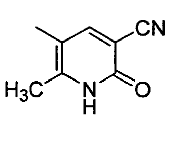 | (14h) | 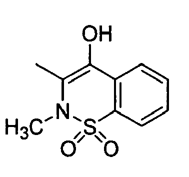 |
| (7h) | 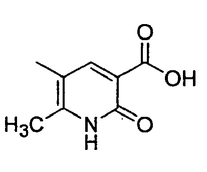 | (15h) | 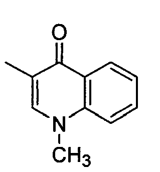 |
| (8h) | 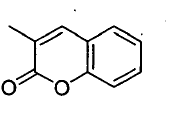 | (16h) | 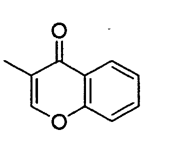 |

Among the compounds (V), examples of the compound (Vi) represented by the compound numbers (1i) to (4i) are shown: in Table 60.

### (Compounds shown in Table 60)

The compound (Vi):

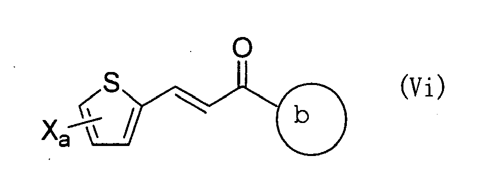

**Table 60**

| | |
|---|---|
| Compound (Vi) Compound No. (1i) | 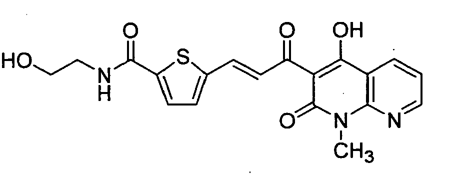 |
| Compound (Vi) Compound No. (2i) | 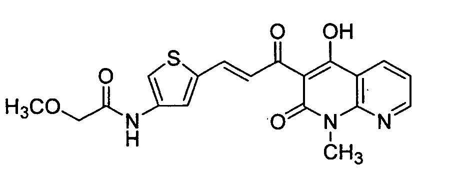 |
| Compound (Vi) Compound No. (3i) | 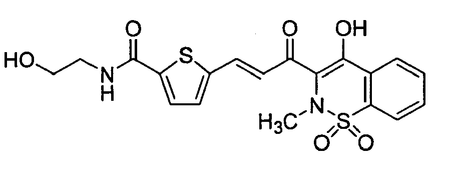 |
| Compound (Vi) Compound No. (4i) | 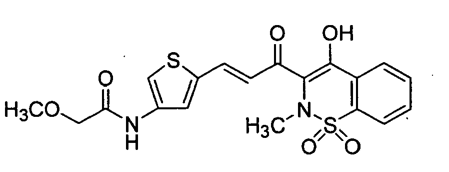 |

The compounds (I) to (VI), (I'), (II') and (V') have the ability to suppress transcription of an extracellular matrix gene such as a Type I collagen gene or a fibronectin gene. The ability is important in improving tissue fibrosis because it decreases expression of an extracellular matrix gene such as a Type I collagen gene or a fibronectin gene to induce a reduction in accumulation of an extracellular matrix such as collagen or fibronectin. Therefore, the compounds (I) to (VI), (I'), (II') and (V') can be utilized as an active ingredient of a composition (medicament, cosmetic, food additive etc.) which can improve tissue fibrosis by decreasing expression of an extracellular matrix gene such as a Type I collagen gene or a fibronectin gene to induce a reduction in accumulation of an extracellular matrix such as collagen or fibronectin.

A disease to which the transcription-suppressing composition of the present invention or the fibrosis-improving composition of the present invention can be applied includes, for example, a disease in which excessive accumulation of an extracellular matrix such as collagen or fibronectin causes fibrosis and then sclerosis of tissues, resulting in decreased function, cicatrization and the like in the tissues such as organs (i.e. fibrosing disease etc.). Specifically, examples of the disease include cirrhosis, chronic pancreatitis, scirrhous gastric cancer, interstitial pulmonary disease, asthma, chronic obstructive pulmonary diseases, glomerular nephritis, lupus nephritis, tubulointerstitial nephritis, IgA nephritis, renal sclerosis, diabetic nephritis, hereditary renal disease, myocardial fibrosis, heart failure, restenosis after PTCA, arterial sclerosis, marrow fibrosis, rheumatoid arthritis, hyperplasia scar after inflammation, postoperative scars or burn scars, atopic dermatitis, hypertrophic scar, hysteromyoma, prostate hypertrophy, scleroderma, Alzheimer disease, sclerotic peritonitis, diabetic retinopathy, I type diabetes, and the like. Incidentally, as an example of cirrhosis, it has been already known that C type or B type hepatitis virus induces chronic inflammation and then increased production of TGF-β, and thereby, hepatic fibrosis (particularly, accumulation of type I and type III collagen) is induced to cause cirrhosis (e.g. see Clin. Liver Dis., 7, 195-210(2003)). As an example of interstitial pulmonary disease, it has been thought that pneumonia caused by mites, viruses, tubercle bacilli or the like induces increased production of TGF-β and then pulmonary fibrosis, and thereby interstitial pulmonary disease is caused. For chronic renal failure such as diabetic nephropathy and IgA nephropathy, it has been already suggested that diabetic nephropathy is caused by increased level of TGF-β in renal glomeruli due to hyperglycemia and thereby induction of renal fibrosis (particularly accumulation of Type I and Type IV collagen), and IgA nephropathy is caused by induction of nephritis due to accumulation of IgA in renal glomeruli followed by increased level of TGF-β, and thereby induction of renal fibrosis (particularly accumulation of Type I and Type IV collagen) (e.g. see Am. J. Physiol. Renal Phsiol., 278, F830-F838(2000), Kidney Int. 64. 149-159(2003)). A db/db mouse, a diabetic nephropathy model animal, develops hyperglycemia by overeating because it has a mutation in a leptin receptor for suppressing ingestion, and then spontaneously develops diabetes. In the db/db mouse, the blood glucose concentration is about 4 times higher than a normal mouse, and fibrosis of renal glomeruli and increased level of TGF-β are found (e.g. see Am. J. Pathol., 158, 1653-1663(2001)). An anti-Thy-1 rat, an IgA nephropathy model animal, is produced by administering an anti-Thy-1 antibody to a normal rat to artificially cause renal fibrosis. It has been shown that renal fibrosis is suppressed by administering an anti-TGF-β receptor antibody to the model animal (e.g. see Kidney Int., 60, 1745-1755(2001)). Although the cause of scleroderma is unknown, it has been found that skin fibrosis is improved by administering a TGF-β inhibitor to a Tsk mouse, which is a model animal therefor (e.g. see J. Invest. Dermatol., 118.461-470(2001)). Thus, a compound which suppresses the activity of TGF-β can be utilized as an active ingredient of a composition (medicament, cosmetic, food additive etc.) for inhibiting the collagen synthesis-promoting activity of TGF-β to suppress tissue fibrosis and thereby providing a fibrosing disease therapeutic effect. On the other hand, it is believed that a cause of heart failure such as left ventricular diastolic failure is cardiac fibrosis under a hypertensive condition. Thus, a compound which suppresses the activity of TGF-β can be utilized as an active ingredient of a composition (medicament etc.) for inhibiting the fibronectin synthesis-promoting activity of TGF-β to suppress tissue fibrosis and thereby providing a heart failure therapeutic effect.

Such transcription-suppressing composition or fibrosis-improving composition of the present invention comprises the compound (I) to (VI), (I'), (II') or (V') and an inert carrier. Such composition usually comprises 0.01% by weight to 99.99% by weight of the compound (I) to (VI), (I'), (II') or (V') and 99.99% by weight to 0.01% by weight of an inert carrier. The inert carrier is a pharmaceutically acceptable carrier or excipient. The transcription-suppressing composition and fibrosis-improving composition of the present invention may further comprise pharmaceutical additives, cosmetic additives, food additives and the like.

The compound (I) to (VI), (I'), (II') or (V') also inhibits the ability of TGF-β to promote transcription of a Type I collagen gene, as shown in Examples 3 and 4 below. That is, the compound (I) to (VI), (I'), (II') or (V') is a TGF-β antagonist having the ability to suppress the activity of TGF-β. Therefore, the compound (I) to (VI), (I'), (II') or (V') can be also utilized as an active ingredient of a composition for suppressing the activity of TGF-β. It has been known that TGF-β has the ability to promote transition from a growth phase (hereinafter, also referred to as hair growth phase in some cases) to a regression phase (hereinafter, also referred to as a hair regression phase in some cases) in the hair life cycle [J. Invest. Dermatol., 111, 948-954(1998), FASEB J., 16, 1967-1969(2002)]. Further, it has been reported that an anti-TGF-β antibody, Fetuin, which is a TGF-β inhibitor, and the like antagonize the suppressing-activity of TGF-β on hair extension and exhibit a promoting-effect on hair extension [J. Invest. Dermaton., 118, 993-997(2002), JP-A 2000-342296]. Therefore, the present compound (and a TGF-β activity-suppressing composition containing the present compound as an active ingredient) may be utilized for inhibiting a promoting effect of TGF-β on transition to a hair regression phase to induce extension of a hair growth phase and thereby providing a hair-growing effect.

Such TGF-β suppressing composition or hair-growing composition of the present invention comprises the compound (I) to (VI), (I'), (II') or (V') and an inert carrier. Such composition usually comprises 0.01% by weight to 99.99% by weight of the compound (I) to (VI), (I'), (II') or (V') and 99.99% by weight to 0.01% by weight of an inert carrier. The inert carrier is a pharmaceutically acceptable carrier or excipient. The TGF-β suppressing composition and hair-growing composition of the present invention may further comprise pharmaceutical additives, cosmetic additives, food additives and the like.

A pharmaceutically acceptable carrier, excipient, pharmaceutical additive, food additive, cosmetic additive, a medicament additive, and the like contained in the above-described composition can be appropriately selected depending on the specific use thereof. In addition, the composition may be in a form of various solids, liquids and the like depending on the specific use thereof.

For example, when the compound (I) to (VI), (I'), (II') or (V') is used as an active ingredient of a medicament, specific examples of the medicament include oral preparations such as powders, fine granules, granules, tablets, syrups, capsules, suspensions, emulsions, extracts and pills; and parenteral preparations such as injections, transdermal absorbing agents such as external liquids and ointments, suppositories and local preparations.

Oral preparations can be prepared using carriers or excipients, and pharmaceutical additives such as binders, disintegrants, surfactants, lubricants, glidants, diluents, preservatives, coloring agents, flavors, stabilizers, humectants, antiseptics, antioxidants and the like, for example, gelatin, sodium alginate, starch, corn starch, white sugar, lactose, glucose, mannit, carboxymethylcellulose, dextrin, polyvinylpyrrolidone, crystalline cellulose, soybean lecithin, sucrose, fatty acid ester, talc, magnesium stearate, polyethylene glycol, magnesium silicate, anhydrous silicic acid and the like, according to a conventional method.

A dose of the oral preparation varies depending on the age, sex and weight of a mammal to be administered, the severity of disease, the kind and dosage form of the composition of the present invention, and the like. Usually, in the case of oral administration, about 1 mg to about 2 g per day, preferably about 5 mg to about 1 g per day of the active ingredient may be administered to an adult human. The daily dose may be also administered at one time or in several divided doses.

Among parenteral preparations, an injection can be prepared using such as a water-soluble solvent such as physiological saline or sterilized water Ringer solution, a water-insoluble solvent such as vegetable oil or fatty acid ester, an isotonic agent such as glucose or sodium chloride, pharmaceutical additives such as a solubilizer, a stabilizer, an antiseptic, a suspending agent and an emulsifying agent, and the like, according to a conventional method. A transdermal absorbing agent such as external liquid or a gel-like ointment, a suppository for rectal administration and the like can be also prepared according to a conventional method. For administering such parenteral preparations, they may be administered by injection (subcutaneously, intravenously etc.), transdermally, or rectally. The local agent can be produced, for example, by incorporating the compound (I) to (VI), (I'), (II') or (V') into a pellet of a sustained-release polymer such as an ethylene vinyl acetate polymer. The pellet may be surgically transplanted into a tissue to be treated.

A dose of the parenteral preparation varies depending on the age, sex and weight of a mammal to be administered, the severity of disease, the kind and dosage form of the composition of the present invention, and the like. Usually, in the case of administration by injection, about 0.1 mg to about 500 mg of the active ingredient may be administered to an adult human. The daily dose may be also administered at one time or in several divided doses.

When the compound (I) to (VI), (I'), (II') or (V') is used by adding to cosmetics, examples of specific forms of cosmetics with the compound added thereto include liquid, emulsion, cream, lotion, ointment, gel, aerosol, mousse and the like. Lotion can be prepared using cosmetic additives such as a suspending agent, an emulsifier, a preservative and the like, according to a conventional method.

A dose of the cosmetic varies depending on the age, sex and weight of a mammal to be administered, the severity of disease, the kind and dosage form of the composition of the present invention, and the like. Usually, about 0.01 mg to about 50 mg of the active ingredient may be administered to an adult human. The daily dose may be also administered at one time or in several divided doses.

When the compound (I) to (VI), (I'), (II') or (V') is used as a food additive, examples of specific forms of a food with the addictive added thereto include powder, a tablet, a beverage, an edible gel or a mixed liquid of the gel and syrup, for example, general beverage and food and luxury food and beverage such as seasonings, Japanese confectionaries, western confectionaries, ice confectionaries, beverage, spreads, pastes, pickles, bottled or canned products, processed domestic animal meats, processed fish meats or marine product, processed dairy or egg products, processed vegetables, processed fruits, processed cereals and the like. Alternatively, the present compound can be also added to feeds or provenders for rearing animals such as livestocks, poultry, honey bee, silkworm, fish and the like.

A dose of the food varies depending on the age, sex and weight of a mammal to be administered, the severity of disease, the kind and dosage form of the composition of the present invention, and the like. Usually, about 0.1 mg to about 500 mg of the active ingredient may be administered to an adult human. The daily dose may be also administered at one time or in several divided doses.

### Examples

The following Examples further illustrate the present invention.

### Example 1

Synthesis of the present aldehyde derivative is described in Examples 1-1 to 1-15.

### Example 1-1

### Synthesis of the present aldehyde derivative [Compound No. (a)]

To a solution of 1.56 g of 5-formylthiophene-2-carboxylic acid in 40 ml of tetrahydrofuran was added 2.11 g of carbonyldiimidazole, and the mixture was stirred at room temperature for 2 hours and 30 minutes. To the mixture was added 1 ml of 2-methoxyethylamine. The mixture was stirred at room temperature for 1 hour and 30 minutes and then concentrated under reduced pressure. The resulting residue was dissolved in 70 ml of ethyl acetate, washed with 2N hydrochloric acid and then an aqueous saturated sodium bicarbonate solution, dried over anhydrous sodium sulfate, and then concentrated under reduced pressure. The resulting residue was subjected to silica gel column chromatography to obtain 0.88 g of 5-formylthiophene-2-carboxylic acid 2-methoxyethylamide [Compound No. (a)] as a white solid.
¹H-NMR(270MHz,DMSO-d₆) δ (ppm): 3.27 (s, 3H), 3.38~3.50 (m, 4H), 7.89 (d, 1H, J=4.1Hz), 8.01 (d, 1H, J=4.1Hz), 8.91 (broad, 1H), 9.95 (s, 1H).

### Example 1-2

### Synthesis of the present aldehyde derivative [Compound No. (b)]

To a solution of 2.86 g of 5-formylfuran-2-carboxylic acid in 30 ml of tetrahydrofuran and 30 ml of dimethylformamide was added 4.22 g of carbonyldiimidazole, and the mixture was stirred at room temperature for 1 hour. To the mixture was added 6 ml of 2-methoxyethylamine, and the mixture was stirred at room temperature overnight. After the mixture was heated under reflux for 5 hours, 2N hydrochloric acid was added thereto, and the mixture was extracted with ethyl acetate. An organic layer was washed with an aqueous saturated sodium chloride solution, dried over anhydrous sodium sulfate, and then concentrated under reduced pressure to obtain 3.01 g of 5-formylfuran-2-carboxylic acid 2-methoxyethylamide [Compound No. (b)] as a white solid.
¹H-NMR (270MHz, DMSO-d₆) δ (ppm): 3.26 (s, 3H), 3.37~3.48 (m, 4H), 7.32 (d, 1H, J=3.6Hz), 7.60 (d, 1H, J=3.6Hz), 8.77 (broad, 1H), 9.70 (s, 1H).

### Example 1-3

### Synthesis of the present aldehyde derivative [Compound No. (c)]

According to the same manner as that of Example 1-1 except that 2.30 g of 5-formyl-1-methyl-1H-pyrrole-2-carboxylic acid was used in place of 5-formylthiophene-2-carboxylic acid, 1.10 g of 5-formyl-1-methyl-1H-pyrrole-2-carboxylic acid 2-methoxyethylamide [Compound No. (c)] was obtained as a yellow crystal.
¹H-NMR (270MHz, DMSO-d₆) δ (ppm): 3.28 (s, 3H), 3.31~3.54 (m, 4H), 4.14 (s, 3H), 6.82 (d, 1H, J=4.1Hz), 7.02 (d, 1H, J=4.1Hz), 8.55 (broad t, 1H, J=4.9Hz), 9.68 (s, 1H).

### Example 1-4

### Synthesis of the present aldehyde derivative [Compound No. (d)]

According to the same manner as that of Example 1-1 except that 2.00 g of 4-formyl-1-methyl-1H-pyrrole-2-carboxylic acid was used in place of 5-formylthiophene-2-carboxylic acid, 0.52 g of 4-formyl-1-methyl-1H-pyrrole-2-carboxylic acid 2-methoxyethylamide [Compound No. (d)] was obtaine as a white crystal.
¹H-NMR (270MHz, CDCl₃) δ (ppm): 3.37 (s, 3H), 3.49~3.65 (m, 4H), 4.00 (s, 3H), 6.38 (broad, 1H), 7.01 (d, 1H, J=1.6Hz), 7.33 (d, 1H,J=1.6Hz), 9.75 (s, 1H).

### Example 1-5

### Synthesis of the present aldehyde derivative [Compound No. (e)

To a solution of 1.56 g of 5-formylthiophene-2-carboxylic acid and 1.15 g of N-hydroxysuccinimide in 15 ml of dimethylformamide was added a solution of 2.06 g of N,N'-dicyclohexylcarbodiimide in 5 ml of dimethylformamide, and the mixture was stirred at room temperature for 30 minutes. Insolubles were filtered and washed with 10 ml of dimethylformamide. To the filtrate was added 1.2 ml of 2-hydroxyethylamine, and the mixture was stirred at room temperature for 1 hour and 30 minutes. Insolubles were filtered, and the filtrate was concentrated under reduced pressure. The residue was dissolved in 50 ml of methanol, and 30 ml of 10% hydrochloric acid was added thereto. The mixture was stirred at room temperature overnight and then concentrated under reduced pressure. The resulting residue was dissolved in 400 ml of ethyl acetate, washed with water, dried over anhydrous sodium sulfate, and then concentrated under reduced pressure to obtain 0.58 g of 5-formylthiophene-2-carboxylic acid 2-hydroxyethylamide [Compound No. (e)] as a yellow solid.
¹H-NMR (270MHz, DMSO-d₆)δ (ppm): 3.29~3.37 (m, 2H), 3.48~3.55 (m, 2H), 4.78 (t, 1H, J=5.7Hz), 7.89 (d, 1H, J=3.9Hz), 8.01 (d, 1H, J=3.9Hz), 8.83 (t, 1H, J=5.4Hz), 9.95 (s, 1H).

### Example 1-6

### Synthesis of the present aldehyde derivative [Compound No. (k)]

To a solution of 0.80 g of 5-formylthiophene-2-carboxylic acid in 10 ml of tetrahydrofuran was added 1.25 g of carbonyldiimidazole, and the mixture was stirred at room temperature for 30 minutes. This reaction solution was added to a mixture of 0.85 g of glycinamide, 1 ml of triethylamine and 10 ml of tetrahydrofuran, and stirred at room temperature overnight. After 10% hydrochloric acid was added thereto, the mixture was extracted with ethyl acetate. An organic layer was washed with an aqueous saturated sodium bicarbonate solution, dried over anhydrous sodium sulfate, and concentrated under reduced pressure. The resulting solid was washed with hexane to obtain 0.21 g of 5-formylthiophene-2-carboxylic acid carbamoylmethylamide [Compound No. (k)] as a yellow solid.
¹H-NMR (270MHz, DMSO-d₆) δ(ppm): 3.82 (d, 2H, J=6.2Hz), 7.09 (broad s, 1H), 7.46 (broad s, 1H), 7.92 (d, 1H, J=4.1Hz), 8.03 (d, 1H, J=4.1Hz), 9.06 (t, 1H, J=5.9Hz), 9.97 (s, 1H).

### Example 1-7

### Synthesis of the present aldehyde derivative [Compound No. (o)]

To a solution of 400 mg of 5-formylthiophene-2-carboxylic acid in dimethylformamide were added 540 mg of 1-ethyl-3-(3-dimethylaminopropyl)carbodiimide hydrochloride and 410 µl of triethylamine, and the mixture was stirred for 30 minutes under ice-cooling. To this reaction solution was added 300 µl of N-(2-methoxyethyl)methylamine, and the mixture was stirred at room temperature. After 10% hydrochloric acid was added thereto, the mixture was extracted with ethyl acetate. An organic layer was washed with an aqueous sodium bicarbonate solution, dried over anhydrous sodium sulfate, and concentrated under reduced under reduced pressure. The resulting residue was washed with ethyl acetate to obtain 221 mg of 5-formylthiophene-2-carboxylic acid N-(2-methoxyethyl)methylamide [Compound No. (o)].
¹H-NMR (300MHz, CDCl₃) δ (ppm): 3.22 (broad s, 3H), 3.42 (s, 3H), 3.55~3.75 (4H), 7.45 (d, 1H, J=3.6Hz), 7.69 (d, 1H, J=3.9Hz), 9.95 (s, 1H).

### Example 1-8

### Synthesis of the present aldehyde derivative [Compound No. (p)]

To a solution of 1.20 g of 5-formylfuran-2-carboxylic acid in 15 ml of tetrahydrofuran and 5 ml of dimethylformamide was added 1.67 g of carbonyldiimidazole, and the mixture was stirred at room temperature. Then 1.11 ml of N-(2-methoxyethyl)methylamine was added thereto, and the mixture was stirred at room temperature for 2 hours and 30 minutes. After the solvent was distilled off and 10% hydrochloric acid was added thereto, the mixture was extracted with ethyl acetate. An organic layer was washed with an aqueous sodium bicarbonate solution and an aqueous saturated sodium chloride solution, dried over anhydrous sodium sulfate, and concentrated under reduced pressure. The resulting residue was subjected to silica gel column chromatography to obtain 5-formylfuran-2-carboxylic acid N-(2-methoxyethyl)methylamide [Compound No. (p)] as a yellow oil.
¹H-NMR (270MHz, CDCl₃) δ (ppm): 3.10 (broad s, 3H), 3.36 (s, 3H), 3.60~3.80 (4H), 7.15~7.25 (1H), 7.25~7.30 (1H), 9.73 (s, 1H).

### Example 1-9

### Synthesis of the present aldehyde derivative [Compound No. (q)]

A solution of 0.95 g of 5-bromomethylthiophene-2-carbaldehyde and 0.31 g of sodium hydroxide in 2.01 g of ethylene glycol was stirred at 55°C for 2 hours. The reaction solution was added to ice water, and then extracted with ethyl acetate. An organic layer was washed with an aqueous saturated sodium chloride solution, dried over anhydrous sodium sulfate, and concentrated under reduced pressure. The resulting residue was subjected to silica gel column chromatography to obtain 0.15 g of 5-[(2-hydroxyethoxy)methyl]thiophene-2-carbaldehyde [Compound No. (q)].
¹H-NMR (270MHz, CDCl₃) δ(ppm): 3.66 (t, 1H, J=4.9Hz), 3.79 (t, 1H, J=4.3Hz), 4.78 (s, 2H), 7.10 (d, 1H, J=3.5Hz), 7.67 (d, 1H, J=4.1Hz), 9.89 (s, 1H).

### Example 1-10

### Synthesis of the present aldehyde derivative [Compound No. (r)]

A solution of 3.52 g of 5-bromomethylthiophene-2-carbaldehyde, 2.74 g of methyl thioglycolate and 2.84 g of potassium carbonate in 22 ml of dimethyl sulfoxide was stirred at room temperature for 2 hours. The reaction solution was added to ice water, and then extracted with ethyl acetate. An organic layer was washed with an aqueous saturated sodium chloride solution, dried over anhydrous sodium sulfate, and concentrated under reduced pressure. The resulting residue was subjected to silica gel column chromatography to obtain 3.16 g of methyl [(5-formylthiophen-2-yl)methylthio]acetate [Compound No. (r)]. ¹H-NMR (270MHz, DMSO-d₆) δ (ppm): 3.36 (s, 3H), 3.62 (s, 2H), 4.15 (s, 2H), 7.21 (d, 1H, J=3.8Hz), 7.89 (d, 1H, J=4.1Hz), 9.86 (s, 1H).

### Example 1-11

### Synthesis of the present aldehyde derivative [Compound No. (s)]

A mixture of 8.21 g of 4-bromothiophene-2-carbaldehyde, 4.60 g of methoxyacetamide, 380 mg of N,N'-dimethylethylenediamine, 25.57 g of potassium carbonate, 829 mg of copper(I) iodide and 55 ml of dioxane was heated under reflux for 5 hours and 45 minutes. The reaction solution was subjected to silica gel column chromatography to obtain 2.52 g of N-(2-formylthiophen-4-yl)-2-methoxyacetamide [Compound No. (s)] as pale yellow powder. ¹H-NMR (270MHz, CDCl₃) δ (ppm): 3.52 (s, 3H), 4.05 (s, 2H), 7.87 (d, 1H, J=1.6Hz), 7.93 (d, 1H, J=1.6Hz), 8.57 (broad s, 1H), 9.78 (s, 1H).

### Example 1-12

### Synthesis of the present aldehyde derivative [Compound No. (t)]

According to the same manner as that of Example 1-11 except that 2.9 g of 2-bromothiophene-5-carbaldehyde was used in place of 4-bromothiophene-2-carbaldeyde, 0.11 g of N-(5-formylthiophen-2-yl)-2-methoxyacetamide [Compound No. (t)] was obtained.
¹H-NMR (270MHz, DMSO-d₆) δ (ppm): 3.37 (s, 3H), 4.15 (s, 2H), 7.01 (d, 1H, J=4.3Hz), 7.81 (d, 1H, J=4.3Hz), 9.78 (s, 1H), 11.72 (broad, 1H).

### Example 1-13

### Synthesis of the present aldehyde derivative [Compound No. (u)]

According to the same manner as that of Example 1-11 except that 2.02 g of 2-methoxyethyl carbamate was used in place of methoxyacetamide, 0.12 g of 2-methoxyethyl N-(2-formylthiophen-4-yl)carbamate [Compound No. (u)] was obtained.
¹H-NMR (300MHz, DMSO-d₆) δ (ppm): 3.28 (s, 3H), 3.57 (t, 2H, J=4.5Hz), 4.22 (t, 2H, J=4.5Hz), 7.72 (s, 1H), 7.85 (s, 1H), 9.88 (s, 1H), 10.31 (broad, 1H).

### Example 1-14

### Synthesis of the present aldehyde derivative [Compound No. (v)]

According to the same manner as that of Example 1-11 except that 3.76 g of 2-bromothiophene-5-carbaldehyde was used in place of 4-bromothiophene-2-carbaldehyde and 2.81 g of 2-methoxyethyl carbamate was used in place of methoxyacetamide, 0.15 g of 2-methoxyethyl N-(5-formylthiophen-2-yl)carbamate [Compound No. (v)] was obtained.
¹H-NMR (300MHz, DMSO-d₆) δ (ppm): 3.28 (s, 3H), 3.57~3.60 (2H), 4.27~4.30 (2H), 6.69 (d, 1H, J=4.2Hz), 7.78 (d, 1H, J=3.9Hz), 9.72 (s, 1H), 11.55 (broad, 1H).

### Example 1-15

### Synthesis of the present aldehyde derivative [Compound No. (w)]

To a solution of 2.50 g of 2-amino-5-formylthiazole and 2.44 g of triethylamine in 30 ml of tetrahydrofuran was added dropwise a solution of 2.22 g of methoxyacetyl chloride in 8 ml of tetrahydrofuran under ice-cooling, and the mixture was stirred at room temperature for 2 hours and 30 minutes. Insolubles were filtered, and the filtrate was added to ice water and then extracted with ethyl acetate. An organic layer was washed with 10% hydrochloric acid and an aqueous saturated sodium chloride solution, dried over anhydrous magnesium sulfate, and concentrated under reduced pressure to obtain 2.83 g of N-(5-formylthiazol-2-yl)-2-methoxyacetamide [Compound No. (w)] as pale yellow powder. ¹H-NMR (270MHz, DMSO-d₆) δ (ppm): 3.36 (s, 3H), 4.22 (s, 2H), 8.42 (s, 1H), 9.97 (s, 1H).

### Example 2

Synthesis of the present compound is described in Examples 2-1 to 2-27.

### Example 2-1

### Synthesis of the present compound [Compound No. (27a-1)]

In a mixture of 2 ml of methanol and 0.02 g of piperidine were dissolved 0.14 g of 3-acetyl-4-hydroxy-1,6-dimethyl-2(1H)-pyridinone and 0.15 g of 5-[(2-hydroxyethoxy)methyl]thiophene-2-carbaldehyde. The solution was heated under reflux for 2 hours and 30 minutes. After cooled to room temperature, the reaction solution was concentrated and then subjected to silica gel column chromatography. The resulting crystal was recrystallized from hexane and ethyl acetate to obtain 0.13 g of 4-hydroxy-3-[3-[5-[(2-hydroxyethoxy)methyl]thiophen-2-yl]-1-oxo-2-propenyl]-1,6-dimethyl-2(1H)-pyridinone [Compound No. (27a-1)] as a tan crystal.
¹H-NMR (270MHz, DMSO-d₆) δ (ppm): 2.40 (s, 3H), 3.40 (s, 3H), 3.49~3.55 (m, 4H), 4.70 (s, 2H), 6.04 (s, 1H), 7.10 (d, 1H, J=4.1Hz), 7.46 (d, 1H, J=3.8Hz), 7.96 (d, 1H, J=15.1Hz), 8.28 (d, 1H, J=15.4Hz), 13.78 (broad, 1H).

### Example 2-2

### Synthesis of the present compound [Compound No. (34a-1)]

According to the same manner as that of Example 2-1 except that 2.56 g of methyl [(5-formylthiophen-2-yl)methylthio]acetate was used in place of 5-[(2-hydroxyethoxy)methyl]thiophene-2-carbaldehyde, 1.22 g of methyl [[5-[3-(4-hydoxy-1,6-dimethyl-2-oxo-1,2-dihydropyridin-3-yl)-3-oxo-1-propenyl]thiophen-2-yl]methylthio]acetate [Compound No. (34a-1)] was obtained as a tan crystal.
¹H-NMR (270MHz, DMSO-d₆) δ (ppm): 2.40 (s, 3H), 3.29 (s, 2H), 3.40 (s, 3H), 3.63 (s, 3H), 4.10 (s, 2H), 6.04 (s, 1H) , 7.05 (d, 1H, J=3.8Hz), 7.43 (d, 1H, J=3.2Hz), 7.94 (d, 1H, J=15.4Hz), 8.26(d, 1H, J=15.7Hz), 16.23 (broad s, 1H).

### Example 2-3

### Synthesis of the present compound [Compound No. (35a-1)]

To a solution of 0.51 g of methyl [[5-[3-(4-hydroxy-1,6-dimethyl-2-oxo-1,2-dihydropyridin-3-yl)-3-oxo-1-propenyl]thiophen-2-yl]metylthio]acetate in 10 ml of methylene chloride was added 0.24 g of m-chloroperbenzoic acid under ice-cooling, and the mixture was stirred under ice-cooling. The reaction solution was concentrated under reduced pressure, and ethyl acetate was added thereto. An organic layer was washed with an aqueous sodium hydrogen carbonate solution and an aqueous saturated sodium chloride solution, and concentrated under reduced pressure. The residue was subjected to silica gel column chromatography to obtain 0.20 g of methyl [[5-[3-(4-hydroxy-1,6-dimethyl-2-oxo-1,2-dihydropyridin-3-yl)-3-oxo-1-propenyl]thiophen-2-yl]methylsulfinyl]acetate [Compound No. (35a-1)] as a yellow crystal.
¹H-NMR (270MHz, DMSO-d₆) δ (ppm): 2.40 (s, 3H), 3.40 (s, 3H), 3.70 (s, 3H), 3.73 (d, 1H, J=12.4Hz), 4.02 (d, 1H, J=14.3Hz), 4.43 (d, 1H, J=13.8Hz), 4.59 (d, 1H, J=13.8Hz), 6.04 (s, 1H), 7.14 (d, 1H, J=3.5Hz), 7.53 (d, 1H, J=3.8Hz), 7.95 (d, 1H, J=15.7Hz), 8.31 (d, 1H, J=15.7Hz), 16.18 (s, 1H).

### Example 2-4

### Synthesis of the present compound [Compound No. (36a-1)]

To a solution of 0.49 g of methyl [[5-[3-(4-hydroxy-1,6-dimethyl-2-oxo-1,2-dihydropyridin-3-yl)-3-oxo-1-propenyl]thiophen-2-yl]methylthio]acetate in 10 ml of methylene chloride was added 0.60 g of m-chloroperbenzoic acid under ice-cooling, and the mixture was stirred for 3 hours under ice-cooling. The reaction solution was treated in the same manner as desccribed in Example 2-3 to obtain 0.28 g of methyl [[5-[3-(4-hydroxy-1,6-dimethyl-2-oxo-1,2-dihydropyridin-3-yl)-3-oxo-1-propenyl]thiophen-2-yl]methylsulfonyl]acetate [Compound No. (36a-1)] as a yellow crystal.
¹H-NMR (270MHz, DMSO-d₆) δ (ppm): 2.41 (s, 3H), 3.40 (s, 3H), 3.74 (s, 3H), 4.44 (s, 2H), 5.00 (s, 2H), 6.05 (s, 1H), 7.22 (d, 1H, J=3.8Hz), 7.53 (d, 1H, J=3.5Hz), 7.96 (d, 1H, J=1.5.7Hz), 8.33 (d, 1H, J=15.7Hz), 16.11 (s, 1H).

### Example 2-5

### Synthesis of the present compound [Compound No. (37a-1)]

To a solution of 0.17 g of methyl [[5-[3-(4-hydroxy-1,6-dimethyl-2-oxo-1,2-dihydropyridin-3-yl)-3-oxo-1-propenyl]thiophen-2-yl]methylsulfinyl]acetate in 5 ml of methanol was added 5 ml of a 1N aqueous sodium hydroxide solution. The mixture was stirred at room temperature. The solvent was distilled off under reduced pressure, and the residue was acidified with 10% hydrochloric acid. Precipitated crystals were filtered and dried to obtain 0.09 g of [[5-[3-(4-hydroxy-1,6-dimethyl-2-oxo-1,2-dihydropyridin-3-yl)-3-oxo-1-propenyl]thiophen-2-yl]methylsulfinyl]acetic acid [Compound No. (37a-1)] as a brown crystal.
¹H-NMR (270MHz, DMSO-d₆) δ (ppm): 2.40 (s, 3H), 3.40 (s, 3H), 3.88~4.04 (2H), 4.36~4.58 (2H), 6.04 (s, 1H), 7.13 (d, 1H, J=3.5Hz), 7.53 (d, 1H, J=3.8Hz), 7.96 (d, 1H, J=15.7Hz), 8.32 (d, 1H, J=15.4Hz), 16.19 (1H).

### Example 2-6

### Synthesis of the present compound [Compound No. (38a-1)]

According to the same manner as that of Example 2-5 except that 0.22 g of methyl [[5-[3-(4-hydroxy-1,6-dimethyl-2-oxo-1,2-dihydropyridin-3-yl)-3-oxo-1-propenyl]thiophen-2-yl]methylsulfonyl]acetate was used in place of methyl [[5-[3-(4-hydroxy-1,6-dimethyl-2-oxo-1,2-dihydropyridin-3-yl)-3-oxo-1-propenyl]thiophen-2-yl]methylsulfinyl]acetate, 0.18 g of [[5-[3-(4-hydroxy-1,6-dimethyl-2-oxo-1,2-dihydropyridin-3-yl)-3-oxo-1-propenyl]thiophen-2-yl]methylsulfonyl]acetic acid [Compound No. (38a-1)] was obtained as a tan crystal. ¹H-NMR (270MHz, DMSO-d₆) δ (ppm): 2.40 (s, 3H), 3.40 (s, 3H), 4.28 (s, 2H), 4.97 (s, 2H), 6.05 (s, 1H) , 7.22 (d, 1H, J=3.5Hz), 7.53 (d, 1H, J=4.1Hz), 7.96 (d, 1H, J=15.7Hz), 8.33 (d, 1H, J=15.7Hz).

### Example 2-7

### Synthesis of the present compound [Compound No. (40a-1)]

According to the same manner as that of Example 2-1 except that 0.15 g of 2-methoxyethyl N-(5-formylthiophen-2-yl)carbamate was used in place of 5-[(2-hydoxyethoxy)methyl]thiophene-2-carbaldehyde and 3 ml of ethanol was used in place of methanol, 0.16 g of 2-methoxyethyl [5-[3-(4-hydroxy-1,6-dimethyl-2-oxo-1,2-dihydropyridin-3-yl)-3-oxo-1-propenyl]thiophen-2-yl]carbamate [Compound No. (40a-1)] was obtained as a red crystal.
¹H-NMR (300MHz, DMSO-d₆) δ (ppm): 2.39 (s, 3H), 3.29 (s, 3H), 3.40 (s, 3H), 3.58~3.60 (2H), 4.27~4.30 (2H), 6.05 (s, 1H), 6.59 (d, 1H, J=3.9Hz), 7.37 (d, 1H, J=4.2Hz), 7.95 (d, 1H, J=15.3Hz), 8.18 (d, 1H, J=15.6Hz), 11.51 (broad, 1H), 16.59 (s, 1H).

### Example 2-8

### Synthesis of the present compound [Compound No. (46a-1)]

According to the same manner as that of Example 2-1 except that 0.11 g of N-(5-formylthiophen-2-yl)-2-methoxyacetamide was used in place of 5-[(2-hydroxyethoxy)methyl]thiophene-2-carbaldehyde and 2 ml of ethanol was used in place of methanol, 0.11 g of 4-hydroxy-3-[3-[5-(methoxyacetylamino)thiophen-2-yl]-1-oxo-2-propenyl]-1,6-dimethyl-2(1H)-pyridinone [Compound No. (46a-1)] was obtained as a red brown crystal.
¹H-NMR (270MHz, DMSO-d₆) δ (ppm): 2.40 (s, 3H), 3.40 (s, 6H), 4.14 (s, 2H), 6.01 (s, 1H), 6.90 (d, 1H, J=3.8Hz), 7.40 (d, 1H, J=4.1Hz), 7.97 (d, 1H, J=15.1Hz), 8.21 (d, 1H, J=15.7Hz).

### Example 2-9

### Synthesis of the present compound [Compound No. (54a-1)]

In a mixture of 6 ml of ethanol and 84 µl of piperidine were dissolved 440 mg of 3-acetyl-4-hydroxy-1,6-dimethyl-2(1H)-pyridinone and 569 mg of 5-formylthiophene-2-carboxylic acid 2-hydroxyethylamide, and the solution was heated under reflux for 5 hours. After cooled to room temperature, precipitated crystals were filtered and washed with ethanol and hexane to obtain 678 mg of 5-[3-(4-hydroxy-1,6-dimethyl-2-oxo-1,2-dihydropyridin-3-yl)-3-oxo-1-propenyl]thiophene-2-carboxylic acid 2-hydroxyethylamide [Compound No. (54a-1)] as a yellow crystal. ¹H-NMR (270MHz, DMSO-d₆) δ (ppm): 2.40 (s, 3H), 3.24 (s, 3H), 3.20~3.40 (m, 2H), 3.45~3.55 (m, 2H), 4.77 (t, 1H, J=5.4Hz), 6.05 (s, 1H), 7.55 (d, 1H, J=4.1Hz), 7.76 (d, 1H, J=4.1Hz), 7.91 (d, 1H, J=15.4Hz), 8.37 (d, 1H, J=15.4Hz), 8.65 (t, 1H, J=5.4Hz), 15.95 (broad, 1H).

### Example 2-10

### Synthesis of the present compound [Compound No. (56a-1)]

According to the same manner as that of Example 2-9 except that 0.30 g of 5-formylthiophene-2-carboxylic acid 2-methoxyethylamide was used in place of 5-formylthiophene-2-carboxylic acid 2-hydroxyethylamide, 0.27 g of 5-[3-(4-hydroxy-1,6-dimethyl-2-oxo-1,2-dihydropyridin-3-yl)-3-oxo-1-propenyl]thiophene-2-carboxylic acid 2-methoxyethylamide [Compound No. (56a-1)] was obtained as a yellow crystal. ¹H-NMR (270MHz, DMSO-d₆) δ (ppm): 2.41 (s, 3H), 3.27 (s, 3H), 3.30 (s, 3H), 3.40~3.53 (m, 4H), 6.06 (s, 1H), 7.56 (d, 1H, J=4.1Hz), 7.78 (d, 1H, J=4.1Hz), 7.93 (d, 1H, J=16.9Hz), 8.38 (d, 1H, J=16.9Hz), 8.74 (t, 1H, J=5.0Hz), 14.00 (broad s, 1H).

### Example 2-11

### Synthesis of the present compound [Compound No. (58a-1)]

According to the same manner as that of Example 2-1 except that 150 mg of 5-formylthiophene-2-carboxylic acid N-(2-methoxyethyl)methylamide was used in place of 5-[(2-hydroxyethoxy)methyl]thiophene-2-carbaldehyde and ethanol was used in place of methanol, 113 mg of [5-[3-(4-hydroxy-1,6-dimethyl-2-oxo-1,2-dihydropyridin-3-yl)-3-oxo-1-propenyl]thiophen-2-yl]carboxylic acid N-(2-methoxyethyl)methylamide [Compound No. (58a-1)] was obtained as a yellow crystal.
¹H-NMR (270MHz, DMSO-d₆) 5 (ppm): 2.40 (s, 3H), 3.14 (3H), 3.27 (s, 3H), 3.40 (s, 3H), 3.55 (t, 2H, J=4.9Hz), 3.65 (t, 2H, J=4.9Hz), 6.05 (s, 1H), 7.51 (d, 1H, J=4.1Hz), 7.54 (d, 1H, J=3.8Hz), 7.94 (d, 1H, J=15.4Hz), 8.35 (d, 1H, J=15.4Hz), 14.00 (broad s, 1H).

### Example 2-12

### Synthesis of the present compound [Compound No. (63a-1)]

According to the same manner as that of Example 2-9 except that 100 mg of 5-formylthiophene-2-carboxylic acid carbamoylmethylamide was used in place of 5-formylthiophene-2-carboxylic acid 2-hydroxyethylamide, 77.6 mg of 5-[3-(4-hydroxy-1,6-dimethyl-2-oxo-1,2-dihydropyridin-3-yl)-3-oxo-1-propenyl]thiophene-2-carboxylic acid carbamoylmethyamide [Compound No. (63a-1)] was obtained as a yellow crystal.
¹H-NMR (270MHz, DMSO-d₆) 5 (ppm): 2.41 (s, 3H), 3.40 (s, 3H), 3.41 (d, 1H, J=5.9Hz), 6.05 (s, 1H), 7.07 (broad, 1H), 7.43 (broad, 1H), 7.57 (d, 1H, J=4.0Hz), 7.79 (d, 1H, J=3.9Hz), 7.93 (d, 1H, J=15.7Hz), 8.39 (d, 1H, J=15.4Hz), 8.88 (t, 1H, J=5.9Hz), 15.97 (broad, 1H).

### Example 2-13

### Synthesis of the present compound [Compound No. (71a-1)]

According to the same manner as that of Example 2-5 except that 0.10 g of methyl [[5-[3-(4-hydroxy-1,6-dimethyl-2-oxo-1,2-dihydropyridin-3-yl)-3-oxo-1-propenyl]thiophen-2-yl]methylthio]acetate was used in place of methyl [[5-[3-(4-hydroxy-1,6-dimethyl-2-oxo-1,2-dihydropyridin-3-yl)-3-oxo-1-propenyl]thiophen-2-yl]methylsulfinyl]acetate, 0.09 g of [[5-[3-(4-hydroxy-1,6-dimethyl-2-oxo-1,2-dihydropyridin-3-yl)-3-oxo-1-propenyl]thiophen-2-yl]methythio]acetic acid [Compound No. (71a-1)] was obtained as a tan crystal.
¹H-NMR (270MHz, DMSO-d₆) δ (ppm): 2.40 (s, 3H), 3.25 (s, 2H), 3.40 (s, 3H), 4.10 (s, 2H), 6.04 (s, 1H), 7.05 (d, 1H, J=3.8Hz), 7.43 (d, 1H, J=3.5Hz), 7.95 (d, 1H, J=15.7Hz), 8.26 (d, 1H, J=15.7Hz), 16.24 (s, 1H).

### Example 2-14

### Synthesis of the present compound [Compound No. (72a-1)]

According to the same manner as that of Example 2-1 except that 0.32 g of 5-[N-(2-dimethylaminoethyl)methylamino]thiophene-2-carbaldehyde was used in place of 5-[(2-hydroxyethoxy)methyl]thiophene-2-carbaldehyde and ethanol was used in place of methanol, 80 mg of 4-hydroxy-3-[3-[5-[N-(2-dimethylaminoethyl)methylamino]thiophen-2-yl]-1-oxo-2-propenyl]-1,6-dimethyl-2(1H)-pyridinone [Compound No. (72a-1)] was obtained as a red brown crystal.
¹H-NMR (270MHz, DMSO-d₆) δ (ppm): 2.19 (s, 6H), 2.36 (s, 3H), 2.45~2.55 (2H), 3.09 (s, 3H), 3.36 (s, 3H), 3.51 (t, 2H, J=8.1Hz), 5.92 (s, 1H), 6.09 (d, 1H, J=5.4Hz), 7.40 (d, 1H, J=5.4Hz), 7.84 (d, 1H, J=16.2Hz), 7.96 (d, 1H, J=16.2Hz).

### Example 2-15

### Synthesis of the present compound [Compound No. (10a-2)]

According to the same manner as that of Example 2-9 except that 0.59 g of 5-formylfuran-2-carboxylic acid 2-methoxyethylamide was used in place of 5-formylhthiophene-2-carboxylic acid 2-hydoxyethylamide, 0.82 g of 5-[3-(4-hydroxy-1,6-dimethyl-2-oxo-1,2-dihydropyridin-3-yl)-3-oxo-1-propenyl]furan-2-carboxylic acid 2-methoxyethylamide [Compound No. (10a-2)] was obtained as a yellow crystal.
¹H-NMR (270MHz, DMSO-d₆) δ (ppm): 2.41 (s, 3H), 3.28 (s, 3H), 3.41~3.47 (m, 7H), 6.05 (s, 1H), 7.10 (d, 1H, J=3.6Hz), 7.26 (d, 1H, J=3.6Hz), 7.61 (d, 1H, J=15.7Hz), 8.35 (d, 1H, J=15.7Hz), 8.60 (broad, 1H), 15.95 (broad s, 1H).

### Example 2-16

### Synthesis of the present compound [Compound No. (12a-2)]

According to the same manner as that of Example 2-1 except that 150 mg of 5-formylfuran-2-carboxylic acid N-(2-methoxyethyl)ethylamide was used in place of 5-[(2-hydroxyethoxy)methyl]thiophene-2-carbaldehyde and ethanol was used in place of methanol, 57 mg of [5-[3-(4-hydroxy-1,6-dimethyl-2-oxo-1,2-dihydropyridin-3-yl)-3-oxo-1-propenyl]furan-2-yl]carboxylic acid N-(2-methoxyethyl)methylamide [Compound No. (12a-2)] was obtained as a yellow crystal.
¹H-NMR (270MHz, DMSO-d₆) δ (ppm): 2.40 (s, 3H), 3.02 (broad s, 3H), 3.28 (s, 3H), 3.40 (s, 3H), 3.50~3.75 (4H), 6.05 (s, 1H), 7.09~7.15 (2H), 7.63 (d, 1H, J=15.7Hz), 8.39 (d, 1H, J=15.9Hz), 16.04 (s, 1H).

### Example 2-17

### Synthesis of the present compound [Compound No. (1a-3)]

According to the same manner as that of Example 2-9 except that 1.17 g of 5-formyl-1-methyl-1H-pyrrole-2-carboxylic acid 2-methoxyethylamide was used in place of 5-formylthiophene-2-carboxylic acid 2-hydroxyethylamide, 0.72 g of 5-[3-(4-hydroxy-1,6-dimethyl-2-oxo-1,2-dihydropyridin-3-yl)-3-oxo-1-propenyl]-1-methyl-1H-pyrrole-2-carboxylic acid 2-methoxyethylamide [Compound No. (1a-3)] was obtained as a yellow crystal.
¹H-NMR (270MHz, DMSO-d₆) δ (ppm): 2.39 (s, 3H), 3.27 (s, 3H), 3.41 (s, 3H), 3.43~3.46 (m, 4H), 3.99 (s, 3H), 6.02 (s, 1H), 6.76 (d, 1H, J=4.3Hz), 6.86 (d, 1H, J=4.3Hz), 7.82 (d, 1H, J=15.5Hz), 8.20 (t, 1H, J=4.8Hz), 8.37 (d, 1H, J=15.5Hz).

### Example 2-18

### Synthesis of the present compound [Compound No. (2a-3)]

According to the same manner as that of Example 2-9 except that 361 mg of 4-formyl-1-methyl-1H-pyrrole-2-carboxylic acid 2-methoxyethylamide was used in place of 5-formylthiophene-2-carboxylic acid 2-hydroxyethylamide, 506 mg of 4-[3-(4-hydroxy-1,6-dimethyl-2-oxo-1,2-dihydropyridin-3-yl)-3-oxo-1-propenyl]-1-methyl-1H-pyrrole-2-carboxylic acid 2-methoxyethylamide [Compound No. (2a-3)] was obtained as a yellow crystal.
¹H-NMR (270MHz, DMSO-d₆) δ (ppm): 2.39 (s, 3H), 3.23 (s, 3H), 3.33 (s, 3H), 3.89 (s, 3H), 3.30~3.50 (m, 4H), 5.99 (s, 1H), 7.25 (d, 1H, J=1.9Hz), 7.46 (d, 1H, J=1.9Hz), 7.76 (d, 1H, J=15.4Hz), 8.24 (d, 1H, J=15.7Hz), 8.37 (t, 1H, J=5.4Hz), 16.71 (broad s, 1H).

### Example 2-19

### Synthesis of the present compound [Compound No. (9a-3)]

According to the same manner as that of Example 2-1 except that 0.15 g of N-(5-formylthiazol-2-yl)-2-methoxyacetamide was used in place of 5-[(2-hydroxyethoxy)methyl]thiophene-2-carbaldehyde and ethanol was used in place of methanol, 0.13 g of 4-hydroxy-3-[3-[2-(methoxyacetylamino)thiazol-5-yl]-1-oxo-2-propenyl]-1,6-dimethyl-2(1H)-pyridinone [Compound No. (9a-3)] was obtained as a yellow crystal.
¹H-NMR (270MHz, DMSO-d₆) δ (ppm): 2.40 (s, 3H), 3.37 (s, 3H), 3.41 (s, 3H), 4.21 (s, 2H), 6.04 (s, 1H), 7.98 (s, 1H), 8.02 (d, 1H, J=18.9Hz), 8.22 (d, 1H, J=16.2Hz), 12.49 (broad s, 1H), 16.25 (s, 1H).

### Example 2-20

### Synthesis of the present compound [Compound No. (10a-3)]

According to the same manner as that of Example 2-1 except that 0.15 g of N-(2-formylthiophen-4-yl)-2-methoxyacetamide was used in place of 5-[(2-hydroxyethoxy)methyl]thiophene-2-carbaldehyde and 2 ml of ethanol was used in place of methanol, and 2 ml of ethanol was used in place of methanol, 0.20 g of 4-hydroxy-3-[3-[4-(methoxyacetylamino)thiophen-2-yl]-1-oxo-2-propenyl]-1,6-dimethyl-2(1H)-pyridinone [Compound No. (10a-3)] was obtained as a tan crystal. ¹H-NMR (300MHz, DMSO-d₆) δ (ppm): 2.40 (s, 3H), 3.38 (s, 3H), 3.40 (s, 3H), 4.01 (s, 2H), 6.04 (s, 1H), 7.69 (s, 1H), 7.79 (s, 1H), 7.92 (d, 1H, J=15.6Hz), 8.34 (d, 1H, J=15.6Hz), 10.34 (s, 1H).

### Example 2-21

### Synthesis of the present compound [Compound No. (11a-3)]

According to the same manner as that of Example 2-1 except that 0.12 g of 2-methoxyethyl N-(2-formylthiophen-4-yl)carbamate was used in place of 5-[(2-hydroxyethoxy)methyl]thiophene-2-carbaldehyde and 2 ml of ethanol was used in place of methanol, 0.09 g of 2-methoxyethyl [4-[3-(4-hydroxy-1,6-dimethyl-2-oxo-1,2-dihydoropyridin-3-yl)-3-oxo-1-propenyl]thiophen-2-yl]carbamate [Compound No. (11a-3)] was obtained as a tan crystal.
¹H-NMR (270MHz, DMSO-d₆) δ (ppm): 2.40 (s, 3H), 3.28 (s, 3H), 3.39 (s, 3H), 3.56 (t, 2H, J=5.4Hz), 4.21 (t, 2H, J=5.4Hz), 6.04 (s, 1H), 7.39 (s, 1H), 7.44 (s, 1H), 7.90 (d, 1H, J=13.5Hz), 8.31 (d, 1H, J=16.2Hz), 10.16 (broad, 1H), 16.18 (s, 1H).

### Example 2-22

### Synthesis of the present compound [Compound No. (10h)]

To a solution of 0.10 g of 5-formylthiophene-2-carboxylic acid 2-methoxyethylamide in 3 ml of ethanol were added 0.10 g of 3-acetyl-4-hydroxy-1-methyl-1H-[1,8]naphthyridine-2-one and 0.015 ml of piperidine, and the mixture was heated under reflux for 4 hours. After cooled to room temperature, precipitated crystals were filtered and washed with ethyl acetate and hexane to obtain 0.13 g of 4-hydroxy-1-methyl-3-[3-[5-[(2-methoxyethyl)aminocarbonyl]thiophen-2-yl]acryloyl]-1H-[1,8]naphthyridine-2-one [Compound No. (10h)] as a tan crystal.
¹H-NMR (270MHz, DMSO-d₆) δ (ppm): 3.28 (s, 3H), 3.41~3.48 (m, 4H), 3.65 (s, 3H), 7.35~7.42 (1H), 7.63~7.65 (1H), 7.79~7.81 (1H), 8.08 (d, 1H, J=15.9Hz), 8.41~8.51 (1H), 8.47 (dd, 1H, J=1.9, 5.9Hz), 8.77~8.78 (2H), 17.75 (broad, 1H).

### Example 2-23

### Synthesis of the present compound [Compound No. (14h)]

To a solution of 500 mg of 5-formylthiophene-2-carboxylic acid 2-methoxyethylamide in 8 ml of ethanol were added 593 mg of 3-acetyl-4-hydroxy-2-methyl-2H-1,2-benzothiazine-1,1-dioxde and 60 mg of piperidine, and the mixture was heated under reflux for 8 hours. After cooled to room temperature, precipitated crystals were filtered and washed with methanol to obtain 777 mg of 4-hydroxy-3-[3-[5-[(2-methoxyethyl)aminocarbonyl]thiophen-2-yl]acryloyl]-2-methyl-2H-1,2-benzothiazine-1,1-dioxide [Compound No. (14h)] as an orange crystal.
¹H-NMR (270MHz, DMSO-d₆) δ (ppm): 2.95 (s, 3H), 3.28 (s, 3H), 3.41~3.46 (m, 4H), 7.18 (d, 1H, J=13.5Hz), 7.74~7.79 (broad, 1H), 7.83 (d, 1H, J=2.7Hz), 7.93~8.06 (m, 4H), 8.14~8.17 (m, 1H), 8.74~8.79 (m, 1H).

### Example 2-24

### Synthesis of the present compound [Compound No. (1i)]

According to the same manner as that of Example 2-22 except that 111 mg of 5-formylthiophene-2-carboxylic acid 2-hydroxyethylamide was used in place of 5-formylthiophene-2-carboxylic acid 2-methoxyethylamide, 151 mg of 4-hydroxy-1-methyl-3-[3-[5-[(2-hydroxyethyl)aminocarbonyl]thiophen-2-yl]acryloyl]-1H-[1,8]naphthyridine-2-one [Compound No. (1i)] was obtained as a yellow crystal.
¹H-NMR (270MHz, DMSO-d₆) δ (ppm): 3.32 (q, 2H, J=5.4Hz), 3.45~3.55 (2H), 3.62 (s, 3H), 4.85 (broad, 1H), 7.35 (dd, 1H, J=5.4, 8.1Hz), 7.60 (d, 1H, J=2.7Hz), 7.77 (d, 1H, J=2.7Hz), 8.04 (d, 1H, J=16.2Hz), 8.39 (d, 1H, J=16.2Hz), 8.45~8.50 (m, 1H), 8.68 (t, 1H, J=5.4Hz), 8.77~8.82 (m, 1H).

### Example 2-25

### Synthesis of the present compound [Compound No. (2i)]

According to the same manner as that of Example 2-22 except that 100 mg of N-(2-formylthiophen-4-yl)-2-methoxyacetamide was used in place of 5-formylthiophene-2-carboxylic acid 2-methoxyethylamide, 131 mg of 4-hydroxy-1-methyl-3-[3-[4-(methoxyacetylamino)thiophen-2-yl]acryloyl]-1H-[1,8]naphthyridine-2-one [Compound No. (2i)] was obtained as a tan crystal.
¹H-NMR (270MHz, DMSO-d₆) δ (ppm): 3.39 (s, 3H), 3.64 (s, 3H), 4.02 (s, 2H), 7.36 (dd, 1H, J=5.4, 8.1Hz), 7.77 (s, 1H), 7.86 (s, 1H), 8.07 (d, 1H, J=13.5Hz), 8.38 (d, 1H, J=13.5Hz), 8.45~8.55 (m, 1H), 8.75~8.85 (m, 1H), 10.37 (broad, 1H).

### Example 2-26

### Synthesis of the present compound [Compound No. (3i)]

According to the same manner as that of Example 2-23 except that 329 mg of 5-formylthiophene-2-carboxylic acid 2-hydroxyethylamide was used in place of 5-formylthiophene-2-carboxylic acid 2-methoxyethylamide, 330 mg of 4-hydroxy-3-[3-[5-[(2-hydroxyethyl)aminocarbonyl]thiophen-2-yl]acryloyl]-2-methyl-2H-1,2-benzothiazine-1,1-dioxide [Compound No. (3i)] was obtained as an orange crystal.
¹H-NMR (270MHz, DMSO-d₆) 5 (ppm): 2.99 (s, 3H), 3.60~3.70 (m, 2H), 3.80~3.90 (2H), 6.53 (broad, 1H), 7.19 (d, 1H, J=16.2Hz), 7.35 (d, 1H, J=2.7Hz), 7.50 (d, 1H, J=2.7Hz), 7.89 (d, 1H, J=16.2Hz), 7.75~7.85 (m, 2H), 7.90~7.95 (m, 1H), 8.15~8.20 (m, 1H).

### Example 2-27

### Synthesis of the present compound [Compound No. (4i)]

According to the same manner as that of Example 2-23 except that 300 mg of N-(2-formylthiophen-4-yl)-2-methoxyacetamide was used in place of 5-formylthiophene-2-carboxylic acid 2-methoxyethylamide, 560 mg of 4-hydroxy-3-[3-[4-(methoxyacetylamino)thiophen-2-yl]acryloyl]-2-methyl-2H-1,2-benzothiazine-1,1-dioxide [Compound No. (4i)] was obtained as an orange crystal.
¹H-NMR (270MHz, DMSO-d₆) δ (ppm): 2.93 (s, 3H), 3.38 (s, 3H), 4.02 (s, 2H), 7.01 (d, 1H, J=16.2Hz), 7.83 (s, 1H), 7.90 (s, 1H), 7.92~8.00 (m, 3H), 8.02 (d, 1H, J=16.2Hz), 8.10~8.18 (m, 1H).

### Example 3 (Preparation of a plasmid having a reporter gene linked to a transcription regulatory region for a Type I collagen gene)

1 x 10⁸ cells of a normal human fetal skin fibroblast (Clontech, catalogue No. CC-2509) were cultured at 37°C overnight under 5% CO₂ atmosphere. After the cultured cells were washed with a sodium phosphate buffer (hereinafter, referred to as PBS) twice, 3 ml of PBS was added thereto and the cells were scraped away the wall of a vessel using a cell scraper (Nalgen, catalogue No. 179693). The scraped cells were collected by centrifugation (1,500 rpm, 4°C, 15 min), and these were suspended in 20 ml of PBS and centrifuged again. To the resulting precipitates were added 11 ml of Solution 2 and 4.8 µl of pronase of DNA Extraction Kit (Stratagene, catalogue No. 200600). After shaken at 60°C for 1 hour, the resulting mixture was allowed to stand in ice for 10 minutes. Then, 4 ml of Solution 3 of the kit was added to the mixture. After mixed, the mixture was allowed to stand in ice for 5 minutes and then centrifuged (3,000 rpm, 4°C, 15 min) to recover a supernatant. To the recovered supernatant was added 2 µl of RNase per 1 ml of the supernatant and the mixture was allowed to stand at 37°C for 15 minutes. To the mixture was added 2-fold volume of ethanol. After mixed, a white thread-like substance (genomic DNA) appeared and the substance was recovered. The recovered genomic DNA was washed with 70% ethanol and then air-dried. The air-dried genomic DNA was dissolved in 500 µl of 10 mM Tris-HCl, 1 mM EDTA (pH 8.0) (hereinafter, referred to as TE).

The resulting genomic DNA-dissolved solution (genomic DNA 1 µg corresponding amount) was mixed with each 1 µl of an oligonucleotide consisting of a base sequence represented by SEQ ID No.: 1 (SEQ ID No. 1: an oligonucleotide primer designed to amplify a collagen promoter DNA: ccaagctagc gaaattatct tttctttcat ag 32) and an oligonucleotide (10 pmol/µl) consisting of a base sequence represented by SEQ ID No. :2 (SEQ ID No. 2: an oligonucleotide primer designed to amplify a collagen promoter DNA: ccaaaagctt gcagtcgtgg ccagtacc 28), 29 µl of distilled water, 5 µl of a buffer attached to TaKaRa LA Taq (TAKARA SHUZO Co., Ltd., catalogue No. RR002A), 5 µl of a Mg²⁺ solution, 5 µl of a dNTP mixture and 0.5 µl of TaKaRa LA Taq (TAKARA SHUZO Co., Ltd., catalogue No. RR002A). After the resulting mixed solution was incubated at 94°C for 5 minutes, the mixed solution was subjected to 30 cycles, in which one cycle consists of incubation at 94°C for 1 minute, at 60°C for 1 minute and then at 72°C for 1 minute. The mixed solution was electrophoresed on a 2% agarose gel to recover about 0.5 kb of a DNA. The recovered DNA was treated with phenol/chloroform and then precipitated with ethanol to recover the DNA. The resulting DNA was dissolved in ultrapure water. To this solution were added 2.5 µl of NheI and 2.5 µl of HindIII, and then incubated at 37°C for 3 hours. Then, the solution was electrophoresed on a 2% agarose gel to recover about 3.5 kb of a DNA. The recovered DNA was precipitated with ethanol to recover again the DNA (hereinafter, referred to as the collagen promoter DNA).

On the other hand, the vector pGL3 (Promega, catalogue No. E1751) having the nucleotide sequence encoding firefly luciferase was digested with NheI and HindIII, and then subjected to agarose gel electrophoresis as described above to recover about 5kb of a DNA. The recovered DNA was precipitated with ethanol to recover the DNA again. To the recovered DNA were added 44 µl of distilled water, 5 µl of Buffer attached to Alkaline Phosphatase (TAKARA SHUZO, catalogue No. 2120A) and 1 µl of Alkaline Phosphatase (TAKARA SHUZO, catalogue No. 2120A). The mixed solution was incubated at 65°C for 30 minutes. Then, the mixed solution was treated with phenol/chloroform twice, and precipitated with ethanol to recover the DNA (hereinafter referred to as the Luc vector DNA). Then, after about 20 ng of the collagen promoter DNA and about 20 ng of the Luc vector DNA were mixed, the same amount of a DNA Ligation kit Ver2 enzyme solution was added and this was incubated for 1 day at 16°C. To the mixed solution was added Escherichia coli 5Hdα (TOYOBO, catalogue No. DNA-903), this was allowed to stand in ice for 30 minutes, and then incubated at 42°C for 45 seconds. The resulting Escherichia coli was seeded on a LB plate containing 50 µg/ml ampicillin sodium (Nacalai, catalogue No. 027-39), and this was allowed to stand at 37°C for 1 day. A single colony appeared and the colony was cultured in 2 ml of a LB medium containing 50 µg/ml ampicillin at 37°C for 12 hours. From the resulting culture solution, a plasmid DNA was prepared using AUTOMATIC DNA ISOLATION SYSTEM PI-50 (KURABO). The nucleotide sequence of the prepared plasmid DNA was analyzed with a DNA sequencer. As a result, it was confirmed that the plasmid (hereinafter, referred to as COL-Luc) had a nucleotide sequence comprising a nucleotide sequence encoding the amino acid sequence of firefly luciferase as a reporter gene linked downstream of the nucleotide sequence -3500 to +57 (the transcription initiation point is +1) of a transcription regulatory region for a human-derived Type I collagen α2 chain gene.

### Example 4 (Measurement of the ability of a test compound to regulate transcription of a Type I collagen gene using the expression level of a report gene as an index)

1 x 10⁶ cells of a normal human fetal skin fibroblast were seeded on a 100 mm dish and cultured at 37°C overnight under 5% CO₂ atmosphere in a Dulbecco's-MEM (Nissui Seiyaku, catalogue No. 05919) medium containing 10(v/v)% heat-inactivated bovine fetal serum (hereinafter, referred to as FBS; Gibco, catalogue No. 21140-079) (hereinafter, this medium is referred to as D-MEM(+)). Then, the medium was replaced with a Dulbecco's-MEM medium not containing FBS (hereinafter, this medium is referred to as D-MEM(-)).

To 300 µl of D-MEM(-) were added 5 µg of COL-Luc and 5 µg of pCMV-β-gal (Invitrogen, catalogue No. 10586-014), and the resulting mixed solution was allowed to stand at room temperature for 5 minutes (solution 1). To 300 µl of D-MEM(-) was added 20 µl of Lipofectine (Gibco, catalogue No. 18292-011), and the resulting mixed solution was allowed to stand at room temperature for 45 minutes (solution 2). Then, the solution 1 and the solution 2 were mixed. After the mixture was allowed to stand at room temperature for 10 minutes, 5.4 ml of D-MEM(-) was added to thereto, followed by mixing. The mixed solution was added to the normal human fetal skin fibroblasts, and the cells were cultured at 37°C under 5% CO₂ atmosphere. After 6 hours, the culture supernatant was removed from the dish, and the cells were washed with PBS twice. To the dish was added 1 ml of PBS containing 0.25% trypsin, and the cells were scraped off the dish. To the scraped cells was added D-MEM(+), and these were mixed well. The mixture was dispensed into a 12-well plate at 1 ml per well, and the plate was incubated at 37°C overnight under 5% CO₂ atmosphere. On the next day, each well was washed with D-MEM(-) twice, and this was replaced with 1 ml of a Dulbecco's-MEM medium containing 0.1% FBS (hereinafter, this medium is referred to as D-MEM (0.1%)).

To the thus cultured cells was added 10 µL of a 1 mM solution of the present compound represented by the compound number (34a-1), (54a-1), (56a-1), (71a-1), (10a-3), (1i) or (3i) in 10% dimethyl sulfoxide (hereinafter, referred to as DMSO) (the present compound final concentration: 10 µM, DMSO final concentration: 0.1%). In addition, to the cultured cells was added 10 µL of a 0.3 mM solution of the present compound represented by the compound number (14h) in 10% dimethyl sulfoxide (hereinafter, referred to as DMSO) (the present compound final concentration: 3 µM, DMSO final concentration: 0.1%). As a control, only 10 µl of DMSO was added.

After one hour, 10 µl of a 0.5 µg/ml aqueous solution of TGF-β (Pepro Tech) or distilled water was added to the well, and the plate was further incubated at 37°C for 40 hours under 5% CO₂ atmosphere. After the incubated cells were washed with PBS twice, 200 µl of a cell lysing agent (Toyo Inc., catalogue No. PD10) was added thereto and the cells were scraped. The scraped cells were recovered as a cell suspension, and the suspension was centrifuged (15,000 rpm, 4°C, 5 min) to recover a supernatant. The recovered supernatant was transferred to a 96-well plate at 50 µl per well, and then 50 µl of a Luc assay solution (20 mM Tricine (pH 7.8), 2.67 mM MgSO₄, 0.1 mM EDTA, 33.3 mM DTT, 270 µM Coenzyme A, 530 µM ATP, 470 µM Luciferin) was automatically dispensed into the plate using MICROLUMAT LB96P (manufactured by EG&G BERTHOLD). Luminescence in each well was measured (Delay: 1.6 second, Meas. Interval: 20 second) .

On the other hand, 50 µl of the recovered supernatant or the cell lysing agent was added to 50 µl of a β-gal substrate solution (5.8 mM o-nitrophenyl-beta-D-galactopyranoside, 1 mM MgCl₂, 45 mM 2-mercaptoethanol) which had been dispensed into a 96-well plate in advance, and the plate was incubated at 37°C for 2 hours. Then, an absorbance in each well was measured using a microplate reader at 420 nm. Based on the value obtained, transcription activity was calculated according to the following equation. $Transcription activity = \left[luminescence amount ( supernatant - added section ) - luminescence amount \left(cell lysing agent-added section\right)\right] / \left[420 nm absorbance ( supernantant - added section ) - 420 nm absorbance \left(cell lysing agent-added section\right)\right]$

Then, based on the calculated transcription activity, an inhibitory effect of a test compound on the ability of TGF-β to promote transcription of a Type I collagen gene was calculated as an inhibition percentage according to the following equation: $Inhibition percentage = \left[transcription activity ( DMSO and TGF - β - added test section ) - transcription activity \left(compound and TGF-β-added test section\right)\right] / [ transcription$ $activity ( DMSO and TGF - β - added test section ) - transcription activity \left(DMSO and TGF-β non-added test section\right) ] \times 100$

The inhibition percentages of the present compounds represented by the compound numbers (34a-1), (54a-1), (56a-1), (71a-1), (10a-3), (1i) and (3i) at the present compound final concentration of 10 µM, and the present compound represented by the compound number (14h) at the present compound final concentration of 3 µM were 70 or more. It was found that the present compounds can inhibit the ability of TGF-β to promote transcription of a Type I collagen gene, and then can suppress transcription of a Type I collagen gene.

### Example 5 (Measurement of the ability of a test compound to suppress TGF-β using the expression level of fibronectin as an index)

5 x 10³ cells/well of a normal human skin fibroblast (Clonetics, catalogue No. CC-2509) were seeded on a 96-well plate (BECTON DICKINSON, catalogue No. 35-3075), and then cultured in an incubator at 37°C and 5% CO₂ overnight. On the next day, the medium was exchanged with 0.1 ml of a D-MEM medium (Nissui Pharmaceutical Co.,Ltd., code No. 05919) containing 0.1% bovine fetal serum. After 1 hour, to the well was added the present compound represented by the compound number (2a-3) at the final concentration of 10 µM or the present compound represented by the compound number (63a-1) at the final concentration of 3 µM. After the cells were cultured for 1 hour, 5 ng/ml (final concentration) of TGF-β (Peprotech, catalogue No.100-21R) was added, followed by further culturing for 26 hours. After the cells were washed with a phosphate buffer twice, a total RNA was isolated using SV96 Total RNA Isolation System (Promega, catalogue No. Z3505). To 5 ml of the isolated total RNA were added 1 µl of 20 µM oligo dT and 4 µl of RNase-free distilled water, incubated at 65°C for 5 minutes, and immediately cooled with an ice. To 10 µl of the solution were added 4 µl of 5 x buffer, 2.4 µl of MgCl₂, 1 µl of 10 mM dNTP, 1 µl of RNasin, 1 µl of Improm II and 0.6 µl of RNase-free distilled water (these are all available from Promega), and the mixture was subjected to a reverse transcription reaction under the condition of 25°C for 5 minutes, 42°C for 1 hour and 70°C for 15 minutes.

To 5 µl of a reverse transcription reaction solution were added each 1 µl of each 20 pmol/µl of primers represented by SEQ ID No. 3 (SEQ ID No. 3: an oligonucleotide designed as a PCR primer in order to detect a DNA of a fibronectin gene: tcgccatcag tagaaggtag ca 22) and SEQ ID No. 4 (SEQ ID No. 4: an oligonucleotide designed as a PCR primer in order to detect a DNA of a fibronectin gene: tatactgaac accaggttgc aagtc 25), and 1.25 µL of a probe for detecting a DNA of a fibronectin gene represented by SEQ ID No. 5 (SEQ ID NO. 5: an oligonucleotide designed as a probe in order to detect a DNA of a fibronectin gene: ctcaaccttc ctgaaactgc aaactccgtc 30) or 1.25 µl of Human GAPDH primer probe (Applied Biosystems, catalogue No. 4310884E). Further, 12.5 µl of TaqMan Universal PCR Master Mix (Applied Biosystems, catalogue No. 4304437) was added, and the mixture was adjusted to 50 µl with sterilized water, and then mixed in a well of Optical 96-Well Reaction Plate (Applied Biosystems, catalogue No. N801-0560). As a standard, a total RNA was prepared from the cells to which only TGF-β had been added, and 250, 125, 62.5, 31.25, 15.625 or 7.8125 ng of the total RNA was subjected to a reverse transcription reaction to obtain a cDNA solution. Then, PCR was performed under the condition of 1 cycle of 50°C for 5 minutes and 40 cycles of 95°C for 15 seconds and 60°C for 1 minute using Gene Amp 7900 (Applied Biosystems). For quantitation, respective standard lines for fibronectin and GAPDH were made. Then the fibronectin amount and the GAPDH amount were calculated, and the transcription amount was calculated according to the following equation. Fibronectin transcription amount = fibronectin amount/GAPDH amount $Inhibition percentage = [ transcription amount ( DMSO and$ $TGF - β - added test section ) - transcription amount \left(compound and TGF-β-added test section\right) ] / \left[transcription amount ( DMSO and TGF - β - added test section ) - transcription amount \left(DMSO and TGF-β non-added test section\right)\right] \times 100$

The inhibition percentages of the present compounds represented by the compound numbers (2a-3) and (63a-1) were 70% or more.

It was confirmed that the present compound suppresses the transcription amount of a fibronectin gene of a skin fibroblast promoted by TGF-β.

### Industrial applicability

According to the present invention, it is possible to develop and provide a composition which decreases expression of an extracellular matrix gene in a tissue to induce a reduction in accumulation of an extracellular matrix and thereby improves tissue fibrosis (i.e. an extracellular matrix accumulation-suppressing agent, a fibrosing disease-treating agent, or heart failure treating agent).

### Sequence Listing Free Text

SEQ ID NO: 1
   Oligonucleotide primer designed for amplifying a collagen promoter DNA
SEQ ID NO: 2
   Oligonucleotide primer designed for amplifying a collagen promoter DNA
SEQ ID NO: 3
   Oligonucleotide primer designed for detecting a fibronectin DNA
SEQ ID NO: 4
   Oligonucleotide primer designed for detecting a fibronectin DNA
SEQ ID NO: 5
   Oligonucleotide primer designed for detecting a fibronectin DNA

## Claims

1. A composition for suppressing transcription of an extracellular matrix gene which comprises an inert carrier and a cinnamoyl compound represented by the formula (I): 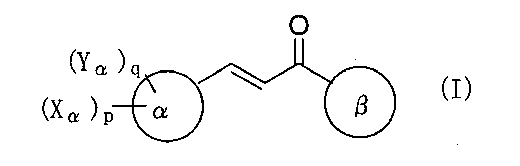 wherein,
I. α represents an aromatic 5-membered ring, or an aromatic 6-membered ring having two or more nitrogen atoms; in (Y_{α})_{q}, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the a ring; in (X_{α})p, Xₐ represents a substituent which does not belong to the following X₀ group, Y₀ group and Z₀ group, p represents 0, 1, 2 or 3 and, when p is not less than 2, X_{α}s are the same or different, and the sum of p and q is not more than 3;
(1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ'')-NRₑ'''-R_{d}- group (wherein Rₑ, Rₑ', Rₑ" and Rₑ''' are the same or different, Rₑ, Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
(2) the Y₀ group: a M_{b0}-R_{d}- group, wherein M_{b0} represents a M_{c0}- group
[wherein M_{co} represents a M_{do}-R_{d}' - group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 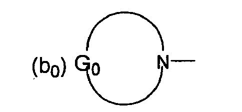 (wherein Go forms an optionally substituted, saturated or unsaturated, nonaromatic 5 to 14-membered hydrocarbon ring or heterocyclic ring), a (c₀)- group represented by 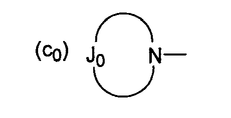 (wherein, J₀ forms a 5 to 7-membered aromatic ring optionally containing a nitrogen atom), a (do)- group represented by 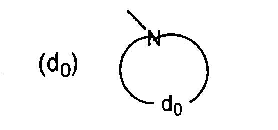 [wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group], or a (e₀)- group represented by 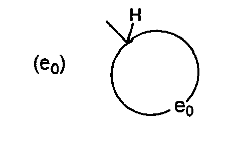 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}; and R_{d}' is the same as or different from R_{d} and has the same meaning as R_{d} has],
a M_{co}-Bₐ- group (wherein M_{c0} and Bₐ are as defined above), a M_{c0}-CO- group (wherein M_{c0} is as defined above), a M_{c0}-CO-O-group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ-group (wherein M_{c0} and Rₑ are as defined above), a M_{c0}RₑN-CO-group (wherein M_{c0} and Rₑ are as defined above), a M_{c0}RₑN-CO-NRₑ' - group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
(3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and
II. β represents
a group represented by formula (I-1): 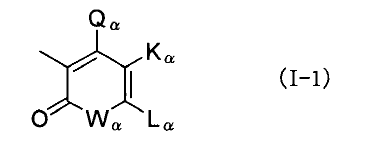
wherein,
(1)Q_{α} represents an optionally substituted hydroxyl group, or an optionally substituted amino group,
(2)W_{α} represents an oxygen atom or a -NT_{α}- group (wherein T_{α} represents a hydrogen atom, or a substituent on the nitrogen atom),
(3)K_{α} and L_{α} are the same or different, and represent a hydrogen atom, or a substituent on the carbon atom, or K_{α} and L_{α} may form an optionally substituted C3-C10 alkylene group or an optionally substituted C4-C10 alkenylene group; a group represented by formula (I-2): 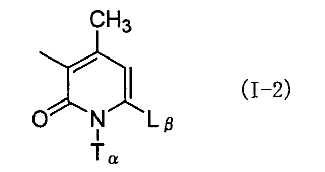 wherein T_{α} is as defined above, and L_{β} represents a hydroxyl group or a methyl group;
a group represented by formula (I-3): 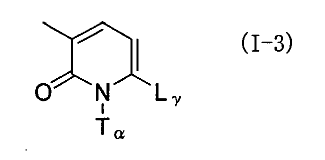 wherein T_{α} is as defined above, and L_{γ} represents a C1-C10 alkyl group;
a group represented by formula (I-4): 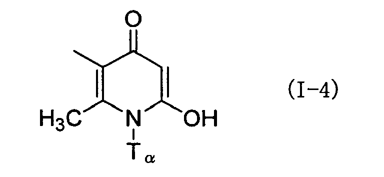 wherein T_{α} is as defined above;
a group represented by formula (I-5): 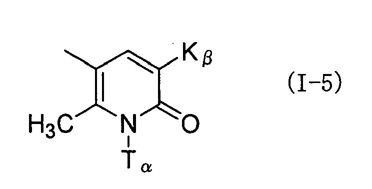 wherein T_{α} is as defined above, and K_{β} represents a cyano group or a UOCO- group (wherein U represents a hydrogen atom or a C1-C10 alkyl group);
a group represented by formula (I-6): 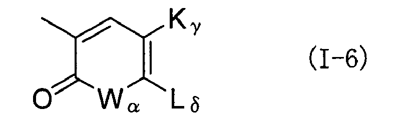 wherein W_{α} is as defined above, and K_{γ} and L_{δ} form an optionally substituted C3-C10 alkylene group or an optionally substituted C4-C10 alkenylene group;
a group represented by formula (I-7): 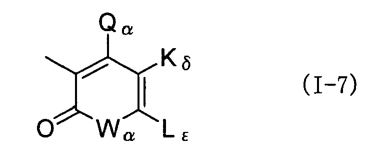 wherein Q_{α} and W_{α} are as defined above, and K_{δ} and L_{ε} form a -V_{α}=V_{α} '-V_{α} ' ' =V_{α} ' ' ' - group (wherein V_{α}, V_{α}', V_{α}' ' and
V_{α}' ' ' are the same or different, and represent an optionally substituted methine group or a -N= group, and at least one of V_{α}, V_{α}', V_{α}' ' and V_{α}' ' ' represents a -N= group);
a group represented by formula (I-8): 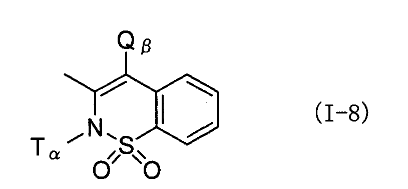 wherein T_{α} is as defined above, and Q_{β} represents an optionally substituted hydroxyl group; or
a group represented by formula (I-9): 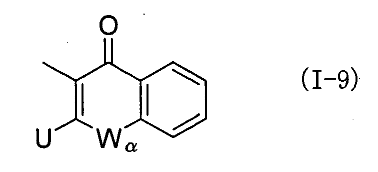 wherein U and W_{α} are as defined above; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

2. A cinnamoyl compound represented by the formula (II): 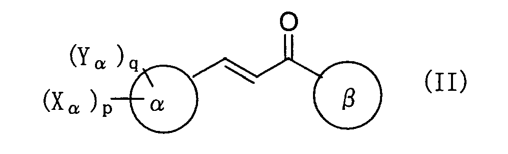 wherein,
I. α represents an aromatic 5-membered ring, or an aromatic 6-membered ring having two or more nitrogen atoms; in (Y_{α})_{q}, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the α ring; in (X_{α})ₚ, X_{α} represents a substituent which does not belong to the following X₀ group, Y₀ group and Z₀ group, p represents 0, 1, 2 or 3 and, when p.is not less than 2, X_{α}s are the same or different, and the sum of p and q is not more than 3;
(1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ''-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ'')-NRₑ'''-R_{d}- group (wherein Rₑ, Rₑ', Rₑ" and Rₑ''' are the same or different, Rₑ, Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ' N-SO₂-R_{d}- group (wherein Rₑ, Rₑ' and R_{d} are as defined above), a C2-C10 alkenyl group or a C2-C10 alkynyl group;
(2) the Y₀ group: a M_{b0}-R_{d}- group, wherein M_{b0} represents a M_{c0}- group [wherein M_{co} represents a M_{do}-R_{d}'-group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 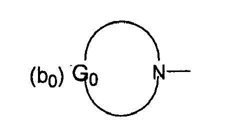 (wherein Go forms an optionally substituted, saturated or unsaturated, nonaromatic 5 to 14-membered hydrocarbon ring or heterocyclic ring), a (c₀)- group represented by 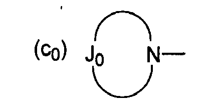 (wherein, J₀ forms a 5 to 7-membered aromatic ring optionally containing a nitrogen atom), a (do)- group represented by 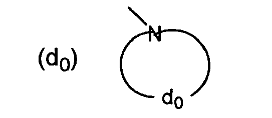 {wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group}, or a (e₀)- group represented by 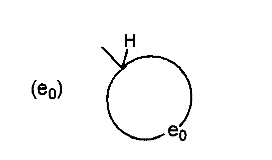 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}, and R_{d}' is the same as or different from R_{d} and has the same meaning as R_{d} has], a M_{c0}-Bₐ- group (wherein M_{c0} and Bₐ are as defined above), a M_{c0}-CO- group (wherein M_{c0} is as defined above), a M_{c0}-CO-O- group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ- group (wherein M_{c0} and Rₑ are as defined above), a M_{c0}RₑN-CO- group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
(3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and
II. β represents
a group represented by formula (II-1): 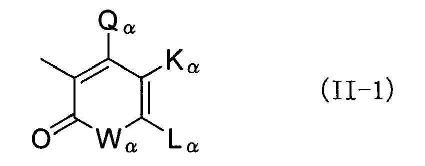
wherein,
(1)Q_{α} represents an optionally substituted hydroxyl group, or an optionally substituted amino group,
(2)W_{α} represents an oxygen atom or a -NT_{α}- group (wherein T_{α} represents a hydrogen atom, or a substituent on the nitrogen atom),
(3)K_{α} and L_{α} are the same or different, and represent a hydrogen atom, or a substituent on the carbon atom, or K_{α} and L_{α} may form an optionally substituted C3-C10 alkylene group or an optionally substituted C4-C10 alkenylene group; a group represented by formula (II-2): 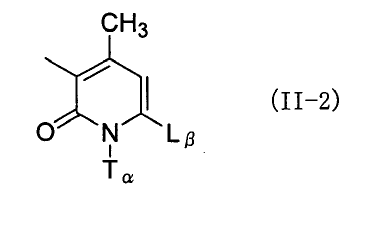 wherein T_{α} is as defined above, and L_{β} represents a hydroxyl group or a methyl group;
a group represented by formula (II-3): 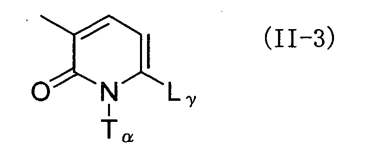 wherein T_{α} is as defined above, and L_{γ} represents a C1-C10 alkyl group;
a group represented by formula (II-4): 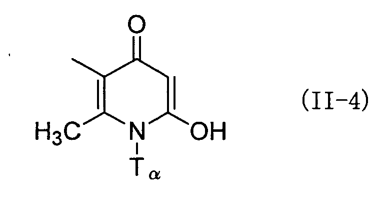 wherein T_{α} is as defined above;
a group represented by formula (II-5): 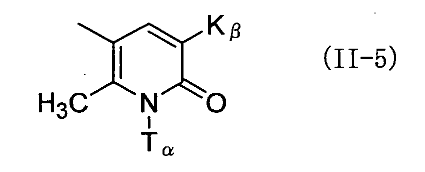 wherein T_{α} is as defined above, and K_{β} represents a cyano group or a UOCO- group (wherein U represents a hydrogen atom or a C1-C10 alkyl group);
a group represented by formula (II-6): 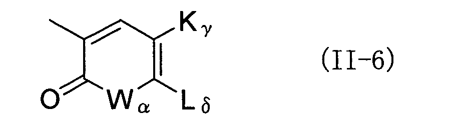 wherein W_{α} is as defined above, and K_{γ} and L_{δ} form an optionally substituted C3-C10 alkylene group or an optionally substituted C4-C10 alkenylene group;
a group represented by formula (II-7) : 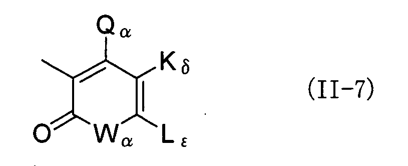 wherein Q_{α} and W_{α} are as defined above, and K_{δ} and L_{ε} form a -V_{α}=V_{α}'-V_{α}'' =V_{α}'''- group (wherein V_{α}, V_{α}', V_{α}'' and V_{α}''' are the same or different, and represent an optionally substituted methine group or a -N= group, and at least one of V_{α}, V_{α}', V_{α}'' and V_{α}' ' ' represents a -N= group);
a group represented by formula (II-8): 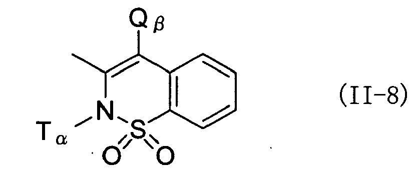 wherein T_{α} is as defined above, and Q_{β} represents an optionally substituted hydroxyl group; or
a group represented by formula (II-9): 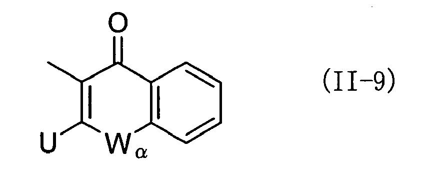 wherein U and W_{α} are as defined above; provided that p and q are not 0 at the same time when α is a furan ring or a thiophene ring; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

3. A cinnamoyl compound represented by the formula (III): 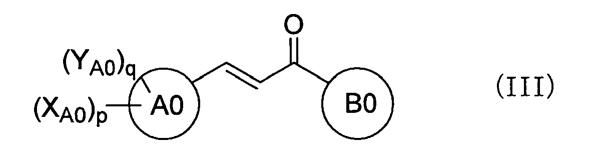 wherein,
I. A0 represents an aromatic 5-membered ring, or an aromatic 6-membered ring having two or more nitrogen atoms,
II. in (X_{A0})ₚ, X_{A0} represents a group included in any group of the following A₀ group to N₀ group, p represents 0, 1, 2 or 3 and, when p is not less than 2, X_{A0}s are the same or different,
(1) the A₀ group:
a D₁-R₄- group, wherein D₁ represnts a (R₁-(O)ₖ-)A₁N-(O)_{k'}- group [wherein R₁ represents a hydrogen atom, or a C1-C10 alkyl group, or a C2-C10 alkyl group substituted with a halogen atom or a R₂-B₁-group (wherein R₂ represents a C1-C10 alkyl group, a C3-C10 alkenyl group or a C3-C10 alkynyl group, and B₁ represents an oxy group, a thio group, a sulfinyl group or a sulfonyl group), or a C3-C10 alkenyl group, or a C3-C10 alkynyl group, k represents 0 or 1, A₁ represents a R₃-(CHR₀)ₘ-(B₂-B₃)_{m'}- group {wherein R₃ represents a hydrogen atom, or a C1-C10 alkyl group optionally substituted with a halogen atom or a R₂-B₁-group (wherein R₂ and B₁ are as defined above), or a C2-C10 alkenyl group, or a C2-C10 alkynyl group, R₀ represents a hydrogen atom, a C1-C10 alkyl group or a C2-C10 haloalkyl group, m represents 0 or 1, B₂ represents a single bond, an oxy group, a thio group or a -N((O)ₙR₁')- group (wherein R₁' is the same as or different from R₁, and has the same meaning as R₁ has, and n represents 0 ro 1), B₃ represents a carbonyl group, a thiocarbonyl group or a sulfonyl group, m' represents 0 or 1, and B₃ is not a sulfonyl group when m is 0 and R₃ is a hydrogen atom}, and k' represents 0 or 1], and R₄ represents a C1-C10 alkylene group, provided that a R₀'R₀"N-R₄- group (wherein R₀' and R₀" are the same as or different from R₀ and have the same meaning as R₀ has, and R₄ is as defined above) is excluded,
a D₂-R₄- group, wherein D₂ represents a cyano group, a R₁R₁'NC(=N-(O)ₙ-A₁)- group (wherein R₁, R₁', n and A₁ are as defined above), an A₁N=C(-OR₂)- group (wherein A₁ and R₂ are as defined above) or a NH₂-CS- group, and R₄ is as defined above,
a D₃-R₄- group, wherein D₃ represents a nitro group or a R₁OSO₂- group (wherein R₁ is as defined above), and R₄ is as defined above, and
a R₁OSO₂- group, wherein R₁ is as defined above;
(2) the B₀ group: an (a₀)- group represented by 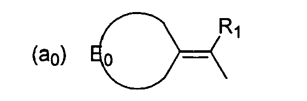 wherein E₀ forms an optionally substituted, saturated or unsaturated, aromatic or nonaromatic 5 to 14-membered hydrocarbon ring or heterocyclic ring, and R₁ is as defined above;
(3) the C₀ group: a C2-C10 alkenyl group substituted with a halogen atom, a R₂-B₁- group (wherein R₂ and B₁ are as defined above), a D₄-R₄- group [wherein D₄ represents a hydroxyl group or an A₁-O- group (wherein A₁ is as defined above), and R₄ is as defined above], a D₅- group [wherein D₅ represents a O=C(R₃)- group (wherein R₃ is as defined above), an A₁-(O)ₙ-N=C(R₃)- group (wherein A₁, n and R₃ are as defined above), a R₁-B₀-CO-R₄-(O)ₙ-N=C(R₃)- group {wherein R₁, R₄, n and R₃ are as defined above, and B₀ represents an oxy group, a thio group or a -N((O)ₘR₁')-group (wherein R₁' and m are as defined above) }, a D₂-R₄-(O)ₙ-N=C(R₃)- group (wherein D₂, R₄, n and R₃ are as defined above) or a R₁A₁N-N=C(R₃)- group (wherein R₁, A₁ and R₃ are as defined above)], a R₁A₁N-O-R₄- group (wherein R₁, A₁ and R₄ are as defined above), a R₁(A₁-(O)ₙ-)N- group (wherein R₁, A₁ and n are as defined above), a D₂- group (wherein D₂ is as defined above) or a D₃- group (wherein D₃ is as defined above);
(4) the D₀ group: a C2-C10 alkynyl group substituted with a (b₀)-R₄- group (in (b₀) 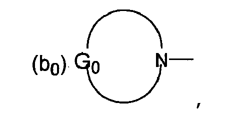 Go forms an optionally substituted, saturated or unsaturated, nonaromatic 5 to 14-membered hydrocarbon ring or heterocyclic ring), a (c₀)-R₄- group (in (c₀) 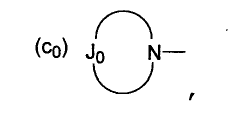 J₀ forms an aromatic 5 to 7-membered ring optionally containing a nitrogen atom and R₄ is as defined above), a halogen atom, a R₂-B₁-R₄- group (wherein R₂, B₁ and R₄ are as defined above), a D₄-R₄- group (wherein D₄ and R₄ are as defined above), a D₅- group (wherein D₅ is as defined above), a D₁-R₄- group (wherein D₁ and R₄ are as defined above), a D₂- group (wherein D₂ is as defined above) or a D₃-R₄- group (wherein D₃ and R₄ are as defined above);
(5) the E₀ group: an A₂-CO-R₅- group, provided that R₅ is not a vinylene group when A₂ is a hydroxyl group [wherein A₂ represents
(i) an A₃-B₄- group
wherein A₃ represents a hydrogen atom, or a C1-C10 alkyl group, or a C2-C10 haloalkyl group, or a C2-C10 alkenyl group optionally substituted with a halogen atom, or a C3-C10 alkynyl group optionally substituted with a halogen atom, or a Rₐ₀-(R₄)ₘ- group (wherein Rₐ₀ represents an optionally substituted 5 to 7-membered aryl group or heteroaryl group, and R₄ and m are as defined above), or a C1-C10 alkyl group substituted with a (b₀)-R₄- group (wherein (b₀) and R₄ are as defined above), a (c₀)-R₄- group (wherein (c₀) and R₄ are as defined above), a R₂-B₁-R₄-group (wherein R₂, B₁ and R₄ are as defined above), a D₄-R₄-group (wherein D₄ and R₄ are as defined above), a D₅- group (wherein D₅ is as defined above), a D₁-R₄- group (wherein D₁ and R₄ are as defined above), a D₂- group (wherein D₂ is as defined above), a D₃-R₄- group (wherein D₃ and R₄ are as defined above) or an A₄-SO₂-R₄- group {wherein A₄ represents a (b₀)- group (wherein (b₀) is as defined above), a (c₀)-group (wherein (c₀) is as defined above) or a R₁R₁'N- group (wherein R₁ and R₁' are as defined above), and R₄ is as defined above}, and
B₄ represents an oxy group, a thio group or a -N((O)ₘR₁)-group (wherein R₁ and m are as defined above), provided that A₃ is not a hydrogen atom when B₄ is a thio group;
(ii) a R₁-B₄-CO-R₄-B₄'- group (wherein R₁, B₄ and R₄ are as defined above, B₄' is the same as or different from B₄ and has the same meaning as B₄ has, provided that R₂ is not a hydrogen atom when B₄ is a thio group) or a D₂-R₄-B₄-group (wherein D₂, R₄ and B₄ are as defined above);
(iii) a R₂-SO₂-NR₁- group (wherein R₂ is as defined above, provided that a hydrogen atom is excluded, and R₁ is as defined above);
(iv) a (b₀)- group, wherein (b₀) is as defined above;
(v) a (c₀)- group, wherein (c₀) is as defined above; or
(vi) a R₁A₁N-NR₁'- group, wherein R₁, A₁ and R₁' are as defined above; and
R₅ represents a C2-C10 alkenylene group optionally substituted with a halogen atom or a C2-C10 alkynylene group];
(6) the F₀ group: an A₅-B₅-R₆- group, wherein
A₅ represents a C2-C10 alkyl group substituted with a D₄- group (wherein D₄ is as defined above), a D₁- group (wherein D₁ is as defined above), a D₃- group (wherein D₃ is as defined above) or an A₄-SO₂- group (wherein A₄ is as defined above), or a C1-C10 alkyl group substituted with a R₂-B₁- group (wherein R₂ and B₁ are as defined above), a D₂-group (wherein D₂ is as defined above), a D₅- group (wherein D₅ is as defined above) or an A₂-CO- group (wherein A₂ is as defined above), and
B₅ represents a B₁- group (wherein B₁ is as defined above) or a -NA₁- group (wherein A₁ is as defined above) and
R₆ represent a single bond or a C1-C10 alkylene group;
(7) the Go group: an A₆-B₅-R₆- group wherein
A₆ represents an (a₀)-R₄- group (wherein (a₀) and R₄ are as defined above), or a C2-C10 alkenyl group, or a C2-C10 alkynyl group, or a C2-C10 alkenyl group substituted with a halogen atom, a R₂-B₁- group (wherein R₂ and B₁ are as defined above), a D₅- group (wherein D₅ is as defined above), a D₂- group (wherein D₂ is as defined above) or an A₂-CO- group (wherein A₂ is as defined above), or a C2-C10 alkynyl group substituted with a halogen atom, a R₂-B₁-group (wherein R₂ and B₁ are as defined above), a D₅- group (wherein D₅ is as defined above), D₂- group (wherein D₂ is as defined above) or an A₂-CO- group (wherein A₂ is as defined above), or a C3-C10 alkenyl group substituted with a (b₀)- group (wherein (b₀) is as defined above), a (c₀)-group (wherein (c₀) is as defined above), a D₄- group (wherein D₄ is as defined above), a D₁- group (wherein D₁ is as defined above) or a D₃- group (wherein D₃ is as defined above), or a C3-C10 alkynyl group substituted with a D₄-group (wherein D₄ is as defined above), a D₁- group (wherein D₁ is as defined above) or a D₃- group (wherein D₃ is as defined above), and
B₅ and R₆ are as defined above;
(8) the H₀ group:
a D₂-N(-(O)ₙ-A₁)-R₆- group (wherein D₂, n, A₁ and R₆ are as defined above),
a D₂- group (wherein D₂ is as defined above, provided that a cyano group is excluded),
a R₁(R₁' (O)ₙ)N-CR₁"=N-R₆- group (wherein R₁, R₁', n and R₆ are as defined above, R₁" is the same as or different from R₁ and has the same meaning as R₁ has),
a R₁-(O)ₙ-N=CR₁'-NR₂-R₆- group (wherein R₁, n, R₁', R₂ and R₆ are as defined above),
a R₂-B₃-NR₁-CO-NR₁'-R₆- group (wherein R₂, B₃, R₁, R₁' and R₆ are as defined above),
a D₂-CO-NR₁-R₆- group (wherein D₂, R₁ and R₆ are as defined above), and
an A₂-COCO-NR₁-R₆- group (wherein A₂, R₁ and R₆ are as defined above);
(9) the I₀ group:
an A₇-B₆-N((O)ₙR₁)-R₆- group [wherein A₇ represents a C2-C10 alkenyl group optionally substituted with a halogen atom, or a C2-C10 alkynyl group, or a C3-C10 haloalkynyl group, or a R₂-B₁-R₄- group (wherein R₂, B₁ and R₄ are as defined above), or a D₄-R₄- group (wherein D₄ and R₄ are as defined above), or a D₅-R₄- group (wherein D₅ and R₄ are as defined above), or a D₁-R₄- group (wherein D₁ and R₄ are as defined above), or a (b₀)-R₄- group (wherein (b₀) and R₄ are as defined above), or a (c₀)-R₄- group (wherein (c₀) and R₄ are as defined above), or a D₂-R₄- group (wherein D₂ and R₄ are as defined above), or a D₃-R₄- group (wherein D₃ and R₄ are as defined above), or an A₄-SO₂-R₄- group (wherein A₄ and R₄ are as defined above), or an A₂-CO-R₄- group (wherein A₂ and R₄ are as defined above), B₆ represents a carbonyl group or a thiocarbonyl group, and n, R₁ and R₆ are as defined above],
an A₈-CS-N((O)ₙR₁)-R₆- group [wherein A₈ represents a hydrogen atom or a C1-C10 alkyl group optionally substituted with a halogen atom, and n, R₁ and R₄ are as defined above],
an A₇'-B₂'-B₃-N((O)ₙR₁) -R₆- group [wherein A₇' represents a C3-C10 alkenyl group optionally substituted with a halogen atom, or a C3-C10 alkynyl group optionally substituted with a halogen atom, or a R₂-B₁-R₄'- group (wherein R₂ and B₁ are as defined above, and R₄' represents a C2-C10 alkylene group), or a D₄-R₄'- group (wherein D₄ and R₄' are as defined above), or a D₁-R₄' - group (wherein D₁ and R₄' are as defined above), or a (b₀)-R₄'- group (wherein (b₀) and R₄' are as defined above), or a (c₀)-R₄'- group (wherein (c₀) and R₄' are as defined above), or a D₂-R₄-group (wherein D₂ and R₄ are as defined above), or a D₃-R₄'-group (wherein D₃ and R₄' are as defined above), or an A₂-CO-R₄- group (wherein A₂ and R₄ are as defined above), B₂' represents an oxy group, a thio group or a -N((O)ₙ,R₁')-group (wherein n' is the same as or different from n and has the same meaning as n has, and R₁' is as defined above), and B₃, n, R₁ and R₆ are as defined above],
an A₈'-B₂'-CS-N((O)ₙR₁)-R₆- group [wherein A₈' represents a C1-C10 alkyl group or a C2-C10 haloalkyl group, B₂' is as defined above, and n, R₁ and R₆ are as defined above],
an A₈'-S-B₃'-N ((O)ₙR₁-R₆- group [wherein A₈' , n, R₁ and R₆ are as defined above, and B₃' represents a carbonyl group or a sulfonyl group], and
an A₇"-SO₂-N((O)ₙR₁)-R₆- group [wherein A₇" represents a C2-C10 alkenyl group, or a C3-C10 alkenyl group substituted with a halogen atom, or a C3-C10 alkynyl group optionally substituted with a halogen atom, or a R₂-B₁-R₄'- group (wherein R₂, B₁ and R₄' are as defined above), or a D₄-R₄'-group (wherein D₄ and R₄' are as defined above), or a D₅-R₄-group (wherein D₅ and R₄ are as defined above), or a D₁-R₄' - group (wherein D₁ and R₄' are as defined above), or a (b₀)-R₄'- group (wherein (b₀) and R₄' are as defined above), or a (c₀)-R₄'- group (wherein (c₀) and R₄' are as defined above), or a D₂-R₄- group (wherein D₂ and R₄ are as defined above), or a NO₂-R₄- group (wherein R₄ is as defined above), or an A₂-CO-R₄- group (wherein A₂ and R₄ are as defined above), and n, R₁ and R₆ are as defined above];
(10) the J₀ group:
an A₇-CO- group (wherein A₇ is as defined above),
an A₉-CS- group (wherein A₉ represents A₇ or A₈),
an A₉' (O)ₘN=C(A₉)- group (wherein A₉' represents A₇' or A₈', and m and A₉ are as defined above),
a D₂-CO- group (wherein D₂ is as defined above),
an A₂-COCO- group (wherein A₂ is as defined above),
an A₉-CO-B₁'-R₆- group (wherein A₉ and R₆ are as defined above, and B₁' represents an oxy group or a thio group, provided that A₉ is not A₈ when B₁' is an oxy group),
an A₉-CS-B₁'-R₆- group (wherein A₉, B₁' and R₆ are as defined above),
an A₇"-SO₂-B₁'-R₆- group (wherein A₇", B₁' and R₆ are as defined above),
an A₈-SO₂-B₁' -R₆- group (wherein A₈, B₁' and R₆ are as defined above, provided that A₈ is not a hydrogen atom),
an A₉'-B₂'-B₃-B₁'-R₆- group (wherein A₉', B₂', B₃, B₁' and R₆ are as defined above), and
a C2-C10 alkenyl group substituted with a (b₀)- group (wherein (b₀) is as defined above) or a (c₀)- group (wherein (c₀) is as defined above);
(11) the K₀ group: an A₁₀-N((O)ₙR₁)-CO-R₆- group, wherein A₁₀ represents a hydrogen atom (provided that n is not 0), an A₇"-SO₂- group (wherein A₇" is as defined above), an A₈-SO₂- group (wherein A₈ is as defined above, provided that A₈ is not a hydrogen atom), an A₉'O- group (wherein A₉' is as defined above, (provided that n is not 1), an A₉'-group (wherein A₉' is as defined above, provided that A₈' is excluded when n is 0), a R₂OCH₂- group (wherein R₂ is as defined above), an A₂-CO-R₄- group (wherein A₂ and R₄ are as defined above) or an A₂-CO-CH(CH₂CO-A₂)- group (wherein A₂ is as defined above), and n, R₁ and R₆ are as defined above;
(12) the L₀ group:
an A₁₀'-N((O)ₙR₁)-SO₂-R₆- group [wherein A₁₀' represents a hydrogen atom (provided that n is not 1), an A₉'O- group (wherein A₉' is as defined above, provided that n is not 0), an A₉'- group (wherein A₉' is as defined above, provided that A₈' is excluded when n is 0), a R₂-CO- group (wherein R₂ is as defined above), an A₂-CO-R₄- group (wherein A₂ and R₄ are as defined above) or an A₂-CO-CH(CH₂CO-A₂)- group (wherein A₂ is as defined above), and n, R₁ and R₆ are as defined above],
an A₉"R₁N-SO₂-N((O)ₙR₁')-R₆- group [wherein A₉" represents a hydrogen atom or an A₉'- group (wherein A₉' is as defined above), and R₁, n, R₁' and R₆ are as defined above] and
a (b₀)-SO₂-N((O)ₙR₁')-R₆- group [wherein (b₀), n, R₁' and R₆ are as defined above];
(13) the M₀ group:
a R₁(R₂S)C=N-R₆- group (wherein R₁, R₂ and R₆ are as defined above),
a R₂B(R₂'B')C=N-R₆- group (wherein R₂ and R₆ are as defined above, R₂' is the same as or different from R₂ and has the same meaning as R₂ has, and B and B' are the same or different and represent an oxy group or a thio group),
a R₁R₁'N-(R₂S)C=N-R₆- group (wherein R₁, R₁', R₂ and R₆ are as defined above),
a R₁N=C(SR₂)-NR₂'-R₆- group (wherein R₁, R₂, R₂' and R₆ are as defined above), and
a R₁(R₁'O)N-R₆- group (wherein R₁, R₁' and R₆ are as defined above);
(14) the N₀ group: a A₁₁-P(=O) (OR₁')-R₄- group, wherein A₁₁ represents a R₁- group (wherein R₁ is as defined above), a R₁O-R₆- group (wherein R₁ and R₆ are as defined above) or a R₁OCO-CHR₀- group (wherein R₁ and R₀ are as defined above), and R₁' and R₄ are as defined above;
III. in (Y_{A0})_{q}, Y_{A0} represents a group included in the following X₀ group and Y₀ group, q represents 0, 1, 2 or 3, the sum of p (wherein p is as defined above) and q is not more than 3, and when q is not less than 2, Y_{A0}s are the same or different, and when q is not less than 2, adjacent two same or different Y_{A0}s may together form a group included in the Z₀ group to be fused to the A0 ring,
(1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ''-R_{d}- group (wherein Rₑ, Rₑ' and Rₑ'' are the same or different, Rₑ and Rₑ' are as defined above, Rₑ'' has the same meaning as Rₑ has, and R_{d} is as defined above), a RₑRₑ'N-C(=NRₑ'')-NRₑ'''-R_{d}- group (wherein Rₑ, Rₑ', Rₑ'' and Rₑ''' are the same or different, Rₑ, Rₑ' and Rₑ'' are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
(2) the Y₀ group: a M_{b0}-R_{d}- group, wherein M_{b0} represents a M_{c0}- group [wherein M_{co} represents a M_{do}-R_{d}'-group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 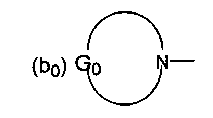 (wherein (b₀) is as defined above), a (c₀)- group represented by 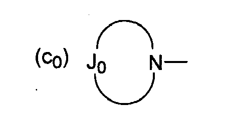 (wherein, (c₀) is as defined above), a (do)- group represented by 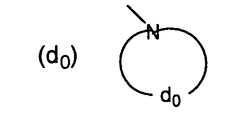 {wherein d₀ forms a 5 to 12-membered hydrocarbon ring which is substituted with a carbonyl group or a thiocarbonyl group and further which may be optionally substituted with an oxy group, a thio group, a -NR₁- group (wherein R₁ is as defined above), a sulfinyl group or a sulfonyl group}, or a (e₀)- group represented by 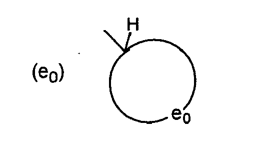 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}, and R_{d}' is the same as or different from R_{d} and has the same meaning as R_{d} has], a M_{c0}-Bₐ- group (wherein M_{c0} and Bₐ are as defined above), a M_{c0}-CO- group (wherein M_{c0} is as defined above), a M_{c0}-CO-O- group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ- group (wherein M_{c0} and Rₑ are as defined above), a M_{c0}RₑN-CO- group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above), or a M_{c0}RₑN-SO₂- group (wherein M_{c0} and Rₑ are as defined above), and R_{d} is as defined above;
(3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the A0 ring; and
IV. B0 is a group represented by formula (III-1): 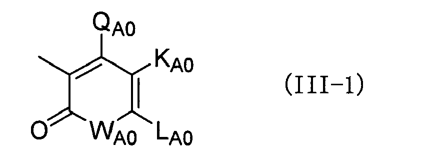 [wherein,
(1) Q_{A0} represents a hydroxyl group, a (b₀)- group (wherein (b₀) is as defined above), an A₉-B₆-B_{c}- group [wherein A₉ and B₆ are as defined above, and B_{c} represents an oxy group or a N-((O)ₘR₁)- group (wherein m and R₁ are as defined above), provided that B_{c} is not a sulfonyl group when A₉ is a hydrogen atom], an A₇"-SO₂-B_{c}- group (wherein A₇" and B_{c} are as defined above), an A₈-SO₂-B_{c}- group (wherein A₈ and B_{c} are as defined above, provided that A₈ is not a hydrogen atom), a R₁R₁'N-SO₂-B_{c}- group (wherein R₁, R₁' and B_{c} are as defined above), a (b₀)-SO₂-B_{c}- group (wherein (b₀) and B_{c} are as defined above), an A₉'-B_{c}- group (wherein A₉' and B_{c} are as defined above), a D₅-R₄-B_{c}- group (wherein D₅, R₄ and B_{c} are as defined above), a M_{c0}-B₃-B_{c}- group (wherein M_{c0}, B₃ and B_{c} are as defined above) or a M_{c0}-B_{c}- group (wherein M_{c0} and B_{c} are as defined above),
(2) W_{A0} represents an oxygen atom or a -NT_{A0}- group wherein T_{A0} represents a hydrogen atom, an A₉'- group (wherein A₉' is as defined above), a D₅-R₄- group (wherein D₅ and R₄ are as defined above) or a M_{cO}- group (wherein M_{cO} is as defined above),
(3) K_{A0} represents a hydrogen atom, a halogen atom or a C1-C10 alkyl group, L_{A0} represents a hydrogen atom, a C1-C10 alkyl group or a M_{bo}- group (wherein M_{bo} is as defined above), or K_{A0} and L_{A0} may form a C3-C10 alkylene group, or a C4-C10 alkenelyne group optionally substit-uted with 1 or more same or different Mₐ- groups (wherein Mₐ is as defined above)],
a group represented by formula (III-2): 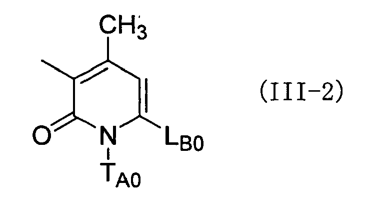 [wherein T_{A0} is as defined above, and L_{B0} represents a hydroxyl group or a methyl group],
a group represented by formula (III-3): 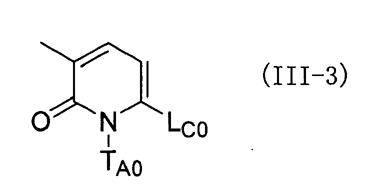 [wherein T_{A0} is as defined above, and L_{C0} represents a Cl-C10 alkyl group],
a group represented by formula (III-4): 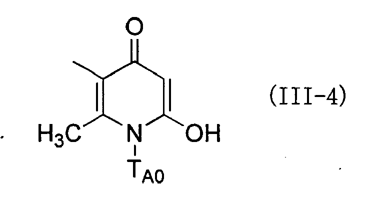 [wherein T_{A0} is as defined above],
a group represented by formula (III-5): 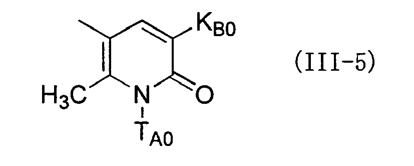 [wherein T_{A0} is as defined above, and K_{B0} represents a cyano group or a UOCO- group (wherein U represents a hydrogen atom or a C1-C10 alkyl group)],
a group represented by formula (III-6): 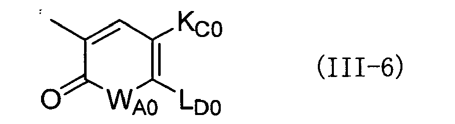 [wherein W_{A0} is as defined above, and K_{C0} and L_{D0} form a C3-C10 alkylene group, or a C4-C10 alkenylyne optionally substituted with 1 or more same or different Mₐ- groups (wherein Mₐ is as defined above)],
a group represented by formula (III-7): 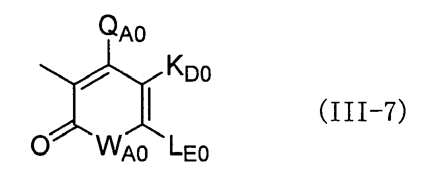 [wherein Q_{A0} and W_{A0} are as defined above, K_{D0} and L_{E0} form a -V_{A0}=V_{A0}'-V_{A0}=V_{A0}'''- group {wherein V_{A0}, V_{A0}' , V_{A0}" and V_{A0}'" are same or different, and represent a methine group optionally substituted with a Mₐ- group (wherein Mₐ is as defined above), or a -N= group, and at least one of V_{A0}, V_{A0}', V_{A0}" and V_{A0}''' represents a -N= group}],
a group represented by formula (III-8): 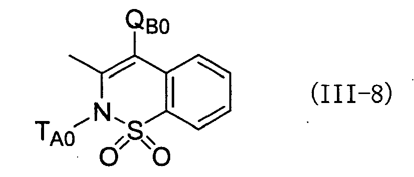 [wherein T_{A0} is as defined above, and Q_{B0} represents a hydroxyl group, a A₉-B₆-O- group (wherein A₉ and B₆ are as defined above), an A₇"-SO₂-O- group (wherein A₇" is as defined above), an A₈-SO₂-O- group, (wherein A₈ is as defined above, provided that A₈ is not a hydrogen), a R₁R₁'N-SO₂-O- group (wherein R₁ and R₁' are as defined above), a (b₀)-SO₂-O- group (wherein (b₀) is as defined above), an A₉'-O- group (wherein A₉' is as defined above), a D₅-R₄-O- group (wherein D₅ and R₄ are as defined above), a M_{cO}-B₃-O- group (wherein M_{cO} and B₃ are as defined above) or a M_{c0}-O- group (wherein M_{c0} is as defined above)], or
a group represented by formula (III-9): 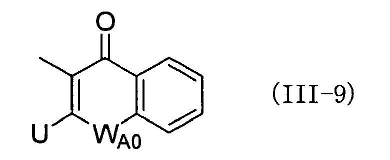 [wherein U and W_{A0} are as defined above], provided that when A0 is a furan ring or a thiophene ring, p and q are not 0 at the same time; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

4. A cinnamoyl compound represented by the formula (IV): 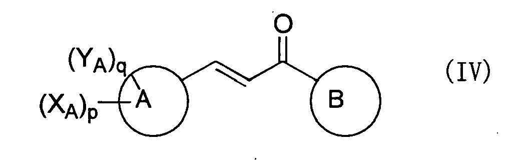 wherein,
I. A represents an aromatic 5-membered ring, or an aromatic 6-membered ring having two or more nitrogen atoms;
II. in (X_{A})ₚ, X_{A} represents a group included in any group of the following A group to the N group, p represents 0, 1, 2 or 3, and when p is not less than 2, X_{A}s are the same or different;
(1) the A group:
a D₁-R₄- group, wherein D₁ represents a (R₁-(O)ₖ-(A₁N-(O)ₖ'- group [wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), or a C3-C10 alkenyl group, or a C3-C10 alkynyl group, k represents 0 or 1, A₁ represents a R₃-(CHR₀)ₘ-(B₂-B₃)_{m'}- group {wherein R₃ represents a hydrogen atom, or a C1-C10 alkyl group optionally substituted with a halogen atom or a R₂-B₁-group (wherein R₂ and B₁ are as defined above), or a C2-C10 alkenyl group, or a C2-C10 alkynyl group, R₀ represents a hydrogen atom, a C1-C10 alkyl group or a C2-C10 haloalkyl group, m represents 0 or 1, B₂ represents a single bond, an oxy group, a thio group or a -N((O)ₙR₁')- group (wherein R₁' is the same as or different from R₁ and has the same meaning as R₁ has, and n represents 0 or 1), B₃ represents a carbonyl group, a thiocarbonyl group or a sulfonyl group, m' represents 0 or 1, and B₃ is not a sulfonyl group when m is 0 and R₃ is a hydrogen atom}, and k' represents 0 or 1], and R₄ represents a C1-C10 alkylene group, provided that a R₀'R₀"N-R₄- group (wherein R₀' and R₀" are the same as or different from R₀ and have the same meaning as R₀ has, and R₄ is as defined above) is excluded,
a D₂-R₄- group, wherein D₂ represents a cyano group, a R₁R₁'NC(=N-(O)ₙ-A₁)- group (wherein R₁, R₁', n and A₁ are as defined above), an A₁N=C(-OR₂)- group (wherein A₁ and R₂ are as defined above) or a NH₂-CS- group, and R₄ is as defined above,
a D₃-R₄- group, wherein D₃ represents a nitro group or a R₁OSO₂- group (wherein R₁ is as defined above), and R₄ is as defined above, and
a R₁OSO₂- group, wherein R₁ is as defined above;
(2) the B group: an (a)-group represented by 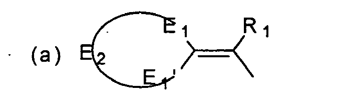 wherein E₁ and E₁' represent a methylene group optionally substituted with a C1-C10 alkyl group or a C1-C10 alkoxy group, or a carbonyl group, provided that E₁ and E₁' are not a carbonyl group at the same time, E₂ represents a C2-C10 alkylene group optionally substituted with an oxy group, a thio group, a sulfinyl group, a sulfonyl group or a - NR₁'- group (wherein R₁' is as defined above), or a C3-C10 alkenylene group optionally substituted with an oxy group, a thio group, a sulfinyl group, a sulfonyl group or a - NR₁'- group (wherein R₁' is as defined above), and R₁ is as defined above;
(3) the C group: a C2-C10 alkenyl group substituted with a halogen atom, a R₂-B₁- group (wherein R₂ and B₁ are as defined above), a D₄-R₄- group [wherein D₄ represents a hydroxyl group or an A₁-O- group (wherein A₁ is as defined above), and R₄ is as defined above], a D₅- group [wherein D₅ represents an O=C(R₃)- group (wherein R₃ is as defined above), an A₁-(O)ₙ-N=C(R₃)- group (wherein A₁, n and R₃ are as defined above), a R₁-B₀-CO-R₄-(O)ₙ-N=C(R₃)- group {wherein R₁, R₄, n and R₃ are as defined above, and B₀ represents an oxy group, a thio group or a -N((O)ₘR₁')-group (wherein R₁' and m are as defined above)}, a D₂-R₄-(O)ₙ-N=C(R₃)- group (wherein D₂, R₄, n and R₃ are as defined above) or a R₁A₁N-N=C(R₃)- group (wherein R₁, A₁ and R₃ are as defined above)], a R₁A₁N-O-R₄- group (wherein R₁, A₁ and R₄ are as defined above), a R₁ (A₁-(O)n-)N- group (wherein R₁, A₁ and n are as defined above), a D₂- group (wherein D₂ is as defined above) or a D₃- group (wherein D₃ is as defined above);
(4) the D group: a C2-C10 alkynyl group substituted with a (b)-R₄- group [wherein in (b) 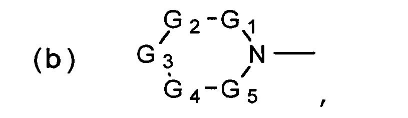 G₁, G₂, G₄ and G₅ represent a methylene group which is connected with the adjacent atom via a single bond and which may be optionally substituted with a methyl group, or a methine group which is connected with the adjacent atom via a double bond and which may be optionally substituted with a methyl group, and G₃ represents a single bond, or a double bond, or a C1-C10 alkylene group optionally substituted with a methyl group, an oxy group, a thio group, a sulfinyl group, a sulfonyl group or a -NR₁- group (wherein R₁ is as defined above), or a C2-C10 alkenylene group optionally substituted with a methyl group, an oxy group, a thio group, a sulfinyl group, a sulfonyl group or a -NR₁- group (wherein R₁ is as defined above); and R₄ is as defined above], a (c)-R₄- group [wherein, in (c) 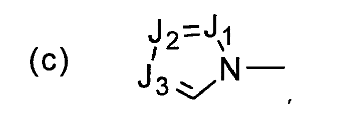 J₁, J₂ and J₃ are the same or different, and represent a methine group optionally substituted with a methyl group, or a nitrogen atom; and R₄ is as defined above), a halogen atom, a R₂-B₁-R₄- group (wherein R₂, B₁ and R₄ are as defined above), a D₄-R₄- group (wherein D₄ and R₄ are as defined above), a D₅- group (wherein D₅ is as defined above), a D₁-R₄- group (wherein D₁ and R₄ are as defined above), a D₂- group (wherein D₂ is as defined above) or a D₃-R₄- group (wherein D₃ and R₄ are as defined above);
(5) the E group: an A₂-CO-R₅- group, provided that R₅ is not a vinylene group when A₂ is a hydroxyl group, [wherein A₂ represents
(i) an A₃-B₄- group
wherein A₃ represents a hydrogen atom, or a C1-C10 alkyl group, or a C2-C10 haloalkyl group, or a C2-C10 alkenyl group optionally substituted with a halogen atom, or a C3-C10 alkynyl group optionally substituted with a halogen atom, or Rₐ-(R₄)ₘ- group (wherein Rₐ represents a phenyl group, a pyridyl group, a furyl group or a thienyl group, which may be optionally substituted with a halogen atom, a C1-C10 alkyl group, a C1-C10 alkoxy group or a nitro group, and R₄ and m are as defined above), or a C1-C10 alkyl group substituted with a (b)-R₄- group (wherein (b) and R₄ are as defined above), a (c)-R₄- group (wherein (c) and R₄ are as defined above), a R₂-B₁-R₄- group (wherein R₂, B₁ and R₄ are as defined above), a D₄-R₄- group (wherein D₄ and R₄ are as defined above), a D₅- group (wherein D₅ is as defined above), a D₁-R₄- group (wherein D₁ and R₄ are as defined above), a D₂- group (wherein D₂ is as defined above), a D₃-R₄- group (wherein D₃ and R₄ are as defined above) or an A₄-SO₂-R₄- group {wherein A₄ represents a (b)- group (wherein (b) is as defined above), a (c)- group (wherein (c) is as defined above) or a R₁R₁'N- group (wherein R₁ and R₁' are as defined above), and R₄ is as defined above}, and B₄ represents an oxy group, a thio group or a -N((O)ₘR₁)-group (wherein R₁ and m are as defined above), provided that A₃ is not a hydrogen atom when B₄ is a thio group;
(ii) a R₁-B₄-CO-R₄-B₄'- group, wherein R₁, B₄ and R₄ are as defined above, B₄' is the same as or different from B₄ and has the same meaning as B₄ has, provided that R₂ is not a hydrogen atom when B₄ is a thio group, or
a D₂-R₄-B₄- group, wherein D₂, R₄ and B₄ are as defined above;
(iii) a R₂-SO₂-NR,- group, wherein R₂ is as defined above, provided that a hydrogen atom is excluded, and R₁ is as defined above;
(iv) a (b)- group, wherein (b) is as defined above,
(v) a (c)- group, wherein (c) is as defined above, or
(vi) a R₁A₁N-NR₁'- group, wherein R₁, A₁ and R₁' are as defined above; and
R₅ represents a C2-C10 alkenylene group optionally substituted with a halogen atom or a C2-C10 alkynylene group];
(6) the F group: an A₅-B₅-R₆- group
[wherein A₅ represents a C2-C10 alkyl group substituted with a D₄- group (wherein D₄ is as defined above), a D₁-group (wherein D₁ is as defined above), a D₃- group (wherein D₃ is as defined above) or an A₄-SO₂- group (wherein A₄ is as defined above), or a C1-C10 alkyl group substituted with a R₂-B₁- group (wherein R₂ and B₁ are as defined above), a D₂- group (wherein D₂ is as defined above), a D₅- group (wherein D₅ is as defined above) or an A₂-CO- group (wherein A₂ is as defined above),
B₅ represents a B₁- group (wherein B₁ is as defined above) or a -NA₁- group (wherein A₁ is as defined above), and
R₆ represents a single bond or a C1-C10 alkylene group];
(7) the G group: an A₆-B₅-R₆- group
[wherein A₆ represents an (a)-R₄- group (wherein (a) and R₄ are as defined above), or a C2-C10 alkenyl group, or a C2-C10 alkynyl group, or a C2-C10 alkenyl group substituted with a halogen atom, a R₂-B₁- group (wherein R₂ and B₁ are as defined above), a D₅- group (wherein D₅ is as defined above), a D₂- group (wherein D₂ is as defined above) or an A₂-CO- group (wherein A₂ is as define above), or a C2-C10 alkynyl group substituted with a halogen atom, a R₂-B₁-group (wherein R₂ and B₁ are as defined above), a D₅- group (wherein D₅ is as defined above), a D₂- group (wherein D₂ is as defined above) or an A₂-CO- group (wherein A₂ is as defined above), or a C3-C10 alkenyl group substituted with a (b)- group (wherein (b) is as defined above), a (c)-group (wherein (c) is as defined above), a D₄- group (wherein D₄ is as defined above), a D₁- group (wherein D₁ is as defined above) or a D₃- group (wherein D₃ is as defined above), or a C3-C10 alkynyl group substituted with a D₄-group (wherein D₄ is as defined above), a D₁- group (wherein D₁ is as defined above) or a D₃- group (wherein D₃ is as defined above), and B₅ and R₆ are as defined above];
(8) the H group:
a D₂-N(-(O)ₙ-A₁)-R₆- group, wherein D₂, n, A₁ and R₆ are as defined above,
a D₂- group, wherein D₂ is as defined above, provided that a cyano group is excluded,
a R₁(R₁'(O)ₙ)N-CR₁"=N-R₆- group, wherein R₁, R₁', n and R₆ are as defined above, R₁" is the same as or different from R₁ and has the same meaning as R₁ has,
a R₁-(O)ₙ-N=CR₁'-NR₂-R₆- group, wherein R₁, n, R₁', R₂ and R₆ are as defined above,
a R₂-B₃-NR₁-CO-NR₁'-R₆- group, wherein R₂, B₃, R₁, R₁' and R₆ are as defined above,
a D₂-CO-NR₁-R₆- group, wherein D₂, R₁ and R₆ are as defined above, or
an A₂-COCO-NR₁-R₆- group, wherein A₂, R₁ and R₆ are as defined above;
(9) the I group:
an A₇-B₆-N((O)ₙR₁)-R₆- group [wherein A₇ represents a C2-C10 alkenyl group optionally substituted with a halogen atom, or a C2-C10 alkynyl group, or a C3-C10 haloalkynyl group, or a R₂-B₁-R₄- group (wherein R₂, B₁ and R₄ are as defined above), or a D₉-R₄- group (wherein D₄ and R₄ are as defined above), or a D₅-R₄- group (wherein D₅ and R₄ are as defined above), or a D₁-R₄- group (wherein D₁ and R₄ are as defined above), or a (b)-R₄- group (wherein (b) and R₄ are as defined above), or a (c)-R₄- group (wherein (c) and R₄ are as defined above), or a D₂-R₄- group (wherein D₂ and R₄ are as defined above), or a D₃-R₄- group (wherein D₃ and R₄ are as defined above), or an A₄-SO₂-R₄- group (wherein A₄ and R₄ are as defined above), or an A₂-CO-R₄- group (wherein A₂ and R₄ are as defined above), B₆ represents a carbonyl group or a thiocarbonyl group, and n, R₁ and R₆ are as defined above],
an A₈-CS-N((O)ₙR₁)-R₆- group [wherein A₈ represents a hydrogen atom or a C1-C10 alkyl group optionally substituted with a halogen atom, and n, R₁ and R₆ are as defined above],
an A₇'-B₂'-B₃-N((O)ₙR₁)-R₆- group, [wherein A₇' represents a C3-C10 alkenyl group optionally substituted with a halogen atom, or a C3-C10 alkynyl group optionally substituted with a halogen atom, or a R₂-B₁-R₄'- group (wherein R₂ and B₁ are as defined above, and R₄' represents a C2-C10 alkylene group), or a D₄-R₄'- group (wherein D₄ and R₄' are as defined above), or a D₁-R₄'- group (wherein D₁ and R₄' are as defined above), or a (b)-R₄'- group (wherein (b) and R₄' are as defined above), or a (c)-R₄'- group (wherein (c) and R₄' are as defined above), or a D₂-R₄-group (wherein D₂ and R₄ are as defined above), or a D₃-R₄' - group (wherein D₃ and R₄' are as defined above), or an A₂-CO-R₄- group (wherein A₂ and R₄ are as defined above), B₂' represents an oxy group, a thio group or a -N((O)ₙ,R₁')-group (wherein n' is the same as or different from n and has the same meaning as n has, and R₁' is as defined above), and B₃, n, R₁ and R₆ are as defined above],
an A₈'-B₂'-CS-N((O)ₙR₁)-R₄- group [wherein A₈' represents a C1-C10 alkyl group or a C2-C10 haloalkyl group, B₂' is as defined above, and n, R₁ and R₆ are as defined above],
an A₈'-S-B₃'-N((O)ₙR₁)-R₆- group [wherein A₈', n, R₁ and R₆ are as defined above, and B₃' represents a carbonyl group or a sulfonyl group] or
an A₇''-SO₂-N((O)ₙR₁)-R₆- group [wherein A₇'' represents a C2-C10 alkenyl group, or a C3-C10 alkenyl group substituted with a halogen atom, or a C3-C10 alkynyl group optionally substituted with a halogen atom, or a R₂-B₁-R₄'- group (wherein R₂, B₁ and R₄' are as defined above), or a D₄-R₄' - group (wherein D₄ and R₄' are as defined above), or a D₅-R₄-group (wherein D₅ and R₄ are as defined above), or a D₁-R₄'-group (wherein D₁ and R₄' are as defined above), or a (b)-R₄'- group (wherein (b) and R₄' are as defined above), or a (c)-R₄'- group (wherein (c) and R₄' are as defined above), or a D₂-R₄- group (wherein D₂ and R₄ are as defined above), or a NO₂-R₄- group (wherein R₄ is as defined above), or an A₂-CO-R₄- group (wherein A₂ and R₄ are as defined above), and n, R₁ and R₄ are as defined above];
(10) the J group:
an A₇-CO- group (wherein A₇ is as defined above),
an A₉-CS- group (wherein A₉ represents A₇ or A₈),
an A₉' (O)ₘN=C(A₉)- group (wherein A₉' represents A₇' or A₈', and m and A₉ are as defined above),
a D₂-CO- group (wherein D₂ is as defined above),
an A₂-COCO- group (wherein A₂ is as defined above),
an A₉-CO-B₁'-R₆- group (wherein A₉ and R₆ are as defined above, and B₁' represents an oxy group or a thio group, provided that A₉ is not A₈ when B₁' is an oxy group),
an A₉-CS-B₁'-R₆- group (wherein A₉, B₁' and R₆ are as defined above),
an A₇"-SO₂-B₁'-R₆- group (wherein A₇", B₁' and R₆ are as defined above),
an A₈-SO₂-B₁'-R₆- group (wherein A₈, B₁' and R₆ are as defined above, provided that A₈ is not a hydrogen atom),
an A₉' -B₂' -B₃-B₁' -R₆- group (wherein A₉' , B₂' , B₃, B₁' and R₆ are as defined above), and
a C2-C10 alkenyl group substituted with a (b)- group (wherein (b) is as defined above) or a (c)- group (wherein (c) is as defined above);
(11) the K group: an A₁₀-N((O)ₙR₁)-CO-R₆- group [wherein A₁₀ represents a hydrogen atom (provided that n is not 0), an A₇"-SO₂- group (wherein A₇" is as defined above), an A₈-SO₂- group (wherein A₈ is as defined above, provided that A₈ is not a hydrogen atom), an A₉'O- group (wherein A₉' is as defined above, provided that n is not 1), an A₉'-group (wherein A₉' is as defined above, provided that A₈' is excluded when n is 0), a R₂OCH₂- group (wherein R₂ is as defined above), an A₂-CO-R₄- group (wherein A₂ and R₄ are as defined above) or an A₂-CO-CH(CH₂CO-A₂)- group (wherein A₂ is as defined above), and n, R₁ and R₆ are as defined above];
(12) the L group:
an A₁₀'-N((O)ₙR₁)-SO₂-R₆- group [wherein A₁₀' represents a hydrogen atom (provided that n is not 0), an A₉'O- group (wherein A₉' is as defined above, provided that n is not 1), an A₉'- group (wherein A₉' is as defined above, provided that A₈' is excluded when n is 0), a R₂-CO- group (wherein R₂ is as defined above), an A₂-CO-R₄- group (wherein A₂ and R₄ are as defined above) or an A₂-CO-CH(CH₂CO-A₂)- group (wherein A₂ is as defined above), and n, R₁ and R₆ are as defined above],
an A₉''R₁N-SO₂-N((O)ₙR₁')-R₆- group [wherein A₉" represents a hydrogen atom or an A₉'- group (wherein A₉' is as defined above), and R₁, n, R₁' and R₆ are as defined above], and
a (b)-SO₂-N((O)ₙR₁')-R₆- group [wherein (b), n, R₁' and R₆ are as defined above];
(13) the M group:
a R₁(R₂S)C=N-R₆- group (wherein R₁, R₂ and R₆ are as defined above),
a R₂B(R₂'B')C=N-R₆- group (wherein R₂ and R₆ are as defined above, R₂' is the same as or different from R₂ and has the same meaning as R₂ has, and B and B' are the same or different and represent an oxy group or a thio group),
a R₁R₁'N-(R₂S)C=N-R₆- group (wherein R₁, R₁', R₂ and R₆ are as defined above),
a R₁N=C(SR₂)-NR₂'-R₆- group (wherein R₁, R₂, R₂' and R₆ are as defined above), and
a R₁(R₁'O)N-R₆- group (wherein R₁, R₁' and R₆ are as defined above);
(14) the N group: an A₁₁-P(=O) (OR₁')-R₄- group
[wherein A₁₁ represents a R₁- group (wherein R₁ is as defined above), a R₁O-R₆- group (wherein R₁ and R₆ are as defined above) or a R₁OCO-CHR₀- group (wherein R₁ and R₀ are as defined above), and R₁' and R₄ are as defined above];
III. in (Y_{A})_{q}, Y_{A} represents a group included in the following X group or Y group, q is 0, 1, 2 or 3, the sum of p (wherein p is as defined above) and q is not more than 3, and when q is not less than 2, Y_{A}s are the same or different, and when q is not less than 2, adjacent two same or different Y_{A}s may together form a group included in the Z group to be fused to the A ring;
(1) the X group: a Mₐ-group, wherein Mₐ represents a R_{b}- group (wherein R_{b} represents a C1-C10 alkyl group optionally substituted with a halogen atom), a halogen atom, a nitro group, a cyano group, a R_{c}-Bₐ-R_{d}- group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH- group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}-group (wherein Rₑ, Rₑ' and R_{d} are as defined above), a RₑRₑ'N-CO-NRₑ"-R_{d}- group (wherein Rₑ, Rₑ' and Rₑ'' are the same or different, Rₑ and Rₑ' are as defined above, Rₑ'' has the same meaning as Rₑ has, and R_{d} is as defined above), a RₑRₑ'N-C(=NRₑ")-NRₑ'''-R_{d}- group (wherein Rₑ, Rₑ', Rₑ" and Rₑ''' are the same or different, Rₑ, Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
(2) the Y group: a M_{b}-R_{d}- group, wherein M_{b} represents a M_{c}-group [wherein M_{c} represents a M_{d}-R_{d}'- group {wherein M_{d} represents a phenyl group optionally substituted with a Mₐ- group (wherein Mₐ is as defined above), a pyridyl group optionally substituted with a Mₐ- group (wherein Mₐ is as defined above), a naphthyl group optionally substituted with a Mₐ- group (wherein Mₐ is as defined above), a (b)-group (wherein (b) is as defined above), a (c)- group (wherein (c) is as defined above), a (d)- group 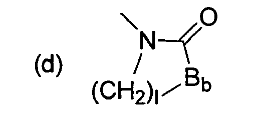 (wherein 1 is 2, 3 or 4, B_{b} represents an oxy group or a thio group) or an (e)- group 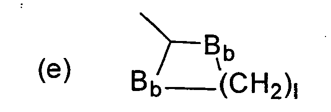 (wherein 1 and B_{b} are as defined above)}, and R_{d}' is the same as or different from R_{d} and has the same meaning as R_{d} has], a M_{c}-Bₐ- group (wherein M_{c} and Bₐ are as defined above), a M_{c}-CO- group (wherein M_{c} is as defined above), a M_{c}-CO-O- group (wherein M_{c} is as defined above), a M_{c}O-CO-group (wherein M_{c} is as defined above), a M_{c}RₑN- group (wherein M_{c} and Rₑ are as defined above), a M_{c}-CO-NRₑ- group (wherein M_{c} and Rₑ are as defined above), a M_{c}O-CO-NRₑ-group (wherein M_{c} and Rₑ are as defined above), a M_{c}RₑN-CO-group (wherein M_{c} and Rₑ are as defined above), a M_{c}RₑN-CO-NRₑ'- group (wherein M_{c}, Rₑ and Rₑ' are as defined above), a M_{c}RₑN-C(=NRₑ')-NRₑ"- group (wherein M_{c}, Rₑ, Rₑ' and Rₑ" are as defined above), a M_{c}-SO₂-NRₑ- group (wherein M_{c} and Rₑ are as defined above) or a M_{c}RₑN-SO₂- group (wherein M_{c} and Rₑ are as defined above), and
R_{d} is as defined above;
(3) Z group: a -Yₐ"=C(Yₐ)-Yₐ'- group (wherein Yₐ represents a hydrogen atom, or a C1-C10 alkyl group optionally substituted with a halogen atom, or a C1-C10 alkoxy group, Yₐ' represents an oxy group, or a thio group, or an imino group optionally substituted with a C1-C10 alkyl group, and Yₐ" represents a -N= group or a methine group),
a C3-C10 alkylene group, and
a -Y_{b}=Y_{b}'-Y_{b}"=Y_{b}'''- group, wherein Y_{b}, Y_{b}', Y_{b}" and Y_{b}''' are the same or different, and represent a methine group optionally substituted with a Mₐ- group (wherein Mₐ is as defined above), or a -N= group;
IV. B is
a group represented by formula (IV-1): 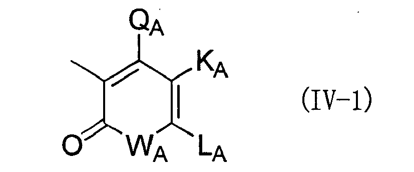 wherein,
(1) Q_{A} represents a hydroxyl group, a (b)- group (wherein (b) is as defined above), an A₉-B₆-B_{c}- group [wherein A₉ and B₆ are as defined above, and B_{c} represents an oxy group or a -N((O)ₘR₁)- group (wherein m and R₁ are as defined above), provided that B_{c} is not a sulfonyl group when A₉ is a hydrogen atom], an A₇"-SO₂-B_{c}- group (wherein A₇" and B_{c} are as defined above), an A₈-SO₂-B_{c}- group (wherein A₈ and B_{c} are as defined above, provided that A_{B} is not a hydrogen atom), a R₁R₁'N-SO₂-B_{c}- group (wherein R₁, R₁' and B_{c} are as defined above), a (b₀)-SO₂-O- group (wherein (b) and B_{c} are as defined above), an A₉'-B_{c}- group (wherein A₉' and B_{c} are as defined above), a D₅-R₄-B_{c}- group (wherein D₅ ,R₄ and B_{c} are as defined above), a M_{c}-B₃-B_{c}-group (wherein M_{c}, B₃ and B_{c} are as defined above) or a M_{c}-B_{c}- group (wherein M_{c} and B_{c} are as defined above),
(2) W_{A} represents an oxygen atom or a -NT_{A}- group [wherein T_{A} represents a hydrogen atom, an A₉'- group (wherein A₉' is as defined above), a D₅-R₄- group (wherein D₅ and R₄ are as defined above) or a M_{c}- group (wherein M_{c} is as defined above)], and
(3) K_{A} represents a hydrogen atom, a halogen atom or a C1-C10 alkyl group, L_{A} represents a hydrogen atom, a C1-C10 alkyl group or a M_{b}- group (wherein M_{b} is as defined above), or K_{A} and L_{A} may form a C3-C10 alkylene group or a - C(Mₐ')=C(Mₐ"-C(Mₐ''')=C(Mₐ'''')- group (wherein Mₐ' , Mₐ'', Mₐ''' and Mₐ'''' are the same or different, and are the same as or different from Mₐ, and represent a hydrogen atom or Mₐ);
a group represented by formula (IV-2): 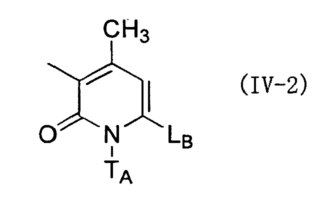
wherein T_{A} is as defined above, and L_{B} represents a hydroxyl group or a methyl group;
a group represented by formula (IV-3): 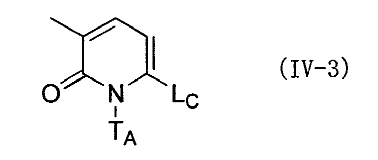 wherein T_{A} is as defined above, and L_{C} represents a C1-C10 alkyl group;
a group represented by formula (IV-4): 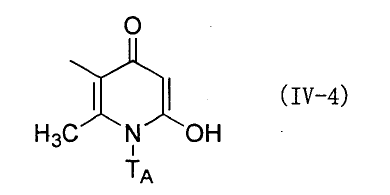 wherein T_{A} is as defined above;
a group represented by formula (IV-5): 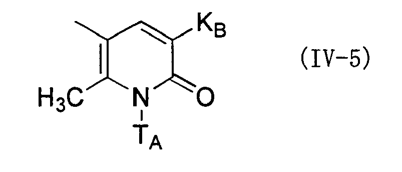 wherein T_{A} is as defined above, and K_{B} represents a cyano group or a UOCO- group (wherein U represents a hydrogen atom or C1-C10 alkyl group);
a group represented by formula (IV-6): 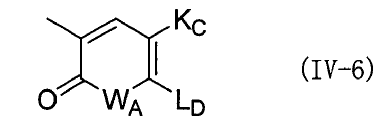 wherein W_{A} is as defined above, and K_{C} and L_{D} form a C3-C10 alkylene group, or a C4-C10 alkenylene group optionally substituted with 1 or more same or different Mₐ- groups (wherein Mₐ is as defined above);
a group represented by formula (IV-7): 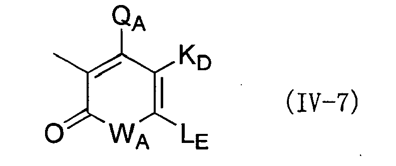 wherein Q_{A} and W_{A} are as defined above, and K_{D} and L_{E} form a -V_{A}=V_{A}'-V_{A}''=V_{A}'''- group {wherein V_{A}, V_{A}' , V_{A}' ' and V_{A}' ' ' are the same or different, and represent a methine group optionally substituted with a Mₐ- group (wherein Mₐ is as defined above), or a -N= group, and at least one of V_{A} V_{A}' V_{A}'' and V_{A}' ' ' represents a -N= group } ;
a group represented by formula (IV-8): 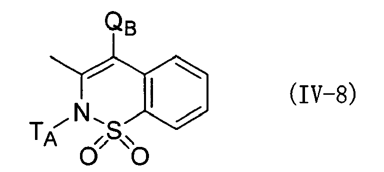
wherein T_{A} is as defined above, Q_{B} represents a hydroxyl group, an Ag-B₆-O- group [wherein A₉ and B₆ are as defined above], an A₇"-SO₂-O- group (wherein A₇" is as defined above), an A₈-SO₂-O- group (wherein A₈ is as defined above, provided that A₈ is not a hydrogen atom), a R₁R₁'N-SO₂-O-group (wherein R₁ and R₁' are as defined above), a (b)-SO₂-O- group (wherein (b) is as defined above), an Ag'-O- group (wherein A₉' is as defined above), a D₅-R₄-O- group (wherein and R₄ are as defined above), a M_{c}-B₃-O- group (wherein M_{c} and B₃ are as defined above), or a M_{c}-O- group (wherein M_{c} is as defined above); or
a group represented by formula (IV-9): 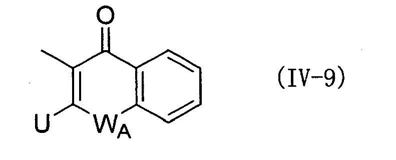 wherein U and W_{A} are as defined above, provided that when A is a furan ring or a thiophene ring, p and q are not 0 at the same time; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range].

5. A cinnamoyl compound represented by the formula (V): 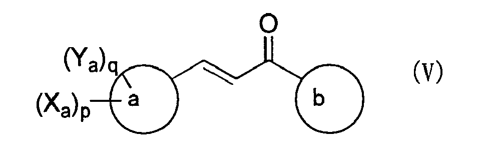 wherein,
I. a represents a thiophene ring, a furan ring, a pyrrole ring, a pyrazole ring, a 1,2,3-triazole ring, a tetrazole ring, an isoxazole ring, a thiazole ring, a pyridazine ring or a pyrimidine ring; Xₐ represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an a₀-r₁-b-r₁'- group {wherein a₀ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a r₂O-CO- group (wherein r₂ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), a₁-NH-CO- group (wherein a₁ represents a C2-C10 alkyl group substituted with a C1-C10 alkoxy group), an a₁'-CO- group (wherein a₁' represents a morpholino group), a rr'N-CH₂- group (wherein r and r' are as defined above), a r₀-(O)₁-CONH-CH₂- group (wherein r₀ represents a C1-C10 alkyl group, and 1 represents 0 or 1), a r-OCH₂- group (wherein r is as defined above), a r₀-CO- group (wherein r₀ is as defined above), a cyano group or a sulfomethyl group, r₁ represents a C1-C10 alkylene group, r₁' represents a single bond or a C1-C10 alkylene group, and b represents an oxy group, a thio group, a sulfinyl group, a sulfonyl group, or an imino group optionally substituted with a methyl group}, or an a₂-y-CO-NH- group (wherein a₂ represents a C2-C10 alkyl group substituted with a C1-C10 alkoxy group, and y represents an oxy group or an imino group), or a rO-COCO-NH- group (wherein r is as defined above), or an a₃-z-NH- group (wherein a₃ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an a₄-Nr'CO- group {wherein a₄ represents a C1-C10 alkoxy group, or a C3-C10 alkenyloxy group, or a r₀-SO₂- group (wherein r₀ is as defined above), or a C2-C10 alkyl group substituted with a hydroxyl group or a C1-C10 alkoxy group, or a C2-C10 alkyl group substituted with a r₀r₀'N- group (wherein r₀ is as defined above, and r₀' is the same as or different from r₀, and represents the same meaning as r₀ has), or a C1-C10 alkyl group substituted with a rO-CO- group (wherein r is as defined above), a cyano group or an aminocarbonyl group, or a rO-CO-(rO-COCH₂)CH- group (wherein r is as defined above), and r' is as defined above}, or an a₅-NHSO₂- group (wherein a₅ represents a C2-C10 alkyl group substituted with a C1-C10 alkoxyl group), or a r₀ON=CH- group (wherein r₀ is as defined above), or a r₀NHCSNH- group (wherein r₀ is as defined above), or a r₀NHC(-Sr₀')=N- group (wherein r₀ and r₀' are as defined above), or a (rO)₂P(=O)CH₂- group (wherein r is as defined above), p represents 0, 1, 2 or 3 and, and when p is not less than 2, Xₐs are same or different,
Yₐ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an a₀'-b'- group (wherein a₀' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a r₀r₀'N- group (wherein r₀ and r₀' are as defined above), or a r₀CO-NH- group (wherein r₀ is as defined above), or a r₀r₀'NCONH- group (wherein r₀ and r₀' are as defined above), or a rr'NCO- group (wherein r and r' are as defined above), or a hydroxyl group, q represents 0, 1, 2 or 3, and when q is not less than 2, Yₐs are the same or different, and when q is not less than 2, adjacent Yₐs may be fused to the a ring to form a 4,5,6,7-tetrahydrobenzo[b]thiophene ring;
II. b is
a group represented by formula (V-1): 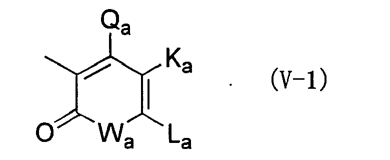
wherein Qₐ represents a rₐ-O- group {rₐ represents a hydrogen atom, or a C1-C10 alkyl group, or a C3-C10 alkenyl group, or a C3-C10 alkynyl, a C1-C10 alkyl group substituted with a r₀r₀'N-CH₂- group (wherein r₀ and r₀' are the same or different), a rOCH₂ group (wherein r is as defined above), a r₀-CO- group (wherein r₀ is as defined above), a C1-C10 alkoxycarbonyl group, a carboxy group, an aminocarbonyl group or a cyano group, or a r₃-r₁- group (wherein r₃ represents a phenyl group or a pyridyl group, and r₁ is as defined above)}, or a piperidino group, or a morpholino group, or a r₄r₄'N-group (wherein r₄ and r₄' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); Wₐ represents an oxygen atom or a - NTₐ- group [wherein Tₐ represents a r_{b}- group (wherein r_{b} is the same as or different from rₐ, and represents the same meaning as rₐ has) or a r₃' - group (wherein r₃' is the same as or different from r₃, and represents the same meaning as r₃ has)], and Kₐ represents a hydrogen atom, halogen atom or C1-C10 alkyl group, and Lₐ represents hydrogen atom or C1-C10 alkyl group, or Kₐ and Lₐ may form a 1,3-butadienylene group;
a group represented by formula (V-2): 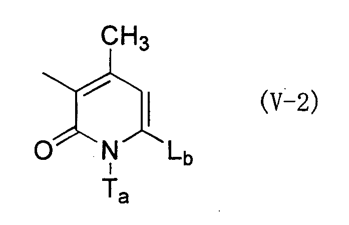 wherein Tₐ is as defined above, and L_{b} represents a hydroxyl group or a methyl group;
a group represented by formula (V-3): 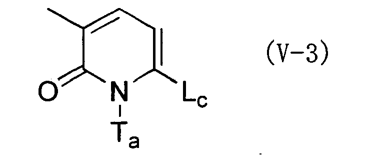 wherein Tₐ is as defined above, and Lₑ represents a C1-C10 alkyl group;
a group represented by formula (V-4): 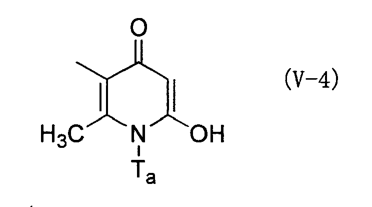 wherein Tₐ is as defined above;
a group represented by formula (V-5): 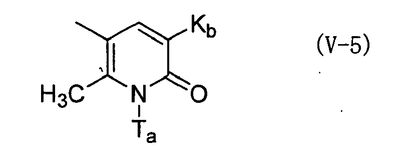 wherein Tₐ is as defined above, and K_{b} represents a cyano group or a UOCO- group (wherein U represents a hydrogen atom or C1-C10 alkyl group);
a group represented by formula (V-6): 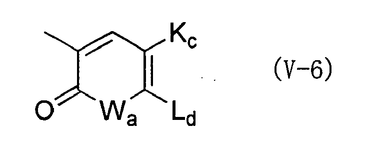 wherein Wₐ is as defined above, and K_{c} and L_{d} form a C3-C10 alkylene group or a C4-C10 alkenylene group;
a group represented by formula (V-7): 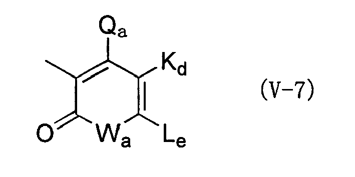 wherein Qₐ and Wₐ are as defined above, and K_{d} and Lₑ form a -Vₐ=Vₐ'-Vₐ''=Vₐ''' - group (wherein Vₐ, Vₐ', Vₐ'' and Vₐ''' are the same or different, and represent a methine group, or a -N= group, and at least one of Vₐ, Vₐ' , Vₐ' ' and Vₐ' ' ' represents a -N= group);
a group represented by formula (V-8): 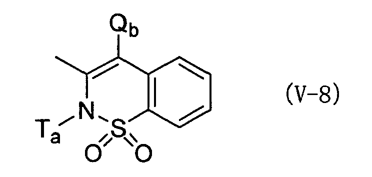 wherein Tₐ is as defined above, and Q_{b} represents a rₐ-O-group (wherein rₐ is as defined above); or
a group represented by formula (V-9): 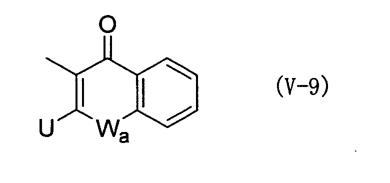 wherein U and Wₐ are as defined above, provided that when A is a furan ring or a thiophene ring, p and q are not 0 at the same time; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

6. A cinnamoyl compound represented by the formula (VI): 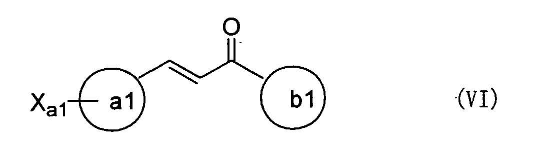 wherein
a1 represents a thiophene ring, a furan ring, a pyrrole ring or a thiazole ring,
Xₐ₁ represents an a₀'-r₁-b₀-r₁'- group {wherein a₀' represents a rO-CO- group (wherein r represents a hydrogen atom or a C1-C10 alkyl group), a r₀r₀'N-CH₂- group (wherein r₀ and r₀' are the same or different and represent a C1-C10 alkyl group), or a hydroxymethyl group, r₁ represents a C1-C10 alkylene group, r₁' represents a single bond or a C1-C10 alkylene group, b₀ represents an oxy group, a thio group, a sulfinyl group, a sulfonyl group, or an imino group substituted with one methyl group}, or an a₂-O-CO-NH-group (wherein a₂ represents a C2-C10 alkyl group substituted with a C1-C10 alkoxy group), or an a₃'-CO-NH-group (wherein a₃' represents a C1-C10 alkyl group substituted with a C1-C10 alkoxy group), or an a₄'-NrCO-group (wherein a₄' represents a C2-C10 alkyl group substituted with a hydroxyl group or a C1-C10 alkoxy group, or a C1-C10 alkyl group substituted with an aminocarbonyl group, and r is as defined above), and
b1 represents a group represented by formula (VI-1): 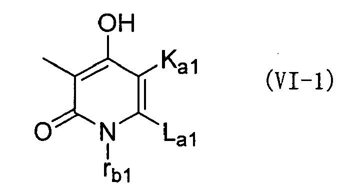 (wherein r_{b1} represents a hydrogen atom or C1-C10 alkyl group, Kₐ₁ represents a hydrogen atom, and Lₐ₁ represents C1-C10 alkyl group, or Kₐ₁ and Lₐ₁ may form a 1,3-butadienylene group),
a group represented by formula (VI-7): 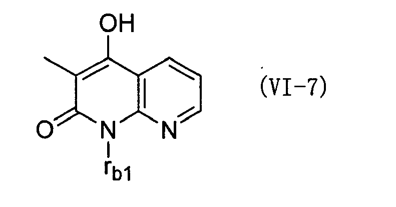 (wherein r_{b1} is as defined above), or
a group represented by formula (VI-8): 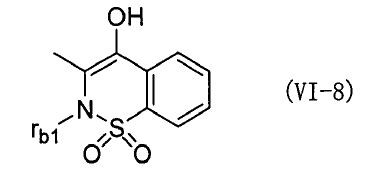 (wherein r_{b1} is as defined above); and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

7. An aldehyde derivative represented by the formula (VII): 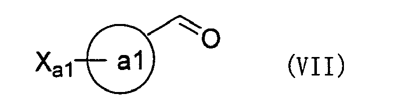 wherein
a1 represents a thiophene group, a furan ring, a pyrrole group or a thiazole ring, and
Xₐ₁ represents an a₀'-r₁-b₀-r₁'- group {wherein a₀' represents a r₀O-CO- group (wherein r₀ represents a C1-C10 alkyl group), a r₀r₀'N-CH₂- group (wherein r₀ and r₀' are the same or different, and r₀' represents the same meaning as that of r₀), or a hydroxymethyl group, r₁ represents a C1-C10 alkylene group, r₁' represents a single bond or a
C1-C10 alkylene group, b₀ represents an oxy group, a thio group, a sulfinyl group, a sulfonyl group, or an imino group substituted with one methyl group}, or an a₂-O-CO-NH-group (wherein a₂ represents a C2-C10 alkyl group substituted with a C1-C10 alkoxy group), or an a₃'-CO-NH-group (wherein a₃' represents a C1-C10 alkyl group substituted with a C1-C10 alkoxy group), or an a₄'-NrCO-group (wherein a₄' represents a C2-C10 alkyl group substituted with a hydroxyl group or a C1-C10 alkoxy group, or a C1-C10 alkyl group substituted with an aminocarbonyl group, r represents a hydrogen atom or a C1-C10 alkyl group),
provided that a1 is not a thiophene ring when a₀' is a dimethylaminomethyl group or a hydroxymethyl group, r₁ is a methylene group, r₁' is a single bond and b₀ is an oxy group, a thio group or an imino group substituted with one methyl group, at the same time, and a1 is not a thiophene ring when a₀' is a methoxycarbonyl group, r₁ is a methylene group, r₁' is a single bond and b₀ is a thio group, at the same time.

8. A process for producing a cinnamoyl compound represented by the formula (VIII''): 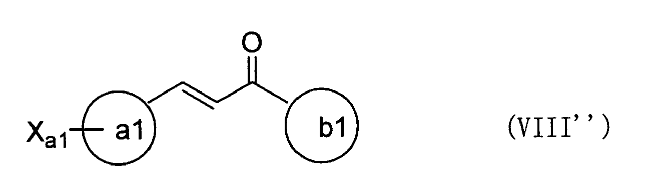 [wherein a1, Xₐ₁ and b1 are as defined below], which comprises reacting an aldehyde derivative represented by the formula (VIII): 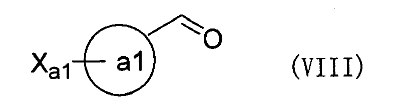 [wherein
a1 represents a thiophene group, a furan ring, a pyrrole group or a thiazole ring, and
Xₐ₁ represents an a₀'-r₁-b₀-r₁'- group {wherein a₀' represents a r₀O-CO- group (wherein r₀ represents a C1-C10 alkyl group), a r₀r₀' N-CH₂- group (wherein r₀ and r₀' are the same or different, and r₀' represents the same meaning as that of r₀), or a hydroxymethyl group, r₁ represents a C1-C10 alkylene group, r₁' represents a single bond or a C1-C10 alkylene group, b₀ represents an oxy group, a thio group, a sulfinyl group, a sulfonyl group, or an imino group substituted with one methyl group}, or an a₂-O-CO-NH-group (wherein a₂ represents a C2-C10 alkyl group substituted with a C1-C10 alkoxy group), or an a₃'-CO-NH-group (wherein a₃' represents a C1-C10 alkyl group substituted with a C1-C10 alkoxy group), or an a₄'-NrCO-group (wherein a₄' represents a C2-C10 alkyl group substituted with a hydroxyl group or a C1-C10 alkoxy group, or a C1-C10 alkyl group substituted with an aminocarbonyl group, r represents a hydrogen atom or a C1-C10 alkyl group),
provided that a1 is not a thiophene ring when a₀' is a dimethylaminomethyl group or a hydroxymethyl group, r₁ is a methylene group, r₁' is a single bond and b₀ is an oxy group, a thio group or an imino group substituted with one methyl group, at the same time, and a1 is not a thiophene ring when a₀' is a methoxycarbonyl group, r₁ is a methylene group, r₁' is a single bond and b₀ is a thio group, at the same time]
with a compound represented by formula (VIII') 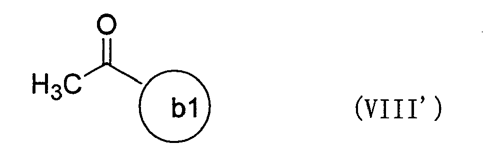 [wherein
b1 is a group represented by formula (VIII-1): 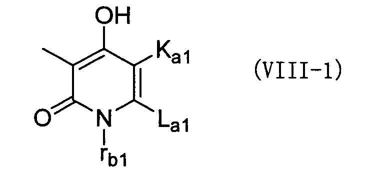 (wherein r_{b1} represents a hydrogen atom or C1-C10 alkyl group, Kₐ₁ represents a hydrogen atom, and Lₐ₁ represents C1-C10 alkyl group, or Kₐ₁ and Lₐ₁ may form a 1,3-butadienylene group),
a group represented by formula (VIII-7): 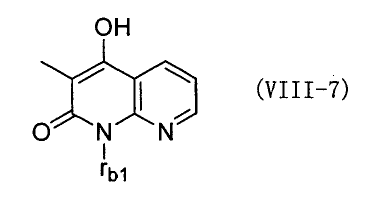 (wherein r_{b1} is as defined above), or
a group represented by formula (VIII-8): 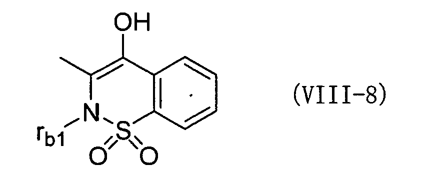 (wherein r_{b1} is as defined above)].

9. A composition for suppressing transcription of an extracellular matrix gene which comprises an inert carrier and a cinnamoyl compound represented by the formula (I'): 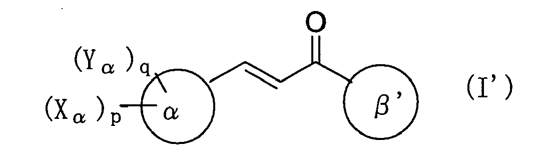 wherein,
I. α represents an aromatic 5-membered ring, or an aromatic 6-membered ring having two or more nitrogen atoms; in (Y_{α})_{q}, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the α ring; in (X_{α})ₚ, X_{α} represents a substituent which does not belong to the following X₀ group, Y₀ group and Z₀ group, p represents 0, 1, 2 or 3 and, when p is not less than 2, X_{α}s are the same or different, and the sum of p and q is not more than 3;
(1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein Rₑ represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ")-NRₑ'''-R_{d}- group (wherein Rₑ, Rₑ', Rₑ" and Rₑ''' are the same or different, R_{e,} Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
(2) the Y₀ group: a M_{b0}-R_{d}- group, wherein M_{b0} represents a M_{c0}- group
[wherein M_{co} represents a M_{do}-R_{d}'- group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 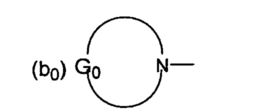 (wherein Go forms an optionally substituted, saturated or unsaturated, nonaromatic 5 to 14-membered hydrocarbon ring or heterocyclic ring), a (c₀)- group represented by 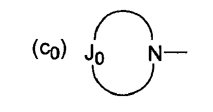 (wherein, J₀ forms a 5 to 7-membered aromatic ring optionally containing a nitrogen atom), a (do)- group represented by 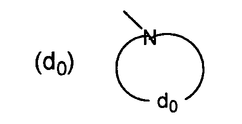 [wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group], or a (e₀)- group represented by 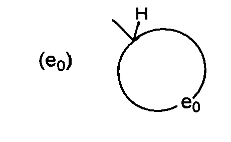 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}; and R_{d}' is the same as or different from R_{d} and has the same meaning as R_{d} has],
a M_{c0}-Bₐ- group (wherein M_{c0} and Bₐ are as defined above), a M_{c0}-CO- group (wherein M_{c0} is as defined above), a M_{c0}-CO-O-group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ-group (wherein M_{c0} and Rₑ are as defined above), a M_{c0}RₑN-CO-group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ"- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
(3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and
II. β' is a group represented by formula (I'-1): 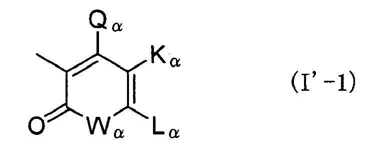 [wherein,
(1)Q_{α} represents an optionally substituted hydroxyl group, or an optionally substituted amino group,
(2)W_{α} represents an oxygen atom or a -NT_{α}- group (wherein T_{α} represents a hydrogen atom, or a substituent on the nitrogen atom),
(3)K_{α} and L_{α} are the same or different, and represent a hydrogen atom, or a substituent on a carbon atom, or K_{α} and L_{α} may form an optionally substituted C3-C10 alkylene group or an optionally substituted C4-C10 alkenylene group], a group represented by formula (I'-2): 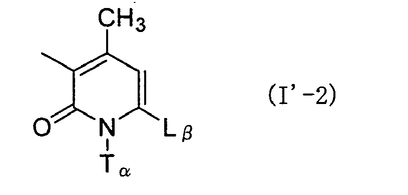 [wherein T_{α} is as defined above, and L_{β} represents a hydroxyl group or a methyl group],
a group represented by formula (I'-3): 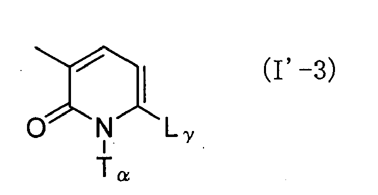 [wherein T_{α} is as defined above, and L_{γ} represents a C1-C10 alkyl group], a group represented by formula (I'-4): 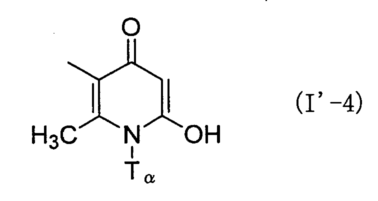 [wherein T_{α} is as defined above], a group represented by formula (I'-5) : 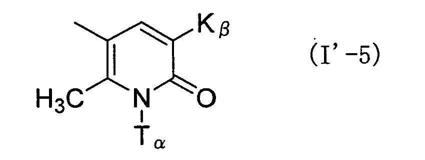 [wherein T_{α} is as defined above, and K_{β} represents a cyano group or a UOCO-group (wherein U represents a hydrogen atom or a C1-C10 alkyl group)], a group represented by formula (I'-6): 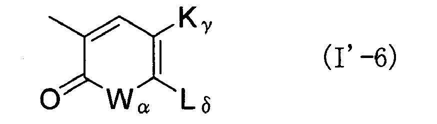 [wherein W_{α} is as defined above, and K_{γ} and L_{δ} form an optionally substituted C3-C10 alkylene group or an optionally substituted C4-C10 alkenylene group],
a group represented by formula (I'-7): 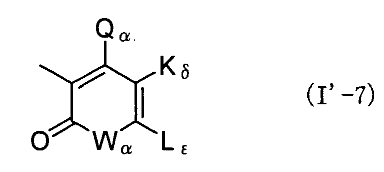 [wherein Q_{α} and W_{α} are as defined above, and K_{δ} and L_{ε} form a -V_{α}=V_{α}'-V_{α}'' =V_{α}''' - group (wherein V_{α}, V_{α}', V_{α}'' and V_{α}''' are the same or different, and represent an optionally substituted methine group or a -N= group, and at least one of V_{α} , V_{α} ' , V_{α} ' ' and V_{α} ' ' ' represents a -N= group)],
a group represented by formula (I'-8): 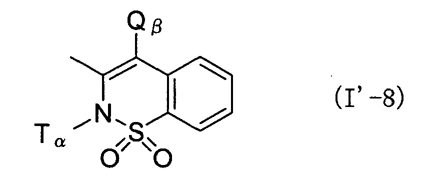 [wherein T_{α} is as defined above, and Q_{β} represents an optionally substituted hydroxyl group], or
a group represented by formula (I'-9): 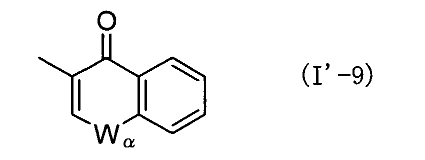 [wherein W_{α} is as defined above]; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

10. A cinnamoyl compound represented by formula (II') : 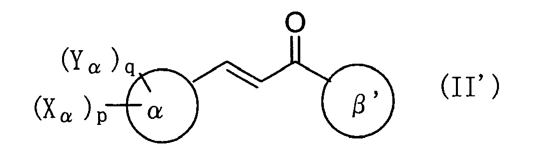 wherein,
I. α represents an aromatic 5-membered ring, or an aromatic 6-membered ring having two or more nitrogen atoms; in (Y_{α})q, Y_{α} represents a group included in the following X₀ group or Y₀ group, q represents 0, 1, 2 or 3 and, when q is not less than 2, Y_{α}s are the same or different and, when q is not less than 2, adjacent two same or different Y_{α}s may together form a group included in the following Z₀ group to be fused to the α ring; in (X_{α})ₚ, X_{α} represents a substituent which does not belong to the following X₀ group, Y₀ group and Z₀ group, p represents 0, 1, 2 or 3 and, when p is not less than 2, X_{α}s are the same or different, and the sum of p and q is not more than 3;
(1) the X₀ group: a Mₐ-group, wherein Mₐ represents a R_{b}- group (wherein R_{b} represents a C1-C10 alkyl group optionally substituted with a halogen atom), a halogen atom, a nitro group, a cyano group, a hydroxyl group, a R_{c}-Bₐ-R_{d}-group (wherein R_{c} represents a C1-C10 alkyl group optionally substituted with a halogen atom, Bₐ represents an oxy group, a thio group, a sulfinyl group or a sulfonyl group, and R_{d} represents a single bond or a C1-C10 alkylene group), an HOR_{d}- group (wherein R_{d} is as defined above), a Rₑ-CO-R_{d}- group (wherein Rₑ represents a hydrogen atom, or a C1-C10 alkyl group optionally substituted with a halogen atom, and R_{d} is as defined above), a Rₑ-CO-O-R_{d}- group (wherein Rₑ and R_{d} are as defined above), a RₑO-CO-R_{d}- group (wherein Rₑ and R_{d} are as defined above), an HO-CO-CH=CH-group, a RₑRₑ'N-R_{d}- group (wherein Rₑ and Rₑ' are the same or different, Rₑ is as defined above, Rₑ' has the same meaning as Rₑ has, and R_{d} is as defined above), a Rₑ-CO-NRₑ'-R_{d}- group (wherein Rₑ, Rₑ' and R_{d} are as defined above), a R_{b}O-CO-N(Rₑ)-R_{d}- group (wherein R_{b}, Rₑ and R_{d} are as defined above), a RₑRₑ'N-CO-R_{d}- group (wherein Rₑ, Rₑ' and R_{d} are as defined above), a RₑRₑ'N-CO-NRₑ"-R_{d}- group (wherein Rₑ, Rₑ' and Rₑ" are the same or different, Rₑ and Rₑ' are as defined above, Rₑ" has the same meaning as Rₑ has, and R_{d} is as defined above), a RₑRₑ'N-C(=NRₑ")-NRₑ'''-R_{d}- group (wherein Rₑ, Rₑ', Rₑ" and Rₑ''' are the same or different, Rₑ, Rₑ' and Rₑ" are as defined above, Rₑ''' has the same meaning as Rₑ has, and R_{d} is as defined above), a R_{b}-SO₂-NRₑ-R_{d}- group (wherein R_{b}, Rₑ and R_{d} are as defined above), a RₑRₑ'N-SO₂-R_{d}- group (wherein Rₑ, Rₑ' and R_{d} are as defined above), a C2-C10 alkenyl group or a C2-C10 alkynyl group;
(2) the Y₀ group: a M_{b0}-R_{d}- group, wherein M_{b0} represents a M_{c0}- group
[wherein M_{co} represents a M_{do}-R_{d}' - group {wherein M_{do} represents a 6 to 10-membered aryl group optionally substituted with a Mₐ- group (wherein Mₐ is as defined above), a 5 to 10-membered heteroaryl group optionally substituted with a Mₐ-group (wherein Mₐ is as defined above), a 3 to 10-membered cyclic hydrocarbon or heterocyclic group optionally substituted with a Mₐ- group (wherein Mₐ is as defined above) and optionally containing an unsaturated bond, a (b₀)- group represented by 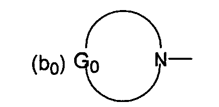 (wherein Go forms an optionally substituted, saturated or unsaturated, nonaromatic 5 to 14-membered hydrocarbon ring or heterocyclic ring), a (c₀)- group represented by 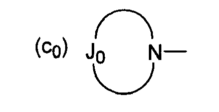 (wherein, J₀ forms a 5 to 7-membered aromatic ring optionally containing a nitrogen atom), a (do)- group represented by 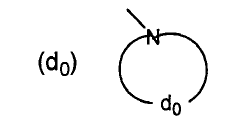 [wherein d₀ forms a 5 to 12-membered hydrocarbon ring which is substituted with a carbonyl group or a thiocarbonyl group and further which may be optionally substituted with an oxy group, a thio group, a -NR₁- group {wherein R₁ represents a hydrogen atom, or a C1-C10 alkyl group, or a C2-C10 alkyl group substituted with a halogen atom or a R₂-B₁- group (wherein R₂ represents a C1-C10 alkyl group, a C3-C10 alkenyl group or a C3-C10 alkynyl group, and B₁ represents an oxy group, a thio group, a sulfinyl group or a sulfonyl group), a C3-C10 alkenyl group, or a C3-C10 alkynyl group}, a sulfinyl group, or a sulfonyl group], or a (e₀)- group represented by 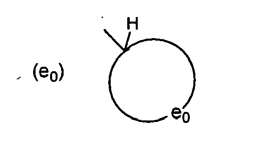 {wherein e₀ forms a 5 to 12-membered hydrocarbon ring optionally substituted with a carbonyl group, a thiocarbonyl group, an oxy group, a thio group, a -NR₁-group (wherein R₁ is as defined above), a sulfinyl group or a sulfonyl group}; and R_{d}' is the same as or different from R_{d} and has the same meaning as R_{d} has], a M_{c0}-Bₐ- group (wherein M_{c0} and Bₐ are as defined above), a M_{c0}-CO- group (wherein M_{c0} is as defined above), a M_{c0}-CO-O-group (wherein M_{c0} is as defined above), a M_{c0}O-CO- group (wherein M_{c0} is as defined above), a M_{c0}RₑN- group (wherein M_{c0} and Rₑ are as defined above), a M_{c0}-CO-NRₑ- group (wherein M_{c0} and Rₑ are as defined above), a M_{c0}O-CO-NRₑ-group (wherein M_{c0} and Rₑ are as defined above), a M_{c0}RₑN-CO-group (wherein M_{c0} and Rₑ are as defined above), a M_{c0}RₑN-CO-NRₑ'- group (wherein M_{c0}, Rₑ and Rₑ' are as defined above), a M_{c0}RₑN-C(=NRₑ')-NRₑ''- group (wherein M_{c0}, Rₑ, Rₑ' and Rₑ" are as defined above), a M_{c0}-SO₂-NRₑ- group (wherein M_{c0} and Rₑ are as defined above) or a M_{c0}RₑN-SO₂- group (wherein M_{c0} and Rₑ are as defined above), and R_{d} is as defined above;
(3) the Z₀ group: a 5- to 12-membered hydrocarbon ring or heterocyclic ring which may be substituted with a halogen atom, a C1-C10 alkoxy group, C3-C10 alkenyloxy group, a C3-C10 alkynyloxy group, a carbonyl group, a thiocarbonyl group, an oxy group, a thio group, a sulfinyl group or a sulfonyl group, and which is aromatic or nonaromatic and monocyclic or fused ring and is fused to the α ring; and
II. β' is a group represented by formula (II'-1): 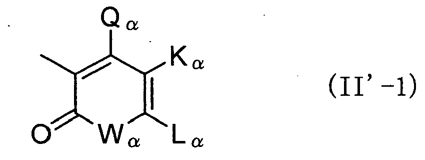 [wherein,
(1)Q_{α} represents an optionally substituted hydroxyl group, or an optionally substituted amino group,
(2)W_{α} represents an oxygen atom or a -NT_{α}- group (wherein T_{α} represents a hydrogen atom, or a substituent on the nitrogen atom),
(3)K_{α} and L_{α} are the same or different, and represent a hydrogen atom, or a substituent on a carbon atom, or K_{α} and L_{α} may form an optionally substituted C3-C10 alkylene group or an optionally substituted C4-C10 alkenylene group],
a group represented by formula (II'-2): 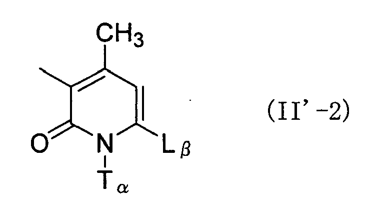 [wherein T_{α} is as defined above, and L_{β} represents a hydroxyl group or a methyl group],
a group represented by formula (II'-3): 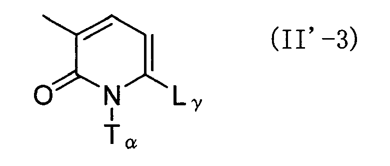 [wherein T_{α} is as defined above, and L_{γ} represents a C1-C10 alkyl group],
a group represented by formula (II'-4): 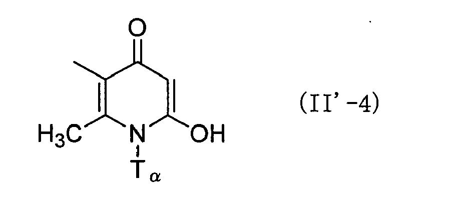 [wherein T_{α} is as defined above],
a group represented by formula (II'-5): 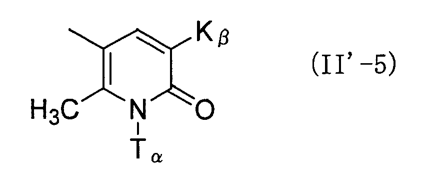 [wherein T_{α} is as defined above, and K_{β} represents a cyano group or a UOCO-group (wherein U represents a hydrogen atom or a C1-C10 alkyl group)],
a group represented by formula (II'-6): 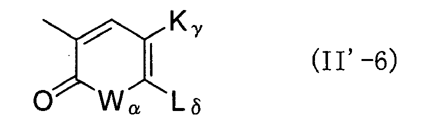 [wherein W_{α} is as defined above, and K_{γ} and L_{δ} form an optionally substituted C3-C10 alkylene group or an optionally substituted C4-C10 alkenylene group],
a group represented by formula (II'-7): 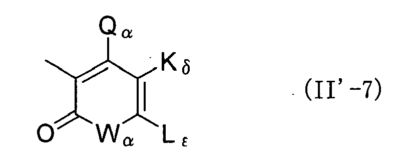 [wherein Q_{α} and W_{α} are as defined above, and K_{δ} and L_{ε} form a -V_{α}=V_{α}'-V_{α}'' =V_{α}''' - group (wherein V_{α}, V_{α}', V_{α}" and
V_{α}''' are the same or different, and represent an optionally substituted methine group or a -N= group, and at least one of V_{α}, V_{α}', V_{α}'' and V_{α}''' represents a -N= group)],
a group represented by formula (II'-8): 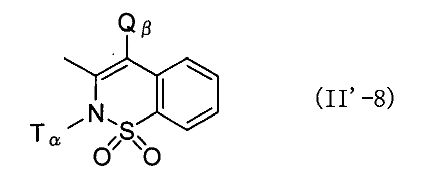 [wherein T_{α} is as defined above, and Q_{β} represents an optionally substituted hydroxyl group], or
a group represented by formula (II'-9): 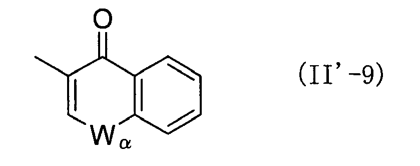 [wherein W_{α} is as defined above], provided that when α is a furan ring or a thiophene ring, p and q are not 0 at the same time; and
the term "as defined above" used for the same symbols among plural substituents means that the plural substituents independently represent the same meaning as that described above and, among the plural substituents, although the selection range of substituents to be selected is the same, selected substituents may be the same or different as long as they are selected within the range.

11. A cinnamoyl compound represented by formula (V'): 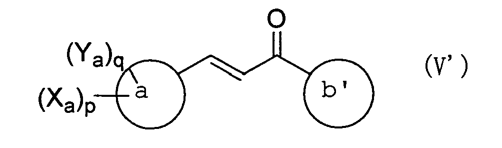 wherein,
I. a represents a thiophene ring, a furan ring, a pyrrole ring or a tetrazole ring; Xₐ represents a C1-C10 alkyl group substituted with a cyano group, or a C1-C10 alkyl group substituted with a tetrahydropyran-4-ylidene group, or a C2-C10 alkenyl group substituted with a halogen atom or a cyano group, or a C2-C10 alkenyl group substituted with a C1-C10 alkoxycarbonyl group, or a C3-C10 alkynyl group substituted with a hydroxyl group, or an a₀-r₁-b-r₁'- group {wherein a₀ represents a methyl group substituted with a C1-C10 alkylthio group, a methyl group substituted with a C1-C10 alkylsulfinyl group, a methyl group substituted with a C1-C10 alkylsulfonyl group, a C2-C10 alkenyl group, a C2-C10 alkynyl group, a r₂O-CO- group (wherein r₂ represents a C1-C10 alkyl group or a C2-C10 alkyl group substituted with a hydroxyl group), a carboxy group, a rr'N-CO- group (wherein r and r' are the same or different, and respresent a hydrogen atom or a C1-C10 alkyl group), a₁-NH-CO- group (wherein a₁ represents a C2-C10 alkyl group substituted with a C1-C10 alkoxy group), an a₁'-CO- group (wherein a₁' represents a morpholino group), a rr'N-CH₂- group (wherein r and r' are as defined above), a r₀-(O)₁-CONH-CH₂- group (wherein r₀ represents a C1-C10 alkyl group, and 1 represents 0 or 1), a r-OCH₂- group (wherein r is as defined above), a r₀-CO- group (wherein r₀ is as defined above), a cyano group or a sulfomethyl group, r₁ represents a C1-C10 alkylene group, r₁' represents a single bond or a C1-C10 alkylene group, and b represents an oxy group, a thio group, a sulfinyl group, a sulfonyl group, or an imino group}, or an a₂-y-CO-NH- group (wherein a₂ represents a C2-C10 alkyl group substituted with a C1-C10 alkoxy group, and y represents an oxy group or an imino group), or a rO-COCO-NH- group (wherein r is as defined above), or an a₃-z-NH- group (wherein a₃ represents a C2-C10 alkenyl group, or a C1-C10 alkyl group substituted with a C1-C10 alkoxy group, a C1-C10 alkoxycarbonyl group, a carboxy group or a cyano group, and z represents a carbonyl group or a sulfonyl group), or an a₄-Nr'CO- group {wherein a₄ represents a C1-C10 alkoxy group, or a C3-C10 alkenyloxy group, or a r₀-SO₂- group (wherein r₀ is as defined above), or a C2-C10 alkyl group substituted with a hydroxyl group or a C1-C10 alkoxy group, or a C2-C10 alkyl group substituted with a r₀r₀'N- group (wherein r₀ is as defined above, and r₀' is the same as or different from r₀, and represents the same meaning as r₀ has), or a C1-C10 alkyl group substituted with a rO-CO- group (wherein r is as defined above), a cyano group or an aminocarbonyl group, or a rO-CO-(rO-COCH₂)CH- group (wherein r is as defined above), and r' is as defined above}, or an a₅-NHSO₂- group (wherein a₅ represents a C2-C10 alkyl group substituted with a Cl-C10 alkoxyl group), or a r₀ON=CH- group (wherein r₀ is as defined above), or a r₀NHCSNH- group (wherein r₀ is as defined above), or a r₀NHC(-Sr₀')=N- group (wherein r₀ and r₀' are as defined above), or a (rO)₂P(=O)CH₂- group (wherein r is as defined above),
p represents 0, 1, 2 or 3 and, and when p is not less than 2, Xₐs are same or different,
Yₐ represents are halogen atom, or a C1-C10 alkyl group optionally substituted with a halogen atom, or a C1-C10 alkyl group optionally substituted with a C1-C10 alkoxy group, or a C2-C10 alkenyl group, or a C2-C10 alkynyl group, or a 2-oxo-oxazolidin-3-yl group, or a [1,3]dioxolan-2-yl group, or a C1-C10 alkoxy group substituted with a morpholino group, or an a₀'-b'- group (wherein a₀' represents a C1-C10 alkyl group optionally substituted with a halogen atom, and b' represents an oxy group, a thio group, a sulfinyl group or a sulfonyl group), or a nitro group, or a cyano group, or a rO-CO- group (wherein r is as defined above), or a r₀r₀'N- group (wherein r₀ and r₀' are as defined above), or a r₀CO-NH- group (wherein r₀ is as defined above), or a r₀r₀'NCONH- group (wherein r₀ and r₀' are as defined above), or a rr'NCO- group (wherein r and r' are as defined above), or a hydroxyl group,
q represents 0, 1, 2 or 3, and when q is not less than 2, Yₐs are the same or different, and when q is not less than 2, adjacent Yₐs may be fused to the a ring to form a 2,3-dihydro-benzo[1,4]dioxine ring;
II. b' is
a group represented by formula (V'-1): 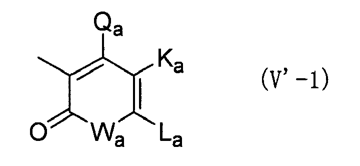
wherein Qₐ represents a rₐ-O- group {rₐ represents a hydrogen atom, or a C1-C10 alkyl group, or a C3-C10 alkenyl group, or a C3-C10 alkynyl, or a C1-C10 alkyl group substituted with a r₀r₀'N-CH₂- group (wherein r₀ and r₀' are the same or different), a rOCH₂ group (wherein r is as defined above), a r₀-CO- group (wherein r₀ is as defined above), a C1-C10 alkoxycarbonyl group, a carboxy group, an aminocarbonyl group or a cyano group, or a r₃-r₁- group (wherein r₃ represents a phenyl group or a pyridyl group, and r₁ is as defined above)}, or a piperidino group, or a morpholino group, or a r₄r₄'N-group (wherein r₄ and r₄' are the same of different, and represent a hydrogen atom, or a C1-C10 alkyl group, or C3-C10 alkenyl group, or a C3-C10 alkynyl group, or a C2-C10 alkyl group substituted with a C1-C10 alkoxy group, provided that they are not a hydrogen atom at the same time); Wₐ represents an oxygen atom or a - NTₐ- group [wherein Tₐ represents a r_{b}- group (wherein r_{b} is the same as or different from rₐ, and represents the same meaning as rₐ has) or a r₃'- group (wherein r₃' is the same as or different from r₃, and represents the same meaning as r₃ has)], and Kₐ represents a hydrogen atom, halogen atom or C1-C10 alkyl group, and Lₐ represents hydrogen atom or C1-C10 alkyl group, or Kₐ and Lₐ may form a 1,3-butadienylene group;
a group represented by formula (V'-2): 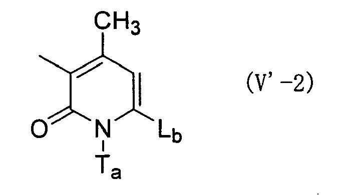 wherein Tₐ is as defined above, and L_{b} represents a hydroxyl group or a methyl group;
a group represented by formula (V'-3): 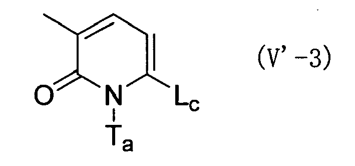 wherein Tₐ is as defined above, and L_{c} represents a C1-C10 alkyl group;
a group represented by formula (V'-4): 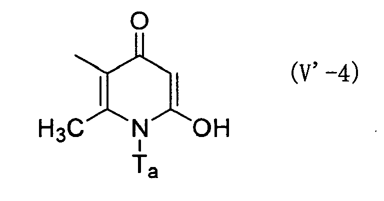 wherein Tₐ is as defined above;
a group represented by formula (V'-5): 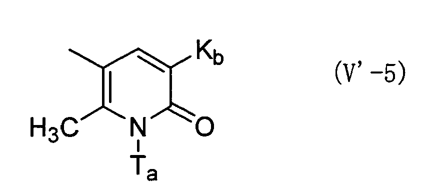 wherein Tₐ is as defined above, and K_{b} represents a cyano group or a UOCO- group (wherein U represents a hydrogen atom or C1-C10 alkyl group);
a group represented by formula (V'-6): 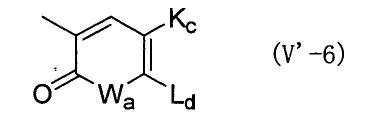 wherein Wₐ is as defined above, and Kₑ and L_{d} form a C3-C10 alkylene group or a C4-C10 alkenylene group;
a group represented by formula (V'-7): 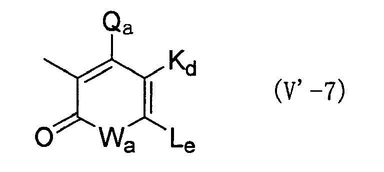 wherein Qₐ and Wₐ are as defined above, and K_{d} and Lₑ form a -Vₐ=Vₐ' -Vₐ' ' =Vₐ' ' ' - group (wherein Vₐ, Vₐ' , Vₐ' ' and Vₐ' ' ' are the same or different, and represent a methine group, or a -N= group, and at least one of Vₐ, Vₐ', Vₐ'' and Vₐ''' represents a -N= group);
a group represented by formula (V'-8): 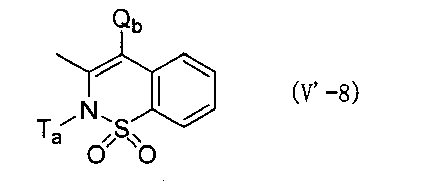 wherein Tₐ is as defined above, and Q_{b} represents a rₐ-O-group (wherein rₐ is as defined above); or
a group represented by formula (V'-9): 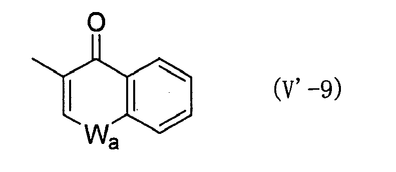 wherein Wₐ is as defined above, provided that when a is a furan ring or a thiophene ring, p and q are not 0 at the same time; and
the term "as defined above" used for the. same symbols among plural substituents means that the plural substituents independently represent the same meaning as that described above and, among the plural substituents, although the selection range of substituents to be selected is the same, selected substituents may be the same or different as long as they are selected within the range.

12. A composition for suppressing transcription of an extracellular matrix gene, which comprises the compound according to any one of claims 2 to 6, 10 and 11, and an inert carrier.

13. A composition for suppressing transcription of an extracellular matrix gene, which comprises the compound according to claim 5 or 11, and an inert carrier.

14. Use of a cinnamoyl compound contained in the composition according to claim 1 or 9 as an active ingredient, as an active ingredient for suppressing transcription of an extracellular matrix gene.

15. Use of the compound according to any one of claims 2 to 6, 10 and 11, as an active ingredient for suppressing transcription of an extracellular matrix gene.

16. Use of the compound according to claim 5 or 11, as an active ingredient for suppressing transcription of an extracellular matrix gene.

17. Use of a cinnamoyl compound contained in the composition according to claim 1 or 9 as an active ingredient, as an active ingredient for decreasing expression of an extracellular matrix gene to induce a reduction in accumulation of an extracellular matrix and thereby improving tissue fibrosis.

18. Use of the compound according to any one of claims 2 to 6, 10 and 11, as an active ingredient for decreasing expression of an extracellular matrix gene to induce a reduction in accumulation of an extracellular matrix and thereby improving tissue fibrosis.

19. A method for improving tissue fibrosis, which comprises administering an effective amount of a cinnamoyl compound contained in the composition according to claim 1 or 9 as an active ingredient to a mammalian patient in need thereof.

20. A method for improving tissue fibrosis, which comprises administering an effective amount of the compound according to any one of claims 2 to 6, 10 and 11 to a mammalian patient in need thereof.

21. An agent for treating chronic renal failure, which comprises the compound according to any one of claims 2 to 6, 10 and 11 and an inert carrier.

22. Use of a cinnamoyl compound contained in the composition according to claim 1 or 9 as an active ingredient, or the compound according to any one of claims 2 to 6, 10 and 11, as an active ingredient for treating chronic renal failure.

23. A method for treating chronic renal failure, which comprises administering an effective amount of a cinnamoyl compound contained in the composition according to claim 1 or 9 as an active ingredient, or the compound according to any one of claims 2 to 6, 10 and 11 to a mammalian patient in need thereof.

24. An agent for treating heart failure, which comprises a cinnamoyl compound contained in the composition according to claim 1 or 9 as an active ingredient, or the compound according to any one of claims 2 to 6, 10 and 11, and an inert carrier.

25. Use of a cinnamoyl compound contained in the composition according to claim 1 or 9 as an active ingredient, or the compound according to claims 2 to 6, 10 and 11, as an active ingredient for treating heart failure.

26. A method for treating heart failure, which comprises administering an effective amount of a cinnamoyl compound contained in the composition according to claim 1 or 9 as an active ingredient, or the composition according to any one of claims 2 to 6, 10 and 11 to a mammalian patient in need thereof.

27. A composition for suppressing the activity of TGF-β, which comprises a cinnamoyl compound contained in the composition according to claim 1 or 9 as an active ingredient, and an inert carrier.

28. A composition for suppressing the activity of TGF-β, which comprises the compound according to any one of claims 2 to 6, 10 and 11, and an inert carrier.

29. Use of a cinnamoyl compound contained in the composition according to claim 1 or 9 as an active ingredient, as an active ingredient for suppressing the activity of TGF-β.

30. Use of the compound according to any one of claims 2 to 6, 10 and 11, as an active ingredient for suppressing the activity of TGF-β.

31. A composition for hair growth which comprises a cinnamoyl compound contained in the composition according to claim 1 or 9 as an active ingredient, or the compound according to any one of claims 2 to 6, 10 and 11, and an inert carrier.

32. Use of a cinnamoyl compound contained in the composition according to claim 1 or 9 as an active ingredient, or the compound according to any one of claims 2 to 6, 10 and 11, as an active ingredient for inhibiting a promoting effect of TGF-β on transition to a hair regression phase to induce extension of a hair growth phase and thereby providing a hair-growing effect.

33. A method for growing hair, which comprises administering an effective amount of a cinnamoyl compound contained in the composition according to claim 1 or 9 as an active ingredient, or the compound according to any one of claims 2 to 6, 10 and 11, to a mammalian patient in need thereof.


SEQUENCE LISTING

<110> Sumitomo Chemical Company Limited

<120> Cinnamoyl compound and use thereof

<130> S13491WO01

<150> JP 2005-057363
<151> 2005-03-02

<160> 5

<210> 1
<211> 32
<212> DNA
<213> Artificial Sequence

<220>
<223> Designed oligonucleotide primer to amplify collagen promoter DNA

<400> 1
ccaagctagc gaaattatct tttctttcat ag     32

<210> 2
<211> 28
<212> DNA

<213> Artificial Sequence

<220>
<223> Designed oligonucleotide primer to amplify collagen promoter DNA

<400> 2
ccaaaagctt gcagtcgtgg ccagtacc     28

<210> 3
<211> 22
<212> DNA
<213> Artificial Sequence

<220>
<223> Designed oligonucleotide primer to detect fibronectin DNA

<400> 3
tcgccatcag tagaaggtag ca  22

<210> 4
<211> 25
<212> DNA
<213> Artificial Sequence

<220>
<223> Designed oligonucleotide primer to detect fibronectin DNA

<400> 4

tatactgaac accaggttgc aagtc 25

<210> 5

<211> 30

<212> DNA

<213> Artificial Sequence

<220>

<223> Designed oligonucleotide primer to detect fibronectin DNA

<400> 5

ctcaaccttc ctgaaactgc aaactccgtc 30